# EUROPEAN PATENT SPECIFICATION

(11) **EP 1 934 357 B1**
(45) Date of publication and mention of the grant of the patent: **16.11.2011**
(21) Application number: 06849359.2
(22) Date of filing: 18.09.2006
(51) Int. Cl.: A01N 63/00

(54) **Methods for controlling pests using RNAi**
Verfahren zum Kontrollieren von Schädlingen mittels RNAi
L'ARN à double brin pour la lutte contre les insectes

(30) Priority: 16.09.2005 US 718034 P; 12.01.2006 US 758191 P; 07.02.2006 US 771160 P; 16.08.2006 US 837910 P; 15.09.2006 WO PCT/IB2006/003446
(43) Date of publication of application: 25.06.2008
(60) Divisional application: 10186041.9
(73) Proprietor: Devgen NV, 9052 Gent-Zwijnaarde (BE)
(72) Inventor: RAEMAEKERS, Romaan, B-9840 De Pinte (BE); FELDMANN, Pascale, B-9030 Mariakerke (BE); PLAETINCK, Geert, B-9820 Bottelare (BE); NOOREN, Irene, NL-3521 Utrecht XH (NL); BLEU, Els, Van, B-8900 Leper (BE); PECQUEUR, Frédéric, F-59320 Sequedin (FR); KUBLER, Laurent, F-59126 Linselles (FR); DAMME, Nicole, B-9770 Kruishoutem (BE); DEGRAVE, Lies, B-8700 Aarsele (BE); REMORY, Isabel, B-9420 Vlekkem (BE)
(74) Representative: De Clercq, Ann G. Y.
(86) International application number: PCT/IB2006/004008
(87) International publication number: WO 2007/083193

(56) References cited:
- WO-A-01/09301
- WO-A-01/34815
- WO-A-01/37654
- WO-A-02/46432
- WO-A-03/004644
- WO-A-2004/022771
- WO-A-2005/019408
- WO-A-2005/049841
- WO-A-2005/071091
- WO-A-2005/110068
- WO-A-2006/045590
- DATABASE UniProt [Online] 30 August 2005 (2005-08-30), "Ribosomal protein S4e." XP002432593 retrieved from EBI accession no. UNIPROT:Q4GXU7 Database accession no. Q4GXU7 & DATABASE EMBL SEQUENCE LIBRARY [Online] Ebi. hinxton; ribosomal protein S4e; rpS4e gene 16 July 2005 (2005-07-16), LONGHORN,S.J.: "Biphyllus lunatus mRNA for ribosomal protein S4e" retrieved from EBI. HINXTON accession no. www.ebi.co.uk Database accession no. AM048926
- LAMBERTON J S ET AL: "VARYING THE NUCLEIC ACID COMPOSITION OF SIRNA MOLECULES DRAMATICALLY VARIES THE DURATION AND DEGREE OF GENE SILENCING" MOLECULAR BIOTECHNOLOGY, TOTOWA, NJ, US, vol. 24, no. 2, June 2003 (2003-06), pages 111-119, XP009043115 ISSN: 1073-6085
- SOARES C A G ET AL: "Capillary feeding of specific dsRNA induces silencing of the isac gene in nymphal Ixodes scapularis ticks" INSECT MOLECULAR BIOLOGY, vol. 14, no. 4, August 2005 (2005-08), pages 443-452, XP002446932 ISSN: 0962-1075

## Description

### FIELD OF THE INVENTION

The present invention relates generally to genetic control of pest infestations. More specifically, the present invention relates to recombinant technologies for repressing or inhibiting expression of target coding sequences in a pest.

### INTRODUCTION

Insect and other pests can cause injury and even death by their bites or stings. Additionally, many pests transmit bacteria and other pathogens that cause diseases. For example, mosquitoes transmit pathogens that cause malaria, yellow fever, encephalitis, and other diseases. The bubonic plague, or black death, is caused by bacteria that infect rats and other rodents. Compositions for controlling microscopic pest infestations have been provided in the form of antibiotic, antiviral, and antifungal compositions. Methods for controlling infestations by pests, such as nematodes and insects, have typically been in the form of chemical compositions that are applied to surfaces on which pests reside, or administered to infested animals in the form of pellets, powders, tablets, pastes, or capsules.

Commercial crops are often the targets of insect attack. Substantial progress has been made in the last a few decades towards developing more efficient methods and compositions for controlling insect infestations in plants. Chemical pesticides have been very effective in eradicating pest infestations. However, there are several disadvantages to using chemical pesticides. Not only are they potentially detrimental to the environment, but chemical pesticides are not selective and can pose harm to non-target flora and fauna. Chemical pesticides persist in the environment and generally are slow to be metabolized, if at all. They accumulate in the food chain, and particularly in the higher predator species. Accumulation of chemical pesticides results in the development of resistance to the agents and in species higher up the evolutionary ladder, they can act as mutagens and/or carcinogens and cause irreversible and deleterious genetic modifications.

Because of the dangers associated with chemical pesticides, biological approaches have been developed for controlling pest infestations. For example, biological control using protein Cry3A from *Bacillus thuringiensis* have effectively controlled Colorado potato beetle larvae either as formulations sprayed onto the foliage or expressed in the leaves of potatoes. An alternative biological agent is double stranded RNA (dsRNA). Over the last few years, downregulation of genes (also referred to as "gene silencing") in multicellular organisms by means of RNA interference has become a well-established technique.

RNA Interference (RNAi) provides a potentially powerful tool for controlling gene expression because of its specificity of target selection and remarkably high efficiency in target mRNA suppression. RNAi refers to the process of sequence-specific posttranscriptional gene silencing mediated by short interfering RNAs (siRNAs) (Zamore, P. et al., Cell 101:25-33 (2000); Fire, A. et al., Nature 391:806 (1998); Hamilton et al., Science 286, 950-951 (1999); Lin et al., Nature 402:128-129 (1999)). While the mechanics underlying RNAi are not fully characterized, it is thought that the presence of dsRNA in cells triggers RNAi by activating the ribonuclease III enzyme Dicer (Zamore, P. et al., (2000); Hammond et al., Nature 404, 293 (2000)). Dicer processes the dsRNA into short pieces called short interfering RNAs (siRNAs), which are about 21 to about 23 nucleotides long and comprise about 19 base pair duplexes (Zamore et al., (2000); Elbashir et al., Genes Dev., 15, 188 (2001)). Following delivery into cells, the siRNA molecules associate with an endonuclease complex, commonly referred to as an RNA-induced silencing complex (RISC), which brings together the antisense strand of the siRNA and the cellular mRNA gene target. RISC cleaves the mRNA, which is then released and degraded. Importantly, RISC is then capable of degrading additional copies of the target mRNA.

Accordingly, the present invention provides methods and compositions for controlling pest infestation by repressing, delaying, or otherwise reducing gene expression within a particular pest.

### SUMMARY OF THE INVENTION

The invention provides a composition comprising a cell comprising a double stranded ribonucleotide produced from the expression of a polynucleotide sequence selected from the group consisting of:
(i) a polynucleotide sequence comprising the nucleic acid sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide comprises annealed complementary strands, one of which has a nucleotide sequence which is complementary to any one of the sequences selected from the group consisting of:
   (i) a polynucleotide sequence consisting of the nucleic acid sequence set forth in any of SEQ ID NOs: 1, 25, 49 to 55, 163 or 166, or the complement thereof,
   (ii) a polynucleotide sequence having at least 85% sequence identity as determined using the BLASTN alignment tool to the nucleic acid sequence set forth in SEQ ID NO 1, or the complement thereof,
   (iii) a polynucleotide sequence consisting of a fragment of at least 21 contiguous nucleotides of any of SEQ ID NOs 1, 25, 49 to 55, 163 or 166, or the complement thereof.

In a preferred embodiment of the composition of the invention, said cell is a bacterial, yeast, or algal cell.

In a preferred embodiment of the composition of the invention, said bacterial cell is killed by heat treatment.

In a preferred embodiment of the invention, said composition is selected from the group consisting of a spray, powder, pellet, gel, capsule, food product, garment bag and book. The invention further provides for a method for controlling insect pest infestation, comprising exposing a pest to a double stranded ribonucleotide produced from the expression of a polynucleotide sequence selected from the group consisting of:
(i) a polynucleotide sequence comprising the nucleic acid sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide comprises annealed complementary strands, one of which has a nucleotide sequence which is complementary to any one of the sequences selected from the group consisting of:
   (i) a polynucleotide sequence consisting of the nucleic acid sequence set forth in any of SEQ ID NOs: 1, 25, 49 to 55, 163 or 166, or the complement thereof,
   (ii) a polynucleotide sequence having at least 85% sequence identity as determined using the BLASTN alignment tool to the nucleic acid sequence set forth in SEQ ID NO 1, or the complement thereof,
   (iii) a polynucleotide sequence consisting of a fragment of at least 21 contiguous nucleotides of any of SEQ ID NOs 1, 25, 49 to 55, 163 or 166, or the complement thereof.

The invention also provides a method for protecting an object from insect pest infestation, comprising treating said object with a composition comprising a double stranded ribonucleotide produced from the expression of a polynucleotide sequence selected from the group consisting of:
(i) a polynucleotide sequence comprising the nucleic acid sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide comprises annealed complementary strands, one of which has a nucleotide sequence which is complementary to any one of the sequences selected from the group consisting of:
   (i) a polynucleotide sequence consisting of the nucleic acid sequence set forth in any of SEQ ID NOs: 1, 25, 49 to 55, 163 or 166, or the complement thereof,
   (ii) a polynucleotide sequence having at least 85% sequence identity as determined using the BLASTN alignment tool to the nucleic acid sequence set forth in SEQ ID NO 1, or the complement thereof,
   (iii) a polynucleotide sequence consisting of a fragment of at least 21 contiguous nucleotides of any of SEQ ID NOs 1, 25, 49 to 55, 163 or 166, or the complement thereof.

Preferably, said said object is selected from the group consisting of wood, tree, book binding, cloth and a food storage container.

Finally, the invention provides for the use of the composition according to the present invention, for preventing or treating an insect infestation.

### BRIEF DESCRIPTION OF THE DRAWINGS

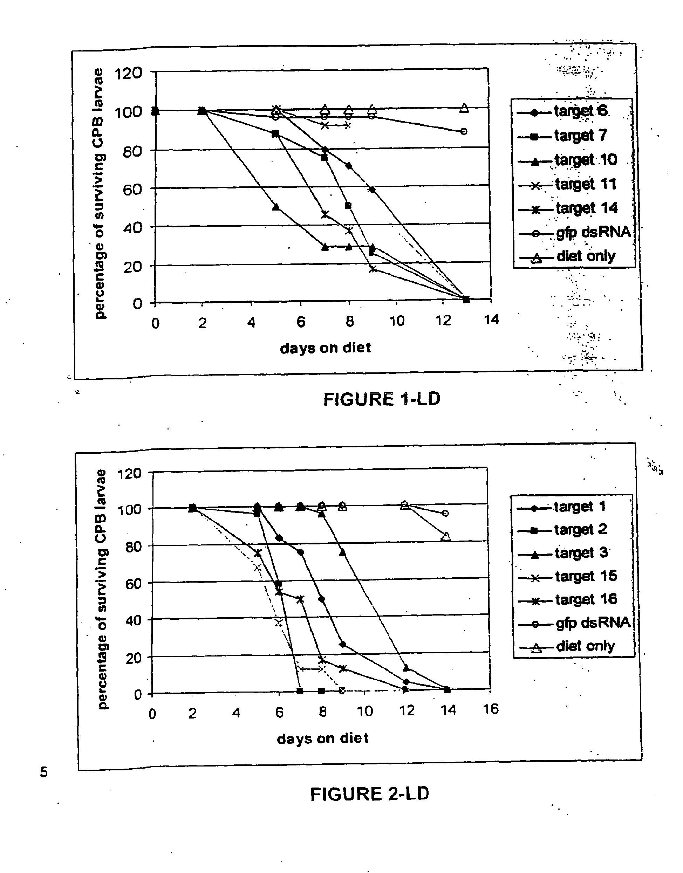

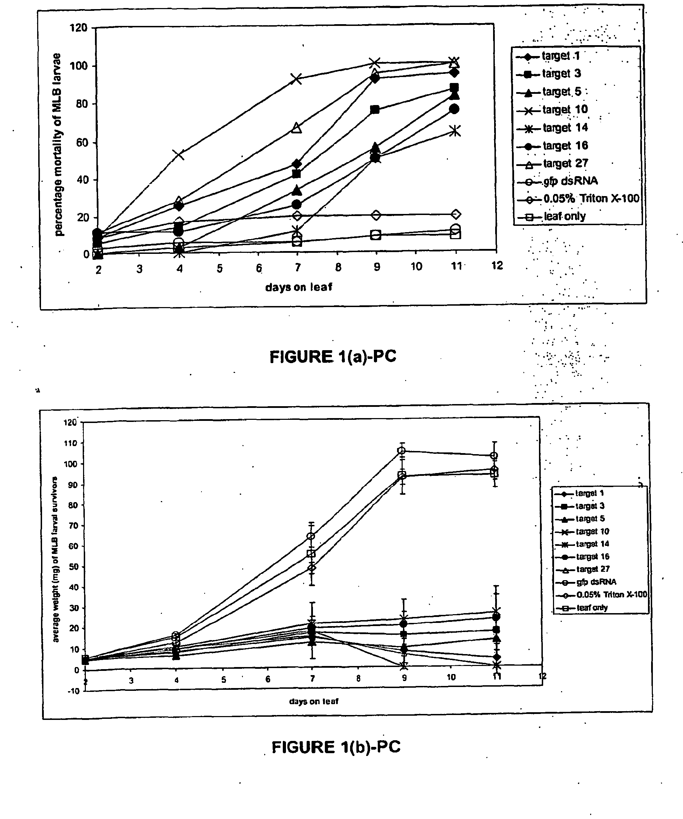

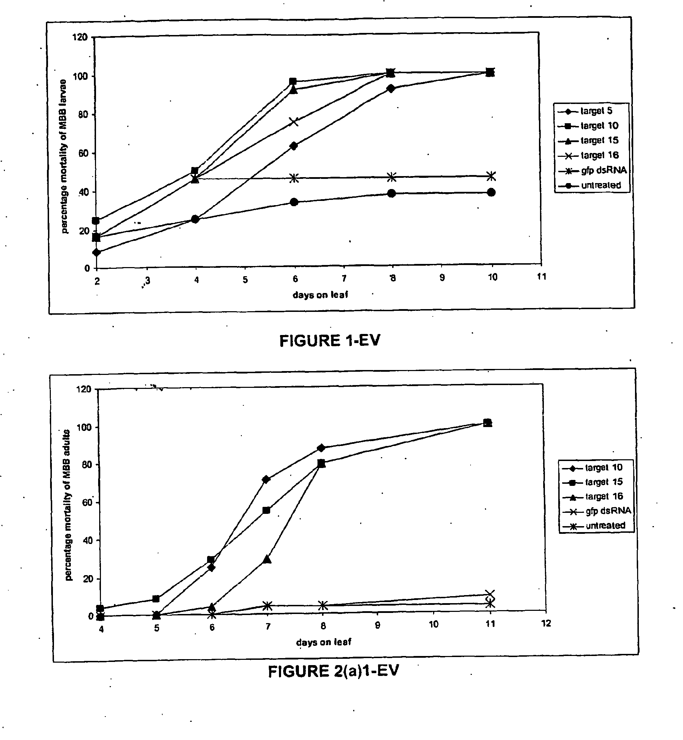

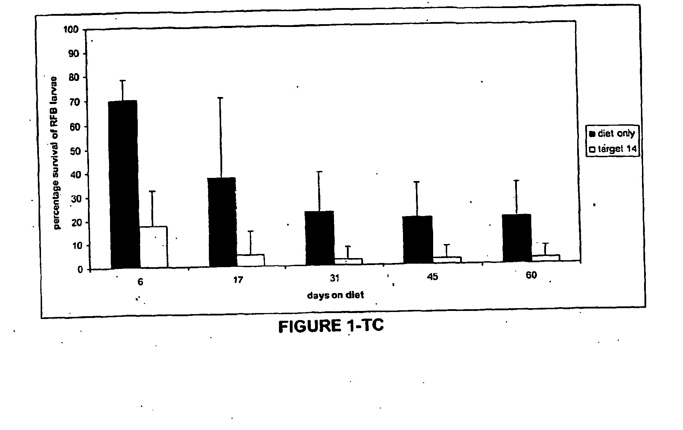

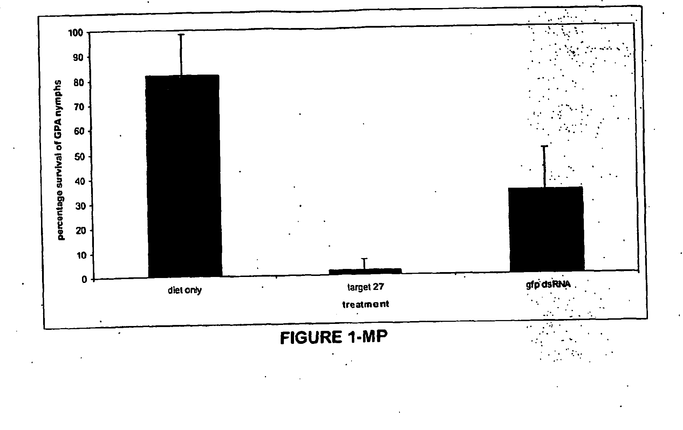

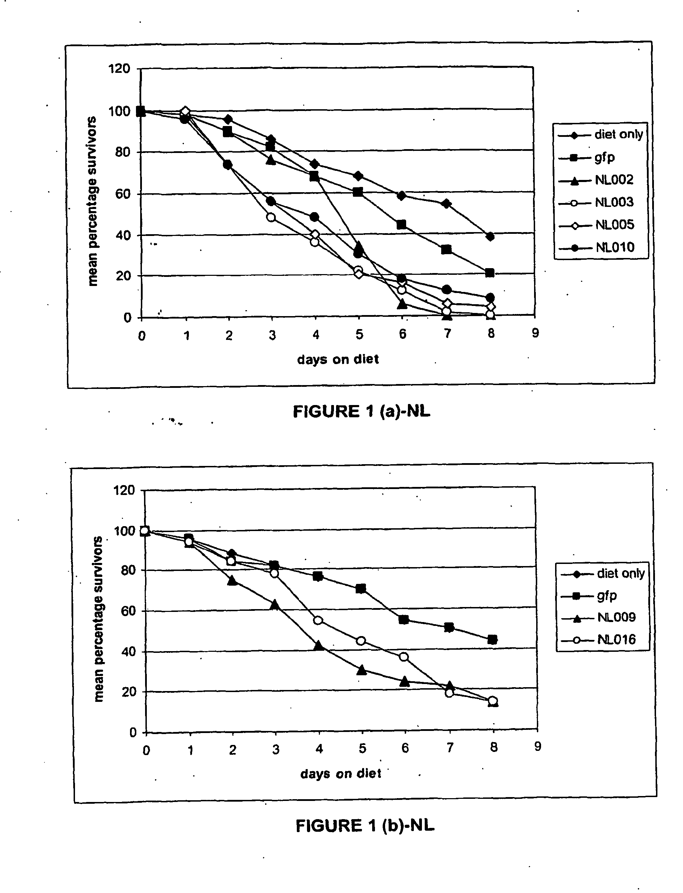

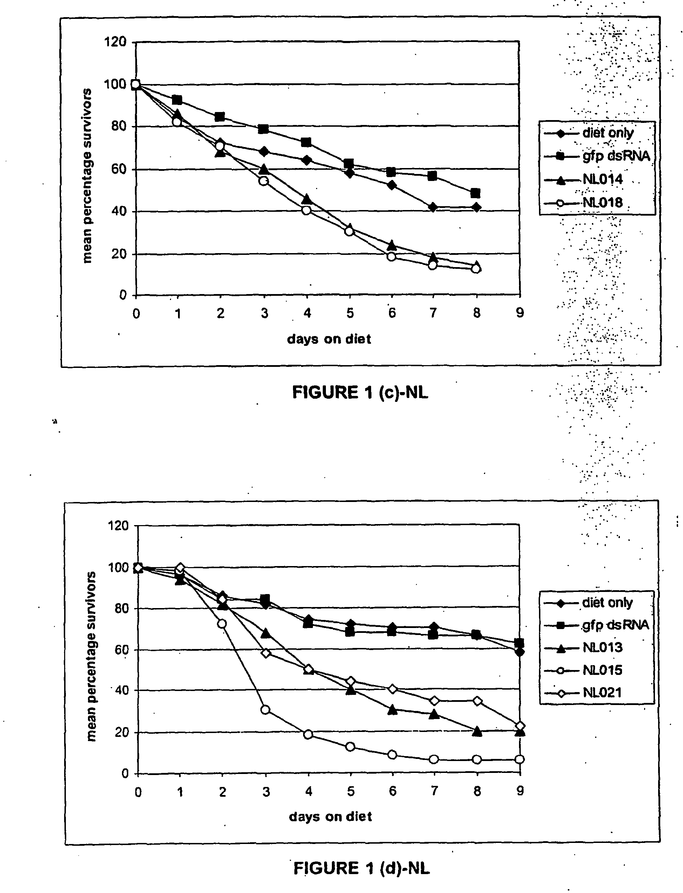

**Figure 1-LD****:** Survival of *L. decemlineata* on artificial diet treated with dsRNA. Insects of the second larval stage were fed diet treated with 50 µl of topically-applied solution of dsRNA (targets or gfp control). Diet was replaced with fresh diet containing topically-applied dsRNA after 7 days. The number of surviving insects were assessed at days 2, 5, 7, 8, 9, & 13. The percentage of surviving larvae was calculated relative to day 0 (start of assay). Target LD006: (SEQ ID NO: 178); Target LD007 (SEQ ID NO: 183); Target LD010 (SEQ ID NO: 188); Target LD011 (SEQ ID NO: 193); Target LD014 (SEQ ID NO: 198); gfp dsRNA (SEQ ID NO: 235).

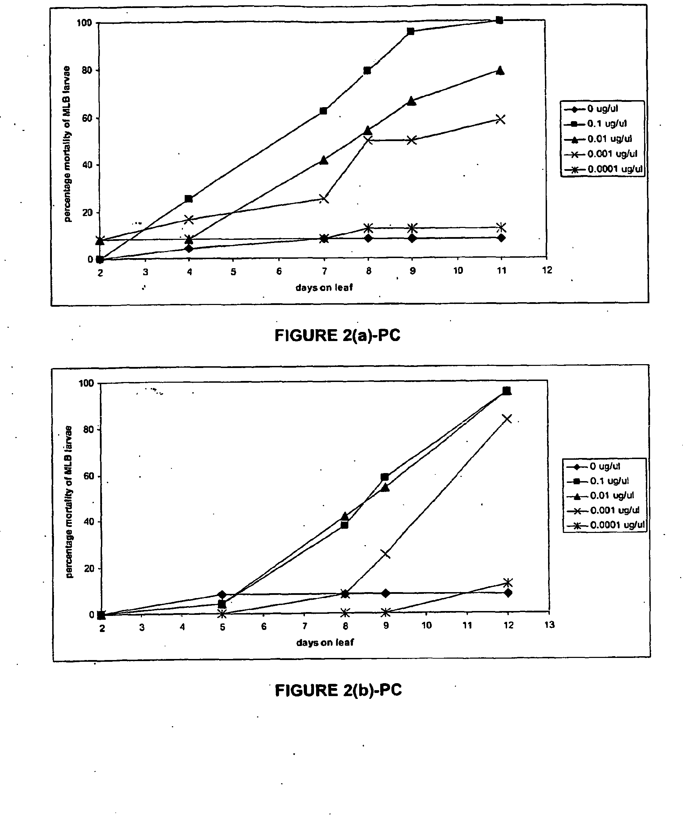

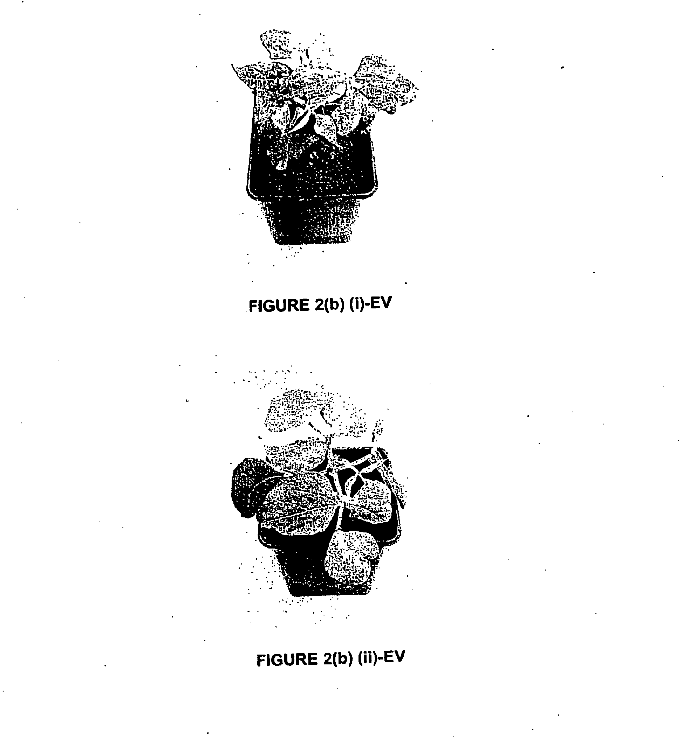

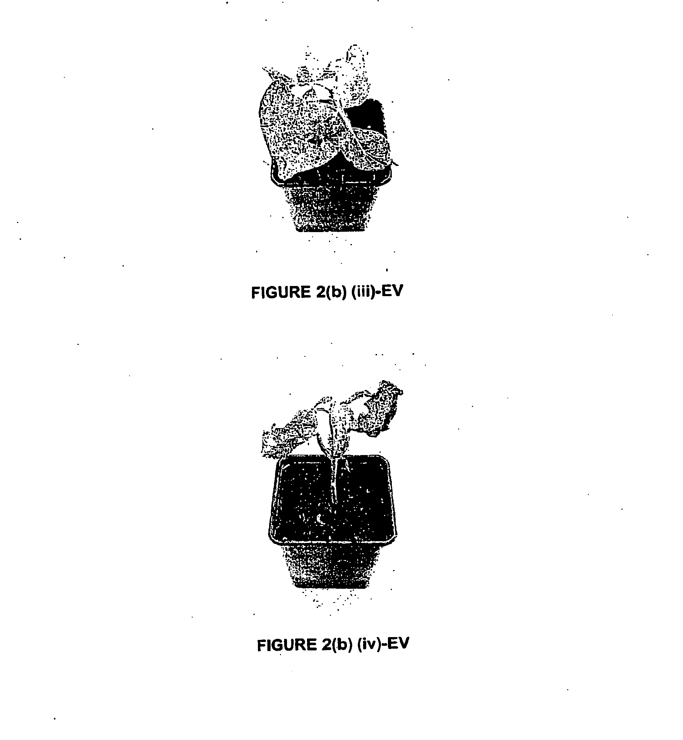

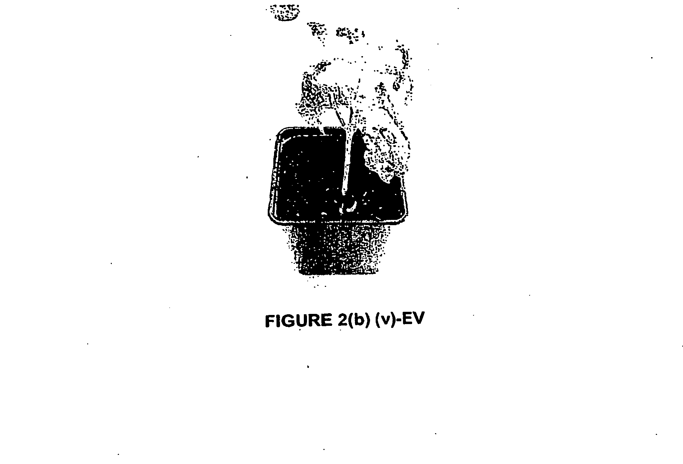

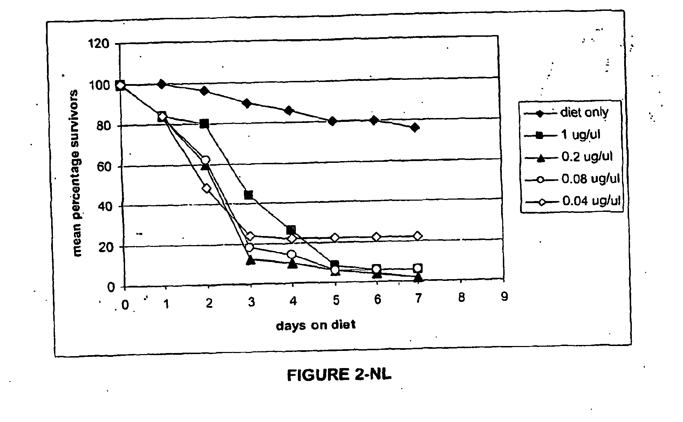

**Figure 2-LD****:** Survival of *L. decemlineata* on artificial diet treated with dsRNA. Insects of the second larval stage were fed diet treated with 50 µl of topically-applied solution of dsRNA (targets or gfp control). Diet was replaced with fresh diet only after 7 days. The number of surviving insects was assessed at days 2, 5, 6, 7, 8, 9, 12, & 14. The percentage of surviving larvae was calculated relative to day 0 (start of assay). Target LD001 (SEQ ID NO: 163); Target LD002 (SEQ ID NO: 168); Target LD003 (SEQ ID NO: 173); Target LD015 (SEQ ID NO: 215); Target LD016 (SEQ ID NO: 220); gfp dsRNA (SEQ ID NO: 235).

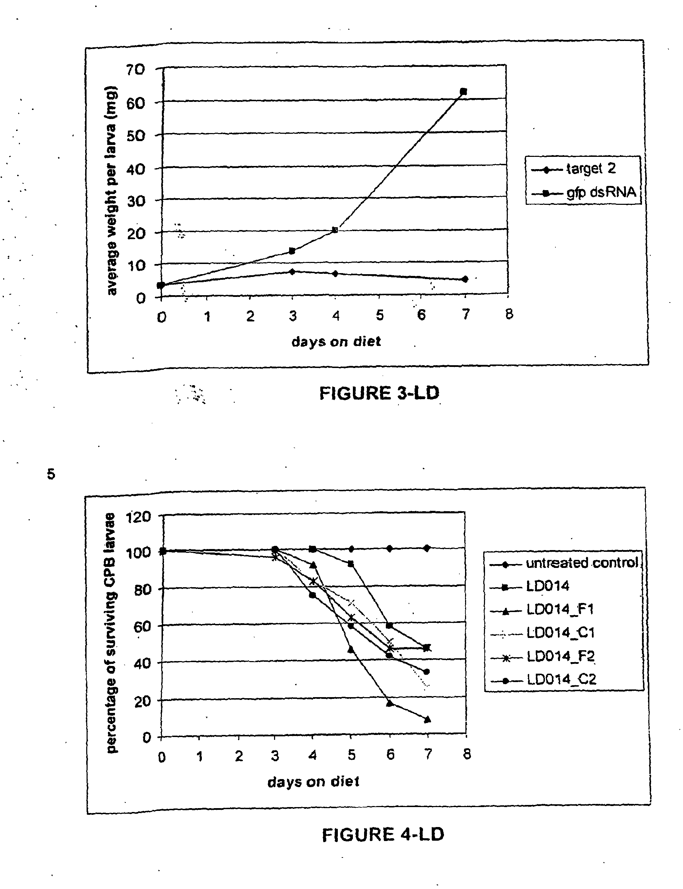

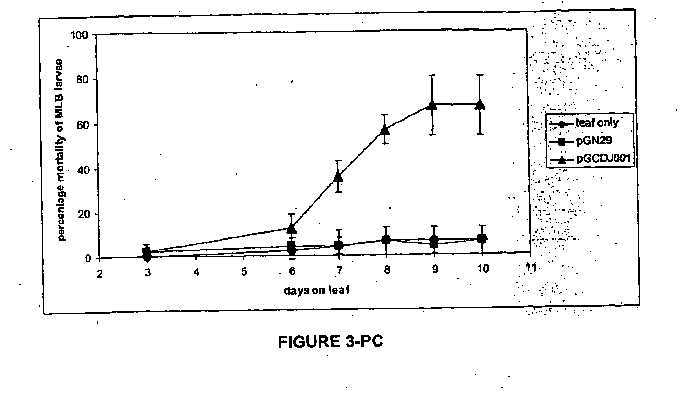

**Figure 3-LD****:** Average weight of *L. decemlineata* larvae on potato leaf discs treated with dsRNA. Insects of the second larval stage were fed leaf discs treated with 20 µl of a topically-applied solution (10 ng/µl) of dsRNA (target LD002 or gfp). After two days the insects were transferred on to untreated leaves every day.
**Figure 4-LD****:** Survival of *L. decemlineata* on artificial diet treated with shorter versions of target LD014 dsRNA and concatemer dsRNA. Insects of the second larval stage were fed diet treated with 50 µl of topically-applied solution of dsRNA (gfp or targets). The number of surviving insects were assessed at days 3, 4, 5, 6, & 7. The percentage of surviving larvae were calculated relative to day 0 (start of assay).

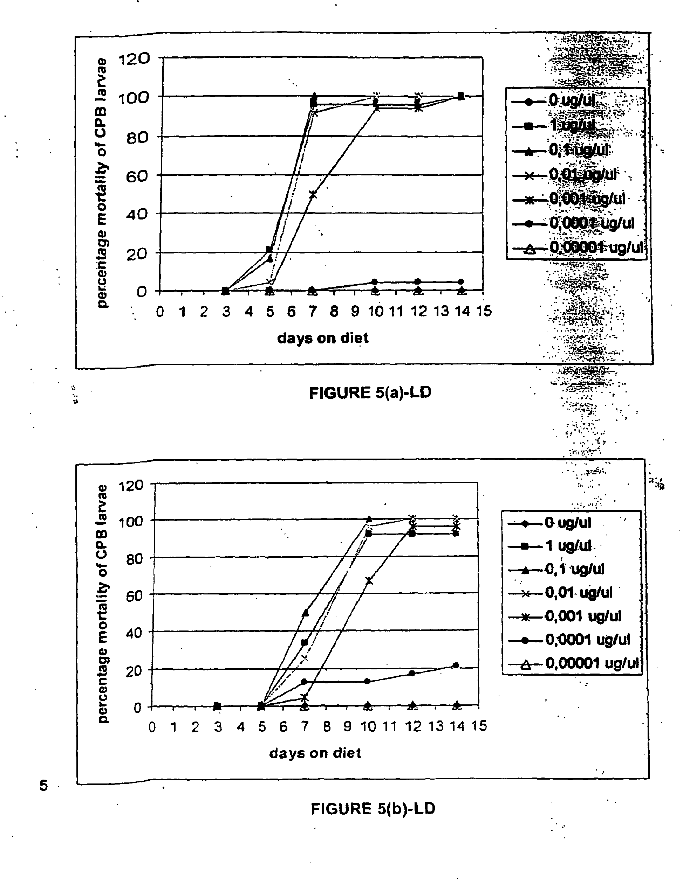

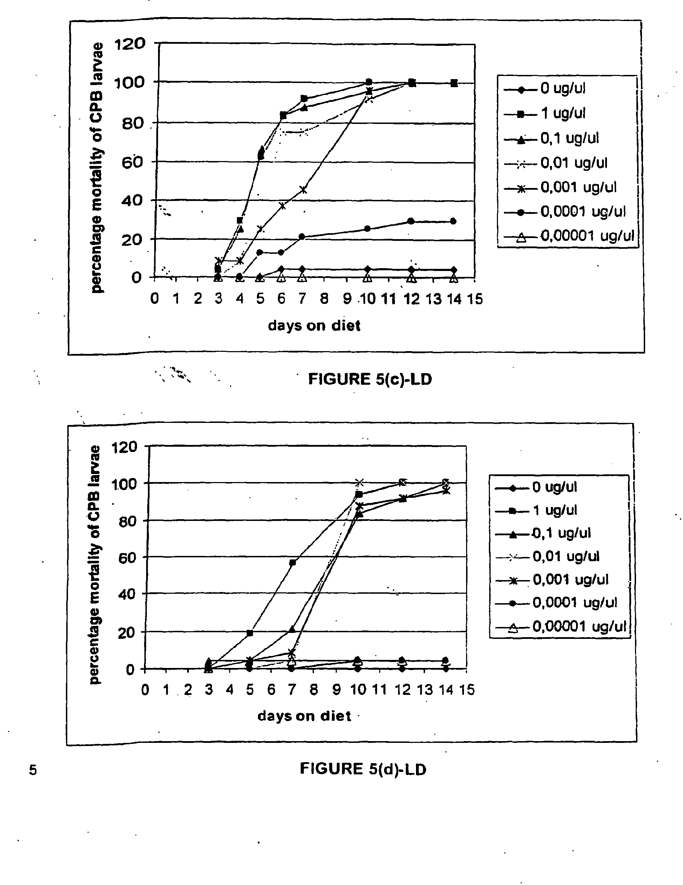

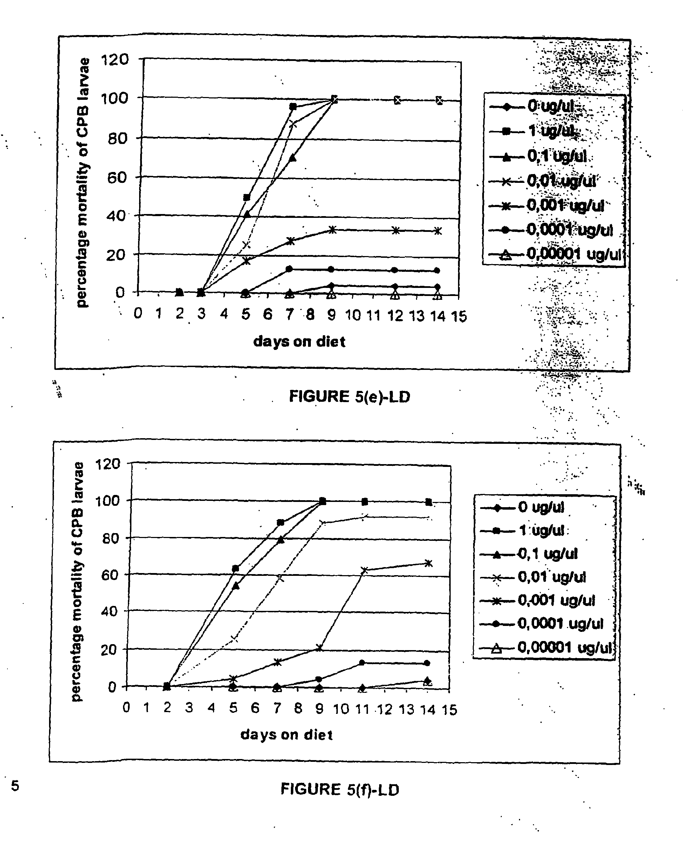

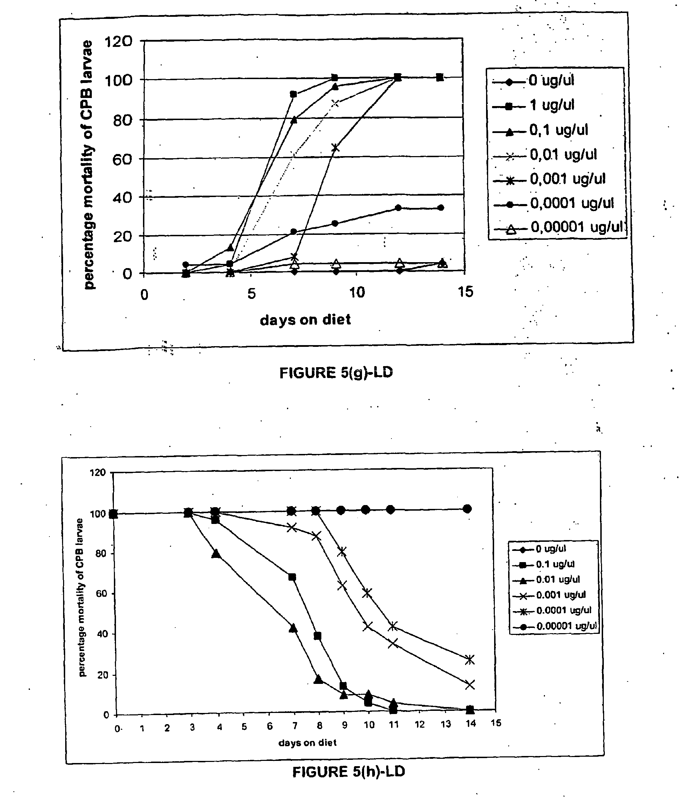

**Figure 5-LD:** Survival of *L. decemlineata* larvae on artificial diet treated with different concentrations of dsRNA of target LD002 (a), target LD007 (b), target LD010 (c), target LD011 (d), target LD014 (e), target LD015 (f), LD016 (g) and target LD027 (h). Insects of the second larval stage were fed diet treated with 50 µl of topically-applied solution of dsRNA. Diet was replaced with fresh diet containing topically-applied dsRNA after 7 days. The number of surviving insects were assessed at regular intervals. The percentage of surviving larvae were calculated relative to day 0 (start of assay).

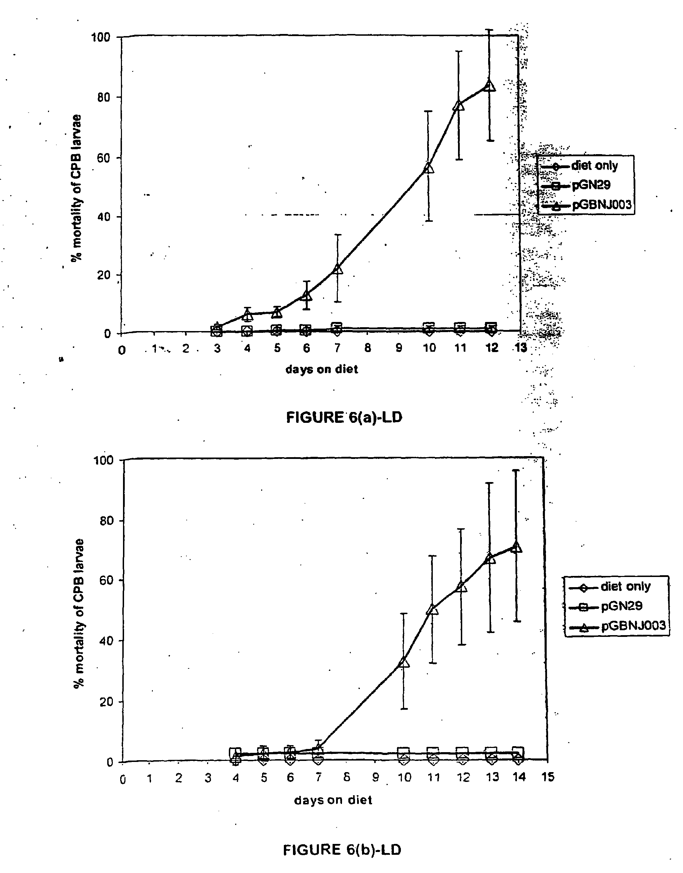

**Figure 6-LD.** Effects of *E. coli* strains expressing dsRNA target LD010 on survival of larvae of the Colorado potato beetle, *Leptinotarsa decemlineata,* over time. The two bacterial strains were tested in separate artificial diet-based bioassays: (a) AB309-105; data points for pGBNJ003 and pGN29 represent average mortality values from 5 different bacterial clones, (b) BL21(DE3); data points for pGBNJ003 and pGN29 represent average mortality values from 5 different and one single bacterial clones, respectively. Error bars represent standard deviations.

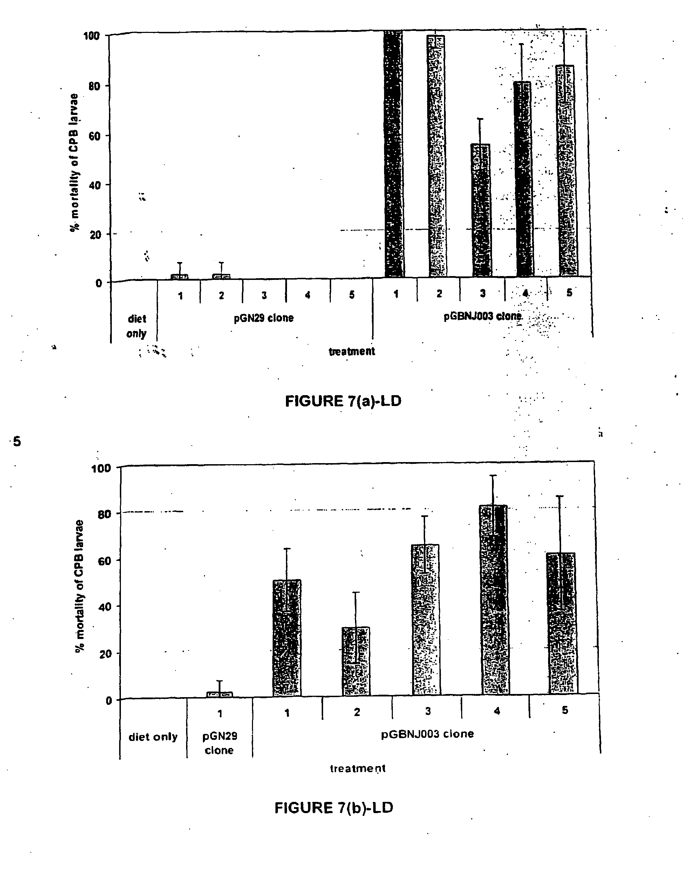

**Figure 7-LD.** Effects of different clones of *E. coli* strains (a) AB309-105 and (b) BL21(DE3) expressing dsRNA target LD010 on survival of larvae of the Colorado potato beetle, *Leptinotarsa decemlineata*, 12 days post infestation. Data points are average mortality values for each clone for pGN29 and pGBNJ003. Clone 1 of AB309-105 harbouring plasmid pGBNJ003 showed 100% mortality towards CPB at this timepoint. Error bars represent standard deviations.

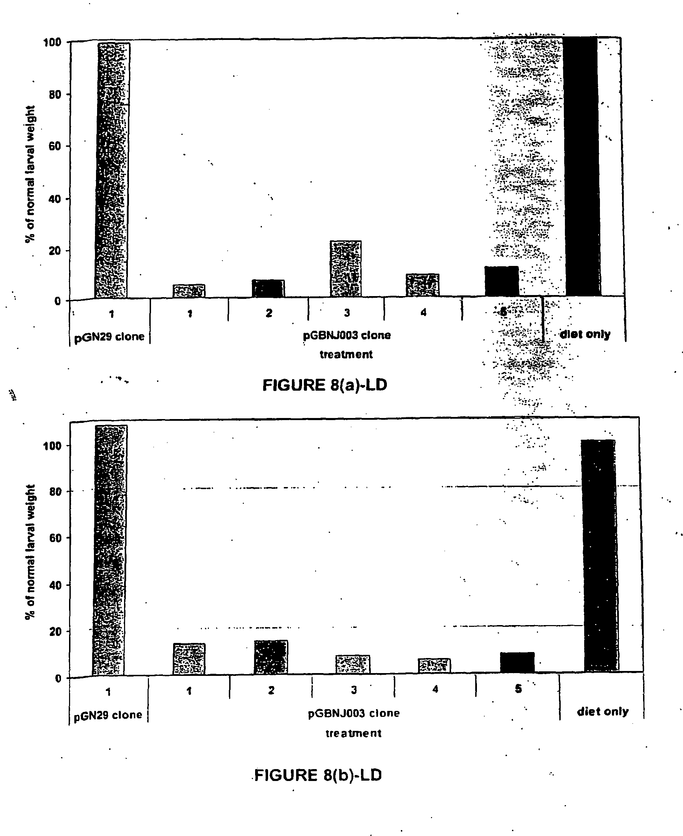

**Figure 8-LD.** Effects of different clones of *E. coli* strains (a) AB309-105 and (b) BL21(DE3) expressing dsRNA target LD010 on growth and development of larval survivors of the Colorado potato beetle, *Leptinotarsa decemlineata,* 7 days post infestation. Data points are % average larval weight values for each clone (one clone for pGN29 and five clones for pGBNJ003) based on the data of Table 10. Diet only treatment represents 100% normal larval weight.

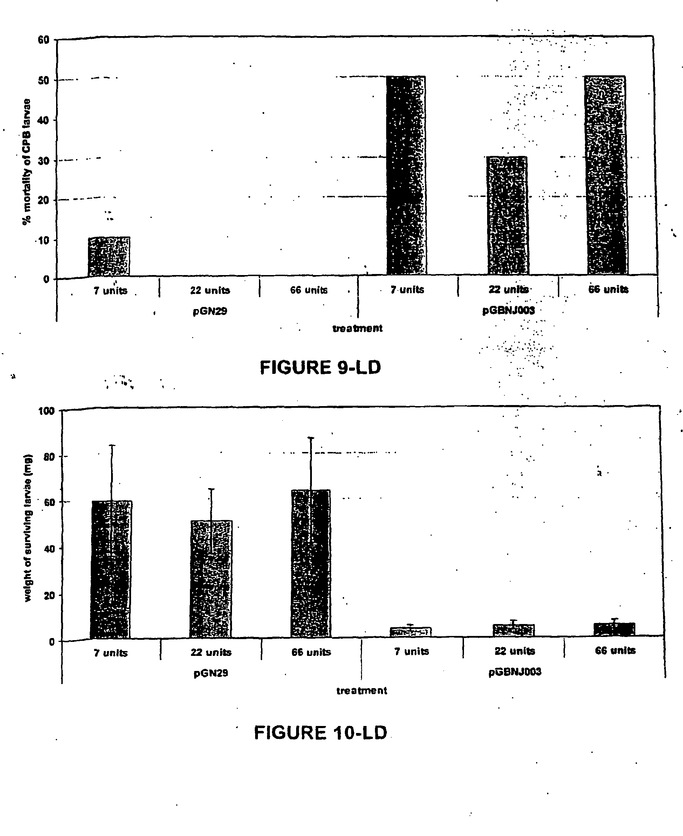

**Figure 9-LD****.** Survival of larvae of the Colorado potato beetle, *Leptinotarsa decemlineata*, on potato plants sprayed by double-stranded RNA-producing bacteria 7 days post infestation. Number of larval survivors were counted and expressed in terms of % mortality. The bacterial host strain used was the RNaseIII-deficient strain AB309-105. Insect gene target was LD010.
**Figure 10-LD****.** Growth/developmental delay of larval survivors of the Colorado potato beetle, *Leptinotarsa decemlineata*, fed on potato plants sprayed with dsRNA-producing bacteria 11 days post infestation. The bacterial host strain used was the RNaseIII-deficient strain AB309-105. Data figures represented as percentage of normal larval weight; 100 % of normal larval weight given for diet only treatment. Insect gene target was LD010. Error bars represent standard deviations.

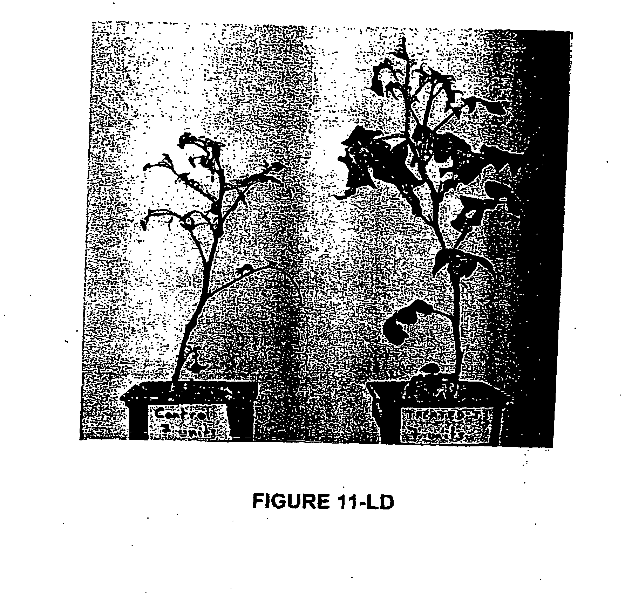

**Figure 11-LD****.** Resistance to potato damage caused by larvae of the Colorado potato beetle, *Leptinotarsa* decemlineata, by double-stranded RNA-producing bacteria 7 days post infestation. Left, plant sprayed with 7 units of bacteria AB309-105 containing the pGN29 plasmid; right, plant sprayed with 7 units of bacteria Ab309-105 containing the pGBNJ003 plasmid. One unit is defined as the equivalent of 1 ml of a bacterial suspension at OD value of 1 at 600 nm. Insect gene target was LD010.

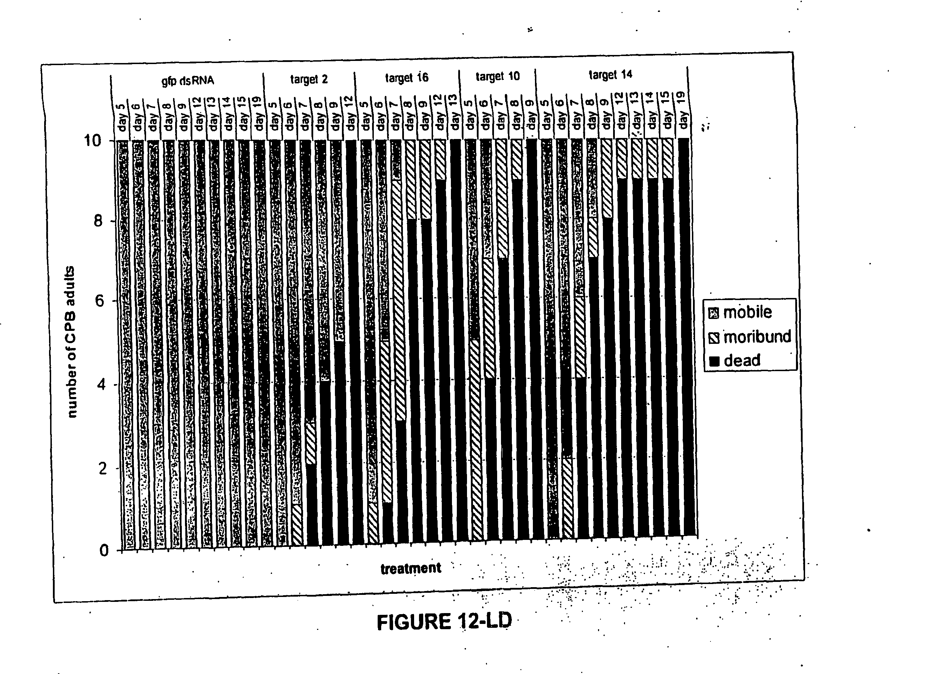

**Figure 12-LD****.** Survival of *L. decemlineata* adults on potato leaf discs treated with dsRNA. Young adult insects were fed double-stranded-RNA-treated leaf discs for the first two days and were then placed on untreated potato foliage. The number of surviving insects were assessed regularly; mobile insects were recorded as insects which were alive and appeared to move normally; moribund insects were recorded as insects which were alive but appeared sick and slow moving - these insects were not able to right themselves once placed on their backs. Target LD002 (SEQ ID NO: 168); Target LD010 (SEQ ID NO: 188); Target LD014 (SEQ ID NO: 198); Target LD016 (SEQ ID NO: 220); gfp dsRNA (SEQ ID NO: 235).

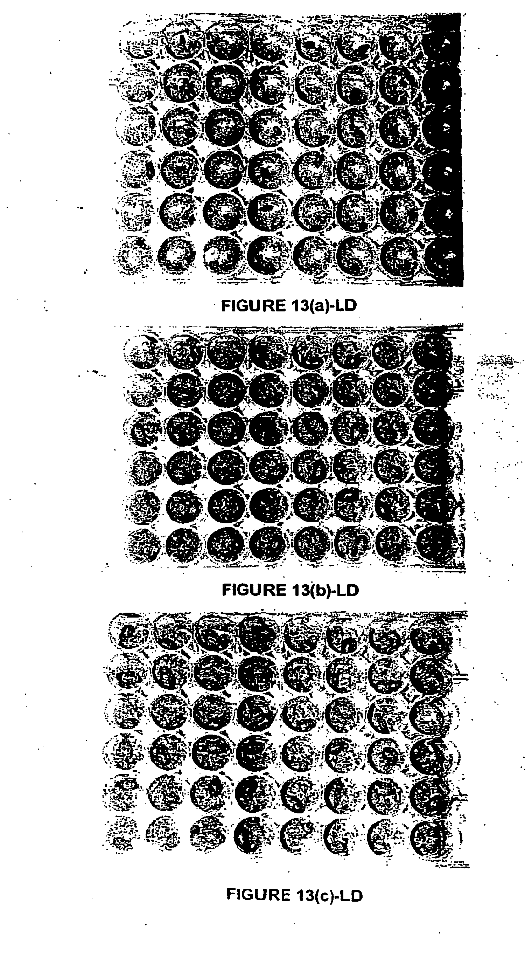

**Figure 13-LD**. Effects of bacterial produced target double-stranded RNA against larvae of *L. decemlineata*. Fifty µl of an OD 1 suspension of heat-treated bacteria expressing dsRNA (SEQ ID NO: 188) was applied topically onto the solid artificial diet in each well of a 48-well plate. CPB larvae at L2 stage were placed in each well. At day 7, a picture was taken of the CPB larvae in a plate containing (a) diet with bacteria expressing target 10 double-stranded RNA, (b) diet with bacteria harbouring the empty vector pGN29, and, (c) diet only.

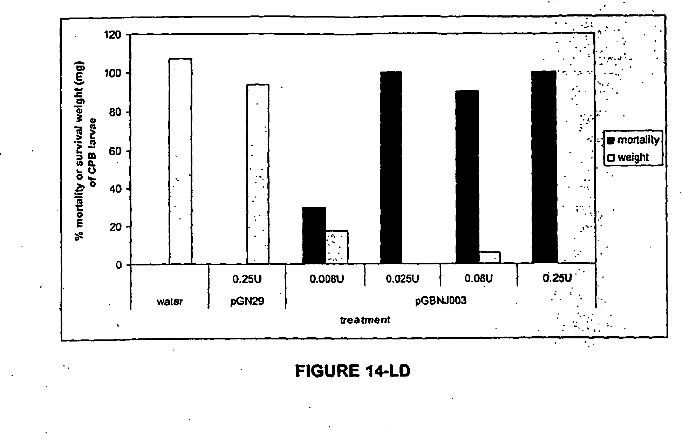

**Figure 14-LD** Effects on CPB larval survival and growth of different amounts of inactivated *E. coli* AB309-105 strain harbouring plasmid pGBNJ003 topically applied to potato foliage prior to insect infestation. Ten L1 larvae were fed treated potato for 7 days. Amount of bacterial suspension sprayed on plants: 0.25 U, 0.08 U, 0.025 U, 0.008 U of target 10 and 0.25 U of pGN29 (negative control; also included is Milli-Q water). One unit (U) is defined as the equivalent bacterial amount present in 1 ml of culture with an optical density value of 1 at 600nm. A total volume of 1.6 ml was sprayed on to each plant. Insect gene target was LD010.

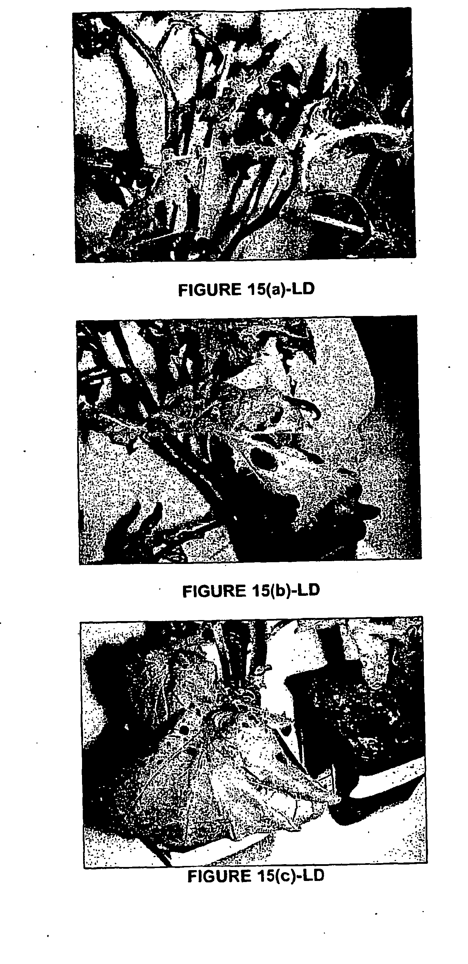

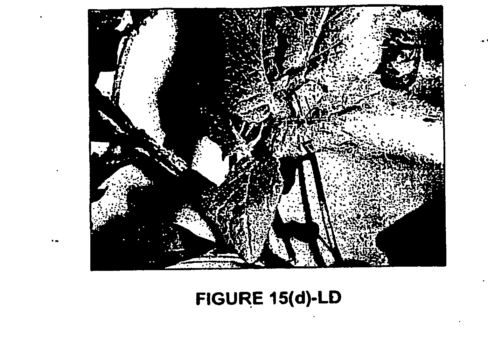

**Figure 15-LD** Resistance to potato damage caused by CPB larvae by inactivated *E. coli* AB309-105 strain harbouring plasmid pGBNJ003 seven days post infestation. **(a)** water, **(b)** 0.25 U *E. coli* AB309-105 harbouring pGN29, **(c)** 0.025 U *E. coli* AB309-105 harbouring pGBNJ003, **(d)** 0.008 U *E. coli* AB309-105 harbouring pGBNJ003. One unit (U) is defined as the equivalent bacterial amount present in 1 ml of culture with an optical density value of 1 at 600nm. A total volume of 1.6 ml was sprayed on to each plant. Insect gene target was LD010.
**Figure 1-PC:** Effects of ingested target dsRNAs on survival and growth of *P. cochleariae* larvae. Neonate larvae were fed oilseed rape leaf discs treated with 25 µl of topically-applied solution of 0.1 µg/µl dsRNA (targets or gfp control). Afer 2 days, the insects were transferred onto fresh dsRNA-treated leaf discs. At day 4, larvae from one replicate for every treatment were collected and placed in a Petri dish containing fresh untreated oilseed rape foliage. The insects were assessed at days 2, 4, 7, 9 & 11. **(a)** Survival of *E. varivestis* larvae on oilseed rape leaf discs treated with dsRNA. The percentage of surviving larvae was calculated relative to day 0 (start of assay). **(b)** Average weights of *P. cochleariae* larvae on oilseed rape leaf discs treated with dsRNA. Insects from each replicate were weighed together and the average weight per larva determined. Error bars represent standard deviations. Target 1: SEQ ID NO: 473; target 3: SEQ ID NO: 478; target 5: SEQ ID NO: 483 --; target 10: SEQ ID NO: 488; target 14: SEQ ID NO: 493; target 16: SEQ ID NO: 498; target 27: SEQ ID NO: 503; gfp dsRNA: SEQ ID NO: 235.
**Figure 2-PC:** Survival of *P. cochleariae* on oilseed rape leaf discs treated with different concentrations of dsRNA of **(a)** target PC010 and **(b)** target PC027. Neonate larvae were placed on leaf discs treated with 25 µl of topically-applied solution of dsRNA. Insects were transferred to fresh treated leaf discs at day 2. At day 4 for target PC010 and day 5 for target PC027, the insects were transferred to untreated leaves. The number of surviving insects were assessed at days 2, 4, 7, 8, 9 & 11 for PC010 and 2, 5, 8, 9 & 12 for PC027. The percentage of surviving larvae was calculated relative to day 0 (start of assay).
**Figure 3-PC****:** Effects of *E. coli* strain AB309-105 expressing dsRNA target PC010 on survival of larvae of the mustard leaf beetle, *P. cochleariae*, over time. Data points for each treatment represent average mortality values from 3 different replicates. Error bars represent standard deviations. Target 10: SEQ ID NO: 488
**Figure 1-EV****:** Survival of *E. varivestis* larvae on bean leaf discs treated with dsRNA. Neonate larvae were fed bean leaf discs treated with 25 µl of topically-applied solution of 1 µg/µl dsRNA (targets or gfp control). Afer 2 days, the insects were transferred onto fresh dsRNA-treated leaf discs. At day 4, larvae from one treatment were collected and placed in a plastic box containing fresh untreated bean foliage. The insects were assessed for mortality at days 2, 4, 6, 8 & 10. The percentage of surviving larvae was calculated relative to day 0 (start of assay). Target 5: SEQ ID NO: 576; target 10: SEQ ID NO: 586; target 15: SEQ ID NO: 591; target 16: SEQ ID NO: 596; gfp dsRNA: SEQ ID NO: 235.
**Figure 2-EV:** Effects of ingested target dsRNAs on surival of *E. varivestis* adults and resistance to snap bean foliar insect damage. **(a)** Surivival of *E. varivestis* adults on bean leaf treated with dsRNA. Adults were fed bean leaf discs treated with 75 µl of topically-applied solution of 0.1 µg/µl dsRNA (targets or gfp control). After 24 hours, the insects were transferred onto fresh dsRNA-treated leaf discs. After a further 24 hours, adults from one treatment were collected and placed in a plastic box containing potted fresh untreated whole bean plants. The insects were assessed for mortality at days 4, 5, 6, 7, 8, & 11. The percentage of surviving adults was calculated relative to day 0 (start of assay). Target 10: SEQ ID NO: 586; target 15: SEQ ID NO: 591; target 16: SEQ ID NO: 596; gfp dsRNA: SEQ ID NO: 235. **(b)** Resistance to bean foliar damage caused by adults of the *E. varivestis* by dsRNA. Whole plants containing insects from one treatment (see (a)) were checked visually for foliar damage on day 9. **(i)** target 10; **(ii)** target 15; **(iii)** target 16; **(iv)** gfp dsRNA; **(v)** untreated.
**Figure 1-TC****:** Survival of *T. castaneum* larvae on artificial diet treated with dsRNA of target 14. Neonate larvae were fed diet based on a flour/milk mix with 1 mg dsRNA target 14. Control was water (without dsRNA) in diet. Four replicates of 10 first instar larvae per replicate were performed for each treatment. The insects were assessed for survival as average percentage means at days 6, 17, 31, 45 and 60. The percentage of surviving larvae was calculated relative to day 0 (start of assay). Error bars represent standard deviations. Target TC014: SEQ ID NO: 878.
**Figure 1-MP****:** Effect of ingested target 27 dsRNA on the survival of *Myzus persicae* nymphs. First instars were placed in feeding chambers containing 50 µl of liquid diet with 2 µg/µl dsRNA (target 27 or gfp dsRNA control). Per treatment, 5 feeding chambers were set up with 10 instars in each feeding chamber. Number of survivors were assessed at 8 days post start of bioassay. Error bars represent standard deviations. Target MP027: SEQ ID NO: 1061; gfp dsRNA: SEQ ID NO: 235.
**Figure 1****-NL:** Survival of *Nilaparvata lugens* on liquid artificial diet treated with dsRNA. Nymphs of the first to second larval stage were fed diet supplemented with 2 mg/ml solution of dsRNA targets in separate bioassays: (a) NL002, NL003, NL005, NL010; (b) NL009, NL016; (c) NL014, NL018; (d) NL013, NL015, NL021. Insect survival on targets were compared to diet only and diet with gfp dsRNA control at same concentration. Diet was replaced with fresh diet containing dsRNA every two days. The number of surviving insects were assessed every day
**Figure 2****-NL:** Survival of *Nilaparvata lugens* on liquid artificial diet treated with different concentrations of target dsRNA NL002. Nymphs of the first to second larval stage were fed diet supplemented with 1, 0.2, 0.08, and 0.04 mg/ml (final concentration) of NL002. Diet was replaced with fresh diet containing dsRNA every two days. The numbers of surviving insects were assessed every day.

### DETAILED DESCRIPTION OF THE INVENTION

The present invention provides a means for controlling pest infestations by exposing a pest to a target coding sequence that post-transcriptionally represses or inhibits a requisite biological function in the pest. Following exposure to a target sequence, the target forms a the dsRNA corresponding to part or whole of an essential pest gene and causes down regulation of the pest target via RNA interference (RNAi). As a result of the down regulation of mRNA, the dsRNA prevents expression of the target pest protein and hence causes death, growth arrest, or sterility of the pest.

The present invention finds application in any area where it is desirable to inhibit viability, growth, development or reproduction of a pest, or to decrease pathogenicity or infectivity of a pest. Practical applications include, but are not limited to, (1) protecting plants against pest infestation; (2) pharmaceutical or veterinary use in humans and animals (for example to control, treat, or prevent insect infections in humans and animals); (3) protecting materials against damage caused by pests; and (4) protecting perishable materials (such as foodstuffs, seed, etc.) against damage caused by pests.

Administering or exposing a double stranded ribonucleic acid molecule to a pest results in one or more of the following attributes: reduction in feeding by the pest, reduction in viability of the pest, death of the pest, inhibition of differentiation and development of the pest, absence of or reduced capacity for sexual reproduction by the pest, muscle formation, juvenile hormone formation, juvenile hormone regulation, ion regulation and transport, maintenance of cell membrane potential, amino acid biosynthesis, amino acid degradation, sperm formation, pheromone synthesis, pheromone sensing, antennae formation, wing formation, leg formation, development and differentiation, egg formation, larval maturation, digestive enzyme formation, haemolymph synthesis, haemolymph maintenance, neurotransmission, cell division, energy metabolism, respiration, apoptosis, and any component of a eukaryotic cells' cytoskeletal structure, such as, for example, actins and tubulins. Any one or any combination of these attributes can result in an effective inhibition of pest infestation.

All technical terms employed in this specification are commonly used in biochemistry, molecular biology and agriculture; hence, they are understood by those skilled in the field to which this invention belongs. Those technical terms can be found, for example in: MOLECULAR CLONING: A LABORATORY MANUAL, 3rd ed., vol. 1-3, ed. Sambrook and Russel, Cold Spring Harbor Laboratory Press, Cold Spring Harbor, N.Y., 2001; CURRENT PROTOCOLS IN MOLECULAR BIOLOGY, ed. Ausubel et al., Greene Publishing Associates and Wiley-Interscience, New York, 1988 (with periodic updates); SHORT PROTOCOLS IN MOLECULAR BIOLOGY: A COMPENDIUM OF METHODS FROM CURRENT PROTOCOLS IN MOLECULAR BIOLOGY, 5th ed., vol. 1-2, ed. Ausubel et al., John Wiley & Sons, Inc., 2002; GENOME ANALYSIS: A LABORATORY MANUAL, vol. 1-2, ed. Green et al., Cold Spring Harbor Laboratory Press, Cold Spring Harbor, N.Y., 1997.

Various techniques using PCR are described, for example, in Innis et al., PCR PROTOCOLS: A GUIDE TO METHODS AND APPLICATIONS, Academic Press, San Diego, 1990 and in Dieffenbach and Dveksler, PCR PRIMER: A LABORATORY MANUAL, 2nd ed., Cold Spring Harbor Laboratory Press, Cold Spring Harbor, N.Y., 2003. PCR-primer pairs can be derived from known sequences by known techniques such as using computer programs intended for that purpose, *e.g.*, Primer, Version 0.5, 1991, Whitehead Institute for Biomedical Research, Cambridge, MA. Methods for chemical synthesis of nucleic acids are discussed, for example, in Beaucage & Caruthers, Tetra. Letts. 22: 1859-62 (1981), and Matteucci & Caruthers, J. Am. Chem. Soc. 103: 3185 (1981).

Restriction enzyme digestions, phosphorylations, ligations, and transformations were done as described in Sambrook et al., MOLECULAR CLONING: A LABORATORY MANUAL, 2nd ed. (1989), Cold Spring Harbor Laboratory Press. All reagents and materials used for the growth and maintenance of bacterial cells were obtained from Aldrich Chemicals (Milwaukee, Wis.), DIFCO Laboratories (Detroit, Mich.), Invitrogen (Gaithersburg, Md.), or Sigma Chemical Company (St. Louis, Mo.) unless otherwise specified.

**Biological activity** refers to the biological behavior and effects of a protein or peptide and its manifestations on a pest. For example, an inventive RNAi may prevent translation of a particular mRNA, thereby inhibiting the biological activity of the protein encoded by the mRNA or other biological activity of the pest.

In the present description, an RNAi molecule may inhibit a biological activity in a pest, resulting in one or more of the following attributes: reduction in feeding by the pest, reduction in viability of the pest, death of the pest, inhibition of differentiation and development of the pest, absence of or reduced capacity for sexual reproduction by the pest, muscle formation, juvenile hormone formation, juvenile hormone regulation, ion regulation and transport, maintenance of cell membrane potential, amino acid biosynthesis, amino acid degradation, sperm formation, pheromone synthesis, pheromone sensing, antennae formation, wing formation, leg formation, development and differentiation, egg formation, larval maturation, digestive enzyme formation, haemolymph synthesis, haemolymph maintenance, neurotransmission, cell division, energy metabolism, respiration, apoptosis, and any component of a eukaryotic cells' cytoskeletal structure, such as, for example, actins and tubulins.

**Complementary DNA** (cDNA) refers to single-stranded DNA synthesized from a mature mRNA template. Though there are several methods, cDNA is most often synthesized from mature (fully spliced) mRNA using the enzyme reverse transcriptase. This enzyme operates on a single strand of mRNA, generating its complementary DNA based on the pairing of RNA base pairs (A, U, G, C) to their DNA complements (T, A, C, G). Two nucleic acid strands are **substantially complementary** when at least 85% of their bases pair.

**Desired Polynucleotide:** a desired polynucleotide is a genetic element, such as a promoter, enhancer, or terminator, or gene or polynucleotide that is to be transcribed and/or translated in a transformed cell that comprises the desired polynucleotide in its genome. If the desired polynucleotide comprises a sequence encoding a protein product, the coding region may be operably linked to regulatory elements, such as to a promoter and a terminator, that bring about expression of an associated messenger RNA transcript and/or a protein product encoded by the desired polynucleotide. Thus, a "desired polynucleotide" may comprise a gene that is operably linked in the 5'- to 3'- orientation, a promoter, a gene that encodes a protein, and a terminator. Alternatively, the desired polynucleotide may comprise a gene or fragment thereof, in a "sense" or "antisense" orientation, the transcription of which produces nucleic acids that may affect expression of an endogenous gene in the host cell. -A desired polynucleotide may also yield upon transcription a double-stranded RNA product upon that initiates RNA interference of a gene to which the desired polynucleotide is associated. -A desired polynucleotide may be positioned within a vector, such that the left and right border sequences flank or are on either side of the desired polynucleotide. -The present invention envisions the stable integration of one or more desired polynucleotides into the genome of at least one host cell. -A desired polynucleotide may be mutated or a variant of its wild-type sequence.- It is understood that all or part of the desired polynucleotide can be integrated into the genome of a host. It also is understood that the term "desired polynucleotide" encompasses one or more of such polynucleotides. -Thus, a vector may comprise one, two, three, four, five, six, seven, eight, nine, ten, or more desired polynucleotides.

**"Exposing"** encompasses any method by which a pest may come into contact with a dsRNA, wherein the dsRNA comprises annealed complementary strands, one of which has a nucleotide sequence which is complementary to at least part of the nucleotide sequence of a pest target gene to be down-regulated. -A pest may be exposed to the dsRNA by direct uptake (e.g. by feeding), which does not require expression of dsRNA within the pest. -Alternatively, a pest may come into direct contact with a composition comprising the dsRNA.- For example, a pest may come into contact with a surface or material treated with a composition comprising a dsRNA. A dsRNA may be expressed by a prokaryotic (for instance, but not limited to, a bacterial) or eukaryotic (for instance, but not limited to, a yeast) host cell or host organism.

**Foreign:** "foreign," with respect to a nucleic acid, means that that nucleic acid is derived from non-host organisms. Foreign DNA or RNA represents nucleic acids that are naturally occurring in the genetic makeup of viruses, mammals, fish or birds, but are not naturally occurring in the host that is to be transformed. Thus, a foreign nucleic acid is one that encodes, for instance, a polypeptide that is not naturally produced by the transformed host. -A foreign nucleic acid does not have to encode a protein product.

**Gene:** refers to a polynucleotide sequence that comprises control and coding sequences necessary for the production of a polypeptide or precursor. The polypeptide can be encoded by a full length coding sequence or by any portion of the coding sequence. A gene may constitute an uninterrupted coding sequence or it may include one or more introns, bound by the appropriate splice junctions. Moreover, a gene may contain one or more modifications in either the coding or the untranslated regions that could affect the biological activity or the chemical structure of the expression product, the rate of expression, or the manner of expression control. Such modifications include, but are not limited to, mutations, insertions, deletions, and substitutions of one or more nucleotides. In this regard, such modified genes may be referred to as "variants" of the "native" gene.

**Genetic element:** a "genetic element" is any discreet nucleotide sequence such as, but not limited to, a promoter, gene, terminator, intron, enhancer, spacer, 5'-untranslated region, 3'-untranslated region, or recombinase recognition site.

**Genetic modification:** stable introduction of a nucleic acid into the genome of certain organisms by applying methods in molecular and cell biology.

**"Gene suppression"** or "down-regulation of gene expression" or "inhibition of gene expression" are used interchangeably and refer to a measurable or observable reduction in gene expression or a complete abolition of detectable gene expression, at the level of protein product and/or mRNA product from the target gene. Down-regulation or inhibition of gene expression is "specific" when down-regulation or inhibition of the target gene occurs without manifest effects on other genes of the pest.

Depending on the nature of the target gene, down-regulation or inhibition of gene expression in cells of a pest can be confirmed by phenotypic analysis of the cell or the whole pest or by measurement of mRNA or protein expression using molecular techniques such as RNA solution hybridization, nuclease protection, Northern hybridization, reverse transcription, gene expression monitoring with a microarray, antibody binding, enzyme-linked immunosorbent assay (ELISA), Western blotting, radioimmunoassay (RIA), other immunoassays, or fluorescence-activated cell analysis (FACS).

**Gymnosperm,** as used herein, refers to a seed plant that bears seed without ovaries. Examples of gymnosperms include conifers, cycads, ginkgos, and ephedras.

**Homology,** as used herein relates to sequences; Protein, or nucleotide sequences are likely to be homologous if they show a "significant" level of sequence similarity or more preferably sequence identity. Truely homologous sequences are related by divergence from a common ancestor gene. Sequence homologs can be of two types:(i) where homologs exist in different species they are known as orthologs. e.g. the α-globin genes in mouse and human are orthologs; (ii) paralogues are homologous genes in within a single species. e.g. the α- and β- globin genes in mouse are paralogs.

**Host cell:** refers to a microorganism, a prokaryotic cell, a eukaryotic cell, or cell line cultured as a unicellular entity that may be, or has been, used as a recipient for a recombinant vector or other transfer of polynucleotides, and includes the progeny of the original cell that has been transfected. The progeny of a single cell may not necessarily be completely identical in morphology or in genomic or total DNA complement as the original parent due to natural, accidental, or deliberate mutation.

**Introduction:** as used herein, refers to the insertion of a nucleic acid sequence into a cell, by methods including infection, transfection, transformation, or transduction.

**Insect pests** as used herein pests are include but are not limited to: from the order Lepidoptera, for example, *Acleris spp., Adoxophyes spp., Aegeria spp., Agrotis spp., Alabama argillaceae, Amylois spp., Anticarsia gemmatalis, Archips spp, Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia Nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni* and *Yponomeuta spp.;*
from the order *Coleoptera,* for example, *Agriotes spp., Anthonomus spp., Atomaria linearis, Chaetocnema tibialis, Cosmopolites spp., Curculio spp., Dennestes spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp.* and *Trogoderma spp.;*
from the order *Orthoptera*, for example, *Blatta spp., Blattella spp., Gryllotalpa spp., Leucophaea maderae, Locusta spp., Periplaneta ssp.*, and *Schistocerca spp.*;
from the order *Isoptera*, for example, *Reticu*/*itemes ssp*; from the order *Psocoptera*, for example, *Liposcelis spp.*; from the order *Anoplura*, for example, *Haematopinus spp.*, *Linognathus spp.*, *Pediculus spp., Pemphigus spp.* and *Phylloxera spp.*;
from the order *Mallophaga*, for example, *Damalinea spp.* and *Trichodectes spp.*;
from the order *Thysanoptera*, for example, *Franklinella spp., Hercinothrips spp.*, *Taeniothrips spp., Thrips palmi, Thrips tabaci* and *Scirtothrips aurantii*;
from the order *Heteroptera*, for example, *Cimex spp.*, *Distantiella theobroma*, *Dysdercus spp., Euchistus spp., Eurygaster spp., Leptocorisa spp., Nezara spp., Piesma spp., Rhodnius spp., Sahlbergella singularis, Scotinophara spp., Triatoma spp., Miridae family spp. such as Lygus hesperus* and *Lygus lineoloris, Lygaeidae* family *spp.* such as *Blissus leucopterus*, and *Pentatomidae* family *spp.*;
from the order *Homoptera*, for example, *Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lacanium corni, Lepidosaphes spp., Macrosiphus spp., Myzus spp., Nehotettix spp., Nilaparvata spp., Paratoria spp., Pemphigus spp., Planococcus spp., Pseudaulacaspis spp., Pseudococcus spp., Psylla ssp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae* and *Unaspis citri*;
from the order *Hymenoptera*, for example, *Acromyrmex, Atta spp., Cephus spp., Diprion spp., Diprionidae, Gilpinia polytoma, Hoplocampa spp., Lasius sppp., Monomorium pharaonis, Neodiprion spp, Solenopsis spp.* and *Vespa ssp.*;
from the order *Diptera*, for example, *Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomysa spp., Lucilia spp., Melanagromyza spp., Musca ssp., Oestrus spp., Orseolia spp., Oscinella frit, Pegomyia hyoscyami, Phorbia spp., Rhagoletis pomonella, Sciara spp., Stomoxys spp., Tabanus spp., Tannia spp.* and *Tipula spp.*,
from the order *Siphonaptera*, for example, *Ceratophyllus spp. und Xenopsylla cheopis* and
from the order *Thysanura*, for example, *Lepisma saccharina*.

**Monocotyledonous plant (monocot)** is a flowering plant having embryos with one cotyledon or seed leaf, parallel leaf veins, and flower parts in multiples of three. Examples of monocots include, but are not limited to turfgrass, maize, rice, oat, wheat, barley, sorghum, orchid, iris, lily, onion, and palm.

**Pest** or **target pest** refers to insects, arachnids, crustaceans, fungi, bacteria, viruses, nematodes, flatworms, roundworms, pinworms, hookworms, tapeworms, trypanosomes, schistosomes, botflies, fleas, ticks, mites, and lice and the like that are pervasive in the human environment. A pest may ingest or contact one or more cells, tissues, or products produced by an organism transformed with a double stranded gene suppression agent, as well as a material or surface treated with a double stranded gene suppression agent.

**Nematodes,** or roundworms, are one of the most common phyla of animals, with over 20,000 different described species (over 15,000 are parasitic). They are ubiquitous in freshwater, marine, and terrestrial environments, where they often outnumber other animals in both individual and species counts, and are found in locations as diverse as Antarctica and oceanic trenches. Further, there are a great many parasitic forms, including pathogens in most plants and animals.

Nematode pests of a particular interest include, for example, *A. caninum, A. ceylancium, H. contortus, O. ostertagi, C. elegans, C. briggsae, P. pacificus, S. stercoralis, S. ratti, P. trichosuri, M. arenaria, M. chitwoodi, M. hapla, M. incognita, M. javanica, M. paraensis, G. rostochiensis, G. pallida, H. glycines, H. schattii, P. penetrans, P. vulnus, R. similis, Z. punctata, A. suum, T. canis, B. malayi, D. immitis, O. volvulus, T. vulpis, T. spiralis, X. index. A. duodenale, A. lumbricoides*, as well as species from the following genera: *Aphelenchoides, Nacobbus, Ditylenchus, Longidorus, Trichodorus*, and *Bursaphelenchus*.

**Normal cell** refers to a cell of an untransformed phenotype or exhibiting a morphology of a non-transformed cell of the tissue type being examined.

**Operably linked:** combining two or more molecules in such a fashion that in combination they function properly in a cell. For instance, a promoter is operably linked to a structural gene when the promoter controls transcription of the structural gene.

**Orthologs** are genes that are related by vertical descent from a common ancestor and encode proteins with the same function in different species. Due to their separation following a speciation event, orthologs may diverge, but usually have similarity at the seqence and structure levels. Two genes that are derived from a common ancestor and encode proteins with similar function are refered to as orthologous. Identification of orthologs is critical for reliable predictions of gene function in newly sequenced genomes.

**"Pest control agent", or "gene suppression agent"** refers to a particular RNA molecule comprising a first RNA segment and a second RNA segment, wherein the complementarity between the first and the second RNA segments results in the ability of the two segments to hybridize *in vivo* and *in vitro* to form a double stranded molecule. It may generally be preferable to include a third RNA segment linking and stabilizing the first and second sequences such that a stem can be formed linked together at one end of each of the first and second segments by the third segment to forms a loop, so that the entire structure forms into a stem and loop structure, or even more tightly hybridizing structures may form into a stem-loop knotted structure. Alternatively, a symmetrical hairpin could be formed without a third segment in which there is no designed loop, but for steric reasons a hairpin would create its own loop when the stem is long enough to stabilize itself. The first and the second RNA segments will generally lie within the length of the RNA molecule and be substantially inverted repeats of each other and linked together by the third RNA segment. The first and the second segments correspond invariably and not respectively to a sense and an antisense sequence with respect to the target RNA transcribed from the target gene in the target insect pest that is suppressed by the ingestion of the dsRNA molecule.

The pest control agent can also be a substantially purified (or isolated) nucleic acid molecule and more specifically nucleic acid molecules or nucleic acid fragment molecules thereof from a genomic DNA (gDNA) or cDNA library. -Alternatively, the fragments may comprise smaller oligonucleotides having from about 15 to about 250 nucleotide residues, and more preferably, about 15 to about 30 nucleotide residues.

**Pesticide** refers to any substance or mixture of substances intended for preventing, destroying, repelling, or mitigating any pest. -A pesticide may be a chemical substance or biological agent used against pests including insects, pathogens, weeds, nematodes, and microbes that compete with humans for food, destroy property, spread disease, or are a nuisance.

**Phenotype** is a distinguishing feature or characteristic of an organism, which may be altered by integrating one or more "desired polynucleotides" and/or screenable/selectable markers into the genome of at least one cell of a transformed organism. The "desired polynucleotide(s)" and/or markers may confer a change in the phenotype of a transformed organism, by modifying any one of a number of genetic, molecular, biochemical, physiological, or morphological characteristics or properties of the transformed cell or organism as a whole.

**Plant and plant tissue:** a "plant" is any of various photosynthetic, eukaryotic, multicellular organisms of the kingdom *Plantae* characteristically producing embryos, containing chloroplasts, and having cellulose cell walls. A part of a plant, i.e., a "plant tissue" may be treated according to the methods of the present disclosure to prevent pest infestation on the plant or on the part of the plant. Many suitable plant tissues can be treated according and include, but are not limited to, somatic embryos, pollen, leaves, stems, calli, stolons, microtubers, and shoots. Thus, the treatment of angiosperm and gymnosperm plants such as acacia, alfalfa, apple, apricot, artichoke, ash tree, asparagus, avocado, banana, barley, beans, beet, birch, beech, blackberry, blueberry, broccoli, brussels sprouts, cabbage, canola, cantaloupe, carrot, cassava, cauliflower, cedar, a cereal, celery, chestnut, cherry, chinese cabbage, citrus, clemintine, clover, coffee, corn, cotton, cowpea, cucumber, cypress, eggplant, elm, endive, eucalyptus, fennel, figes, fir, geranium, grape, grapefruit, groundnuts, ground cherry, gum hemlock, hickory, kale, kiwifruit, kohlrabi, larch, lettuce, leek, lemon, lime, locust, pine, maidenhair, maize, mango, maple, melon, millet, mushroom, mustard, nuts, oak, oats, okra, onion, orange, an ornamental plant or flower or tree, papaya, palm, parsley, parsnip, pea, peach, peanut, pear, peat, pepper, persimmon, pigeon pea, pine, pineapple, plantain, plum, pomegranate, potato, pumpkin, radicchio, radish, rapeseed, raspberry, rice, rye, sorghum, , sallow, soybean, spinach, spruce, squash, strawberry, sugarbeet, sugarcane, sunflower, sweet potato, sweet corn, tangerine, tea, tobacco, tomato, trees, triticale, turf grasses, turnips, a vine, walnut, watercress, watermelon, wheat, yams, yew, and zucchini can be envisaged.

According to the present disclosure "plant tissue" also encompasses plant cells. -Plant cells include suspension cultures, callus, embryos, meristematic regions, callus tissue, leaves, roots, shoots, gametophytes, sporophytes, pollen, seeds and microspores.- Plant tissues may be at various stages of maturity and may be grown in liquid or solid culture, or in soil or suitable media in pots, greenhouses or fields. -A plant tissue also refers to any clone of such a plant, seed, progeny, propagule whether generated sexually or asexually, and descendents of any of these, such as cuttings or seed.

**Promoter** is intended to mean a nucleic acid, preferably DNA that binds RNA polymerase and/or other transcription regulatory elements. -As with any promoter, the promoters will facilitate or control the transcription of DNA or RNA to generate an mRNA molecule from a nucleic acid molecule that is operably linked to the promoter. As stated earlier, the RNA generated may code for a protein or polypeptide or may code for an RNA interfering, or antisense molecule.

**Polynucleotide** is a nucleotide sequence, comprising a gene coding sequence or a fragment thereof, a promoter, an intron, an enhancer region, a polyadenylation site, a translation initiation site, 5' or 3' untranslated regions, a reporter gene, a selectable marker or the like. The polynucleotide may comprise single stranded or double stranded DNA or RNA. The polynucleotide may comprise modified bases or a modified backbone. The polynucleotide may be genomic, an RNA transcript (such as an mRNA) or a processed nucleotide sequence (such as a cDNA). The polynucleotide may comprise a sequence in either sense or antisense orientations.

**An isolated polynucleotide** is a polynucleotide sequence that is not in its native state, e.g., the polynucleotide is comprised of a nucleotide sequence not found in nature or the polynucleotide is separated from nucleotide sequences with which it typically is in proximity or is next to nucleotide sequences with which it typically is not in proximity.

**Recombinant nucleotide sequence** refers to a nucleic acid molecule that contains a genetically engineered modification through manipulation via mutagenesis, restriction enzymes, and the like.

**RNA interference (RNAi)** refers to sequence-specific or gene-specific suppression of gene expression (protein synthesis) that is mediated by short interfering RNA **(siRNA).**

**Sequence identity:** as used herein, "sequence identity" or "identity" in the context of two nucleic acid sequences includes reference to the residues in the two sequences which are the same when aligned for maximum correspondence over a specified region.

As used herein, percentage of sequence identity means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity.

"Sequence identity" has an art-recognized meaning and can be calculated using published techniques. -See COMPUTATIONAL MOLECULAR BIOLOGY, Lesk, ed. (Oxford University Press, 1988), BIOCOMPUTING: INFORMATICS AND GENOME PROJECTS, Smith, ed. (Academic Press, 1993), COMPUTER ANALYSIS OF SEQUENCE DATA, PART I, Griffin & Griffin, eds., (Humana Press, 1994), SEQUENCE ANALYSIS IN MOLECULAR BIOLOGY, Von Heinje ed., Academic Press (1987), SEQUENCE ANALYSIS PRIMER, Gribskov & Devereux, eds. (Macmillan Stockton Press, 1991), and Carillo & Lipton, SIAM J. Applied Math. 48: 1073 (1988). -Methods commonly employed to determine identity or similarity between two sequences include but are not limited to those disclosed in GUIDE To HUGE COMPUTERS, Bishop, ed., (Academic Press, 1994) and Carillo & Lipton, *supra*. Methods to determine identity and similarity are codified in computer programs. -Preferred computer program methods to determine identity and similarity between two sequences include but are not limited to the GCG program package (Devereux et al., Nucleic Acids Research 12: 387 (1984)), BLASTP, BLASTN, FASTA (Atschul et al., J. Mol. Biol. 215: 403 (1990)), and FASTDB (Brutlag et al., Comp. App. Biosci. 6: 237 (1990)).

**Short hairpin RNA (shRNA)** are short single-stranded RNAs having a high degree of secondary structure such that a portion of the RNA strand forms a hairpin loop.

**Short interfering RNA (siRNA)** refers to double-stranded RNA molecules from about 10 to about 30 nucleotides long that are named for their ability to specifically interfere with gene protein expression.

**Target sequence** refers to a nucleotide sequence in a pest that is selected for suppression or inhibition by double stranded RNA technology. -A target sequence encodes an essential feature or biological activity within a pest.

**Transcriptional terminators:** -The expression DNA constructs typically have a transcriptional termination region at the opposite end from the transcription initiation regulatory region.- The transcriptional termination region may be selected, for stability of the mRNA to enhance expression and/or for the addition of polyadenylation tails added to the gene transcription product. -Translation of a nascent polypeptide undergoes termination when any of the three chain-termination codons enters the A site on the ribosome. -Translation termination codons are UAA, UAG, and UGA.

**Transformation:-** A process by which a nucleic acid is stably inserted into the genome of an organism. Transformation may occur under natural or artificial conditions using various methods well known in the art. Transformation may rely on any known method for the insertion of nucleic acid sequences into a prokaryotic or eukaryotic host cell, including microorganism-mediated transformation, viral infection, whiskers, electroporation, microinjection, polyethylene glycol-treatment, heat shock, lipofection, and particle bombardment.

**Transgenic organism** comprises at least one cell in which an exogenous nucleic acid has been stably integrated. -A transgenic organism is for instance a bacterial, or eukaryotic, such as a yeast, host cell or host organism. The bacterium can be chosen from the group comprising Gram-negative and Gram-positive bacteria, such as, but not limited to, *Escherichia* spp. (e.g. *E. coli*), Bacillus spp. (e.g. *B. thuringiensis*), *Rhizobium* spp., *Lactobacilllus* spp., *Lactococcus* spp., etc.. The yeast can be chosen from the group comprising *Saccharomyces* spp., etc.

**Variant:-** a "variant," as used herein, is understood to mean a nucleotide sequence that deviates from the standard, or given, nucleotide or amino acid sequence of a particular gene or protein.- The terms, "isoform," "isotype," and "analog" also refer to "variant" forms of a nucleotide sequence. -"Variant" may also refer to a "shuffled gene" such as those described in Maxygen-assigned patents.

It is understood that the present invention is not limited to the particular methodology, protocols, vectors, and reagents, etc., described herein, as these may vary. -It is also to be understood that the terminology used herein is used for the purpose of describing particular embodiments only, and is not intended to limit the scope of the present invention. -It must be noted that as used herein and in the appended claims, the singular forms "a," "an," and "the" include plural reference unless the context clearly dictates otherwise. -Thus, for example, a reference to "a gene" is a reference to one or more genes and includes equivalents thereof known to those skilled in the art and so forth.

### I. Target Pests

The present invention provides methodology and constructs for controlling insect pest infestations by administering, or otherwise exposing, to a pest a target coding sequence that post-transcriptionally represses or inhibits a requisite biological function in the pest. As used herein, the term "pest" refers to insects, arachnids, crustaceans, fungi, bacteria, viruses, nematodes, flatworms, roundworms, pinworms, hookworms, tapeworms, trypanosomes, schistosomes, botflies, fleas, ticks, mites, and lice and the like that are pervasive in the human environment. -A pest may ingest or contact one or more cells, tissues, or products produced by an organism transformed with a double stranded gene suppression agent, as well as a surface or material treated with a double stranded gene suppression agent.

A "pest resistance" trait is a characteristic of a transgenic host that causes the host to be resistant to attack from a pest that typically inflicts damage to the host. Such pest resistance can arise from a natural mutation or more typically from incorporation of recombinant DNA that confers pest resistance. To impart pest resistance to a transgenic host, a recombinant DNA can, for example, be transcribed into a RNA molecule that forms a dsRNA molecule within the tissues or fluids of the recombinant host.- The dsRNA molecule is comprised in part of a segment of RNA that is identical to a corresponding RNA segment encoded from a DNA sequence within a pest that prefers to feed on the recombinant host. Expression of the gene within the target pest is suppressed by the dsRNA, and the suppression of expression of the gene in the target pest results in the host being pest resistant.

Suitable pests include any organism that causes damage to another organism.- The invention contemplates insect, pests in particular.

**Insect** as used herein can be any insect, meaning any organism belonging to the Kingdom Animals, more specific to the Phylum Arthropoda, and to the Class Insecta or the Class Arachnida. The methods of the invention are applicable to all insects and that are susceptible to gene silencing by RNA interference and that are capable of internalising double-stranded RNA from their immediate environment.

The insect may belong to the following orders: Acari, Araneae, Anoplura, Coleoptera, Collembola, Dermaptera, Dictyoptera, Diplura, Diptera, Embioptera, Ephemeroptera, Grylloblatodea, Hemiptera, Homoptera, Hymenoptera, Isoptera, Lepidoptera, Mallophaga, Mecoptera, Neuroptera, Odonata, Orthoptera, Phasmida, Plecoptera, Protura, Psocoptera, Siphonaptera, Siphunculata, Thysanura, Strepsiptera, Thysanoptera, Trichoptera, and Zoraptera.

The insect may be chosen from the group consisting of:
(1) an insect which is a plant pest, such as but not limited to *Nilaparvata* spp. (e.g. *N. lugens (brown planthopper)); Laodelphax* spp. (e.g. *L. striatellus* (small brown planthopper)); *Nephotettix* spp. (e.g. *N. virescens* or *N. cincticeps* (green leafhopper), or *N.nigropictus* (rice leafhopper)); *Sogatella* spp. (e.g. *S. furcifera* (white-backed planthopper)); *Blissus* spp. (e.g. *B. leucopterus leucopterus* (chinch bug)); *Scotinophora* spp. (e.g. *S. vermidulate* (rice blackbug)); *Acrosternum* spp. (e.g. *A. hilare* (green stink bug)); *Parnara* spp. (e.g. *P. guttata* (rice skipper)); *Chilo* spp. (e.g. *C. suppressalis* (rice striped stem borer), *C. auricilius* (gold-fringed stem borer), or *C. polychrysus* (dark-headed stem borer)); *Chilotraea* spp. (e.g. *C. polychrysa* (rice stalk borer)); *Sesamia* spp. (e.g. *S. inferens* (pink rice borer)); *Tryporyza* spp. (e.g. *T. innotata* (white rice borer), or *T. incertulas* (yellow rice borer)); *Cnaphalocrocis* spp. (e.g. *C. medinalis* (rice leafroller)); *Agromyza* spp. (e.g. *A. oryzae* (leafminer), or *A. parvicornis* (corn blot leafminer)); *Diatraea* spp. (e.g. *D. saccharalis* (sugarcane borer), *or D. grandiosella* (southwestern corn borer)); *Narnaga* spp. (e.g. *N. aenescens* (green rice caterpillar)); *Xanthodes* spp. (e.g. *X. transversa* (green caterpillar)); *Spodoptera* spp. (e.g. *S. frugiperda* (fall armyworm), *S. exigua* (beet armyworm), *S. littoralis* (climbing cutworm) or *S. praefica* (western yellowstriped armyworm)); *Mythimna* spp. (e.g. *Mythmna (Pseudaletia) seperata* (armyworm)); *Helicoverpa* spp. (e.g. *H. zea* (corn earworm)); *Colaspis* spp. (e.g. *C. brunnea* (grape colaspis)); *Lissorhoptrus* spp. (e.g. *L. oryzophilus* (rice water weevil)); *Echinocnemus* spp. (e.g. *E. squamos* (rice plant weevil)); *Diclodispa* spp. (e.g. *D. armigera* (rice hispa)); *Oulema* spp. (e.g. *O. oryzae* (leaf beetle); *Sitophilus* spp. (e.g. *S. oryzae* (rice weevil)); *Pachydiplosis* spp. (e.g. *P. oryzae* (rice gall midge)); *Hydrellia* spp. (e.g. *H. griseola* (small rice leafminer), or *H. sasakii* (rice stem maggot)); *Chlorops* spp. (e.g. *C. oryzae* (stem maggot)); *Ostrinia* spp. (e.g. *O. nubilalis* (European corn borer)); *Agrotis* spp. (e.g. *A.ipsilon* (black cutworm)); *Elasmopalpus* spp. (e.g. *E. lignosellus* (lesser cornstalk borer)); *Melanotus* spp. (wireworms); *Cyclocephala* spp. (e.g. *C. borealis* (northern masked chafer), or *C. immaculata* (southern masked chafer)); *Popillia* spp. (e.g. *P. japonica* (Japanese beetle)); *Chaetocnema* spp. (e.g. *C. pulicaria* (corn flea beetle)); *Sphenophorus* spp. (e.g. *S. maidis* (maize billbug)); *Rhopalosiphum* spp. (e.g. *R. maidis* (corn leaf aphid)); *Anuraphis* spp. (e.g. *A. maidiradicis* (corn root aphid)); *Melanoplus* spp. (e.g. *M. femurrubrum* (redlegged grasshopper) *M. differentialis* (differential grasshopper) or *M. sanguinipes* (migratory grasshopper)); *Hylemya* spp. (e.g. *H. platura* (seedcorn maggot)); *Anaphothrips* spp. (e.g. *A. obscrurus* (grass thrips)); *Solenopsis* spp. (e.g. *S. milesta* (thief ant)); *or* spp. (e.g. *T. urticae* (twospotted spider mite), *T. cinnabarinus* (carmine spider mite); *Helicoverpa spp. (e.g. H. zea* (cotton bollworm), or *H. armigera* (American bollworm)); *Pectinophora* spp. (e.g *P. gossypiella* (pink bollworm)); *Earias* spp. (e.g. *E. vittella* (spotted bollworm)); *Heliothis* spp. (e.g. *H. virescens* (tobacco budworm)); *Anthonomus* spp. (e.g. *A. grandis* (boll weevil)); *Pseudatomoscelis* spp. (e.g. *P. seriatus* (cotton fleahopper)); *Trialeurodes* spp. (e.g. *T. abutiloneus* (banded-winged whitefly) *T. vaporariorum* (greenhouse whitefly)); *Bemisia* spp. (e.g. *B. argentifolii* (silverleaf whitefly)); *Aphis* spp. (e.g. *A. gossypii* (cotton aphid), *A. mellifera); Lygus* spp. (e.g. *L. lineolaris* (tarnished plant bug) or *L. hesperus* (western tarnished plant bug)); *Euschistus* spp. (e.g. *E. conspersus* (consperse stink bug)); *Chlorochroa* spp. (e.g. *C*. *sayi* (Say stinkbug)); *Nezara* spp. (e.g. *N. viridula* (southern green stinkbug)); *Trips* spp. (e.g. *T. tabaci* (onion thrips)); *Frankliniella* spp. (e.g. *F. fusca* (tobacco thrips), or *F*. *occidentalis* (western flower thrips)); *Leptinotarsa* spp. (e.g. *L. decemlineata* (Colorado potato beetle), *L. juncta* (false potato beetle), or *L*. *texana* (Texan false potato beetle)); *Lema* spp. (e.g. *L. trilineata* (three-lined potato beetle)); *Epitrix* spp. (e.g. *E. cucumeris* (potato flea beetle), *E. hirtipennis* (flea beetle), or *E. tuberis* (tuber flea beetle)); *Epicauta* spp. (e.g. *E. vittata* (striped blister beetle)); *Empoasca* spp. (e.g. *E. fabae* (potato leafhopper)); *Myzus* spp. (e.g. *M. persicae* (green peach aphid)); *Paratrioza* spp. (e.g. *P. cockerelli* (psyllid)); *Conoderus* spp. (e.g. *C. falli* (southern potato wireworm), or *C*. *vespertinus* (tobacco wireworm)); *Phthorimaea* spp. (e.g. *P. operculella* (potato tuberworm)); *Macrosiphum spp. (e.g. M. euphorbiae* (potato aphid)); *Thyanta spp. (e.g. T. pallidovirens* (redshouldered stinkbug)); *Phthorimaea spp. (e.g. P. operculella* (potato tuberworm)); *Helicoverpa spp. (e.g. H. zea* (tomato fruitworm); *Keiferia spp. (e.g. K. lycopersicella* (tomato pinworm)); *Limonius* spp. (wireworms); *Manduca spp. (e.g. M. sexta* (tobacco hornworm), or *M quinquemaculata* (tomato homworm)); *Liriomyza spp. (e.g. L. sativae, L. trifolli* or *L. huidobrensis* (leafminer)); *Drosophilla* spp. (e.g. *D. melanogaster, D. yakuba, D. pseudoobscura* or *D. simulans*)*; Carabus* spp. (e.g. *C*. *granulatus); Chironomus* spp. (e.g. *C. tentanus); Ctenocephalides* spp. (e.g. *C*. *felis* (cat flea)); *Diaprepes* spp. (e.g. *D. abbreviatus* (root weevil)); *Ips* spp. (e.g. *I. pini* (pine engraver)); *Tribolium* spp. (e.g *T. castaneum* (red floor beetle)); *Glossina* spp. (e.g. *G. morsitans* (tsetse fly)); *Anopheles* spp. (e.g. *A. gambiae* (malaria mosquito)); *Helicoverpa* spp. (e.g. *H. armigera* (African Bollworm)); *Acyrthosiphon* spp. (e.g. *A. pisum* (pea aphid)); *Apis* spp. (e.g. *A. melifera* (honey bee)); *Homalodisca* spp. (e.g. H. *coagulate* (glassy-winged sharpshooter)); Aedes spp. (e.g. Ae. aegypti *(yellow fever mosquito)); Bombyx spp. (e.g. B. mori (silkworm), B. mandarina); Locusta spp. (e.g. L. migratoria* (migratory locust)); *Boophilus* spp. (e.g. *B. microplus* (cattle tick)); *Acanthoscurria* spp. (e.g. *A. gomesiana* (red-haired chololate bird eater)); *Diploptera* spp. (e.g. *D. punctata* (pacific beetle cockroach)); *Heliconius* spp. (e.g. *H. erato* (red passion flower butterfly) or *H. melpomene* (postman butterfly)); *Curculio* spp. (e.g. *C*. *glandium* (acorn weevil)); *Plutella* spp. (e.g. *P. xylostella* (diamontback moth)); *Amblyomma* spp. (e.g. *A. variegatum* (cattle tick)); *Anteraea* spp. (e.g. *A. yamamai* (silkmoth)); *Belgica* spp. (e.g. *B. antartica), Bemisa* spp. (e.g. *B. tabaci), Bicyclus* spp., *Biphillus* spp., *Callosobruchus* spp., *Choristoneura* spp., *Cicindela* spp., *Culex spp.,Culicoides* spp., *Diaphorina* spp., *Diaprepes* spp., *Euclidia* spp., *Glossina* spp., *Gryllus* spp., *Hydropsyche* spp., *Julodis* spp., *Lonomia* spp., *Lutzomyia* spp., *Lysiphebus* spp, *Meladema* spp, *Mycetophagus* spp., *Nasonia* spp., *Oncometopia* spp., *Papilio* spp., *Pediculus* spp., *Plodia* spp., *Rhynchosciara* spp., *Sphaerius* spp., *Toxopter a* spp., *Trichoplusa* spp., and *Armigeres* spp. (e.g. *A. subalbatus);*
(2) an insect capable of infesting or injuring humans and/or animals such as, but not limited to those with piercing-sucking mouthparts, as found in Hemiptera and some Hymenoptera and Diptera such as mosquitos, bees, wasps, lice, fleas and ants, as well as members of the Arachnidae such as ticks and mitesorder, class or familiy of Acarina (ticks and mites) e.g. representatives of the families *Argasidae, Dermanyssidae, Ixodidae, Psoroptidae* or *Sarcoptidae* and representatives of the species *Amblyomma* spp., *Anocentor* spp., *Argas* spp., *Boophilus* spp., *Cheyletiel*/*a* spp., *Chorioptes* spp., *Demodex* spp., *Dermacentor* spp., *Dennanyssus* spp., *Haemophysalis* spp., *Hyalomma* spp., *Ixodes* spp., *Lynxacarus spp., Mesostigmata* spp., *Notoedres* spp., *Ornithodoros* spp., *Ornithonyssus* spp., *Otobius* spp., *otodectes* spp., *Pneumonyssus* spp., *Psoroptes* spp., *Rhipicephalus* spp., *Sarcoptes* spp., or *Trombicula* spp. ; Anoplura (sucking and biting lice) e.g. representatives of the species *Bovicola* spp., *Haematopinus* spp., *Linognathus* spp., *Menopon* spp., *Pediculus* spp., *Pemphigus* spp., *Phylloxera* spp., or *Solenopotes* spp. ; Diptera (flies) e.g. representatives of the species *Aedes* spp., *Anopheles* spp., *Calliphora* spp., *Chrysomyia* spp., *Chrysops* spp., *Cochliomyia* spp., *Culex* spp., *Culicoides* spp., *Cuterebra* spp., *Dermatobia* spp., *Gastroplailus* spp., *Glossina* spp., *Haematobia spp. , Haematopota* spp., *Hippobosca* spp., *Hypoderma* spp., *Lucilia* spp., *Lyperosia* spp., *Melophagus* spp., *Oestrus* spp., *Phaenicia* spp., *Phlebotomus* spp., *Phonnia* spp., *Sarcophaga* spp., *Simulium* spp., *Stomoxys* spp., *Tabanus* spp., *Tannia* spp. or *Tipula* spp.; Mallophaga (biting lice) e.g. representatives of the species *Damalina* spp., *Felicola* spp., *Heterodoxus* spp. or *Trichodectes* spp.; or Siphonaptera (wingless insects) e.g. representatives of the species *Ceratophyllus* spp., spp., *Pulex* spp., or *Xenopsylla* spp; Cimicidae (true bugs) e.g. representatives of the species *Cimex* spp., *Tritominae* spp., *Rhodinius* spp., or *Triatoma* spp.
   and
(3) an insect that causes unwanted damage to substrates or materials, such as insects that attack foodstuffs, seeds, wood, paint, plastic, clothing etc.

The methods disclosed herein are applicable to all nematodes and fungi that are susceptible to gene silencing by RNA interference and that are capable of internalising double-stranded RNA from their immediate environment.

In one embodiment, the nematode may belong to the family of the Heteroderidae, encompassing the genera Heterodera and Globodera.

Fungal pests of particular interest include but are not limited to the following. In one embodiment, the fungus may be a mold, or more particularly a filamentous fungus. In other embodiments of the invention, the fungus may be a yeast:

### II. Identification of Target Sequences

The present invention relates to a method for identifying and obtaining a nucleic acid comprising a nucleotide sequence for producing a dsRNA or siRNA. For example, such a method comprises: (a) probing a cDNA or genomic. DNA library with a hybridization probe comprising all or a portion of a nucleotide sequence or a homolog thereof from a targeted pest; (b) identifying a DNA clone that hybridizes with the hybridization probe; (c) isolating the DNA clone identified in step (b); and (d) sequencing the cDNA or genomic DNA fragment that comprises the clone isolated in step (c) wherein the sequenced nucleic acid molecule transcribes all or a substantial portion of the RNA nucleotide acid sequence or a homolog thereof.

Additionally, the present invention relates to a method for obtaining a nucleic acid fragment comprising a nucleotide sequence for producing a substantial portion of a dsRNA or siRNA comprising: (a) synthesizing first and a second oligonucleotide primers corresponding to a portion of one of the nucleotide sequences from a targeted pest; and (b) amplifying a cDNA or genomic DNA template in a cloning vector using the first and second oligonucleotide primers of step (a) wherein the amplified nucleic acid molecule transcribes a substantial portion of a dsRNA or siRNA of the present invention.

In practicing the present disclosure a target gene may be derived from any pest that causes damage to another organism. Several criteria may be employed in the selection of preferred target genes. The gene is one whose protein product has a rapid turnover rate, so that dsRNA inhibition will result in a rapid decrease in protein levels. In certain embodiments it is advantageous to select a gene for which a small drop in expression level results in deleterious effects for the recipient pest. If it is desired to target a broad range of insect species, for example, a gene is selected that is highly conserved across these species. Conversely, for the purpose of conferring specificity, a gene is selected that contains regions that are poorly conserved between individual insect species, or between insects and other organisms. In certain embodiments it may be desirable to select a gene that has no known homologs in other organisms.

As used herein, the term "derived from" refers to a specified nucleotide sequence that may be obtained from a particular specified source or species, albeit not necessarily directly from that specified source or species.

In one embodiment, a gene is selected that is expressed in the insect gut. Targeting genes expressed in the gut avoids the requirement for the dsRNA to spread within the insect. Target genes may include, for example, those that share substantial homologies to the nucleotide sequences of known gut-expressed genes that encode protein components of the plasma membrane proton V-ATPase (Dow *et al.,* 1997,; Dow, 1999). This protein complex is the sole energizer of epithelial ion transport and is responsible for alkalinization of the midgut lumen. The V-ATPase is also expressed in the Malpighian tubule, an outgrowth of the insect hindgut that functions in fluid balance and detoxification of foreign compounds in a manner analogous to a kidney organ of a mammal.

In another embodiment, a gene is selected that is essentially involved in the growth, development, and reproduction of an insect. Exemplary genes include but are not limited to the structural subunits of ribosomal proteins and a beta-coatamer gene, CHD3 gene. Ribosomal proteins such as S4 (RpS4) and S9(RpS9) are structural constituents of the ribosome involved in protein biosynthesis and which are components of the cytosolic small ribosomal subunit, the ribosomal proteins such as L9 and L19 are structural constituent of ribosome involved in protein biosynthesis which is localised to the ribosome. The beta-coatamer gene in *C*. *elegans* encodes a protein which is a subunit of a multimeric complex that forms a membrane vesicle coat Similar sequences have been found in diverse organisms such as *Arabidopsis tha*/*iana, Drosophila melanogaster,* and *Saccharomyces cerevisiae.* Related sequences are found in diverse organisms such as *Leplinotarsa decemlineata, Phaedon cochleariae, Epilachna varivetis, Anthonomus grandis, Tribolium castaneum, Myzus persicae, Nilaparvata lugens, Chilo suppresralis, Plutella xylostella* and *Acheta domesticus.* Other target genes may include, for example, those that play important roles in viability, growth, development, reproduction, and infectivity. These target genes include, for example, house keeping genes, transcription factors, and insect specific genes or lethal knockout mutations in *Caenorhabditis* or *Drosophila.* The target genes may also be those that are from other organisms, *e.g.,* from a nematode (*e.g., Meloidogyne* spp. or *Heterodera* spp.), other insects or arachnidae (e.g. *Leptinotarsa* spp., *Phaedon* spp., *Epilachna* spp.; *Anthonomus* spp., *Tribolium* spp., *Myzus* spp., *Nilaparvata* spp., *Chilo* spp., *Plutella* spp., or *Acheta* spp.,. Additionally, the nucleotide sequences for use as a target sequence may also be derived from viral, bacterial, fungal, insect or fungal genes whose functions have been established from literature and the nucleotide sequences of which share substantial similarity with the target genes in the genome of an insect.

For many of the insects that are potential targets for control there may be limited information regarding the sequences of most genes or the phenotype resulting from mutation of particular genes. Therefore, genes may be selected based on available information available concerning corresponding genes in a model organism, such as *Caenorhabditis* or *Drosophila,* or in some other insect species. Genes may also be selected based on available sequence information for other species, such as nematode or fungal species, in which the genes have been characterized. In some cases it will be possible to obtain the sequence of a corresponding gene from a target insect by searching databases, such as GenBank, using either the name of the gene or the gene sequence. Once the sequence is obtained, PCR may be used to amplify an appropriately selected segment of the gene in the insect

In order to obtain a DNA segment from the corresponding gene in an insect species, for example, PCR primers may be designed based on the sequence as found in *C*. *elegans* or *Drosophila,* or an insect from which the gene has already been cloned. The primers are designed to amplify a DNA segment of sufficient length. Amplification conditions are selected so that amplification will occur even if the primers do not exactly match the target sequence. Alternately, the gene, or a portion thereof, may be cloned from a genomic DNA or cDNA library prepared from the insect pest species, using a known insect gene as a probe. Techniques for performing PCR and cloning from libraries are known. Further details of the process by which DNA segments from target insect pest species may be isolated based on the sequence of genes previously cloned from an insect species are provided in the Examples. One of ordinary skill in the art will recognize that a variety of techniques may be used to isolate gene segments from insect pest species that correspond to genes previously isolated from other species.

### III. Methods for inhibiting or suppressing a target gene

The present invention provides methods for inhibiting gene expression of one or multiple target genes in a target pest using stabilized dsRNA methods. The invention is particularly useful for modulating eukaryotic gene expression, in particular modulating the expression of genes present in pests that exhibit a digestive system pH level that is from about 4.5 to about 9.5, more preferably from about 5.0 to about 8.0, and even more preferably from about 6.5 to about 7.5. For pests with a digestive system that exhibits pH levels outside of these ranges, delivery methods may be desired for use that do not require ingestion of dsRNA molecules.

The methods encompass the simultaneous or sequential provision of two or more different double-stranded RNAs or RNA constructs to the same insect, so as to achieve down-regulation or inhibition of multiple target genes or to achieve a more potent inhibition of a single target gene.

Alternatively, multiple targets are hit by the provision of one double-stranded RNA that hits multiple target sequences, and a single target is more efficiently inhibited by the presence of more than one copy of the double stranded RNA fragment corresponding to the target gene. Thus, in one embodiment the double-stranded RNA construct comprises multiple dsRNA regions, at least one strand of each dsRNA region comprising a nucleotide sequence that is complementary to at least part of a target nucleotide sequence of an insect target gene. The dsRNA regions in the RNA construct may be complementary to the same or to different target genes and/or the dsRNA regions may be complementary to targets from the same or from different insect species. Use of such dsRNA constructs in a plant host cell, thus establishes a more potent resistance to a single or to multiple insect species in the plant. In one embodiment, the double stranded RNA region comprises multiple copies of the nucleotide sequence that is complementary to the target gene. Alternatively, the dsRNA hits more than one target sequence of the same target gene. The invention thus relates to isolated double stranded RNA constructs comprising at least two copies of said nucleotide sequence complementary to at least part of a nucleotide sequence of an insect target. DsRNA that hits more than one of the above-mentioned targets, or a combination of different dsRNA against different of the above mentioned targets can be developed. Suitable dsRNA nucleotides and dsRNA constructs are described in WO2006/046148 by applicant, which is incorporated herein in its entirity.

The terms "hit", "hits", and "hitting" are alternative wordings to indicate that at least one of the strands of the dsRNA is complementary to, and as such may bind to, the target gene or nucleotide sequence.

The modulatory effect of dsRNA is applicable to a variety of genes expressed in the pests including, for example, endogenous genes responsible for cellular metabolism or cellular transformation, including house keeping genes, transcription factors, and other genes which encode polypeptides involved in cellular metabolism.

As used herein, the phrase "inhibition of gene expression" or "inhibiting expression of a target gene in the cell of an pest" refers to the absence (or observable decrease) in the level of protein and/or mRNA product from the target gene. Specificity refers to the ability to inhibit the target gene without manifest effects on other genes of the cell and without any effects on any gene within the cell that is producing the dsRNA molecule. The inhibition of gene expression of the target gene in the pest may result in novel phenotypic traits in the pest.

"Gene suppression" refers to any of the well-known methods for reducing the levels of gene transcription to mRNA and/or subsequent translation of the mRNA. Gene suppression is also intended to mean the reduction of protein expression from a gene or a coding sequence including posttranscriptional gene suppression and transcriptional suppression. Posttranscriptional gene suppression is mediated by the homology between of all or a part of a mRNA transcribed from a gene or coding sequence targeted for suppression and the corresponding double stranded RNA used for suppression, and refers to the substantial and measurable reduction of the amount of available mRNA available in the cell for binding by ribosomes. The transcribed RNA can be in the sense orientation to effect what is called co-suppression, in the anti-sense orientation to effect what is called anti-sense suppression, or in both orientations producing a dsRNA to effect what is called RNA interference (RNAi).

Transcriptional suppression is mediated by the presence in the cell of a dsRNA gene suppression agent exhibiting substantial sequence identity to a promoter DNA sequence or the complement thereof to effect what is referred to as promoter trans suppression. -Gene suppression may be effective against a native host gene associated with a trait, e.g., to provide hosts with reduced levels of a protein encoded by the native gene or with enhanced or reduced levels of an affected metabolite. -Gene suppression can also be effective against target genes in pests that may ingest or contact material containing gene suppression agents, specifically designed to inhibit or suppress the expression of one or more homologous or complementary sequences in the cells of the pest.

A beneficial method of post transcriptional gene suppression in hosts employs both sense-oriented and anti-sense-oriented, transcribed RNA which is stabilized, e.g., as a hairpin and stem and loop structure. -A preferred DNA construct for effecting post transcriptional gene suppression is one in which a first segment encodes an RNA exhibiting an anti-sense orientation exhibiting substantial identity to a segment of a gene targeted for suppression, which is linked to a second segment in sense orientation encoding an RNA exhibiting substantial complementarity to the first segment. Such a construct forms a stem and loop structure by hybridization of the first segment with the second segment and a loop structure from the nucleotide sequences linking the two segments -(see WO94/01550, WO98/05770, US 2002/0048814, and US 2003/0018993).

According to the present disclosure, there is envisaged a nucleotide sequence, for which *in vitro* expression results in transcription of a stabilized RNA sequence that is substantially homologous to an RNA molecule of a targeted gene in a pest that comprises an RNA sequence encoded by a nucleotide sequence within the genome of the pest. -Thus, after the pest uptakes the stabilized RNA sequence, or is otherwise exposed to the dsRNA, a down-regulation of the nucleotide sequence corresponding to the target gene in the cells of a target pest is affected.

Inhibition of a target gene using the stabilized dsRNA technology is sequence-specific in that nucleotide sequences corresponding to the duplex region of the RNA are targeted for genetic inhibition. -RNA containing a nucleotide sequences identical to a portion of the target gene is preferred for inhibition. -RNA sequences with insertions, deletions, and single point mutations relative to the target sequence have also been found to be effective for inhibition. In performance of the present invention, it is preferred that the inhibitory dsRNA and the portion of the target gene share at least from about 85% sequence identity, or from about 90% sequence identity, or from about 95% sequence identity, or from about 99% sequence identity, or even about 100% sequence identity. Alternatively, the duplex region of the RNA may be defined functionally as a nucleotide sequence that is capable of hybridizing with a portion of the target gene transcript. -A less than full length sequence exhibiting a greater homology compensates for a longer less homologous sequence. -The length of the identical nucleotide sequences may be at least about 25, 50, 100, 200, 300, 400, 500 or at least about 1000 bases. Normally, a sequence of greater than 20-100 nucleotides should be used, though a sequence of greater than about 200-300 nucleotides would be preferred, and a sequence of greater than about 500-1000 nucleotides would be especially preferred depending on the size of the target gene. -The is has the advantage of being able to tolerate sequence variations that might be expected due to genetic mutation, strain polymorphism, or evolutionary divergence. -The introduced nucleic acid molecule may not need to be absolute homology, may not need to be full length, relative to either the primary transcription product or fully processed mRNA of the target gene. -Therefore, those skilled in the art need to realize that, as disclosed herein, 100% sequence identity between the RNA and the target gene is not required to practice the methods disclosed herein.

### IV. Methods for preparing dsRNA

dsRNA molecules may be synthesized either *in vivo* or *in vitro.* -The dsRNA may be formed by a single self-complementary RNA strand or from two complementary RNA strands. -Endogenous RNA polymerase of the cell may mediate transcription *in vivo,* or cloned RNA polymerase can be used for transcription *in vivo* or *in vitro.* Inhibition may be targeted by specific transcription in an organ, tissue, or cell type; stimulation of an environmental condition (e.g., infection, stress, temperature, chemical inducers); and/or engineering transcription at a developmental stage or age. The RNA strands may or may not be polyadenylated; the RNA strands may or may not be capable of being translated into a polypeptide by a cell's translational apparatus.

A RNA, dsRNA, siRNA, or miRNA may be produced chemically or enzymatically by one skilled in the art through manual or automated reactions or *in vivo* in another organism. RNA may also be produced by partial or total organic synthesis; any modified ribonucleotide can be introduced by *in vitro* enzymatic or organic synthesis. -The RNA may be synthesized by a cellular RNA polymerase or a bacteriophage RNA polymerase (e.g., T3, T7, SP6). The use and production of an expression construct are known in the art (see, for example, WO 97/32016; U.S. Pat. No's. 5,593, 874, 5,698,425, 5,712,135, 5,789,214, and 5,804,693). -If synthesized chemically or by *in vitro* enzymatic synthesis, the RNA may be purified prior to introduction into the cell. -For example, RNA can be purified from a mixture by extraction with a solvent or resin, precipitation, electrophoresis, chromatography, or a combination thereof. -Alternatively, the RNA may be used with no or a minimum of purification to avoid losses due to sample processing. The RNA may be dried for storage or dissolved in an aqueous solution. -The solution may contain buffers or salts to promote annealing, and/or stabilization of the duplex strands.

### V. Polynucleotide Sequences

Envisaged are nucleotide sequences, the expression of which results in an RNA sequence which is substantially homologous to an RNA molecule of a targeted gene in a pest that comprises an RNA sequence encoded by a nucleotide sequence within the genome of the pest. -Thus, after ingestion of the dsRNA sequence down-regulation of the nucleotide sequence of the target gene in the cells of the pest may be obtained resulting in a deleterious effect on the maintenance, viability, proliferation, reproduction, and infestation of the pest.

Each "nucleotide sequence" set forth herein is presented as a sequence of deoxyribonucleotides (abbreviated A, G, C and T). However, by "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C and U) where each thymidine deoxynucleotide (T) in the specified deoxynucleotide sequence in is replaced by the ribonucleotide uridine (U).

As used herein, "nucleic acid" refers to a single or double-stranded polymer of deoxyribonucleotide or ribonucleotide bases read from the 5' to the 3' end.- A nucleic acid may also optionally contain non-naturally occurring or altered nucleotide bases that permit correct read through by a polymerase and do not reduce expression of a polypeptide encoded by that nucleic acid. "Nucleotide sequence" or "nucleic acid sequence" refers to both the sense and antisense strands of a nucleic acid as either individual single strands or in the duplex.

The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA) and the term "deoxyribonucleic acid" (DNA) is inclusive of cDNA and genomic DNA and DNA-RNA hybrids.

The words "nucleic acid segment", "nucleotide sequence segment", or more generally "segment" will be understood by those in the art as a functional term that includes both genomic sequences, ribosomal RNA sequences, transfer RNA sequences, messenger RNA sequences, operon sequences and smaller engineered nucleotide sequences that express or may be adapted to express, proteins, polypeptides or peptides.

Accordingly, the present invention relates to an isolated nucleic acid molecule comprising a polynucleotide having a sequence selected from the group consisting of any of the polynucleotide sequences of SEQ ID NOs: 1, 25, 49-55, 163, or 166 . The invention also relates to functional fragments of the polynucleotide sequences of SEQ ID NOs: 1, 25, 49-55, 163, or 166 . The invention further relates to complementary nucleic acids, or fragments thereof, to any of the polynucleotide sequences of SEQ ID NOs: 1, 25, 49-55, 163, or 166 , as well as a nucleic acid, comprising at least 21 contiguous bases, which hybridizes to any of the polynucleotide sequences of SEQ ID NOs: 1, 25, 49-55, 163, or 166

The present invention also relates to orthologous sequences, and complements and fragments thereof, of the polynucleotide sequences of SEQ ID NOs: 1, 25, 49-55, 163, or Accordingly, the invention relates to target genes which are insect orthologs of a gene comprising a nucleotide sequence as represented in any of SEQ ID NOs: 1, 25. 49-55. 163. or 166 By way of example, insect orthologues may comprise a nucleotide sequence as represented in any of SEQ ID NOs: 49-55. 275-309. 621-657. 813-828. 908-957. 1161-1192. 1730-1770. 2120-2174. 2384-2417 or a fragment thereof of at least 21, 22, 23, 24, 25, 26 or 27 nucleotides . A non-limiting list of insect or arachnida orthologs genes or sequences comprising at least a fragment of 15, preferably at least 17 bp of one of the sequences of the invention is given in Tables 4.

The invention also relates to target genes which are nematode orthologs of a gene comprising a nucleotide sequence as represented in any of SEQ ID NOs: 1, 25, 49-55, 163, or By way of example, nematode orthologs may comprise a nucleotide sequence as represented in any of SEQ ID NOs: 124, 435, 739-741, 1011-1013. 1438-1441. 1988. 2291-2295 or a fragment of at nucleotides thereof According to another aspect of the methods described herein for controlling nematode growth in an organism, or for preventing nematode infestation of an organism susceptible to nemataode infection, comprising contacting nematode cells with a double-stranded RNA, wherein the double-stranded RNA comprises annealed complementary strands, one of which has a nucleotide sequence which is complementary to at least part of the nucleotide sequence of a target gene comprising a fragment of at least nucleotides of any of the sequences as represented in SEQ ID NOs: 1, 25, 49-55, 163, or 166 , whereby the double-stranded RNA is taken up by the fungus and thereby controls growth or prevents infestation. The disclosure also relates to nematode-resistant transgenic plants comprising a fragment of at least 21 nucleotides of any of the sequences as represented in SEQ ID NOs: 1, 25. 49-55, 163, or 166 A non-limiting list of nematode orthologs genes or sequences comprising at least a fragment of 15, preferably at least 17 bp of one of the sequences is given in Tables 5.

According to another embodiment, the disclosure relates to target genes which are fungal orthologs of a gene comprising a nucleotide sequence as represented in any of SEQ ID NO:s 1. 25. 49-55. 163. or 166 By way of example, fungal orthologs may comprise a nucleotide sequence as represented in any of SEQ ID NOs: 136. 447. 752-755. 855. 1474-1479, 2299-2310, 244 or a fragment of at least 21,22, 23, 24, 25, 26 or 27 nucleotides thereof-. According to another aspect, the disclosure thus relates to any of the methods described herein for controlling fungal growth on a cell or an organism, or for preventing fungal infestation of a cell or an organism susceptible to fungal infection, comprising contacting fungal cells with a double-stranded RNA, wherein the double-stranded RNA comprises annealed complementary strands, one of which has a nucleotide sequence which is complementary to at least part of the nucleotide sequence of a target gene comprising a fragment of at least 17, 18, 19, 20 or 21 nucleotides of any of the sequences as represented in SEQ ID NOs: 1, 25, 49-55, 163. or 166 whereby the double-stranded RNA is taken up by the fungus and thereby controls growth or prevents infestation. The disclosure also relates to fungal-resistant transgenic plants comprising a fragment of at least 21 of any of the sequences as represented in SEQ ID NOs: 1, 25, 49-55, 163, or 166 A non-limiting list of fungal orthologs genes or sequences comprising at least a fragment of 15, preferably at least 17 bp of one of said sequences is given in Tables 6.

In a further embodiment, a dsRNA molecule of the invention comprises any of SEQ ID NOs: 1, 25, 49-55, 163, or 166 though the sequences set forth in SEQ ID NOs: 1, 25, 49-55, 163, or 166 are not limiting. -A dsRNA molecule of the invention can comprise any contiguous target gene from a pest species (e.g., about 25 or more, or about 21, 22, 23, 24, or 25 or more contiguous nucleotides).

By "isolated" nucleic acid molecule(s) is intended a nucleic acid molecule, DNA or RNA, which has been removed from its native environment. For example, recombinant DNA molecules contained in a DNA construct are considered isolated for the purposes of the present disclosure. Further examples of isolated DNA molecules include recombinant DNA molecules maintained in heterologous host cells or purified (partially or substantially) DNA molecules in solution. -Isolated RNA molecules include in vitro RNA transcripts of the DNA molecules of the present disclosure. -Isolated nucleic acid molecules, further include such molecules produced synthetically.

Nucleic acid molecules may be in the form of RNA, such as mRNA, or in the form of DNA, including, for instance, cDNA and genomic DNA obtained by cloning or produced synthetically. -The DNA or RNA may be double-stranded or single-stranded.- Single-stranded DNA may be the coding strand, also known as the sense strand, or it may be the non-coding strand, also referred to as the anti-sense strand.

### VI. Sequence Analysis

Unless otherwise indicated, all nucleotide sequences determined by sequencing a DNA molecule herein were determined using an automated DNA sequencer (such as the Model 373 from Applied Biosystems, Inc.). -Therefore, as is known in the art for any DNA sequence determined by this automated approach, any nucleotide sequence determined herein may contain some errors. -Nucleotide sequences determined by automation are typically at least about 95% identical, more typically at least about 96% to at least about 99.9% identical to the actual nucleotide sequence of the sequenced DNA molecule. -The actual sequence can be more precisely determined by other approaches including manual DNA sequencing methods well known in the art. -As is also known in the art, a single insertion or deletion in a determined nucleotide sequence compared to the actual sequence will cause a frame shift in translation of the nucleotide sequence such that the predicted amino acid sequence encoded by a determined nucleotide sequence may be completely different from the amino acid sequence actually encoded by the sequenced DNA molecule, beginning at the point of such an insertion or deletion.

In another aspect, the invention relates to an isolated nucleic acid molecule comprising a polynucleotide which hybridizes under stringent hybridization conditions to a portion of the polynucleotide in a nucleic acid molecule described above. -By a polynucleotide which hybridizes to a "portion" of a polynucleotide is intended a polynucleotide (either DNA or RNA) hybridizing to at least about 15 nucleotides, and more preferably at least about 20 nucleotides, and still more preferably at least about 30 nucleotides, and even more preferably more than 30 nucleotides of the reference polynucleotide. -These fragments that hybridize to the reference fragments are useful as diagnostic probes and primers. Two sequences hybridize when they form a double-stranded complex in a hybridization solution of 6X SSC, 0.5% SDS, 5X Denhardt's solution and 100µg of non-specific carrier DNA. See Ausubel *et al.,* section 2.9, supplement 27 (1994). -Sequences may hybridize at "moderate stringency," which is defined as a temperature of 60 °C in a hybridization solution of 6X SSC, 0.5% SDS, 5X Denhardt's solution and 100µg of non-specific carrier DNA. -For "high stringency" hybridization, the temperature is increased to 68 °C. Following the moderate stringency hybridization reaction, the nucleotides are washed in a solution of 2X SSC plus 0.05% SDS for five times at room temperature, with subsequent washes with 0.1X SSC plus 0.1% SDS at 60 °C for 1h. -For high stringency, the wash temperature is increased to 68 °C.-Hybridized nucleotides are those that are detected using 1 ng of a radiolabeled probe having a specific radioactivity of 10,000 cpm/ng, where the hybridized nucleotides are clearly visible following exposure to X-ray film at -70 °C for no more than 72 hours.

The present application is directed to such nucleic acid molecules which are at least 85%, 90%, 95%, 96%, 97%, 98%, 99% or 100% identical to a nucleic acid sequence described in any of SEQ ID NOs: -Preferred, however, are nucleic acid molecules which are at least 95%, 96%, 97%, 98%, 99% or 100% identical to the nucleic acid sequence shown in of SEQ ID NOs: 1 -Differences between two nucleic acid sequences may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 95%, 96%, 97%, 98% or 99% identical to a reference nucleotide sequence refers to a comparison made between two molecules using standard algorithms well known in the art and can be determined conventionally using publicly available computer programs such as the BLASTN algorithm. See Altschul et al., Nucleic Acids Res. 25:3389-3402 (1997).

A nucleic acid can comprises an antisense strand having about 15 to about 30 (e.g., about 15, 16, 17, 18, 19, 20, 21, 22, 23, 24, 25, 26, 27, 28, 29, or 30) nucleotides, wherein the antisense strand is complementary to a RNA sequence or a portion thereof encoding a protein that controls cell cycle or homologous recombination, and wherein said siRNA further comprises a sense strand having about 15 to about 30 (e.g., about 15, 16, 17, 18, 19, 20, 21, 22, 23, 24, 25, 26, 27, 28, 29, or 30) nucleotides, and wherein said sense strand and said antisense strand are distinct nucleotide sequences where at least about 15 nucleotides in each strand are complementary to the other strand.

In one embodiment, the present invention provides double-stranded nucleic acid molecules that mediate RNA interference gene silencing. In another embodiment, the siRNA molecules of the invention consist of duplex nucleic acid molecules containing about 15 to about 30 base pairs between oligonucleotides comprising about nucleotides. In yet another embodiment, siNA molecules comprise duplex nucleic acid molecules with overhanging ends of about 1 to about 32 (e.g., about 1, 2, or 3) nucleotides, for example, about 21-nucleotide duplexes with about 19 base pairs and 3'-terminal mononucleotide, dinucleotide, or trinucleotide overhangs. In yet another embodiment, siNA molecules of the invention comprise duplex nucleic acid molecules with blunt ends, where both ends are blunt, or alternatively, where one of the ends is blunt.

An siNA molecule of the may comprise modified nucleotides while maintaining the ability to mediate RNAi. The modified nucleotides can be used to improve in vitro or in vivo characteristics such as stability, activity, and/or bioavailability. For example, a siNA molecule can comprise modified nucleotides as a percentage of the total number of nucleotides present in the siNA molecule. As such, a siNA molecule can generally comprise about 5% to about 100% modified nucleotides (e.g., about 5%, 10%, 15%, 20%, 25%, 30%, 35%, 40%, 45%, 50%, 55%, 60%, 65%, 70%, 75%, 80%, 85%, 90%, 95% or 100% modified nucleotides). The actual percentage of modified nucleotides present in a given siNA molecule will depend on the total number of nucleotides present in the siNA. If the siNA molecule is single stranded, the percent modification can be based upon the total number of nucleotides present in the single stranded siNA molecules. Likewise, if the siNA molecule is double stranded, the percent modification can be based upon the total number of nucleotides present in the sense strand, antisense strand, or both the sense and antisense strands.

### VII. Nucleic Acid Constructs

A recombinant nucleic acid vector may, for example, be a linear or a closed circular plasmid. The vector system may be a single vector or plasmid or two or more vectors or plasmids that together contain the total nucleic acid to be introduced into the genome of the bacterial host. In addition, a bacterial vector may be an expression vector. Nucleic acid molecules as set forth in SEQ ID NOs: 1, 25, 49-55, 163, or 166 or fragments thereof can, for example, be suitably inserted into a vector under the control of a suitable promoter that functions in one or more microbial hosts to drive expression of a linked coding sequence or other DNA sequence. Many vectors are available for this purpose, and selection of the appropriate vector will depend mainly on the size of the nucleic acid to be inserted into the vector and the particular host cell to be transformed with the vector.- Each vector contains various components depending on its function (amplification of DNA or expression of DNA) and the particular host cell with which it is compatible. -The vector components for bacterial transformation generally include, but are not limited to, one or more of the following: a signal sequence, an origin of replication, one or more selectable marker genes, and an inducible promoter allowing the expression of exogenous DNA.

### Promoters

"Operably linked", as used in reference to a regulatory sequence and a structural nucleotide sequence, means that the regulatory sequence causes regulated expression of the linked structural nucleotide sequence. "Regulatory sequences" or "control elements" refer to nucleotide sequences located upstream (5' noncoding sequences), within, or downstream (3' non-translated sequences) of a structural nucleotide sequence, and which influence the timing and level or amount of transcription, RNA processing or stability, or translation of the associated structural nucleotide sequence. -Regulatory sequences may include promoters, translation leader sequences, introns, enhancers, stem-loop structures, repressor binding sequences, and polyadenylation recognition sequences and the like.

An expression vector for producing a mRNA can also contain an inducible promoter that is recognized by the host bacterial organism and is operably linked to the nucleic acid encoding, for example, the nucleic acid molecule coding the *D. v. virgifera* mRNA or fragment thereof of interest. Inducible promoters suitable for use with bacterial hosts include β-lactamase promoter, *E. coli* λ phage PL and PR promoters, and *E. coli* galactose promoter, arabinose promoter, alkaline phosphatase promoter, tryptophan (trp) promoter, and the lactose operon promoter and variations thereof and hybrid promoters such as the tac promoter. However, other known bacterial inducible promoters are suitable.

In certain embodiments, the genes can be derived from different insects in order to broaden the range of insects against which the agent is effective. -When multiple genes are targeted for suppression or a combination of expression and suppression, a polycistronic DNA element can be fabricated as illustrated and disclosed in Fillatti, Application Publication No. US 2004-0029283.

### Selectable marker genes

A recombinant DNA vector or construct will typically comprise a selectable marker that confers a selectable phenotype on transformed cells. Selectable markers may also be used to select for cells that contain the exogenous nucleic acids encoding polypeptides or proteins. -The marker may encode biocide resistance, such as antibiotic resistance (*e.g*., kanamycin, G418 bleomycin, hygromycin, *etc*.). -Examples of selectable markers include, but are not limited to, a neo gene which codes for kanamycin resistance and can be selected for using kanamycin, G418, *etc*., a bar gene which codes for bialaphos resistance; a nitrilase gene which confers resistance to bromoxynil, and a methotrexate resistant DHFR gene. -Examples of such selectable markers are illustrated in U.S. Patents 5,550,318; 5,633,435; 5,780,708 and 6,118,047.

A recombinant vector or construct may also include a screenable marker. Screenable markers may be used to monitor expression. -Exemplary screenable markers include a β-glucuronidase or uidA gene (GUS) which encodes an enzyme for which various chromogenic substrates are known (Jefferson, 1987; Jefferson *et al.,* 1987); a β-lactamase gene (Sutcliffe *et al.,* 1978), a gene which encodes an enzyme for which various chromogenic substrates are known (*e.g*., PADAC, a chromogenic cephalosporin); a luciferase gene (Ow *et al.,* 1986) a xylE gene (Zukowsky *et al.,* 1983) which encodes a catechol dioxygenase that can convert chromogenic catechols; an α-amylase gene (Ikatu *et al*., 1990); a tyrosinase gene (Katz *et al.,* 1983) which encodes an enzyme capable of oxidizing tyrosine to DOPA and dopaquinone which in turn condenses to melanin; an a - galactosidase, which catalyzes a chromogenic α-galactose substrate.

A transformation vector can be readily prepared using methods available in the art. The transformation vector comprises one or more nucleotide sequences that is/are capable of being transcribed to an RNA molecule and that is/are substantially homologous and/or complementary to one or more nucleotide sequences encoded by the genome of the insect, such that upon uptake of the RNA there is down-regulation of expression of at least one of the respective nucleotide sequences of the genome of the pest.

### VIII. Methods for Genetic Engineering

The present invention relates to the introduction of a nucleotide sequence into a organism to achieve pest inhibitory levels of expression of one or more dsRNA molecules. -The inventive polynucleotides and polypeptides may be introduced into a host cell, such as bacterial or yeast cell, by standard procedures known in the art for introducing recombinant sequences into a target host cell. Such procedures include, but are not limited to, transfection, infection, transformation, natural uptake, calcium phosphate, electroporation, microinjection biolistics and microorganism-mediated transformation protocols. -The methods chosen vary with the host organism.

A transgenic organism is one that comprises at least one cell it its genome in which an exogenous nucleic acid has been stably integrated. -Thus, a transgenic organism may contain only genetically modified cells in certain parts of its structure.

Accordingly, the present invention also relates to a transgenic cell or organism comprising any of the nucleotide sequences or recombinant DNA constructs described herein. The invention further relates to prokaryotic cells (such as, but not limited to, gram-positive and gram-negative bacterial cells) and eukaryotic cells (such as, but not limited to, yeast cells or plant cells).

For example, the present invention relates to introducing a target gene into a bacterium, such as *Lactobacillus.* -The nucleic acid constructs can be integrated into a bacterial genome with an integrating vector.- Integrating vectors typically contain at least one sequence homologous to the bacterial chromosome that allows the vector to integrate. Integrations appear to result from recombinations between homologous DNA in the vector and the bacterial chromosome. -For example, integrating vectors constructed with DNA from various Bacillus strains integrate into the Bacillus chromosome (EP 0 127,328). -Integrating vectors may also be comprised of bacteriophage or transposon sequences. Suicide vectors are also known in the art.

Construction of suitable vectors containing one or more of the above-listed components employs standard recombinant DNA techniques. -Isolated plasmids or DNA fragments are cleaved, tailored, and re-ligated in the form desired to generate the plasmids required. -Examples of available bacterial expression vectors include, but are not limited to, the multifunctional *E. coli* cloning and expression vectors such as Bluescript™ (Stratagene, La Jolla, CA), in which, for example, a *D. v. virgifera* protein or fragment thereof, may be ligated into the vector in frame with sequences for the amino-terminal Met and the subsequent 7 residues of β-galactosidase so that a hybrid protein is produced; pIN vectors (Van Heeke and Schuster, 1989); and the like.

The invention also relates to introducing a target gene into a yeast cell. A yeast recombinant construct can typically include one or more of the following: a promoter sequence, fusion partner sequence, leader sequence, transcription termination sequence, a selectable marker. These elements can be combined into an expression cassette, which may be maintained in a replicon, such as an extrachromosomal element (*e.g*., plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 (Botstein *et al.*, 1979), pCl/1 (Brake *et al.*, 1984), and YRp17 (Stinchcomb *et al.,* 1982). In addition, a replicon may be either a high or low copy number plasmid. A high copy number plasmid will generally have a copy number ranging from about 5 to about 200, and typically about 10 to about 150. A host containing a high copy number plasmid will preferably have at least about 10, and more preferably at least about 20.

Useful yeast promoter sequences can be derived from genes encoding enzymes in the metabolic pathway. Examples of such genes include alcohol dehydrogenase (ADH) (EP 0 284044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EP 0 3215447). The yeast PHO5 gene, encoding acid phosphatase, also provides useful promoter sequences (Myanohara *et al.,* 1983). In addition, synthetic promoters that do not occur in nature also function as yeast promoters. Examples of such hybrid promoters include the ADH regulatory sequence linked to the GAP transcription activation region (U.S. Patent No. 4,876,197 and 4,880,734). Examples of transcription terminator sequences and other yeast-recognized termination sequences, such as those coding for glycolytic enzymes, are known to those of skill in the art.

Alternatively, the expression constructs can be integrated into the yeast genome with an integrating vector. Integrating vectors typically contain at least one sequence homologous to a yeast chromosome that allows the vector to integrate, and preferably contain two homologous sequences flanking the expression construct. Integrations appear to result from recombinations between homologous DNA in the vector and the yeast chromosome (Orr-Weaver *et al.,* 1983). An integrating vector may be directed to a specific locus in yeast by selecting the appropriate homologous sequence for inclusion in the vector. See Orr-Weaver *et al.*, supra. One or more expression constructs may integrate, possibly affecting levels of recombinant protein, produced (Rine *et al.*, 1983).

### IX. Quantifying inhibition of target gene expression

Inhibition of target gene expression may be quantified by measuring either the endogenous target RNA or the protein produced by translation of the target RNA and the consequences of inhibition can be confirmed by examination of the outward properties of the cell or organism. Techniques for quantifying RNA and proteins are well known to one of ordinary skill in the art. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, and tetracyclin, and the like.

In certain embodiments gene expression is inhibited by at least 10%, preferably by at least 33%, more preferably by at least 50%, and yet more preferably by at least 80%. Gene expression can be inhibited by at least 80%, more preferably by at least 90%, more preferably by at least 95%, or by at least 99% within cells in the pest so a significant inhibition takes place. -Significant inhibition is intended to refer to sufficient inhibition that results in a detectable phenotype (*e.g*., cessation of larval growth, paralysis or mortality, *etc*.) or a detectable decrease in RNA and/or protein corresponding to the target gene being inhibited. Although in certain conditions inhibition occurs in substantially all cells of the pest, in other preferred embodiments inhibition occurs in only a subset of cells expressing the gene. For example, if the target gene plays an essential role in cells in an insect alimentary tract, inhibition of the gene within these cells is sufficient to exert a deleterious effect on the insect.

### X. Exposing pest to dsRNA

A pest can be exposed to a dsRNA in any suitable manner that permits administering the dsRNA to the pest. For example, the pest can be contacted with the dsRNA in pure or substantially pure form, for example an aqueous solution containing the dsRNA. In one embodiment, the insect may be simply "soaked" or "sprayed" with an aqueous solution comprising the dsRNA. Alternatively, the pest may be "sprayed" with a solution comprising a dsRNA.

Alternatively, the dsRNA may be linked to a food component of the pest, such as a food component for a mammalian pathogenic pest, in order to increase uptake of the dsRNA by the insect. Ingestion by a pest permits delivery of the pest control agents to the pest and results in down-regulation of a target gene in the host. -Methods for oral introduction may include, for example, directly mixing dsRNA with a, pest's food, as well as engineered approaches in which a species that is used as food is engineered to express the dsRNA or siRNA, then fed to the pest to be affected. For example, a bacteria, such as *Lactobacillus*, may be transformed with a target sequence and then fed to a pest. In one embodiment, for example, the dsRNA or siRNA molecules may be incorporated into, or overlaid on the top of, the insect's diet.

In other embodiments the pest may be contacted with a composition containing the inventive dsRNA. -The composition may, in addition to the dsRNA, contain further excipients, diluents, or carriers.

The dsRNA may also be incorporated in the medium in which the pest grows or infests. For example, a dsRNA may be incorporated into a food container or protective wrapping as a means for inhibiting pest infestation. -Wood, for example, may be treated with a solution comprising a dsRNA to prevent pest infestation.

In other embodiments, the dsRNA is expressed in a bacterial or fungal cell and the bacterial or fungal cell is taken up or eaten by the insect species.

As illustrated in the examples, bacteria can be engineered to produce any of the dsRNA or dsRNA constructs of the invention. These bacteria can be eaten by the insect species. When taken up, the dsRNA can initiate an RNAi response, leading to the degradation of the target mRNA and weakening or killing of the feeding insect. Alternatively, dsRNA producing bacteria or yeast cells can be sprayed directly onto the crops.

Some bacteria have a very close interaction with the host plant, such as, but not limited to, symbiotic *Rhizobium* with the *Legminosea* (for example Soy). Such recombinant bacteria could be mixed with the seeds (for instance as a coating) and used as soil improvers.

A virus such as a baculovirus which specifically infects insects may be also be used. This ensures safety for mammals, especially humans, since the virus will not infect the mammal, so no unwanted RNAi effect will occur..

Possible applications include intensive greenhouse cultures, for instance crops that are less interesting from a GMO point of view, as well as broader field crops such as soy.

This approach has several advantages, eg: since the problem of possible dicing by a plant host is not present, it allows the delivery of large dsRNA fragments into the gut lumen of the feeding pest; the use of bacteria as insecticides does not involve the generation of transgenic crops, especially for certain crops where transgenic variants are difficult to obtain; there is a broad and flexible application in that different crops can be simultaneously treated on the same field and/or different pests can be simultaneously targeted, for instance by combining different bacteria producing distinct dsRNAs.

### XI. Products

Numerous products can encompass a dsRNA for use in controlling pests. For example, pharmaceutical or veterinary compositions for treating or preventing a pest disease or infection of humans or animals, respectively. -Such compositions comprise at least one dsRNA or RNA construct, or nucleotide sequence or recombinant DNA construct encoding the dsRNA or RNA construct, wherein the RNA comprises annealed complementary strands, one of which has a nucleotide sequence which corresponds to a target nucleotide sequence of an pest target gene that causes the disease or infection, and at least one carrier, excipient, or diluent suitable for pharmaceutical use.

Alternatively, a pharmaceutical or veterinary composition may be used as a composition suitable for topical use, such as application on the skin of an animal or human. For example, a dsRNA may be used in a liquid composition to be applied to the skin as drops, gel, aerosol, cream, ointment, etc. -Additionally, a dsRNA may be integrated into a transdermal patch or other medical device for treating or preventing a disease or condition. Other conventional pharmaceutical dosage forms may also be produced, including tablets, capsules, pessaries, transdermal patches, suppositories, etc. -The chosen form will depend upon the nature of the target pest and hence the nature of the disease it is desired to treat.

Oral vaccines, for example, can be produced using the inventive constructs and methods. -For example, a vaccine can be constructed by producing a dsRNA in bacteria (e.g. *lactobacillus*) which can be included in food and functions as an oral vaccine against insect infection. -Accordingly, methods for treating and/or preventing a pest disease or condition can be developed, comprising administering to a subject in need of such treatment and/or prevention, any of the compositions as herein described, said composition comprising at least one double-stranded RNA or double stranded RNA construct comprising annealed complementary strands, one of which has a nucleotide sequence which is complementary to at least part of a nucleotide sequence of a pest target gene that causes the disease or condition.

While the inventive compositions may be used for treating a disease or condition in a subject patient, the compositions and methods may also be used as a means for protecting a substrate or material from pest infestation. -The nature of the excipients included in the composition and the physical form of the composition may vary depending upon the nature of the substrate that it is desired to treat.

For example, such a composition may be a coating or a powder that can be applied to a substrate as a means for protecting the substrate from infestation by an insect and thereby preventing pest-induced damage to the substrate or material. -Thus, in one embodiment, the composition is in the form of a coating on a suitable surface which adheres to, and is eventually ingested by an insect which comes into contact with the coating. Such a composition can be used to protect any substrate or material that is susceptible to infestation by or damage caused by a pest, for example foodstuffs and other perishable materials, and substrates such as wood.

For example, the composition may be a liquid that is brushed or sprayed onto or imprinted into the material or substrate to be treated. Thus, a human user can spray the insect or the substrate directly with the composition

For example, houses and other wood products can be destroyed by termites, powder post beetles, and carpenter ants. -By treating wood or house siding with a composition comprising a dsRNA, it may be possible to reduce pest infestation. -Likewise, a tree trunk may be treated with a composition comprising a dsRNA.

Flour beetles, grain weevils, meal moths, and other pests feed on stored grain, cereals, pet food, powdered chocolate, and almost everything else in the kitchen pantry that is not protected. -Accordingly, for treating cereal boxes and other food storage containers and wrapping with a composition comprising a target dsRNA can be developed.

Larvae of clothes moths eat clothes made from animal products, such as fur, silk and wool. Thus, it may be desirable to treat hangers, closet organizers, and garment bags with the inventive dsRNA. -Book lice and silverfish are pests of libraries because they eat the starchy glue in the bindings of books. -Accordingly, compositions for treating books from pest infestation and destruction can be developed.

In one embodiment, the composition is in the form of a bait. -The bait is designed to lure the insect to come into contact with the composition. Upon coming into contact therewith, the composition is then internalized by the insect, by ingestion for example and mediates RNAi to thus kill the insect. The bait may depend on the species being targeted. -An attractant may also be used.- The attractant may be a pheromone, such as a male or female pheromone for example. The attractant acts to lure the insect to the bait, and may be targeted for a particular insect or may attract a whole range of insects. The bait may be in any suitable form, such as a solid, paste, pellet or powdered form.

The bait may also be carried away by the insect back to the colony. The bait may then act as a food source for other members of the colony, thus providing an effective control of a large number of insects and potentially an entire insect pest colony. This is an advantage associated with use of the double stranded RNA or bacteria expressing the dsRNA of the invention, because the delayed action of the RNAi mediated effects on the pests allows the bait to be carried back to the colony, thus delivering maximal impact in terms of exposure to the insects.

The baits may be provided in a suitable "housing" or "trap". -Such housings and traps are commercially available and existing traps may be adapted to include the compositions of the invention. -The housing or trap may be box-shaped for example, and may be provided in pre-formed condition or may be formed of foldable cardboard for example. -Suitable materials for a housing or trap include plastics and cardboard, particularly corrugated cardboard. The inside surfaces of the traps may be lined with a sticky substance in order to restrict movement of the insect once inside the trap. The housing or trap may contain a suitable trough inside which can hold the bait in place. -A trap is distinguished from a housing because the insect can not readily leave a trap following entry, whereas a housing acts as a "feeding station" which provides the insect arachnid with a preferred environment in which they can feed and feel safe from predators.

It is clear that numerous products and substrates can be treated with the inventive compositions for reducing pest infestation. -Of course, the nature of the excipients and the physical form of the composition may vary depending upon the nature of the substrate that is desired to treat. For example, the composition may be a liquid that is brushed or sprayed onto or imprinted into the material or substrate to be treated, or a coating that is applied to the material or substrate to be treated.

Specific examples are presented below of methods for identifying target sequences and introducing the sequences into various cells and compositions. They are meant to be exemplary and not as limitations on the present invention.

### Example 1: Silencing C.elegans target genes in C. elegans in High Throughout Screening

A *C. elegans* genome wide library was prepared in the pGN9A vector (WO 01/88121) between two identical T7-promoters and terminators, driving its expression in the sense and antisense direction upon expression of the T7 polymerase, which was induced by IPTG.

This library was transformed into the bacterial strain AB301-105 (DE3) in 96 well plate format. For the genome wide screening, these bacterial cells were fed to the nuclease deficient *C*. *elegant nuc-1(e1392)* strain.

Feeding the dsRNA produced in the bacterial strain AB301-105 (DE3), to *C*. *elegans nuc-1 (e1392)* worms, was performed in a 96 well plate format as follows: *nuc-1* eggs were transferred to a separate plate and allowed to hatch simultaneously at 20 °C for synchronization of the L1 generation. 96 well plates were filled with 100 µL liquid growth medium comprising IPTG and with 10 µL bacterial cell culture of OD₆₀₀1 AB301-105 (DE3) of the C. elegans dsRNA library carrying each a vector with a C. elegans genomic fragment for expression of the dsRNA. To each well, 4 of the synchronized L1 worms were added and were incubated at 25 °C for at least 4 to 5 days. These experiments were performed in quadruplicate. In the screen 6 controls were used:
- pGN29 = negative control, wild type
- pGZ1 = *unc-22* = twitcher phenotype
- pGZ18 = chitin synthase = embryonic lethal
- pGZ25 = *pos-1* = embryonic lethal
- pGZ59 = *bli-4D* = acute lethal
- ACC = acetyl co-enzym A carboxylase = acute lethal

After 5 days, the phenotype of the *C*. *elegans nuc-1 (e1392)* worms fed with the bacteria producing dsRNA were compared to the phenotype of worms fed with the empty vector (pGN29) and the other controls. The worms that were fed with the dsRNA were screened for lethality (acute or larval) lethality for the parent (Po) generation, (embryonic) lethality for the first filial (F1) generation, or for growth retardation of Po as follows: (i) Acute lethality of Po: L1's have not developed and are dead, this phenotype never gives progeny and the well looks quite empty; (ii) (Larval) lethality of Po: Po died in a later stage than L1, this phenotype also never gives progeny. Dead larvae or dead adult worms are found in the wells; (iii) Lethality for F1: L1's have developed until adult stage and are still alive. This phenotype has no progeny. This can be due to sterility, embryonic lethality (dead eggs on the bottom of well), embryonic arrest or larval arrest (eventually ends up being lethal): (iv) Arrested in growth and growth retardation/delay: Compared to a well with normal development and normal # of progeny.

For the target sequences presented in Table 1A, it was concluded that dsRNA mediated silencing of the C. *elegans* target gene in nematodes, such as *C*. *elegans*, had a fatal effect on the growth and viability of the worm.

Subsequent to the above dsRNA silencing experiment, a more detailed phenotyping experiment was conducted in *C*. *elegans* in a high throughput format on 24 well plates. The dsRNA library produced in bacterial strain AB301-105 (DE3), as described above, was fed to *C*. *elegans nuc-1 (e1392)* worms on 24 well plates as folluws: *nuc-1* eggs were transferred to a separate plate and allowed to hatch simultaneously at 20 C for synchronization of the L1 generation. Subsequently 100 of the synchronized L1 worms were soaked in a mixture of 500 µL S-complete fed medium, comprising 5 µg/mL cholesterol, 4 µL/mL PEG and 1mM IPTG, and 500 µL of bacterial cell culture of OD₆₀₀1 AB301-105 (DE3) of the *C*. *elegans* dsRNA library carrying each a vector with a *C*. *elegans* genomic fragment for expression of the dsRNA. The soaked L1 worms were rolled for 2 hours at 25 C.

After centrifugation and removal of 950 µL of the supernatant, 5 µL of the remaining and resuspended pellet (comprising about 10 to 15 worms) was transferred in the middle of each well of a 24 well plate, filled with a layer of agar LB broth. -The inoculated plate was incubated at 25°C for 2 days. At the adult stage, 1 adult worm was singled and incubated at 25°C for 2 days for inspection of its progeny. The other adult worms are inspected *in situ* on the original 24 well plate. -These experiments were performed in quadruplicate.

This detailed phenotypic screen was repeated with a second batch of worms, the only difference being that the worms of the second batch were incubated at 20 C for 3 days.

The phenotype of the worms fed with *C*. *elegans* dsRNA was compared to the phenotype of *C*. *elegans nuc-1 (e1392)* worms fed with the empty vector.

Based on this experiment, it was concluded that silencing the *C*. *elegans* target genes as represented in **Table 1A** had a fatal effect on the growth and viability of the worm and that the target gene is essential to the viability of nematodes. Therefore these genes are good target genes to control (kill or prevent from growing) nematodes via dsRNA mediated gene silencing. Accordingly, the present invention relates to the use of nematode orthologs of the above *C*. *elegans* target gene, to control nematode infestation, such as nematode infestation of plants.

### Example 2: Identification of D. melanogaster orthologs

As described above in Example 1, numerous *C*. *elegans* lethal sequences were identified and can be used for identifying orthologs in other species and genera.- For example, the *C*. *elegans* lethal sequences can be used to identify orthologous *D*. *melanogasters* sequences. That is, each *C*. *elegans* sequence can be querried against a public database, such as GenBank, for orthologous sequences in *D. melanogaster.* -Potential *D*. *melanogaster* orthologs were selected that share a high degree of sequence homology (E value preferably less than or equal to 1E-30) and the sequences are blast reciprocal best hits, the latter means that the sequences from different organisms (e.g. *C*. *elegans* and *D. melanogaster*) are each other's top blast hits. For example, sequence C from *C*. *elegans* is compared against sequences in *D. melanogaster* using BLAST. If sequence C has the *D*. *melanogaster* sequence D as best hit and when D is compared to all the sequences of *C*. *elegans,* also turns out to be sequence C, then D and C are reciprocal best hits. This criterium is often used to define orthology, meaning similar sequences of different species, having similar function. The *D. melanogaster* sequence identifiers are represented in Table 1A.

### Example 3: Leptinotarsa decemlineata (Colorado potato beetle)

### A. Cloning partial gene sequences from Leptinotarsa decemlineata

High quality, intact RNA was isolated from 4 different larval stages of *Leptinotarsa decemlineata* (Colorado potato beetle; source: Jeroen van Schaik, Entocare CV Biologische Gewasbescherming, Postbus 162, 6700 AD Wageningen, the Netherlands) using TRIzol Reagent (Cat. Nr. 15596-026/15596-018, Invitrogen, Rockville, Maryland, USA) following the manufacturer's instructions. Genomic DNA present in the RNA preparation was removed by DNase treatment following the manufacturer's instructions (Cat. Nr. 1700, Promega). -cDNA was generated using a commercially available kit (SuperScript^{™} III Reverse Transcriptase, Cat. Nr. 18080044, Invitrogen, Rockville, Maryland, USA) following the manufacturer's instructions.

To isolate cDNA sequences comprising a portion of the LD001, LD002, LD003, LD006, LD007, LD010, LD011, LD014, LD015, LD016 and LD018 genes, a series of PCR reactions with degenerate primers were performed using Amplitaq Gold (Cat. Nr. N8080240, Applied Biosystems) following the manufacturer's instructions.

The sequences of the degenerate primers used for amplification of each of the genes are given in **Table 2-LD**, which displays *Leptinotarsa decemlineata* target genes including primer sequences and cDNA sequences obtained. These primers were used in respective PCR reactions with the following conditions: 10 minutes at 95°C, followed by 40 cycles of 30 seconds at 95°C, 1 minute at 55°C and 1 minute at 72°C, followed by 10 minutes at 72°C. The resulting PCR fragments were analyzed on agarose gel, purified (QIAquick Gel Extraction kit, Cat. Nr. 28706, Qiagen), cloned into the pCR8/GW/topo vector (Cat. Nr. K2500 20, Invitrogen), and sequenced. The sequences of the resulting PCR products are represented by the respectiveSEQ ID NOs as given in **Table 2-LD** and are referred to as the partial sequences. The corresponding partial amino acid sequence are represented by the respective SEQ ID NOs as given in **Table 3-LD**, where the start of the reading frame is indicated in brackets.

### B. dsRNA production of the Leptinotarsa decemlineata genes

dsRNA was synthesized in milligram amounts using the commercially available kit T7 Ribomax^{™} Express RNAi System (Cat. Nr. P 1700, Promega). First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-LD**. The conditions in the PCR reactions were as follows: 4 minutes at 95°C, followed by 35 cycles of 30 seconds at 95°C, 30 seconds at 55°C and 1 minute at 72°C, followed by 10 minutes at 72°C. The anti-sense T7 template was generated using specific forward and specific T7 reverse primers in a PCR reaction with the same conditions as described above. The sequences of the respective primers for amplifying the anti-sense template for each of the target genes are given in **Table 8-LD**. The resulting PCR products were analyzed on agarose gel and purified by PCR purification kit (Qiaquick PCR Purification Kit, Cat. Nr. 28106, Qiagen) and NaClO₄ precipitation. The generated T7 forward and reverse templates were mixed to be transcribed and the resulting RNA strands were annealed, DNase and RNase treated, and purified by sodium acetate, following the manufacturer's instructions. The sense strand of the resulting dsRNA for each of the target genes is given in **Table 8-LD**. Table 8-LD displays sequences for preparing ds RNA fragments of *Leptinotarsa decemlineata* target sequences and concatemer sequences, including primer sequences.

### C. Screening dsRNA targets using artificial diet for activity against Leptinotarsa decemlineata

Artificial diet for the Colorado potato beetle was prepared as follows (adapted from Gelman et al., 2001, J. Ins. Sc., vol. 1, no. 7, 1-10): water and agar were autoclaved, and the remaining ingredients for a total volume of 1L (water: 768 ml: agar: 14g: rolled oats: 40 g: Torula yeast: 60g; lactalbumin hydrolysate: 30g; casein: 10g ; fructose: 20g; Wesson salt mixture: 4g; tomato fruit powder: 12.5 g; potato leaf powder: 25g; b-sitosterol: 1g; sorbic acid: 0.8g; methyl paraben: 0.8g; Vanderzant vitamin mix: 12g; neomycin sulfate: 0.2g; aureomycin: 0.130g; rifampicin: 0.130g; chloramphenicol; 0.130g; nystatin; 0.050g; soybean oil: 2ml; wheat germ oil: 2ml) were added when the temperature dropped to 55°C. At this temperature, the ingredients were mixed well before the diet was aliquoted into 24-well plates (Nunc) with a quantity of 1ml of diet per well. The artificial diet was allowed to solidify by cooling at room temperature. Diet was stored at 4 °C for up to three weeks.

Fifty µl of a solution of dsRNA at a concentration of 1 mg/ml was applied topically onto the solid artificial diet in the wells of the multiwell plate. The diet was dried in a laminair flow cabin. Per treatment, twenty-four Colorado potato beetle larvae (2^{nd} stage), with two insects per well, were tested. The plates were stored in the insect rearing chamber at 25 ± 2 °C, 60-% relative humidity, with a 16:8 hours light:dark photoperiod. The beetles were assessed as live or dead every 1, 2 or 3 days. After seven days, for targets LD006, LD007, LD010, LD011, and LD014, the diet was replaced with fresh diet with topically applied dsRNA at the same concentration (1 mg/ml); for targets LD001, LD002, LD003, LD015, and LD016, the diet was replaced with fresh diet only. The dsRNA targets were compared to diet only or diet with topically applied dsRNA corresponding to a fragment of the GFP (green fluorescent protein) coding sequence (SEQ ID NO: 235).

Feeding artificial diet containing intact naked dsRNAs to *L. decemlineata* larvae resulted in significant increases in larval mortalities as indicated in two separate bioassays (Figures 1LD-2LD).

All dsRNAs tested resulted ultimately in 100 % mortality after 7 to 14 days. Diet with or without GFP dsRNA sustained the insects throughout the bioassays with very little or no mortality.

Typically, in all assays observed, CPB second-stage larvae fed normally on diet with or without dsRNA for 2 days and molted to the third larval stage. At this new larval stage the CPB were observed to reduce significantly or stop altogether their feeding, with an increase in mortality as a result.

### D. Bioassay of dsRNA targets using potato leaf discs for activity against the Leptinotarsa decemlineata

An alternative bioassay method was employed using potato leaf material rather than artificial diet as food source for CPB. Discs of approximately 1.1 cm in diameter (or 0.95 cm²) were cut out off leaves of 2 to 3-week old potato plants using a suitably-sized cork borer. Treated leaf discs were prepared by applying 20 µl of a 10 ng/µl solution of target LD002 dsRNA or control gfp dsRNA on the adaxial leaf surface. The leaf discs were allowed to dry and placed individually in 24 wells of a 24-well multiplate (Nunc). A single second-larval stage CPB was placed into each well, which was then covered with tissue paper and a multiwell plastic lid. The plate containing the insects and leaf discs were kept in an insect chamber at 28°C with a photoperiod of 16h light/8h dark. The insects were allowed to feed on the leaf discs for 2 days after which the insects were transferred to a new plate containing fresh treated leaf discs. Thereafter, the insects were transferred to a plate containing untreated leaf discs every day until day 7. Insect mortality and weight scores were recorded.

Feeding potato leaf discs with surface-applied intact naked dsRNA of target LD002 to *L. decemlineata* larvae resulted in a significant increase in larval mortalities (i.e. at day 7 all insects were dead; 100 % mortality) whereas control gfp dsRNA had no effect on CPB survival. Target LD002 dsRNA severely affected the growth of the larvae after 2 to 3 days whereas the larvae fed with gfp dsRNA at the same concentration developed as normal **(****Figure 3-LD****)**.

### E. Screening shorter versions of dsRNAs using artificial diet for activity against Leptinotarsa decemlineata

This example exemplifies the finding that shorter (60 or 100bp) dsRNA fragments on their own or as concatemer constructs are sufficient in causing toxicity towards the Colorado potato beetle.

LD014, a target known to induce lethality in Colorado potato beetle, was selected for this example. This gene encodes a V-ATPase subunit E (SEQ ID NO: 15).

A 100 base pair fragment, LD014_F1, at position 195-294 on SEQ ID NO: 15 (SEQ ID NO: 159) and a 60 base pair fragment, LD014_F2, at position 235-294 on SEQ ID NO: 15 (SEQ ID NO: 160) were further selected.

Two concatemers of 300 base pairs, LD014_C1 and LD014_C2, were designed (SEQ ID NO: 161 and SEQ ID NO: 162). LD014_C1 contained 3 repeats of the 100 base pair fragment described above (SEQ ID NO: 159) and LD014_C2 contained 5 repeats of the 60 base pair fragment described above (SEQ ID NO: 160).

The fragments LD014_F1 and LD014_F2 were synthesized as sense and antisense primers. These primers were annealed to create the double strands DNA molecules prior to cloning. *Xba*I and *Xma*I restrictions sites were included at the 5' and 3' ends of the primers, respectively, to facilitate the cloning.

The concatemers were made as 300 base pairs synthetic genes. *Xba*I and *Xma*I restrictions sites were included at the 5' and 3' ends of the synthetic DNA fragments, respectively, to facilite the cloning.

The 4 DNA molecules, i.e. the 2 single units (LD014_F1 & LD014_F2) and the 2 concatemers (LD014_C1 & LD014_C2), were digested with *Xba*I and *Xma*I and subcloned in pBluescriptII SK+ linearised by *Xba*I and *Xma*I digests, resulting in recombinant plasmids p1, p2, p3, & p4, respectively.

Double-stranded RNA production: dsRNA was synthesized using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter. For LD014_F1, the sense T7 template was generated using the specific T7 forward primer oGBM159 and the specific reverse primer oGBM164 (represented herein as SEQ ID NO: 204 and SEQ ID NO: 205, respectively) in a PCR reaction . The anti-sense T7 template was generated using the specific forward primer oGBM163 and the specific T7 reverse primer oGBM160 (represented herein as SEQ ID NO: 206 and SEQ ID NO: 207, respectively) in a PCR reaction. The resulting PCR products were analyzed on agarose gel and purified by PCR purification kit and NaClO₄ precipitation. -The generated T7 forward and reverse templates were mixed to be transcribed and the resulting RNA, strands were annealed, Dnase and Rnase treated, and purified by sodium acetate . The sense strand of the resulting dsRNA is herein represented by SEQ ID NO: 203.

For LD014_F2, the sense T7 template was generated using the specific T7 forward primer oGBM161 and the specific reverse primer oGBM166 (represented herein as SEQ ID NO: 209 and SEQ ID NO: 210, respectively) in a PCR reaction . The anti-sense T7 template was generated using the specific forward primer oGBM165 and the specific T7 reverse primer oGBM162 (represented herein as SEQ ID NO: 211 and SEQ ID NO: 212, respectively) in a PCR reaction. The resulting PCR products were analyzed on agarose gel and purified by PCR purification kit and NaClO₄ precipitation. The generated T7 forward and reverse templates were mixed to be transcribed and the resulting RNA strands were annealed, Dnase and Rnase treated, and purified by sodium acetate . The sense strand of the resulting dsRNA is herein represented by SEQ ID NO: 208.

Also for the concatemers, separate single 5' T7 RNA polymerase promoter templates were generated in two separate PCR reactions, each reaction containing the target sequence in a different orientation relative to the T7 promoter. The recombinant plasmids p3 and p4 containing LD014_C1 & LD014_C2 were linearised with *Xba*I or *Xma*I, the two linear fragments for each construct purified and used as template for the *in vitro* transcription assay, using the T7 promoters flanking the cloning sites. Double-stranded RNA was prepared by *in vitro* transcription using the T7 RiboMAX^{™} Express RNAi System (Promega). The sense strands of the resulting dsRNA for LD014_C1 and LD014_C2 are herein represented by SEQ ID NO: 213 and2114, respectively.

Shorter sequences of target LD014 and concatemers were able to induce lethality in *Leptinotarsa decemlineata*, as shown in **Figure 4-LD**.

### GF. Screening dsRNAs at different concentrations using artificial diet for activity against Leptinotarsa decemlineata

Fifty µl of a solution of dsRNA at serial ten-fold concentrations from 1 µg/µl (for target LD027 from 0.1 µg/µl)down to 0.01 ng/µl was applied topically onto the solid artificial diet in the wells of a 24-well plate (Nunc). The diet was dried in a laminair flow cabin. Per treatment, twenty-four Colorado potato beetle larvae (2^{nd} stage), with two insects per well, were tested. The plates were stored in the insect rearing chamber at 25 ± 2 °C, 60-% relative humidity, with a 16:8 hours light:dark photoperiod. The beetles were assessed as live or dead at regular intervals up to day 14. After seven days, the diet was replaced with fresh diet with topically applied dsRNA at the same concentrations. The dsRNA targets were compared to diet only.

Feeding artificial diet containing intact naked dsRNAs of different targets to *L*. *decemlineata* larvae resulted in high larval mortalities at concentrations as low as between 0.1 and 10 ng dsRNA/µl as shown in **Figure 5-LD**.

### G. Cloning of a CPB gene fragment in a vector suitable for bacterial production of insect-active double-stranded RNA

While any efficient bacterial promoter may be used, a DNA fragment corresponding to an MLB gene target was cloned in a vector for the expression of double-stranded RNA in a bacterial host (See WO 00/01846).

The sequences of the specific primers used for the amplification of target genes are provided in Table 8. -The template used is the pCR8/GW/topo vector containing any of target sequences. The primers are used in a PCR reaction . The resulting PCR fragment is analyzed on agarose gel, purified, blunt-end cloned into *Srf* I-linearized pGNA49A vector (reference to WO00188121A1), and sequenced.- The sequence of the resulting PCR product corresponds to the respective sequence as given in Table 8. -The recombinant vector harboring this sequence is named pGBNJ003.

The sequences of the specific primers used for the amplification of target gene fragment LD010 are provided in Table 8 (forward primer SEQ ID NO: 191 and reverse primer SEQ ID NO: 190). The template used was the pCR8/GW/topo vector containing the LD010 sequence (SEQ ID NO: 11). The primers were used in a PCR , blunt-end cloned into *Srf* I-linearized pGNA49A vector (reference to WO 00/188121A1), and sequenced.- The sequence of the resulting PCR product corresponds to SEQ ID NO: 188 as given in Table 8.- The recombinant vector harboring this sequence was named pGBNJ003.

### H. Expression and production of a double-stranded RNA target in two strains of Escherichia coli: (1) AB309-105, and, (2) BL21(DE3)

The procedures described below were followed in order to express suitable levels of insect-active double-stranded RNA of target LD010 in bacteria. An RNaseIII-deficient strain, AB309-105, was used in comparison to wild-type RNaseIII-containing bacteria, BL21(DE3).

### Transformation ofAB309-105 and BL21(DE3)

Three hundred ng of the plasmid was added to and gently mixed in a 50 µl aliquot of ice-chilled chemically competent *E*. coli strain AB309-105 or BL21(DE3). The cells were incubated on ice for 20 minutes before subjecting them to a heat shock treatment of 37 °C for 5 minutes, after which the cells were placed back on ice for a further 5 minutes. Four hundred and fifty µl of room temperature SOC medium was added to the cells and the suspension incubated on a shaker (250 rpm) at 37 °C for 1 hour. One hundred µl of the bacterial cell suspension was transferred to a 500 ml conical flask containing 150 ml of liquid Luria-Bertani (LB) broth supplemented with 100 µg/ml carbenicillin antibiotic. The culture was incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) was made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture was measured using an appropriate spectrophotometer and adjusted to a value of 1 by the addition of fresh LB broth. Fifty ml of this culture was transferred to a 50 ml Falcon tube and the culture then centrifuged at 3000 g at 15 °C for 10 minutes. The supernatant was removed and the bacterial pellet resuspended in 50 ml of fresh S complete medium (SNC medium plus 5 µg/ml cholesterol) supplemented with 100 µg/ml carbenicillin and 1 mM IPTG. The bacteria were induced for 2 to 4 hours at room temperature.

### Heat treatment of bacteria

Bacteria were killed by heat treatment in order to minimize the risk of contamination of the artificial diet in the test plates. However, heat treatment of bacteria expressing double-stranded RNA is not a prerequisite for inducing toxicity towards the insects due to RNA interference. The induced bacterial culture was centrifuged at 3000 g at room temperature for 10 minutes, the supernatant discarded and the pellet subjected to 80 °C for 20 minutes in a water bath. After heat treatment, the bacterial pellet was resuspended in 1.5 ml MilliQ water and the suspension transferred to a microfuge tube. Several tubes were prepared and used in the bioassays for each refreshment. The tubes were stored at -20 °C until further use.

### I. Laboratory trials to test Escherichia coli expressing dsRNA target LD010 against Leptinotarsa decemlineata

Two bioassay methods were employed to test double-stranded RNA produced in *Escherichia coli* against larvae of the Colorado potato beetle: (1) artificial diet-based bioassay, and, (2) plant-based bioassay.

### Artificial diet-based bioassays

Artificial diet for the Colorado potato beetle was prepared as described previously in Example 43C. A half milliliter of diet was dispensed into each of the wells of a 48-well multiwell test plate (Nunc). For every treatment, fifty µl of an OD 1 suspension of heat-treated bacteria (which is equivalent to approximately 5 x 10⁷ bacterial cells) expressing dsRNA was applied topically onto the solid diet in the wells and the plates were allowed to dry in a laminair flow cabin. Per treatment, forty-eight 2^{nd} stage Colorado potato beetle larvae, one in each well containing diet and bacteria, were tested. Each row of a plate (i.e. 8 wells) was considered as one replicate. The plates were kept in the insect rearing chamber at 25 ± 2 °C, 60 ± 5-% relative humidity, with a 16:8 hours light:dark photoperiod. After every 4 days, the beetles were transferred to fresh diet containing topically-applied bacteria. The beetles were assessed as alive or dead every one or three days post infestation. For the survivors, growth and development in terms of larval weight was recorded on day 7 post infestation.

For RNaseIII-deficient *E*. *coli* strain AB309-105, bacteria containing plasmid pGBNJ003 and those containing the empty vector pGN29 (reference to WO 00/188121A1) were tested in bioassays for CPB toxicity. Bacteria harboring the pGBNJ003 plasmid showed a clear increase in insect mortality with time, whereas little or no mortality was observed for pGN29 and diet only control **(****Figures 6a-LD** **&** **7a-LD****)**. The growth and development of Colorado potato beetle larval survivors, 7 days after feeding on artificial diet containing bacteria expressing dsRNA target LD010, was severely impeded **(Table 10-LD**, **Figure 8a-LD****)**.

For *E. coli* strain BL21(DE3), bacteria containing plasmid pGBNJ003 and those containing the empty vector pGN29 were tested against the Colorado potato beetle larvae. Similar detrimental effects were observed on larvae fed diet supplemented with BL21(DE3) bacteria as for the RNAseIII-deficient strain, AB309-105 **(****Figures 6b-LD** **&** **7b-LD****).** However, the number of survivors for the five clones were higher for BL21(DE3) than for AB309-105; at day 12, average mortality values were approximately 25 % lower for this strain compared to the RNase III deficient strain. Also, the average weights of survivors fed on diet containing BL21(DE3) expressing dsRNA corresponding to target LD010 was severely reduced **(Table 10-LD,** **Figure 8b-LD****)**.

The delay in growth and development of the CPB larvae fed on diet containing either of the two bacterial strains harboring plasmid pGBNJ003 was directly correlated to feeding inhibition since no frass was visible in the wells of refreshed plates from day 4 onwards when compared to bacteria harboring the empty vector pGN29 or the diet only plate. This observation was similar to that where CPB was fed on *in vitro* transcribed double-stranded RNA topically applied to artificial diet (see Example 3D); here, cessation of feeding occurred from day 2 onwards on treated diet.

### Plant-based bioassays

Whole potato plants were sprayed with suspensions of chemically induced bacteria expressing dsRNA prior to feeding the plants to CPB larvae. The potato plants of variety 'line 5' were grown from tubers to the 8-12 unfolded leaf stage in a plant growth room chamber with the following conditions: 25 ± 2°C, 60 % relative humidity, 16:8 hour light:dark photoperiod. The plants were caged by placing a 500 ml plastic bottle upside down over the plant with the neck of the bottle firmly placed in the soil in a pot and the base cut open and covered with a fine nylon mesh to permit aeration, reduce condensation inside and prevent larval escape. Fifteen Colorado potato beetle larvae at the L1 stage were placed on each treated plant in the cage. Plants were treated with a suspension of *E*. *coli* AB309-105 harboring the pGBNJ003 plasmids (clone 1; **Figure 7a-LD**) or pGN29 plasmid (clone 1; see **Figure 7a-LD**). Different quantities of bacteria were applied to the plants: 66, 22, and 7 units, where one unit is defined as 10⁹ bacterial cells in 1 ml of a bacterial suspension at optical density value of 1 at 600 nm wavelength. In each case, a total volume of 1.6 ml was sprayed on the plant with the aid of a vaporizer. One plant was used per treatment in this trial. The number of survivors were counted and the weight of each survivor recorded.

Spraying plants with a suspension of *E*. *coli* bacterial strain AB309-105 expressing target dsRNA from pGBNJ003 led to a dramatic increase in insect mortality when compared to pGN29 control. The mortality count was maintained when the amount of bacteria cell suspension was diluted 9-fold (**Figure 9-LD**). The average weights of the larval survivors at day 11 on plants sprayed with bacteria harboring the pGBNJ003 vector were approximately 10-fold less than that of pGN29 (**Figure 10-LD**). Feeding damage by CPB larvae of the potato plant sprayed with bacteria containing the pGBNJ003 plasmid was much reduced when compared to the damage incurred on a potato plant sprayed with bacteria containing the empty vector pGN29 (**Figure 11-LD**).

These experiments showed that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification was provided for the effective protection of plants/crops from insect damage by the use of a spray of a formulation consisting of bacteria expressing double-stranded RNA corresponding to an insect gene target.

### J. Testing various culture suspension densities of Escherichia coli expressing dsRNA target LD010 against Leptinotarsa decemlineata

Preparation and treatment of bacterial cultures are described in Example 3J. Threefold serial dilutions of cultures (starting from 0.25 unit equivalents) of *Escherichia coli* RNAseIII-deficient strain AB309-105 expressing double-stranded RNA of target LD010 were applied to foliages of the potato plant of variety 'Bintje' at the 8-12 unfolded leaf stage. Ten L1 larvae of the *L. decemlineata* were placed on the treated plants with one plant per treatment. Scoring for insect mortality and growth impediment was done on day 7 (i.e., 7 days post infestation).

As shown in **Figure 14-LD**, high CPB larval mortality (90 to 100-%) was recorded after 1 week when insects were fed potato plants treated with a topical application by fine spray of heat-inactivated cultures of *E coli* harboring plasmid pGBNJ003 (for target 10 dsRNA expression) at densities 0.25, 0.08 and 0.025 bacterial units. At 0.008 units, about a third of the insects were dead, however, the surviving insects were significantly smaller than those in the control groups (*E*. *coli* harbouring the empty vector pGN29 and water only). Feeding damage by CPB larvae of the potato plant sprayed with bacteria containing the pGBNJ003 plasmid at concentrations 0.025 or 0.008 units was much reduced when compared to the damage incurred on a potato plant sprayed with bacteria containing the empty vector pGN29 (**Figure 15-LD**).

### K. Adults are extremely susceptible to orally ingested dsRNA corresponding to target genes.

The example provided below highlights the finding that adult insects (and not only insects of the larval stage) are extremely susceptible to orally ingested dsRNA corresponding to target genes.

Four targets were chosen for this experiment: targets 2, 10, 14 and 16 (SEQ ID NO: 168, 188, 198 and 220, respectively). GFP fragment dsRNA (SEQ ID NO: 235) was used as a control. Young adults (2 to 3 days old) were picked at random from our laboratory-reared culture with no bias towards insect gender. Ten adults were chosen per treatment. The adults were prestarved for at least 6 hours before the onset of the treatment. On the first day of treatment, each adult was fed four potato leaf discs (diameter 1.5 cm²) which were pretreated with a topical application of 25 µl of 0.1 µg/µl target dsRNA (synthesized as described in Example 3A; topical application as described in Example 3E) per disc. Each adult was confined to a small petridish (diameter 3 cm) in order to make sure that all insects have ingested equal amounts of food and thus received equal doses of dsRNA. The following day, each adult was again fed four treated leaf discs as described above. On the third day, all ten adults per treatment were collected and placed together in a cage consisting of a plastic box (dimensions 30 cm x 20 cm x 15 cm) with a fine nylon mesh built into the lid to provide good aeration. Inside the box, some moistened filter paper was placed in the base. Some (untreated) potato foliage was placed on top of the paper to maintain the adults during the experiment. From day 5, regular assessments were carried out to count the number of dead, alive (mobile) and moribund insects. For insect moribundity, adults were laid on their backs to check whether they could right themselves within several minutes; an insect was considered moribund only if it was not able to turn onto its front.

Clear specific toxic effects of double-stranded RNA correpsonding to different targets towards adults of the Colorado potato beetle, *Leptinotarsa decemlineata,* were demonstrated in this experiment (**Figure 12-LD**). Double-stranded RNA corresponding to a gfp fragment showed no toxicity towards CPB adults on the day of the final assessment (day 19). This experiment clearly showed that the survival of CPB adults was severely reduced only after a few days of exposure to dsRNA when delivered orally. For example, for target 10, on day 5, 5 out of 10 adults were moribund (sick and slow moving); on day 6, 4 out of 10 adults were dead with three of the survivors moribund; on day 9 all adults were observed dead.

As a consequence of this experiment, the application of target double-stranded RNAs against insect pests may be broadened to include the two life stages of an insect pest (i.e. larvae and adults) which could cause extensive crop damage, as is the case with the Colorado potato beetle.

### Example 4: Phaedon cochleariae (mustard leaf beetle)

### A. Cloning of a partial sequence of the Phaedon cochleariae (mustard leaf beetle) PC001, PC003, PC005, PC010, PC014, PC016 and PC027 genes via family PCR

High quality, intact RNA was isolated from the third larval stage of *Phaedon cochleariae* (mustard leaf beetle; source: Dr. Caroline Muller, Julius-von-Sachs-Institute for Biosciences, Chemical Ecology Group, University of Wuerzburg, Julius-von-Sachs-Platz 3, D-97082 Wuerzburg, Germany) using TRIzol Reagent . Genomic DNA present in the RNA preparation was removed by DNas treatment following the manufacturer's instructions. cDNA was generated using a commercially available kit (SuperScript^{™} III Reverse Transcriptase ).

To isolate cDNA sequences comprising a portion of the PC001, PC003, PC005, PC010, PC014, PC016 and PC027 genes, a series of PCR reactions with degenerate primers were performed using Amplitaq Gold .

The sequences of the degenerate primers used for amplification of each of the *Phaedon cochleariae* target genes are given in **Table 2-PC** including cDNA sequences obtained.

These primers were used in respective PCR reactions . The resulting PCR fragments were analyzed on agarose gel, purified , cloned into the pCR4/TOPO vector (Cat. Nr. K4530-20, Invitrogen) and sequenced. The sequences of the resulting PCR products are represented by the respective SEQ ID NOs as given in **Table 2-PC** and are referred to as the partial sequences.

The corresponding partial amino acid sequence are represented by the respective SEQ ID NOs as given in **Table 3-PC** , and the start of the reading frame is indicated in brackets.

### B. dsRNA production of the Phaedon cochleariae genes

dsRNA was synthesized in milligram amounts using the commercially available kit T7 Ribomax^{™} Express RNAi System (Cat. Nr. P1700, Promega). First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the *Phaedon cochleariae* target genes are given in **Table 8-PC.** The anti-sense T7 template was generated using specific forward and specific T7 reverse primers in a PCR reaction. The sequences of the respective primers for amplifying the anti-sense template for each of the target genes are given in **Table 8-PC.** The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-PC.**

### C. Laboratory trials to test dsRNA targets, using oilseed rape leaf discs for activity against Phaedon cochleariae larvae

The example provided below is an exemplification of the finding that the mustard leaf beetle (MLB) larvae are susceptible to orally ingested dsRNA corresponding to own target genes.

To test the different double-stranded RNA samples against MLB larvae, a leaf disc assay was employed using oilseed rape (*Brassica napus* variety SW Oban; source: Nick Balaam, Sw Seed Ltd., 49 North Road, Abington, Cambridge, CB1 6AS, UK) leaf material as food source. The insect cultures were maintained on the same variety of oilseed rape in the insect chamber at 25 ± 2 °C and 60 ± 5 % relative humidity with a photoperiod of 16h light/8h dark. Discs of approximately 1.1 cm in diameter (or 0.95 cm²) were cut out off leaves of 4- to 6-week old rape plants using a suitably-sized cork borer. Double-stranded RNA samples were diluted to 0.1 µg/µl in Milli-Q water containing 0.05% Triton X-100. Treated leaf discs were prepared by applying 25 µl of the diluted solution of target PC001, PC003, PC005, PC010, PC014, PC016, PC027 dsRNA and control gfp dsRNA or 0.05 % Triton X-100 on the adaxial leaf surfaces. The leaf discs were left to dry and placed individually in each of the 24 wells of a 24-well multiplate containing 1 ml of gellified 2% agar which helps to prevent the leaf disc from drying out. Two neonate MLB larvae were placed into each well of the plate, which was then covered with a multiwell plastic lid. The plate (one treatment containing 48 insects) was divided into 4 replicates of 12 insects per replicate (each row). The plate containing the insects and leaf discs were kept in an insect chamber at 25 ± 2 °C and 60 ± 5 % relative humidity with a photoperiod of 16h light/8h dark. The insects were fed leaf discs for 2 days after which they were transferred to a new plate containing freshly treated leaf discs. Thereafter, 4 days after the start of the bioassay, the insects from each replicate were collected and transferred to a Petri dish containing untreated fresh oilseed rape leaves. Larval mortality and average weight were recorded at days 2, 4 7, 9 and 11.

*P. cochleariae* larvae fed on intact naked target dsRNA-treated oilseed rape leaves resulted in significant increases in larval mortalities for all targets tested, as indicated in Figure 1(a). Tested double-stranded RNA for target PC010 led to 100 % larval mortality at day 9 and for target PC027 at day 11. For all other targets, signficantly high mortality values were reached at day 11 when compared to control gfp dsRNA, 0.05% Trition X-100 alone or untreated leaf only: (average value in percentage ± confidence interval with alpha 0.05) PC001 (94.4 ± 8.2); PC003 (86.1 ± 4.1); PC005 (83.3 ± 7.8); PC014 (63.9 ± 20.6); PC016 (75.0 ± 16.8); gfp dsRNA (11.1 ± 8.2); 0.05% Triton X-100 (19.4 ± 10.5); leaf only (8.3 ± 10.5).

Larval survivors were assessed based on their average weight. For all targets tested, the mustard leaf beetle larvae had significantly reduced average weights after day 4 of the bioassay; insects fed control gfp dsRNA or 0.05% Triton X-100 alone developed normally, as for the larvae on leaf only **(Figure 1(b)-PC).**

### D. Laboratory trials to screen dsRNAs at different concentrations using oilseed rape leaf discs for activity against Phaedon cochleariae larvae

Twenty-five µl of a solution of dsRNA from target PC010 or PC027 at serial ten-fold concentrations from 0.1 µg/µl down to 0.1 ng/µl was applied topically onto the oilseed rape leaf disc, as described in Example 4D above. As a negative control, 0.05% Triton X-100 only was administered to the leaf disc. Per treatment, twenty-four mustard leaf beetle neonate larvae, with two insects per well, were tested. The plates were stored in the insect rearing chamber at 25 ± 2 °C, 60 ± 5 % relative humidity, with a 16:8 hours light:dark photoperiod. At day 2, the larvae were transferred on to a new plate containing fresh dsRNA-treated leaf discs. At day 4 for target PC010 and day 5 for target PC027, insects from each replicate were transferred to a Petri dish containing abundant untreated leaf material. The beetles were assessed as live or dead on days 2, 4, 7, 8, 9, and 11 for target PC010, and 2, 5, 8, 9 and 12 for target PC027.

Feeding oilseed rape leaf discs containing intact naked dsRNAs of the two different targets, PC010 and PC027, to *P. cochleariae* larvae resulted in high mortalities at concentrations down to as low as 1 ng dsRNA/µl solution, as shown in Figures 2 (a) and (b). Average mortality values in percentage ± confidence interval with alpha 0.05 for different concentrations of dsRNA for target PC010 at day 11, 0 µg/µl: 8.3 ± 9.4; 0.1 µg/µl: 100; 0.01 µg/µl: 79.2 ± 20.6; 0.001 µg/µl: 58.3 ± 9.4; 0.0001 µg/µl: 12.5 ± 15.6; and for target PC027 at day 12, 0 µg/µl: 8.3 ± 9.4; 0.1 µg/µl: 95.8 ± 8.2; 0.01 µg/µl: 95.8 ± 8.2; 0.001 µg/µl: 83.3 ± 13.3; 0.0001 µg/µl 12.5 ± 8.2.

### E. Cloning of a MLB gene fragment in a vector suitable for bacterial production of insect-active double-stranded RNA

What follows is an example of cloning a DNA fragment corresponding to an MLB gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-PC**. The template used is the pCR8/GW/topo vector containing any of target sequences. The primers are used in a PCR The resulting PCR fragment is analyzed on agarose gel, purified, blunt-end cloned into *Srf* I-linearized pGNA49A vector (reference to WO00188121A1), and sequenced. The sequence of the resulting PCR product corresponds to the respective sequence as given in **Table PC**. The recombinant vector harbouring this sequence is named pGBNJ00 .

The sequences of the specific primers used for the amplification of target gene fragment PC010 are provided in **Table 8-PC**. The template used was the pCR8/GW/topo vector containing the PC010 sequence (SEQ ID NO: 253). The primers were used in a touchdown PCR The resulting PCR fragment was analyzed on agarose gel, purified, blunt-end cloned into *Srf* I-linearized pGNA49A vector (reference to WO00188121A1), and sequenced. The sequence of the resulting PCR product corresponds to SEQ ID NO: 488 as given in **Table 8-PC**. The recombinant vector harbouring this sequence was named pGCDJ001.

### F. Expression and production of a double-stranded RNA target in two strains of Escherichia coli AB309-105

The procedures described below are followed in order to express suitable levels of insect-active double-stranded RNA of insect target in bacteria. In this experiment, an RNaseIII-deficient strain, AB309-105 is used.

### Transformation of AB309-105

Three hundred ng of the plasmid were added to and gently mixed in a 50 µl aliquot of ice-chilled chemically competent *E. coli* strain AB309-105. The cells were incubated on ice for 20 minutes before subjecting them to a heat shock treatment of 37 °C for 5 minutes, after which the cells were placed back on ice for a further 5 minutes. Four hundred and fifty µl of room temperature SOC medium was added to the cells and the suspension incubated on a shaker (250 rpm) at 37°C for 1 hour. One hundred µl of the bacterial cell suspension was transferred to a 500 ml conical flask containing 150 ml of liquid Luria-Bertani (LB) broth supplemented with 100 µg/ml carbenicillin antibiotic. The culture was incubated on an Innova 4430 shaker (250 rpm) at 37°C overnight (16 to 18 hours).

### Chemical induction of double-stranded RNA expression in AB309-105

Expression of double-stranded RNA from the recombinant vector, pGBNJ003, in the bacterial strain AB309-105 was made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture was measured using an appropriate spectrophotometer and adjusted to a value of 1 by the addition of fresh LB broth. Fifty ml of this culture was transferred to a 50 ml Falcon tube and the culture then centrifuged at 3000 g at 15°C for 10 minutes. The supernatant was removed and the bacterial pellet resuspended in 50 ml of fresh S complete medium (SNC medium plus 5 µg/ml cholesterol) supplemented with 100 µg/ml carbenicillin and 1 mM IPTG. The bacteria were induced for 2 to 4 hours at room temperature.

### Heat treatment of bacteria

Bacteria were killed by heat treatment in order to minimize the risk of contamination of the artificial diet in the test plates. However, heat treatment of bacteria expressing double-stranded RNA is not a prerequisite for inducing toxicity towards the insects due to RNA interference. The induced bacterial culture was centrifuged at 3000 g at room temperature for 10 minutes, the supernatant discarded and the pellet subjected to 80°C for 20 minutes in a water bath. After heat treatment, the bacterial pellet was resuspended in a total volume of 50 ml of 0.05% Triton X-100 solution. The tube was stored at 4 °C until further use

### G. Laboratory trials to test Escherichia coli expressing dsRNA targets against Phaedon cochleariae

### Leaf disc bioassays

The leaf-disc bioassay method was employed to test double-stranded RNA from target PC010 produced in *Escherichia coli* (from plasmid pGCDJ001) against larvae of the mustard leaf beetle. Leaf discs were prepared from oilseed rape foliage, as described in Example 4. Twenty µl of a bacterial suspension, with an optical density measurement of 1 at 600 nm wavelength, was pipetted onto each disc. The leaf disc was placed in a well of a 24-multiwell plate containing 1 ml gellified agar. On each leaf disc were added two neonate larvae. For each treatment, 3 replicates of 16 neonate larvae per replicate were prepared. The plates were kept in the insect rearing chamber at 25 ± 2 °C and 60 ± 5-% relative humidity, with a 16:8 hours light:dark photoperiod. At day 3 (i.e. 3 days post start of bioassay), larvae were transferred to a new plate containing fresh treated (same dosage) leaf discs. The leaf material was refreshed every other day from day 5 onwards. The bioassay was scored on mortality and average weight Negative controls were leaf discs treated with bacteria harbouring plasmid pGN29 (empty vector) and leaf only.

A clear increase in mortality of *P. cochleariae* larvae with time was shown after the insects were fed on oilseed rape leaves treated with a suspension of RNaseIII-deficient *E*. *coli* strain AB309-105 containing plasmid pGCDJ001, whereas very little or no insect mortality was observed in the case of bacteria with plasmid pGN29 or leaf only control **(****Figure 3-PC****).**

### Plant-based bioassays

Whole plants are sprayed with suspensions of chemically induced bacteria expressing dsRNA prior to feeding the plants to MLB. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. MLB are placed on each treated plant in the cage. Plants are treated with a suspension of *E*. *coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E*. *coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 5: Epilachna varivetis (Mexican bean beetle)

### A. Cloning Epilachna varivetis partial gene sequences

High quality, intact RNA was isolated from 4 different larval stages of *Epilachna varivetis* (Mexican bean beetle; source: Thomas Dorsey, Supervising Entomologist, New Jersey Department of Agriculture, Division of Plant Industry, Bureau of Biological Pest Control, Phillip Alampi Beneficial Insect Laboratory, PO Box 330, Trenton, New Jersey 08625-0330, USA) using TRIzol Reagent . Genomic DNA present in the RNA preparation was removed by DNase treatment . cDNA was generated using a commercially available kit (SuperScript ^{™} III Reverse Transcriptase

To isolate cDNA sequences comprising a portion of the EV005, EV009, EV010, EV015 and EV016 genes, a series of PCR reactions with degenerate primers were performed using Amplitaq Gold .

The sequences of the degenerate primers used for amplification of each of the *Epilachna varivetis* target genes are given in **Table 2-EV**, including cDNA sequences obtained. These primers were used in respective PCR reactions . The resulting PCR fragments were analyzed on agarose gel, purified (QIAquick Gel Extraction), cloned into the pCR4/TOPO vector (Cat. Nr. K4530-20, Invitrogen), and sequenced. The sequences of the resulting PCR products are represented by the respective SEQ ID NO:s as given in **Table 2-EV** and are referred to as the partial sequences. The corresponding partial amino acid sequences are represented by the respective SEQ ID NO:s as given in **Table 3-EV**, where the start of the reading frame is indicated in brackets.

### B. dsRNA production of the Epilachna varivetis genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-EV**. The anti-sense T7 template was generated using specific forward and specific T7 reverse primers in a PCR reaction. The sequences of the respective primers for amplifying the anti-sense template for each of the target genes are given in **Table 8-EV**. The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-EV**.

### C. Laboratory trials to test dsRNA targets using bean leaf discs for activity against Epilachna varivetis larvae

The example provided below is an exemplification of the finding that the Mexican bean beetle (MBB) larvae are susceptible to orally ingested dsRNA corresponding to own target genes.

To test the different double-stranded RNA samples against MBB larvae, a leaf disc assay was employed using snap bean (*Phaseolus vulgaris* variety Montano; source: Aveve NV, Belgium) leaf material as food source. The same variety of beans was used to maintain insect cultures in the insect chamber at 25 ± 2 °C and 60 ± 5 % relative humidity with a photoperiod of 16h light/8h dark. Discs of approximately 1.1 cm in diameter (or 0.95 cm²) were cut out off leaves of 1- to 2-week old bean plants using a suitably-sized cork borer. Double-stranded RNA samples were diluted to 1 µg/µl in Milli-Q water containing 0.05% Triton X-100. Treated leaf discs were prepared by applying 25 µl of the diluted solution of target Ev005, Ev010, Ev015, Ev016 dsRNA and control gfp dsRNA or 0.05 % Triton X-100 on the adaxial leaf surface. The leaf discs were left to dry and placed individually in each of the 24 wells of a 24-well multiplate containing 1 ml of gellified 2 % agar which helps to prevent the leaf disc from drying out. A single neonate MBB larva was placed into each well of a plate, which was then covered with a multiwell plastic lid. The plate was divided into 3 replicates of 8 insects per replicate (row). The plate containing the insects and leaf discs were kept in an insect chamber at 25 ± 2 °C and 60 ± 5 % relative humidity with a photoperiod of 16h light/8h dark. The insects were fed on the leaf discs for 2 days after which the insects were transferred to a new plate containing freshly treated leaf discs. Thereafter, 4 days after the start of the bioassay, the insects were transferred to a petriplate containing untreated fresh bean leaves every day until day 10. Insect mortality was recorded at day 2 and every other day thereafter.

Feeding snap bean leaves containing surface-applied intact naked target dsRNAs to E. *varivestis* larvae resulted in significant increases in larval mortalities, as indicated in Figure 1. Tested double-stranded RNAs of targets Ev010, Ev015, & Ev016 led to 100 % mortality after 8 days, whereas dsRNA of target Ev005 took 10 days to kill all larvae. The majority of the insects fed on treated leaf discs containing control gfp dsRNA or only the surfactant Triton X-100 were sustained throughout the bioassay **(****Figure 1-EV****)**.

### D. Laboratory trials to test dsRNA targets using bean leaf discs for activity against Epilachna varivestis adults

The example provided below is an exemplification of the finding that the Mexican bean beetle adults are susceptible to orally ingested dsRNA corresponding to own target genes.

In a similar bioassay set-up as for Mexican bean beetle larvae, adult MBBs were tested against double-stranded RNAs topically-applied to bean leaf discs. Test dsRNA from each target Ev010, Ev015 and Ev016 was diluted in 0.05-% Triton X-100 to a final concentration of 0.1 µg/µl. Bean leaf discs were treated by topical application of 30 µl of the test solution onto each disc. The discs were allowed to dry completely before placing each on a slice of gellified 2-% agar in each well of a 24-well multiwell plate. Three-day-old adults were collected from the culture cages and fed nothing for 7-8 hours prior to placing one adult to each well of the bioassay plate (thus 24 adults per treatment). The plates were kept in the insect rearing chamber (under the same conditions as for MBB larvae for 24 hours) after which the adults were transferred to a new plate containing fresh dsRNA-treated leaf discs. After a further 24 hours, the adults from each treatment were collected and placed in a plastic box with dimensions 30 cm x 15 cm x 10 cm containing two potted and untreated 3-week-old bean plants. Insect mortality was assessed from day 4 until day 11.

All three target dsRNAs (Ev010, Ev015 and Ev016) ingested by adults of *Epilachna varivestis* resulted in significant increases in mortality from day 4 (4 days post bioassay start), as shown in **Figure 2(a)-EV.** From day 5, dramatic changes in feeding patterns were observed between insects fed initially with target-dsRNA-treated bean leaf discs and those that were fed discs containing control gfp dsRNA or surfactant Triton X-100. Reductions in foliar damage by MBB adults of untreated bean plants were clearly visible for all three targets when compared to gfp dsRNA and surfactant only controls, albeit at varying levels; insects fed target 15 caused the least damage to bean foliage **(Figure 2(b)-EV).**

### E. Cloning of a MBB gene fragment in a vector suitable for bacterial production of insect-active double-stranded RNA

What follows is an example of cloning a DNA fragment corresponding to an MBB gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-EV.** The template used is the pCR8/GW/topo vector containing any of target sequences. The primers are used in a PCR reaction The resulting PCR fragment is analyzed on agarose gel, purified, blunt-end cloned into *Srf*I-linearized pGNA49A vector (reference to WO00188121A1), and sequenced. The sequence of the resulting PCR product corresponds to the respective sequence as given in **Table 8-EV.** The recombinant vector harbouring this sequence is named pGBNJ00XX.

### F. Expression and production of a double-stranded RNA target in two strains of Escherichia coil: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, HL21(DE3).

### Transformation of AB309-105 and BL21 (DE3)

Three hundred ng of the plasmid are added to and gently mixed in a 50 µl aliquot of ice-chilled chemically competent *E.* coli strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21 (DE3)

Expression of double-stranded RNA from the recombinant vector, pGBNJ003, in the bacterial strain Tab309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15°C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimize the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### G. Laboratory trials to test Escherichia coli expressing dsRNA targets against Epilachna varivetis

### Plant-based bioassays

Whole plants are sprayed with suspensions of chemically induced bacteria expressing dsRNA prior to feeding the plants to MBB. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. MMB are placed on each treated plant in the cage. Plants are treated with a suspension of *E. coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E. coli* bacterial strain AB309-105 expressing target dsRNA from pGBNJ003 lead to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 6: Anthonomus grandis (cotton boll weevil)

### A. Cloning Anthonomus grandis partial sequences

High quality, intact RNA was isolated from the 3 instars of *Anthonomus grandis* (cotton boll weevil; source: Dr. Gary Benzon, Benzon Research Inc., 7 Kuhn Drive, Carlisle, Pennsylvania 17013, USA) using TRIzol Reagent . Genomic DNA present in the RNA preparation was removed by DNase treatment following the manufacturer's instructions. cDNA was generated using a commercially available kit (SuperScript^{™} III Reverse Transcriptase,

To isolate cDNA sequences comprising a portion of the AG001, AGO05, AG010, AG014 and AG016 genes, a series ofPCR reactions with degenerate primers were performed using Amplitaq Gold .

The sequences of the degenerate primers used for amplification of each of the genes are given in **Table 2-AG.** These primers were used in respective PCR reactions The resulting PCR fragments were analyzed on agarose gel, purified, cloned into the pCR8/GW/TOPO vector and sequenced. The sequences of the resulting PCR products are represented by the respective SEQ ID NO:s as given in **Table 2-AG** and are referred to as the partial sequences. The corresponding partial amino acid sequence are represented by the respective SEQ ID NO:s as given in **Table 3-AG.**

### B. dsRNA production of the Anthonomus grandis (cotton boll weevil) genes

dsRNA was synthesized in milligram amounts using the commercially available kit T7 Ribomax^{™} Express RNAi System.First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-AG.** A touchdown PCR was performed . The anti-sense T7 template was generated using specific forward and specific T7 reverse primers in a PCR reaction . The sequences of the respective primers for amplifying the anti-sense template for each of the target genes are given in **Table 8-AG.** The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate, . The sense strand of the resulting dsRNA for each of the target genes is given in **Table 8-AG.**

### C. Laboratory trials to test dsRNA targets, using artificial diet for activity against the larvae of the house cricket, Acheta domesticus

House crickets, *Acheta domesticus*, were maintained at Insect Investigations Ltd. (origin: Blades Biological Ltd., Kent, UK). The insects were reared on bran pellets and cabbage leaves. Mixed sex nymphs of equal size and no more than 5 days old were selected for use in the trial. Double-stranded RNA was mixed with a wheat-based pelleted rodent diet (rat and mouse standard diet, B & K Universal Ltd., Grimston, Aldbrough, Hull, UK). The diet, BK001P, contains the following ingredients in descending order by weight: wheat, soya, wheatfeed, barley, pellet binder, rodent 5 vit min, fat blend, dicalcium phosphate, mould carb. The pelleted rodent diet was finely ground and heat-treated in a microwave oven prior to mixing, in order to inactivate any enzyme components. All rodent diet was taken from the same batch in order to ensure consistency. The ground diet and dsRNA were mixed thoroughly and formed into small pellets of equal weight, which were allowed to dry overnight at room temperature.

Double-stranded RNA samples from targets and gfp control at concentrations 10 µg/µl are applied in the ratio 1 g ground diet plus 1 ml dsRNA solution, thereby resulting in an application rate of 10 mg dsRNA per g pellet. Pellets are replaced weekly. The insects are provided with treated pellets for the first three weeks of the trial. Thereafter untreated pellets are provided. Insects are maintained within lidded plastic containers (9 cm diameter, 4.5 cm deep), ten per container. Each arena contains one treated bait pellet and one water source (damp cotton wool ball), each placed in a separate small weigh boat. The water is replenished *ad lib* throughout the experiment.

Assessments are made at twice weekly intervals, with no more than four days between assessments, until all the control insects had either died or moulted to the adult stage (84 days). At each assessment the insects are assessed as live or dead, and examined for abnormalities. From day 46 onwards, once moulting to adult commences, all insects (live and dead) are assessed as nyumph or adult. Surviving insects are weighed on day 55 of the trial. Four replicates are performed for each of the treatments. During the trial the test conditions are 25 to 33 °C and 20 to 25 % relative humidity, with a 12:12 hour light:dark photoperiod.

### D. Cloning of a CBW gene fragment in a vector suitable for bacterial production of insect-active double-stranded RNA

What follows is an example of cloning a DNA fragment corresponding to CBW gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-AG.** The template used is the pCR8/GW/topo vector containing any of target sequences. The primers are used in a PCR reaction . The resulting PCR fragment is analyzed on agarose gel, purified, blunt-end cloned into *Srf* I-linearized pGNA49A vector (reference to WO00188121A1), and sequenced.- The sequence of the resulting PCR product corresponds to the respective sequence as given in **Table 8**. **AG.** The recombinant vector harbouring this sequence is named pGBNJ00XX.

### E. Expression and production of a double-stranded RNA target In two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21 (DE3).

### Transformation of AB309-105 and BL21 (DE3)

Three hundred ng of the plasmid are added to and gently mixed in a 50 µl aliquot of ice-chilled chemically competent *E.* coli strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Anthonomus grandis

### Plant-based bioassays

Whole plants are sprayed with suspensions of chemically induced bacteria expressing dsRNA prior to feeding the plants to CBW. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. CBW are placed on each treated plant in the cage. Plants are treated with a suspension of *E. coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E. coli* bacterial strain AB309-105 expressing target dsRNA from pGBNJ003 lead to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 7: Tribolium castaneum (red flour beetle)

### A. Cloning Tribolium castaneum partial sequences

High quality, intact RNA was isolated from all the different insect stages of *Tribolium castaneum* (red flour beetle; source: Dr. Lara Senior, Insect Investigations Ltd., Capital Business Park, Wentloog, Cardiff, CF3 2PX Wales, UK) using TRIzol Reagent . Genomic DNA present in the RNA preparation was removed by DNase treatment following the manafacturer's instructions. cDNA was generated using a commercially available kit (SuperScript^{™} III Reverse Transcriptase,

To isolate cDNA sequences comprising a portion of the TC001, TC002, TC010, TC014 and TC015 genes, a series ofPCR reactions with degenerate primers were performed using Amplitaq Golf .

The sequences of the degenerate primers used for amplification of each of the genes are given in **Table 2-TC.** These primers were used in respective PCR The resulting PCR fragments were analyzed on agarose gel, purified, cloned into the pCR8/GW/TOPO vector (Cat. Nr. K2500-20, Invitrogen), and sequenced. The sequences of the resulting PCR products are represented by the respective SEQ ID NO:s as given in **Table 2-TC** and are referred to as the partial sequences. The corresponding partial amino acid sequences are represented by the respective SEQ ID NO:s as given in **Table 3-TC.**

### B. dsRNA production of the Tribolium castaneum genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-TC.** The anti-sense T7 template was generated using specific forward and specific T7 reverse primers in a PCR reaction. The sequences of the respective primers for amplifying the anti-sense template for each of the target genes are given in **Table 8-TC.** The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-TC.**

### C. Laboratory trials to test dsRNA targets, using artificial diet for activity against Tribolium castaneum larvae

The example provided below is an exemplification of the finding that the red flour beetle (RFB) larvae are susceptible to orally ingested dsRNA corresponding to own target genes.

Red flour beetles, *Tribolium castaneum,* were maintained at Insect Investigations Ltd. (origin: Imperial College of Science, Technology and Medicine, Silwood Park, Berkshire, UK). Insects were cultured according to company SOP/251/01. Briefly, the beetles were housed in plastic jars or tanks. These have an open top to allow ventilation. A piece of netting was fitted over the top and secured with an elastic band to prevent escape. The larval rearing medium (flour) was placed in the container where the beetles can breed. The stored product beetle colonies were maintained in a controlled temperature room at 25 ± 3 °C with a 16:8 hour light:dark cycle.

Double-stranded RNA from target TC014 (with sequence corresponding to SEQ ID NO: -799) was incorporated into a mixture of flour and milk powder (wholemeal flour: powdered milk in the ratio 4:1) and left to dry overnight. Each replicate was prepared separately: 100 µl of a 10 µg/µl dsRNA solution (1 mg dsRNA) was added to 0.1 g flour/milk mixture. The dried mixture was ground to a fine powder. Insects were maintained within Petri dishes (55 mm diameter), lined with a double layer of filter paper. The treated diet was placed between the two filter paper layers. Ten first instar, mixed sex larvae were placed in each dish (replicate). Four replicates were performed for each treatment. Control was Milli-Q water. Assessments (number of survivors) were made on a regular basis. During the trial, the test conditions were 25 - 33 °C and 20 - 25-% relative humidity, with a 12:12 hour light:dark photoperiod.

Survival of larvae of *T. castaneum* over time on artificial diet treated with target TC014 dsRNA was significantly reduced when compared to diet only control, as shown in Figure 1.

### D. Cloning of a RFB gene fragment in a vector suitable for bacterial production of insect-active double-stranded RNA

What follows is an example of cloning a DNA fragment corresponding to an RFB gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-TC.** The template used is the pCR8/GW/topo vector containing any of target sequences. The primers are used in a PCR reaction . The resulting PCR fragment is analyzed on agarose gel, purified (QIAquick Gel Extraction kit, cloned into *Srf*I-linearized pGNA49A vector (reference to WO00188121A1), and sequenced. The sequence of the resulting PCR product corresponds to the respective sequence as given in **Table 8-TC.**- The recombinant vector harbouring this sequence is named pGBNJ00 XX.

### E. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21(DE3).

### Transformation of AB309-105 and BL21(DE3)

Three hundred ng of the plasmid are added to and gently mixed in a 50 µl aliquot of ice-chilled chemically competent *E.* coli strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Tribolium castaneum

### Plant-based bioassays

Whole plants are sprayed with suspensions of chemically induced bacteria expressing dsRNA prior to feeding the plants to RFB. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. RFB are placed on each treated plant in the cage. Plants are treated with a suspension of *E. coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E. coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 8: Myzus persicae (green peach aphid)

### A. Cloning Myzus persicae partial sequences

High quality, intact RNA was isolated from nymphs of *Myzus persicae* (green peach aphid; source: Dr. Rachel Down, Insect & Pathogen Interactions, Central Science Laboratory, Sand Hutton, York, YO41 1LZ, UK) using TRizol Reagent . Genomic DNA present in the RNA preparation was removed by DNase treatment . cDNA was generated using a commercially available kit (Superscript ^{™} III Reverse Transcriptase.

To isolate cDNA sequences comprising a portion of the MP001, MP002, MP010, MP016 and MP027 genes, a series ofPCR reactions with degenerate primers were performed using Amplitaq Gold.

The sequences of the degenerate primers used for amplification of each of the genes are given in **Table 2-MP**. These primers were used in respective PCR. The resulting PCR fragments were analyzed on agarose gel, purified, cloned into the pCR8/GW/TOPO vector (Cat. Nr. K2500-20, Invitrogen), and sequenced. The sequences of the resulting PCR products are represented by the respective SEQ ID NO:s as given in **Table 2-MP** and are referred to as the partial sequences. The corresponding partial amino acid sequences are represented by the respective SEQ ID NO:s as given in **Table 3-MP**.

### B. dsRNA production of Myzus persicae genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-MP**. A touchdown PCR was performed. The anti-sense T7 template was generated using specific forward and specific T7 reverse primers in a PCR reaction with the same conditions as described above. The sequences of the respective primers for amplifying the anti-sense template for each of the target genes are given in **Table 8-MP**. The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate The sense strand of the resulting dsRNA for each of the target genes is given in **Table 8-MP**.

### C. Laboratory trials to test dsRNA targets using liquid artificial diet for activity against Myzus persicae

Liquid artificial diet for the green peach aphid, *Myzus persicae*, was prepared based on the diet suitable for pea aphids (*Acyrthosiphon pisum*), as described by Febvay et al. (1988) [Influence of the amino acid balance on the improvement of an artificial diet for a biotype of Acyrthosiphon pisum (Homoptera: Aphididae). Can. J. Zool. 66: 2449-2453], but with some modifications. The amino acids component of the diet was prepared as follows: in mg/100ml, alanine 178.71, beta-alanine 6.22, arginine 244.9, asparagine 298.55, aspartic acid 88.25, cysteine 29.59, glutamic acid 149.36, glutamine 445.61, glycine 166.56, histidine 136.02, isoleucine 164.75, leucine 231.56, lysine hydrochloride 351.09, methionine 72.35, ornithine (HCl) 9.41, phenylalanine 293, proline 129.33, serine 124.28, threonine 127.16, tryptophane 42.75, tyrosine 38.63, L-valine 190.85. The amino acids were dissolved in 30 ml Milli-Q H₂O except for tyrosine which was first dissolved in a few drops of 1 M HCl before adding to the amino acid mix. The vitamin mix component of the diet was prepared as a 5 x concentrate stock as follows: in mg/L, amino benzoic acid 100, ascorbic acid 1000, biotin 1, calcium panthothenate 50, choline chloride 500, folic acid 10, myoinositol 420, nicotinic acid 100, pyridoxine hydrochloride 25, riboflavin 5, thiamine hydrochloride 25. The riboflavin was dissolved in 1 ml H2O at 50 °C and then added to the vitamin mix stock. The vitamin mix was aliquoted in 20 ml per aliquot and stored at -20 °C. One aliquot of vitamin mix was added to the amino acid solution. Sucrose and MgSO₄.7H₂O was added with the following amounts to the mix: 20 g and 242 mg, respectively. Trace metal stock solution was prepared as follows: in mg/100ml, CuSO₄.5H₂O 4.7, FeCl₃.6H₂O 44.5, MnCl₂.4H2O 6.5, NaCl 25.4, ZnCl₂ 8.3. Ten ml of the trace metal solution and 250 mg KH₂PO₄ was added to the diet and Milli-Q water was added to a final liquid diet volume of 100 ml. The pH of the diet was adjusted to 7 with 1 M KOH solution. The liquid diet was filter-sterilised through an 0.22 µm filter disc (Millipore).

Green peach aphids (*Myzus persicae*; source: Dr. Rachel Down, Insect & Pathogen Interactions, Central Science Laboratory, Sand Hutton, York, YO41 1LZ, UK) were reared on 4- to 6-week-old oilseed rape (*Brassica napus* variety SW Oban; source: Nick Balaam, Sw Seed Ltd., 49 North Road, Abington, Cambridge, CB1 6AS, LTK) in aluminium-framed cages containing 70 µm mesh in a controlled environment chamber with the following conditions: 23 ±2 °C and 60 ±5 % relative humidity, with a 16:8 hours light:dark photoperiod.

One day prior to the start of the bioassay, adults were collected from the rearing cages and placed on fresh detached oilseed rape leaves in a Petri dish and left overnight in the insect chamber. The following day, first-instar nymphs were picked and transferred to feeding chambers. A feeding chamber comprised of 10 first instar nymphs placed in a small Petri dish (with diameter 3 cm) covered with a single layer of thinly stretched parafilm M onto which 50 µl of diet was added. The chamber was sealed with a second layer of parafilm and incubated under the same conditions as the adult cultures. Diet with dsRNA was refreshed every other day and the insects' survival assessed on day 8 i.e. 8^{th} day post bioassay start. Per treatment, 5 bioassay feeding chambers (replicates) were set up simultaneously. Test and control (gfp) dsRNA solutions were incorporated into the diet to a final concentration of 2 µg/µl. The feeding chambers were kept at 23 ±2 °C and 60 ±5 % relative humidity, with a 16:8 hours light: dark photoperiod. A Mann-Whitney test was determined by GraphPad Prism version 4 to establish whether the medians do differ significantly between target 27 (MP027) and gfp dsRNA.

In the bioassay, feeding liquid artificial diet supplemented with intact naked dsRNA from target 27 (SEQ ID NO: 1061) to nymphs of *Myzus persicae* using a feeding chamber, resulted in a significant increase in mortality, as shown in Figure 1. Average percentage survivors for target 27, gfp dsRNA and diet only treatment were 2, 34 and 82, respectively. Comparison of target 027 with gfp dsRNA groups using the Mann-Whitney test resulted in an one-tailed P-value of 0.004 which indicates that the median of target 027 is significantly different (P < 0.05) from the expected larger median of gfp dsRNA. The green peach aphids on the liquid diet with incorporated target 27 dsRNA were noticeably smaller than those that were fed on diet only or with gfp dsRNA control (data not presented).

### D. Cloning of a GPA gene fragment in a vector suitable for bacterial production of insect-active double-stranded RNA

What follows is an example of cloning a DNA fragment corresponding to a GPA gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-MP.** The template used is the pCR8/GW/topo vector containing any of target sequences. The primers are used in a PCR reaction The resulting PCR fragment is analyzed on agarose gel, purified (QIAquick Gel Extraction kit), blunt-end cloned into *Srf* I-linearized pGNA49A vector (reference to WO00188121A1), and sequenced. The sequence of the resulting PCR product corresponds to the respective sequence as given in **Table 8-MP**. The recombinant vector harbouring this sequence is named pGBNJ00XX.

### E. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21(DE3).

### Transformation of AB309-105 and BL21(DE3)

Three hundred ng of the plasmid are added to and gently mixed in a 50 µl aliquot of ice-chilled chemically competent *E.* coli strain AB309-105 or BL21(DE3). The cells are incubated on ice for 20 minutes before subjecting them to a heat shock treatment of 37°C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Myzus persicae

### Plant-based bioassays

Whole plants are sprayed with suspensions of chemically induced bacteria expressing dsRNA prior to feeding the plants to GPA. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. GPA are placed on each treated plant in the cage. Plants are treated with a suspension of *E. coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E. coli* bacterial strain AB309-105 expressing target dsRNA from pGBNJ003 lead to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 9: Nilaparvata lugens (Brown Plant Hopper)

### A. Cloning Nilaparvata lugens partial sequences

From high quality total RNA of *Nilaparvata lugens* (source: Dr. J. A. Gatehouse, Dept. Biological Sciences, Durham University, UK) cDNA was generated using a commercially available kit (SuperScriptTM III Reverse Transcriptase ).

To isolate cDNA sequences comprising a portion of the *Nilaparvata lugens* NL001, NL002, NL003, NL004, NL005, NL006, NL007, NL008, NL009, NL010, NL011, NL012, NL013, NL014, NL015, NL016, NL018, NL019, NL021, NL022, and NL027 genes, a series of PCR reactions with degenerate primers were performed using Amplitaq Gold.

The sequences of the degenerate primers used for amplification of each of the genes are given in **Table 2-NL.** These primers were used in respective PCR . The resulting PCR fragments were analyzed on agarose gel, purified , cloned into the pCR8/GW/topo vector (Cat. Nr. K2500 20, Invitrogen), and sequenced.- The sequences of the resulting PCR products are represented by the respective SEQ ID NO:s as given in **Table 2-NL** and are referred to as the partial sequences.- The corresponding partial amino acid sequences are represented by the respective SEQ ID NO:s as given in **Table 3-NL.**

### B. Cloning of a partial sequence of the Nilaparvata lugens NL023 gene via EST sequence

From high quality total RNA of *Nilaparvata lugens* (source: Dr. J. A. Gatehouse, Dept. Biological Sciences, Durham University, UK) cDNA was generated using a commercially available kit (SuperScript^{™} III Reverse Transcriptase ).

A partial cDNA sequence, NL023, was amplified from *Nilaparvala lugens* cDNA which corresponded to a *Nilaparvata lugens* EST sequence in the public database Genbank with accession number CAH65679.2. To isolate cDNA sequences comprising a portion of the NL023 gene, a series of PCR reactions with EST based specific primers were performed using PerfectShot™ ExTaq.

For NL023, the specific primers oGBKW002 and oGBKW003 (represented herein as SEQ ID NO: 1157 and SEQ ID NO: 1158, respectively) were used in two independent PCR . The resulting PCR products were analyzed on agarose gel, purified , cloned into the pCR4-TOPO vector (Cat N°. K4575-40, Invitrogen) and sequenced. The consensus sequence resulting from the sequencing of both PCR products is herein represented by SEQ ID NO: -1111 and is referred to as the partial sequence of the NL023 gene. The corresponding partial amino acid sequence is herein reperesented as SEQ ID NO: 1112.

### C. dsRNA production of Nilaparvata lugens genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in Table 4. The anti-sense T7 template was generated using specific forward and specific T7 reverse primers in a PCR reaction. The sequences of the respective primers for amplifying the anti-sense template for each of the target genes are given in **Table 4-NL.** The resulting PCR products were analyzed on agarose gel and purified by PCR purification kit . The generated T7 forward and reverse templates were mixed to be transcribed and the resulting RNA strands were annealed, DNase and RNase treated, and purified by sodium acetate, but with RNA pellet washed twice in 70% ethanol. The sense strand of the resulting dsRNA for each of the target genes is given in **Table 8-NL.**

The template DNA used for the PCR reactions with T7 primers on the green fluorescent protein (gfp) control was the plasmid pPD96.12 (the Fire Lab, http://genome-www.stanford.edu/group/fire/), which contains the wild-type gfp coding sequence interspersed by 3 synthetic introns. Double-stranded RNA was synthesized using the commercially available kit T7 RiboMAX^{™} Express RNAi System (Cat.N°. P1700, Promega). First two separate single 5' T7 RNA polymerase promoter templates were generated in two separate PCR reactions, each reaction containing the target sequence in a different orientation relative to the T7 promoter. For gfp, the sense T7 template was generated using the specific T7 FW primer oGAU183 and the specific RV primer oGAU182 (represented herein as SEQ ID NO: 236 and SEQ ID NO: 237 , respectively) in a PCR reaction The anti-sense T7 template was generated using the specific FW primer oGAU181 and the specific T7 RV primer oGAU184 (represented herein as SEQ ID NO: 238 and SEQ ID NO: 239-, respectively) in a PCR reaction. The resulting PCR products were analyzed on agarose gel and purified . The generated T7 FW and RV templates were mixed to be transcribed and the resulting RNA strands were annealed, DNase and RNase treated, and purified by precipitation with sodium acetate and isopropanol, but with RNA pellet washed twice in 70% ethanol. The sense strands of the resulting dsRNA is herein represented by SEQ ID NO: 235.

### D. Laboratory trials to screen dsRNA targets using liquid artificial diet for activity against Nilaparvata lugens

Liquid artificial diet (MMD-1) for the rice brown planthopper, *Nilaparvata lugens*, was prepared as described by Koyama (1988) [Artificial rearing and nutritional physiology of the planthoppers and leafhoppers (Homoptera: Delphacidae and Deltocephalidae) on a holidic diet. JARQ 22: 20-27], but with a modification in final concentration of diet component sucrose: 14.4-% (weight over volume) was used. Diet components were prepared as separate concentrates: 10 x mineral stock (stored at 4 °C), 2 x amino acid stock (stored at -20 °C) and 10 x vitamin stock (stored at -20 °C). The stock components were mixed immediately prior to the start of a bioassay to 4/3 x concentration to allow dilution with the test dsRNA solution (4 x concentration), pH adjusted to 6.5, and filter-sterilised into approximately 500 µl aliquots.

Rice brown planthopper (*Nilaparvata lugens*) was reared on two-to-three month old rice (*Oryza sativa* cv Taichung Native 1) plants in a controlled environment chamber. 27 ± 2 °C, 80-% relative humidity, with a 16:8 hours light:dark photoperiod. A feeding chamber comprised 10 first or second instar nymphs placed in a small petri dish (with diameter 3 cm) covered with a single layer of thinly stretched parafilm M onto which 50 µl of diet was added. The chamber was sealed with a second layer of parafilm and incubated under the same conditions as the adult cultures but with no direct light exposure. Diet with dsRNA was refreshed every other day and the insects' survival assessed daily. Per treatment, 5 bioassay feeding chambers (replicates) were set up simultaneously. Test and control (gfp) dsRNA solutions were incorporated into the diet to a final concentration of 2 mg/ml. The feeding chambers were kept at 27 ± 2 °C, 80-% relative humidity, with a 16:8 hours light:dark photoperiod. Insect survival data were analysed using the Kaplan-Meier survival curve model and the survival between groups were compared using the logrank test (Prism version 4.0).

Feeding liquid artificial diet supplemented with intact naked dsRNAs to *Nilaparvata lugens in vitro* using a feeding chamber resulted in significant increases in nymphal mortalities as shown in four separate bioassays **(Figures 1(a)-(d)-NL****; Tables 10(a)-(d-)-NL)**. These results demonstrate that dsRNAs corresponding to different essential BPH genes showed significant toxicity towards the rice brown planthopper.

Effect of gfp dsRNA on BPH survival in these bioassays is not significantly different to survival on diet only

**Tables 10(a)-(d-)-NL** show a summary of the survival of *Nilaparvata lugens* on artificial diet supplemented with 2 mg/ml (final concentration) of the following targets; in **Table 10(a)-NL**: NL002, NL003, NL005, NL010; in **Table 10(b)-NL** NL009, NL016; in **Table 10(c)-NL** NL014, NL018; and in **Table 10(d)-NL** NL013, NL015, NL021. In the survival analysis column, the effect of RNAi is indicated as follows: + = significantly decreased survival compared to gfp dsRNA control (alpha < 0.05); - = no significant difference in survival compared to gfp dsRNA control. Survival curves were compared (between diet only and diet supplemented with test dsRNA, gfp dsRNA and test dsRNA, and diet only and gfp dsRNA) using the logrank test.

### E. Laboratory trials to screen dsRNAs at different concentrations using artificial diet for activity against Nilaparvata lugens

Fifty µl of liquid artificial diet supplemented with different concentrations of target NL002 dsRNA, namely 1, 0.2, 0.08, and 0.04 mg/ml (final concentration), was applied to the brown planthopper feeding chambers. Diet with dsRNA was refreshed every other day and the insects' survival assessed daily. Per treatment, 5 bioassay feeding chambers (replicates) were set up simultaneously. The feeding chambers were kept at 27 ± 2 °C, 80-% relative humidity, with a 16:8 hours light:dark photoperiod. Insect survival data were analysed using the Kaplan-Meier survival curve model and the survival between groups were compared using the logrank test (Prism version 4.0).

Feeding liquid artificial diet supplemented with intact naked dsRNAs of target NL002 at different concentrations resulted in significantly higher BPH mortalities at final concentrations of as low as 0.04 mg dsRNA per ml diet when compared with survival on diet only, as shown in **Figure 2****-NL** and **Table 11-NL. Table 11-NL** summarizes the survival of *Nilaparvata lugens* artificial diet feeding trial supplemented with 1, 0.2, 0.08, & 0.04 mg/ml (final concentration) of target NL002. In the survival analysis column the effect of RNAi is indicated as follows: + = significantly decreases survival compared to diet only control (alpha < 0.05); - = no significant differences in survival compared to diet only control. Survival curves were compared using the logrank test.

### F. Cloning of a BPH gene fragment in a vector suitable for bacterial production of insect-active double-stranded RNA

What follows is an example of cloning a DNA fragment corresponding to a BPH gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in Table 8. The template used is the pCR8/GW/topo vector containing any of target sequences. The primers are used in a PCR reaction The resulting PCR fragment is analyzed on agarose gel, purified, blunt-end cloned into *Srf* I-linearized pGNA49A vector (reference to WO00188121A1), and sequenced. The sequence of the resulting PCR product corresponds to the respective sequence as given in **Table 8-NL**. The recombinant vector harbouring this sequence is named pGBNJ00.

### G. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21 (DE3).

### Transformation of AB309-105 and HL21(DE3)

Three hundred ng of the plasmid are added to and gently mixed in a 50 µl aliquot of ice-chilled chemically competent *E. coli* strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### H. Laboratory trials to test Escherichia coli expressing dsRNA targets against Nilaparvata lugens

### Plant-based bioassays

Whole plants are sprayed with suspensions of chemically induced bacteria expressing dsRNA prior to feeding the plants to BPH. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. BPH are placed on each treated plant in the cage. Plants are treated with a suspension of *E*. *coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E. coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 10: Chilo suppressalis (rice striped stem borer)

### A. Cloning of partial sequence of the Chilo suppressalis genes via family PCR

High quality, intact RNA was isolated from the 4 different larval stages of *Chilo suppressalis* (rice striped stem borer) using TRIzol Reagent . Genomic DNA present in the RNA preparation was removed by DNase treatment . cDNA was generated using a commercially available kit (SuperScript ^{™} III Reverse Transcriptase.

To isolate cDNA sequences comprising a portion of the CS001, CS002, CS003, CS006, CS007, CS009, CS011, CS013, CS014, CS015, CS016 and CS018 genes, a series of PCR reactions with degenerate primers were performed using Amplitaq Gold.

The sequences of the degenerate primers used for amplification of each of the genes are given in **Table 2-CS**. These primers were used in respective PCR reactions . The resulting PCR fragments were analyzed on agarose gel, purified , cloned into the pCR4/TOPO vector (Cat. Nr. K2500-20, Invitrogen), and sequenced. The sequences of the resulting PCR products are represented by the respective SEQ ID NO:s as given in **Table 2-CS** and are referred to as the partial sequences. The corresponding partial amino acid sequences are represented by the respective SEQ ID NO:s as given in **Table 3-CS**.

### B. dsRNA production of the Chilo suppressalis genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-CS**.

The antisense T7 template was generated using specific forward and specific T7 reverse primers in a PCR reaction. The sequences of the respective primers for amplifying the anti-sense template for each of the target genes are given in **Table 8-CS.** The resulting PCR products were analyzed on agarose gel and purified by PCR purification kit and NaClO₄ precipitation.- The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-CS**.

### C. Laboratory trials to test dsRNA targets, using artificial diet for activity against Chilo suppressalis larvae

Rice striped stem borers, *Chilo suppressalis*, (origin: Syngenta, Stein, Switzerland) were maintained on a modified artificial diet based on that described by Kamano and Sato, 1985 (in: Handbook of Insect Rearing. Volumes I & II. P Singh and RF Moore, eds., Elsevier Science Publishers, Amsterdam and New York, 1985, pp 448). Briefly, a litre diet was made up as follows: 20 g of agar added to 980 ml of Milli-Q water and autoclaved; the agar solution was cooled down to approximately 55 °C and the remaining ingredients were added and mixed thoroughly: 40 g corn flour (Polenta), 20 g cellulose, 30 g sucrose, 30 g casein, 20 g wheat germ (toasted), 8 g Wesson salt mixture, 12 g Vanderzant vitamin mix, 1.8 g sorbic acid, 1.6 g nipagin (methylparaben), 0.3 g aureomycin, 0.4 g cholesterol and 0.6 g L-cysteine. The diet was cooled down to approx. 45 °C and poured into rearing trays or cups. The diet was left to set in a horizontal laminair flow cabin. Rice leaf sections with oviposited eggs were removed from a cage housing adult moths and pinned to the solid diet in the rearing cup or tray. Eggs were left to hatch and neonate larvae were available for bioassays and the maintenance of the insect cultures. During the trials and rearings, the conditions were 28 ± 2 °C and 80 ± 5 % relative humidity, with a 16:8 hour light:dark photoperiod.

The same artificial diet is used for the bioassays but in this case the diet is poured equally in 24 multiwell plates, with each well containing 1 ml diet. Once the diet is set, the test formulations are applied to the diet's surface (2 cm²), at the rate of 50 µl of 1 µg/µl dsRNA of target. The dsRNA solutions are left to dry and two first instar moth larvae are placed in each well. After 7 days, the larvae are transferred to fresh treated diet in multiwell plates. At day 14 (i.e. 14 days post bioassay start) the number of live and dead insects is recorded and examined for abnormalities. Twenty-four larvae in total are tested per treatment.

An alternative bioassay is performed in which treated rice leaves are fed to neonate larvae of the rice striped stem borer. Small leaf sections of *Indica* rice variety Taichung native 1 are dipped in 0.05 % Triton X-100 solution containing 1 µg/µl of target dsRNA, left to dry and each section placed in a well of a 24 multiwell plate containing gellified 2 % agar. Two neonates are transferred from the rearing tray to each dsRNA treated leaf section (24 larvae per treatment). After 4 and 8 days, the larvae are transferred to fresh treated rice leaf sections. The number of live and dead larvae are assessed on days 4, 8 and 12; any abnormalities are also recorded.

### D. Cloning of a SSB gene fragment in a vector suitable for bacterial production of insect-active double-stranded RNA

What follows is an example of cloning a DNA fragment corresponding to an SSB gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in Table 8. The template used is the pCR8/GW/topo vector containing any of target sequences. The primers are used in a PCR reaction. The resulting PCR fragment is analyzed on agarose gel, purified, blunt-end cloned into *Srf* I-linearized pGNA49A vector (reference to WO00188121A1), and sequenced. The sequence of the resulting PCR product corresponds to the respective sequence as given in **Table 8-CS**. The recombinant vector harbouring this sequence is named pGBNJ00XX.

### E. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21 (DE3). Transformation of AB309-105 and BL21(DE3)

Three hundred ng of the plasmid are added to and gently mixed in a 50 µl aliquot of ice-chilled chemically competent E.. coli strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Chilo suppressalis

### Plant-based bioassays

Whole plants are sprayed with suspensions of chemically induced bacteria expressing dsRNA prior to feeding the plants to SSB. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. SSB are placed on each treated plant in the cage. Plants are treated with a suspension of *E. coli* AB309-105 harbouring the pGBNJ0001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E*. *coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the elective protection of plants/crops from insect damage by the use of a spray of a formulation consisting of bacteria expressing double-stranded RNA corresponding to an insect gene target.

### Example 911: Plutella xylostella (Diamondback moth)

### A. Cloning of a partial sequence of the Plutella xylostella

High quality, intact RNA was isolated from all the different larval stages of Plutella xylostella (Diamondback moth; source: Dr. Lara Senior, Insect Investigations Ltd., Capital Business Park, Wentloog, Cardiff, CF3 2PX, Wales, UK) using TRIzol Reagent.

Genomic DNA present in the RNA preparation was removed by DNase treatment. cDNA was generated using a commercially available kit (SuperScript ^{™} III Reverse Transcriptase).

To isolate cDNA sequences comprising a portion of the PX001, PX009, PX010, PX015, PX016 genes, a series of PCR reactions with degenerate primers were performed using Amplitaq Gold.

The sequences of the degenerate primers used for amplification of each of the genes are given in **Table 2-PX**. These primers were used in respective PCR. The resulting PCR fragments were analyzed on agarose gel, purified, cloned into the pCR8/GW/TOPO vector (Cat. Nr. K2500-20, Invitrogen) and sequenced. The sequences of the resulting PCR products are represented by the respective SEQ ID NO:s as given in **Table 2-PX** and are referred to as the partial sequences. The corresponding partial amino acid sequence are represented by the respective SEQ ID NO:s as given in **Table 3-PX**.

### B. dsRNA production of the Plutella xylostella genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-PX**. The anti-sense T7 template was generated using specific forward and specific T7 reverse primers in a PCR reaction. The sequences of the respective primers for amplifying the anti-sense template for each of the target genes are given in **Table 8-PX**. The resulting PCR products were analyzed on agarose gel and purified by PCR purification kit. The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-PX**.

### C. Laboratory trials to test dsRNA targets, using artificial diet for activity against Plutella xylostella larvae

Diamond-back moths, *Plutella xylostella*, were maintained at Insect Investigations Ltd. (origin: Newcastle University, Newcastle-upon-Tyne, UK). The insects were reared on cabbage leaves. First instar, mixed sex larvae (approximately 1 day old) were selected for use in the trial. Insects were maintained in Eppendorf tubes (1.5 ml capacity). Commercially available Diamond-back moth diet (Bio-Serv, NJ, USA), prepared following the manafacturer's instructions, was placed in the lid of each tube (0.25 ml capacity, 8 mm diameter). While still liquid, the diet was smoother over to remove excess and produce an even surface.

Once the diet has set the test formulations are applied to the diet's surface, at the rate of 25 µl undiluted formulation (1 µg/µl dsRNA of targets) per replicate. The test formulations are allowed to dry and one first instar moth larva is placed in each tube. The larva is placed on the surface of the diet in the lid and the tube carefully closed. The tubes are stored upside down, on their lids such that each larva remains on the surface of the diet Twice weekly the larvae are transferred to new Eppendorf tubes with fresh diet. The insects are provided with treated diet for the first two weeks of the trial and thereafter with untreated diet.

Assessments are made twice weekly for a total of 38 days at which point all larvae are dead. At each assessment the insects are assessed as live or dead and examined for abnormalities. Forty single larva replicates are performed for each of the treatments. During the trial the test conditions are 23 to 26 °C and 50 to 65-% relative humidity, with a 16:8 hour light:dark photoperiod.

### D. Cloning of a DBM gene fragment in a vector suitable for bacterial production of insect-active double-stranded RNA

What follows is an example of cloning a DNA fragment corresponding to a DBM gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in **Table 8-PX**. The template used is the pCR8/GW/topo vector containing any of target sequences. The primers are used in a PCR reaction.

The resulting PCR fragment is analyzed on agarose gel, purified, blunt-end cloned into *Srf* I-linearized pGNA49A vector (reference to WO00188121A1), and sequenced. The sequence of the resulting PCR product corresponds to the respective sequence as given in **Table 8-PX**. The recombinant vector harbouring this sequence is named pGBNJ00XX.

### E. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21(DE3).

### Transformation of AB309-105 and BL21(DE3)

Three hundred ng of the plasmid are added to and gently mixed in a 50 µl aliquot of ice-chilled chemically competent *E*. coli strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Plutella xylostella

### Plant-based bioassays

Whole plants are sprayed with suspensions of chemically induced bacteria expressing dsRNA prior to feeding the plants to DBM. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. DBM are placed on each treated plant in the cage. Plants are treated with a suspension of *E*. *coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between I and 10 ml s sprayed on the plant with the aid of a vaporizer. One plant is used per treatment in this trial. The number of survivors are counted and the weight of each survivor recorded.

Spraying plants with a suspension of *E*. *coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 12: Acheta domesticus (house cricket)

### A. Cloning Acheta domesticus partial sequences

High quality, intact RNA was isolated from all the different insect stages of *Acheta domesticus* (house cricket; source: Dr. Lara Senior, Insect Investigations Ltd., Capital Business Park, Wentloog, Cardiff, CF3 2PX, Wales, UK) using TRIzol Reagents. Genomic DNA present in the RNA preparation was removed by DNase treatment. cDNA was generated using a commercially available kit (SuperScript^{™} III Reverse Transcriptase.

To isolate cDNA sequences comprising a portion of the AD001, AD002, AD009, AD015 and AD016 genes, a series of PCR reactions with degenerate primers were performed using Amplitaq Gold.

The sequences of the degenerate primers used for amplification of each of the genes are given in **Table 2-AD**. These primers were used in respective PCR reactions resulting PCR fragments were analyzed on agarose gel, purified, cloned into the pCR8/GW/topo vector (Cat. Nr. K2500 20, Invitrogen) and sequenced. The sequences of the resulting PCR products are represented by the respective SEQ ID NO:s as given in **Table 2-AD** and are referred to as the partial sequences. The corresponding partial amino acid sequence are represented by the respective SEQ ID NO:s as given in **Table 3-AD**.

### B. dsRNA production of the Acheta domesticus genes

dsRNA was synthesized in milligram amounts using the commercially available kit T7 Ribomax^{™} Express RNAi System. First two separate single 5' T7 RNA polymerase promoter templates were generated in two separate PCR reactions, each reaction containing the target sequence in a different orientation relative to the T7 promoter.

For each of the target genes, the sense T7 template was generated using specific T7 forward and specific reverse primers. The sequences of the respective primers for amplifying the sense template for each of the target genes are given in **Table 8-AD**. The anti-sense T7 template was generated using specific forward and specific T7 reverse primers in a PCR reaction. The sequences of the respective primers for amplifying the anti-sense template for each of the target genes are given in **Table 8-AD**. The resulting PCR products were analyzed on agarose gel and purified by PCR purification kit and NaClO₄ precipitation. The generated T7 forward and reverse templates were mixed to be transcribed and the resulting RNA strands were annealed, DNase and RNase treated, and purified by sodium acetate. The sense strand of the resulting dsRNA for each of the target genes is given in **Table 8-AD**.

### C. Laboratory trials to test dsRNA targets, using artificial diet for activity against Acheta domesticus larvae

House crickets, *Acheta domesticus,* were maintained at Insect Investigations Ltd. (origin: Blades Biological Ltd., Kent, UK). The insects were reared on bran pellets and cabbage leaves. Mixed sex nymphs of equal size and no more than 5 days old were selected for use in the trial. Double-stranded RNA is mixed with a wheat-based pelleted rodent diet (rat and mouse standard diet, B & K Universal Ltd., Grimston, Aldbrough, Hull, UK). The diet, BK001P, contains the following ingredients in descending order by weight: wheat, soya, wheatfeed, barley, pellet binder, rodent 5 vit min, fat blend, dicalcium phosphate, mould carb. The pelleted rodent diet is finely ground and heat-treated in a microwave oven prior to mixing, in order to inactivate any enzyme components. All rodent diet is taken from the same batch in order to ensure consistency. The ground diet and dsRNA are mixed thoroughly and formed into small pellets of equal weight, which are allowed to dry overnight at room temperature.

Double-stranded RNA samples from targets and gfp control at concentrations 10 µg/µl were applied in the ratio 1 g ground diet plus 1 ml dsRNA solution, thereby resulting in an application rate of 10 mg dsRNA per g pellet. Pellets are replaced weekly. The insects are provided with treated pellets for the first three weeks of the trial. Thereafter untreated pellets are provided. Insects are maintained within lidded plastic containers (9 cm diameter, 4.5 cm deep), ten per container. Each arena contains one treated bait pellet and one water source (damp cotton wool ball), each placed in a separate small weigh boat. The water is replenished *ad lib* throughout the experiment.

Assessments are made at twice weekly intervals, with no more than four days between assessments, until all the control insects had either died or moulted to the adult stage (84 days). At each assessment the insects are assessed as live or dead, and examined for abnormalities. From day 46 onwards, once moulting to adult has commenced, all insects (live and dead) are assessed as nymph or adult. Surviving insects are weighed on day 55 of the trial. Four replicates are performed for each of the treatments. During the trial the test conditions are 25 to 33 °C and 20 to 25-% relative humidity, with a 12:12 hour light:dark photoperiod.

### D. Cloning of a HC gene fragment in a vector suitable for bacterial production of insect active double-stranded RNA

What follows is an example of cloning a DNA fragment corresponding to a HC gene target in a vector for the expression of double-stranded RNA in a bacterial host, although any vector comprising a T7 promoter or any other promoter for efficient transcription in bacteria, may be used (reference to WO0001846).

The sequences of the specific primers used for the amplification of target genes are provided in Table 8. The template used is the pCR8/GW/topo vector containing any of target sequences. The primers are used in a PCR reaction with the following conditions: 5 minutes at 98°C, followed by 30 cycles of 10 seconds at 98°C, 30 seconds at 55°C and 2 minutes at 72°C, followed by 10 minutes at 72°C. -The resulting PCR fragment is analyzed on agarose gel, purified blunt-end cloned into *Srf* I-linearized pGNA49A vector (reference to WO00188121A1), and sequenced.- The sequence of the resulting PCR product corresponds to the respective sequence as given in **Table 8-AD**.- The recombinant vector harbouring this sequence is named pGBNJ00XX.

### E. Expression and production of a double-stranded RNA target in two strains of Escherichia coli: (1) AB309-105, and, (2) BL21(DE3)

The procedures described below are followed in order to express suitable levels of insect-active double-stranded RNA of insect target in bacteria. An RNaseIII-deficient strain, AB309-105, is used in comparison to wild-type RNaseIII-containing bacteria, BL21 (DE3).

### Transformation of AB309-105 and BL21(DE3)

Three hundred ng of the plasmid are added to and gently mixed in a 50 µl aliquot of ice-chilled chemically competent *E*. coli strain AB309-105 or BL21 (DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGHNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

### F. Laboratory trials to test Escherichia coli expressing dsRNA targets against Acheta domestics

### Plant-based bioassays

Whole plants are sprayed with suspensions of chemically induced bacteria expressing dsRNA prior to feeding the plants to HC. The plants are grown from in a plant growth room chamber. The plants are caged by placing a 500 ml plastic bottle upside down over the plant with the neck of the bottle firmly placed in the soil in a pot and the base cut open and covered with a fine nylon mesh to permit aeration, reduce condensation inside and prevent insect escape. HC are placed on each treated plant in the cage. Plants are treated with a suspension of *E*. *coli* AB309-105 harbouring the pGBNJ001 plasmids or pGN29 plasmid. Different quantities of bacteria are applied to the plants: for instance 66, 22, and 7 units, where one unit is defined as 10⁹ bacterial cells in 1 ml of a bacterial suspension at optical density value of 1 at 600 nm wavelength. In each case, a total volume of between 1 and 10 ml s sprayed on the plant with the aid of a vaporizer. One plant is used per treatment in this trial. The number of survivors are counted and the weight of each survivor recorded.

Spraying plants with a suspension of *E*. *coli* bacterial strain AB309-105 expressing target dsRNA from pGBNJ003 leed to a dramatic increase in insect mortality when compared to pGN29 control. These experiments show that double-stranded RNA corresponding to an insect gene target sequence produced in either wild-type or RNaseIII-deficient bacterial expression systems is toxic towards the insect in terms of substantial increases in insect mortality and growth/development delay for larval survivors. It is also clear from these experiments that an exemplification is provided for the effective protection of plants/crops from insect damage by the use of a spray of a formulation consisting of bacteria expressing double-stranded RNA corresponding to an insect gene target.

### Example 13: Pyricu/aria grisea (rice blast)

### A. Cloning P. grisea partial sequences

High quality, intact RNA is isolated from different growth stages of *P. grisea* using TRIzol Reagent. Genomic DNA present in the RNA preparation is removed by DNase treatment following the manafacturer's instructions. cDNA is generated using a commercially available kit (SuperScript ^{™} III Reverse Transcriptase,

To isolate cDNA sequences comprising a portion of a target gene, PCR is performed with degenerate primers using Amplitaq Gold. The resultant PCR products are fractionated and sequenced.

### B. dsRNA production of P. grisea genes

dsRNA is synthesized in milligram amounts using a commercially available kit, such as T7 Ribomax^{™} Express RNAi System. The resulting PCR products are analyzed on an agarose gel and purified by a PCR purification kit and NaClO₄ precipitation.- The produced T7 forward and reverse templates are mixed and the resulting RNA strands are annealed, then DNase and RNase treated, and purified by sodium acetate.

### C. Expression and production of a double-stranded RNA target in two strains of Escherichia coli: (1) AB309-105, and, (2) BL21(DE3)

The procedures described below are followed in order to express suitable levels of fungal double-stranded RNA of fungal target in bacteria. An RNaseIII-deficient strain, AB309-105, is used in comparison to wild-type RNaseIII-containing bacteria, BL21(DE3).

### Transformation of AB309-105 and BL21 (DE3)

Three hundred ng of the plasmid are added to and gently mixed in a 50 µl aliquot of ice-chilled chemically competent *E. coli* strain AB309-105 or BL21(DE3). The cells are incubated on ice for 20 minutes before subjecting them to a heat shock treatment of 37 °C for 5 minutes, after which the cells are placed back on ice for a further 5 minutes. Four hundred and fifty µl of room temperature SOC medium is added to the cells and the suspension incubated on a shaker (250 rpm) at 37 °C for 1 hour. One hundred µl of the bacterial cell suspension is transferred to a 500 ml conical flask containing 150 ml of liquid Luria-Bertani (LB) broth supplemented with 100 µg/ml carbenicillin antibiotic. The culture is incubated on an Innova 4430 shaker (250 rpm) at 37 °C overnight (16 to 18 hours).

### Chemical induction of double-stranded RNA expression in AB309-105 and BL21(DE3)

Expression of double-stranded RNA from the recombinant vector, pGBNJ003, in the bacterial strain AB309-105 or BL21(DE3) is made possible since all the genetic components for controlled expression are present. In the presence of the chemical inducer isopropylthiogalactoside, or IPTG, the T7 polymerase will drive the transcription of the target sequence in both antisense and sense directions since these are flanked by oppositely oriented T7 promoters.

The optical density at 600 nm of the overnight bacterial culture is measured using an appropriate spectrophotometer and adjusted to a value of 1 by the addition of fresh LB broth. Fifty ml of this culture is transferred to a 50 ml Falcon tube and the culture then centrifuged at 3000 g at 15 °C for 10 minutes. The supernatant is removed and the bacterial pellet resuspended in 50 ml of fresh S complete medium (SNC medium plus 5 µg/ml cholesterol) supplemented with 100 µg/ml carbenicillin and 1 mM IPTG. The bacteria are induced for 2 to 4 hours at room temperature.

### Heat treatment of bacteria

Bacteria are killed by heat treatment in order to minimise the risk of contamination of the artificial diet in the test plates. However, heat treatment of bacteria expressing double-stranded RNA is not a prerequisite for inducing toxicity towards the insects due to RNA interference. The induced bacterial culture is centrifuged at 3000 g at room temperature for 10 minutes, the supernatant discarded and the pellet subjected to 80 °C for 20 minutes in a water bath. After heat treatment, the bacterial pellet is resuspended in 1.5 ml MilliQ water and the suspension transferred to a microfuge tube. Several tubes are prepared and used in the bioassays for each refreshment. The tubes are stored at -20 °C until further use.

**Table 1A**

| **C.elegans id** | **D. melanogaster id** | **description** | **devgen RNAi screen** |
|---|---|---|---|
| B0250.1 | CG1263 | large ribosomal subunit L8 protein. | Acute lethal or lethal |
| B0336.10 | CG3661 | large ribosomal subunit L23 protein. | or Acute lethal or lethal |
| B0336.2 | CG8385 | ADP-ribosylation factor | Acute lethal or lethal |
| B0464.1 | CG3821 | Putative aspartyl(D) tRNA synthetase. | Acute lethal or lethal |
| C01G8.5 | CG10701 | Ortholog of the ERM family of cytosketetal linkers | Acute lethal or lethal |
| C01H6.5 | CG33183 | Nuclear hormone receptor that is required in all larval molts | Acute lethal or lethal |
| C02C6.1 | CG18102 | Member of the DYNamin related gene class | Acute lethal or lethal |
| C03D6.8 | CG6764 | Large ribosomal subunit L24 | Acute lethal or lethal |
| C041F12.4 | C6253 | protein (Rlp24p) rpl-14 encodes a large ribosomal subunit L14 protein. | Acute lethal or lethal |
| C04H5.6 | CG10689 | Product with RNA helicase activity (EC:2.7.7.-) involved in nuclear mRNA splicing, via spliceosome which is a component of the spliceosome complex | Embryonic lethal or sterile |
| C1389.3 | CG14813 | Delta subunit of the coatomer (COPI) complex | Acute lethal or lethal |
| C17H12.14 | CG1088 | Member of the Vacuolar H ATPase gene class | Acute lethal or lethal |
| C26E6.4 | CG3180 | DNA-directed RNA polymerase II | Acute lethal or lethal |
| F23F12.6 | CG16916 | Triple A ATPase subunit of the 26S proteasome's 19S regulatory particle (RP) base subcomplex | Acute lethal or lethal |
| F5789.10 | CG10149 | class Member of the proteasome Regulatory Particle, Non-ATPase-like gene | Acute lethal or lethal |
| K11D9.2 | CG3725 | sarco-endoplasmic reticulum | Embryonic lethal or sterile |
| T20G5.1 | CG9012 | Ca[2+] ATPase homolog Clathrin heavy chain | Acute lethal or lethal |
| T20H4.3 | C5394 | Predicted cytoplasmic prolyl-tRNA synthetase (ProRS) | Acute lethal or lethal |
| T21E12.4 | CG7507 | Cytoplasmic dynein heavy chain homolog | Acute lethal or lethal |
| C05C10.3 | CG1140 | Ortholoque to the human gene 3-OXOACID COA TRANSFERASE | Acute lethal or lethal |
| C09D4.5 | CG2746 | Ribosomal protein L19, structural constituent of ribosome involved in protein biosynthesis which is localised to the ribosome | Acute lethal or lethal |
| C09E10.2 | CG31140 | Orthologue of diacylglyerol kinase involved in movement, egg laying, and synaptic transmission, and is expressed in neurons. | Acute lethal or lethal |
| C13B9.3 | CG14813 | Delta subunit of the coatomer (COPI) | Acute lethal or lethal |
| C1489.7 | CG12775 | Large ribosomal subunit L21 protein (RPL-21 involved in protein biosynthesis | Acute lethal or lethal |
| C15H11.7 | CG30382 | Type 6 alpha subunit of the 26S proteasome's 20S protease core particle (CP) | Acute lethal or lethal |
| C17E4.9 | CG9261 | Protein involved with Na+/K+- exchanging ATPase complex | Embryonic lethal or sterile |
| C17H12.14 | CG1088 | V-ATPase E subunit | Acute lethal or lethal |
| C23G10.4 | CG11888 | Non-ATPase subunit of the 26S proteasome's 19S regulatory paritcle base subcomplex (RPN-2) | Acute lethal or lethal |
| C26D10.2 | CG7269 | Product with helicase activity involved in nuclear RNA splicing, via spliceosome which is localized to the nucleus | Acute lethal or lethal |
| C26E6.4 | CG3180 | RNA polymerase II 140kD subunit (Rpl11 40), DNA-directed RNA polymerase activity (EC:2.7.7.6) involved in transcription from Pol II promoter which is a component of the DNA-directed RNA polymerase II, core complex | Acute lethal or lethal |
| C26F1.4 | CG15697 | Product with function in protein biosynthesis and ubiquitin in protein degradation. | Acute lethal or lethal |
| C30C11.1 | CG12220 | Unknown function | Acute lethal or lethal |
| C30C11.2 | CG10484 | Member of the proteasome Regulatory Particle, Non-ATPase-like gene class | Acute lethal or lethal |
| C36A4.2 | CG13977 | cytochrome P450 | Acute lethal or lethal |
| C37C3.6 | CG33103 | Orthologous to thrombospondin, papilin and lacunin | Acute lethal or lethal |
| C37H5.8 | CG8542 | Member of the Heat Shock Protein gene class | Acute lethal or lethal |
| C39F7.4 | CG3320 | Rab-protein 1 involved in cell adhesion | Acute lethal or lethal |
| C41 C4.8 | CG2331 | Transitional endoplasmic reticulum ATPase TER94, Golgi organization and bioqenesis | Growth delay or arrested in growth |
| C42D8.5 | CG8827 | ACE-like protein | Acute lethal or lethal |
| C47E12.5 | CG1782 | Ubiquitin-activating enzyme,function in an ATP-dependent reaction that activates ubiquitin prior to its conjugation to proteins that will subsequently be degraded by the 26S proteasome. | Acute lethal or lethal |
| C47E8.5 | CG1242 | Member of the abnormal DAuer Formation gene class | Acute lethal or lethal |
| C49H3.11 | CG5920 | Small ribosomal subunit S2 protein. | Acute lethal or lethal |
| C52E4.4 | CG1341 | Member of the proteasome Regulatory Particle, ATPase-like gene class | Acute lethal or lethal |
| C56C10.3 | CG8055 | Carrier protein with putatively involved in intracellular protein transport | Growth delay or arrested in growth |
| CD4.6 | CG4904 | Type 1 alpha Subunit of the 26S proteasome's 20S protease core particle (CP). | Acute lethal or lethal |
| D1007.12 | CG9282 | Large ribosomal subunit L24 protein. | Acute lethal or lethal |
| D1054.2 | CG5266 | of the Proteasome Alpha Subunit gene class | Acute lethal or lethal |
| D1081.8 | CG6905 | MYB transforming protein | Acute lethal or lethal |
| F07D10.1 | CG7726 | Large ribosomal subunit L11 protein (RPL-11.2 ) involved in protein biosynthesis. | Acute lethal or lethal |
| F11C3.3 | CG17927 | Muscle myosin heavy chain (MHC B) | Acute lethal or lethal |
| F13B10.2 | CG4863 | Large ribosomal subunit L3 protein (rpl-3) | Acute lethal or lethal |
| F16A11.2 | CG9987 | Mathanococcus hypothetical protein 0682 like | Acute lethal or lethal |
| F20B6.2 | CG17369 | V-ATPase B subunit | growth delay or arrested in growth |
| F23F12.6 | CG16916 | Triple A ATPase subunit of the 26S proteasome's 19S regulatory particle (RP) base subcomplex (RPT-3) | Acute lethal or lethal |
| F25H5.4 | CG2238 | Translation elongation factor 2 (EF-2), a GTP-binding protein involved in protein synthesis | Growth delay or arrested in growth |
| F26D10.3 | CG4264 | Member of the Heat Shock Protein gene class | Acute lethal or lethal |
| F28C6.7 | CG6846 | Large ribosomal subunit L26 protein (RPL-26) involved in protein biosynthesis | Embryonic lethal or sterile |
| F28D1.7 | CG8415 | Small ribosomal subunit S23 protein (RPS-23) involved in protein biosynthesis | Acute lethal or lethal |
| F29G9.5 | CG5289 | Member of the proteasome Regulatory Particle, ATPase-like gene class | Acute lethal or lethal |
| F32H2.5 | CG3523 | Mitochondrial protein | Acute lethal or lethal |
| F37C12.11 | CG2986 | Small ribosomal subunit S21 protein (RPS-21) involved in protein biosynthesis | Acute lethal or lethal |
| F37C12.4 | CG7622 | Large ribosomal subunit L36 protein (RPL-36) involved in protein biosynthesis | Acute lethal or lethal |
| F37C12.9 | CG1527 | Small ribosomal subunit S14 protein (RPS-14) involved in protein biosynthesis | Acute lethal or lethal |
| F38E11.5 | CG6699 | beta' (beta-prime) subunit of the coatomer (COPI) complex | Acute lethal or lethal |
| F39B2.6 | CG10305 | Small ribosomal subunit S26 protein (RPS-26) involved in protein biosynthesis | Acute lethal or lethal |
| F39H11.5 | CG12000 | Member of the Proteasome Beta Subunit gene class | Acute lethal or lethal |
| F40F8.10 | CG3395 | Ribosomal protein S9 (RpS9), structural constituent of ribosome involved in protein biosynthesis which is a component of the cytosolic small ribosomal subunit | Acute lethal or lethal |
| F42C5.8 | CG7808 | Small ribosomal subunit S8 protein (RPS-8) involved in protein biosynthesis | Acute lethal or lethal |
| F49C12.8 | CG5378 | Member of the proteasome Regulatory Particle, Non-ATPase-like gene class | Acute lethal or lethal |
| F53A3.3 | CG2033 | Small ribosomal subunit S15a protein. | Acute lethal or lethal |
| F53G12.10 | CG4897 | large ribosomal subunit L7 protein (rpl-7) | Acute lethal or lethal |
| F54A3.3 | CG8977 | Unknown function | Acute lethal or lethal |
| F54E2.3 | CG1915 | Product with sallimus (sis), myosin-light-chain kinase activity (EC:2.7.1.117) involved in mitotic chromosome condensation which is localized to the nucleus | |
| F54E7.2 | CG11271 | Small ribosomal subunit S12 protein (RPS-12) involved in protein biosynthesis | Acute lethal or lethal |
| F55A11.2 | CG4214 | Member of the SYNtaxin gene class | Acute lethal or lethal |
| F55A3.3 | CG1828 | transcritpion factor | Acute lethal or lethal |
| F55C10.1 | CG11217 | Ortholog of calcineurin B, the regulatory subunit of the protein phosphatase 2B | Acute lethal or lethal |
| F56F3.5 | CG2168 | rps-1 encodes a small ribosomal subunit S3A protein. | Acute lethal or lethal |
| F57B9.10 | CG10149 | Member of the proteasome Regulatory Particle, Non-ATPase-like gene class | Acute lethal or lethal |
| F58F12.1 | CG2968 | ATP synthase | Acute lethal or lethal |
| F59E10.3 | CG3948 | Zeta subunit of the coatomer (COPI) complex | Acute lethal or lethal |
| JC8.3 | CG3195 | Large ribosomal subunit L12 protein (rpl-12) | Acute lethal or lethal |
| K01 G5.4 | CG1404 | Putative RAN small monomeric GTPase (cell adhesion) | Acute lethal or lethal |
| K04F10.4 | CG18734 | Subtilase | Acute lethal or lethal |
| K05C4.1 | CG12323 | Member of the Proteasome Beta Subunit gene class | Acute lethal or lethal |
| K07D4.3 | CG18174 | Putative proteasome regulators particle, lid subcomplex, rpn11 | Acute lethal or lethal |
| K11D9.2 | CG3725 | Sarco-endoplasmic reticulum Ca[2+] ATPase | Embryonic lethal or sterile; Acute lethal or lethal |
| M03F4.2 | CG4027 | An actin that is expressed in body wall and vulval muscles and the spermatheca. | Acute lethal or lethal |
| R06A4.9 | CG1109 | six WD40 repeats | Acute lethal or lethal |
| R10E11.1 | CG15319 | Putative transcriptional cofactor | Acute lethal or lethal |
| R12E2.3 | CG3416 | Protein with endopeptidase activity involved in proteolysis and peptidolysis | Acute lethal or lethal |
| F10C1.2 | CG10119 | Member of the Intermediate Filament, B gene class | Embryonic lethal or sterile |
| F35G12.8 | C11397 | Homolog of the SMC4 subunit of mitotic condensin | Embryonic lethal or sterile |
| F53G12.1 | CG5771 | ATPase homologue | Embryonic lethal or sterile |
| F54E7.3 | CG5055 | PDZ domain-containing protein | Embryonic lethal or sterile |
| H28016.1 | CG3612 | ATP synthase | Growth delay or arrested in growth |
| K12C11.2 | CG4494 | Member of the SUMO (ubiquitin-related) homolog gene class | Embryonic lethal or sterile |
| R12E2.3 | CG3416 | Member of the proteasome Regulatory Particle, Non-ATPase-like gene class | Acute lethal or lethal |
| R13A5.8 | CG6141 | Ribosomal protein L9, structural constituent of ribosome involved in protein biosynthesis which is localised to the ribosome | Acute lethal or lethal |
| T01C3.6 | CG4046 | rps-16 encodes a small ribosomal subunit S16 protein. | Acute lethal or lethal |
| T01H3.1 | CG7007 | proteolipid protein PPA1 like protein | Acute lethal or lethal |
| T05C12.7 | CG5374 | cytosolic chaperonin | Acute lethal or lethal |
| T05H4.6 | CG5605 | eukaryotic peptide chain release factor subunit 1 | Acute lethal or lethal |
| T10H9.4 | CG17248 | N-synaptobrevin; v-SNARE, vesicle-mediated transport, synaptic vesicle | |
| T14F9.1 | CG17332 | ATPase subunit | Growth delay or arrested in growth |
| T20G5.1 | CG9012 | Clathrin heavy chain | Acute lethal or lethal |
| T21B10.7 | CG7033 | t-complex protein 1 | Embryonic lethal or sterile |
| W09B12.1 | CG17907 | Acetylcholineesterase | |
| T27F2.1 | CG8264 | Member of the mammalian SKIP (Ski interacting protein) homolog gene class | Acute lethal or lethal |
| ZC434.5 | CG5394 | predicted mitochondrial glutamyl-tRNA synthetase (GluRS) | Acute lethal or lethal |
| B0511.6 | C6375 | helicase | Embryonic lethal or sterile |
| DY3.2 | CG10119 | Nuclear lamin; LMN-1 protein | Growth delay or arrested in growth |
| R13G10.1 | CG11397 | homolog of the SMC4 subunit of mitotic condensin | Wild Type. |
| T26E3.7 | C3612 | Predicted mitochondrial protein. | Growth delay or arrested in growth |
| Y113G7A.3 | CG1250 | GTPase activator, ER to Golgi prot transport, component of the Golgi stack | Acute lethal or lethal |
| Y43B11AR.4 | CG11276 | Ribosomal protein S4 (RpS4), structural constituent of ribosome involved in protein biosynthesis which is a component of the cytosolic small ribosomal subunit | Acute lethal or lethal |
| Y46G5A.4 | CG5931 | Y46G5A.4 gene | Acute lethal or lethal |
| Y71F9AL.17 | C7961 | Alpha subunit of the coatomer (COPI) complex | Acute lethal or lethal |
| Y76B12C.7 | CG10110 | Gene cleavage and polyadenylation specificity factor | Embryonic lethal or sterile |
| Y37D8A.10 | CG1751 | Unknown function | Embryonic lethal or sterile |
| CG7765 | C06G3.2 | Member of the Kinesin-Like Protein gene class | |
| CG10922 | C44E4.4 | RNA-binding protein | Embryonic lethal or sterile |
| CG4145 | F01G12.5 | alpha-2 type IV collagen | Embryonic lethal or sterile |
| CG13391 | F28H1.3 | apredicted cytoplasmic alanyl-tRNA synthetase (AlaRS) | Growth delay or arrested in growth |
| CG7765 | R05D3.7 | Member of the UNCoordinated gene class | Embryonic lethal or sterile |
| CG7398 | R06A4.4 | Member of the Importin Beta family gene class | Embryonic lethal or sterile |
| CG7436 | T17E9.2 | Unknown function | Embryonic lethal or sterile |
| CG2666 | T25G3.2 | Putative chitin synthase | Embryonic lethal or sterile |
| CG17603 | W04A8.7 | TATA-binding protein associated factor TAF1L (TAFII250) | Embryonic lethal or sterile |

**Table 1-LD/PC/AG/TC/MP/NL/CS/PX/AD**

| **Target ID** | **Dm identifier** | **SEQ ID NO NA** | **SEQ ID NO AA** | **Function (based on Flybase)** |
|---|---|---|---|---|
| LD001 | CG11276 | 1 | 2 | Ribosomal protein S4 (RpS4), structural constituent of ribosome involved in protein biosynthesis which is a component of the cytosolic small ribosomal subunit |
| PC001 | CG11276 | 247 | 248 | Ribosomal protein S4 (RpS4), structural constituent of ribosome involved in protein biosynthesis which is a component of the cytosolic small ribosomal subunit |
| AG001 | CG11276 | 601 | 602 | Ribosomal protein S4 (RpS4), structural constituent of ribosome involved in protein biosynthesis which is a component of the cytosolic small ribosomal subunit |
| TC001 | CG11276 | 793 | 794 | Ribosomal protein S4 (RpS4), structural constituent of ribosome involved in protein biosynthesis which is a component of the cytosolic small ribosomal subunit |
| MP001 | CG11276 | 888 | 889 | Ribosomal protein S4 (RpS4), structural constituent of ribosome involved in protein biosynthesis which is a component of the cytosolic; small ribosomal subunit |
| NL001 | CG11276 | 1071 | 1072 | Ribosomal protein S4 (RpS4), structural constituent of ribosome involved in protein biosynthesis which is a component of the cytosolic small ribosomal subunit |
| CS001 | CG11276 | 1682 | 1683 | Ribosomal protein S4 (RpS4), structural constituent of ribosome involved in protein biosynthesis which is a component of the cytosolic small ribosomal subunit |
| PX001 | CG11276 | 2100 | 2101 | Ribosomal protein S4 (RpS4), structural constituent of ribosome involved in protein biosynthesis which is a component of the cytosolic small ribosomal subunit |
| AD001 | CG11276 | 2364 | 2365 | Ribosomal protein S4 (RpS4), structural constituent of ribosome involved in protein biosynthesis which is a component of the cytosolic small ribosomal subunit |

**Table 2-LD**

| **Target ID** | **Primer Forward 5' → 3'** | **Primer Reverse 5'→3'** | **cDNA Sequence (sense strand) 5' →3'** |
|---|---|---|---|
| LD001 | SEQ ID NO: 25 | SEQ ID NO: 26 | SEQ ID NO: 1 |
| | GGCCCCAAGAA GCATTTGAAGC G | TAGCGGATGGT GCGDCCRTCRT G | GGCCCCAAGAAGCATTTGAAGCGTTTGAATGCCCCAAAAGCATGGATGTTGGATAAATTGG GAGGTGTTTTCGCACCTCGCCCATCTACAGGACCTCACAAATTGCGAGAGTCTTTGCCCTT GGTGATCTTCCTACGTAACCGATTGAAGTATGCTTTGACTAACAGCGAAGTTACTAAGATTG TTATGCAAAGGTTAATCAAAGTAGATGGAAAAGTGAGGACCGACTCCAATTACCCTGCTGG GTTTATGGATGTTATTACCATTGAAAAAACTGGTGAATTTTTCCGACTCATCTATGATGTTAA AGGACGATTTGCAGTGCATCGTATTACTGCTGAGGAAGCAAAGTACAAACTATGCAAAGTC AGGAGGATGCAAACTGGCCCCAAAGGAATTCCCTTCATAGTGACACACGACGGCCGCACC ATCCGCTA |
| LD002 | SEQ ID NO: 27 | SEQ ID NO: 28 GCAATGTCATC | SEQ ID NO: 3 |
| LD003 | SEQ ID NO: 29 | SEQ ID NO: 30 | SEQ ID NO: 5 |
| LD006 | SEQ ID NO:31 | SEQ ID NO:32 | SEQ ID NO: 7 |
| LD007 | SEQ ID NO: 33 | SEQ ID NO: 34 | SEQ ID NO: 9 |
| LD010 | SEQ ID NO: 35 | SEQ ID NO: 36 | SEQ ID NO: 11 |
| LD011 | SEQ ID NO: 37 | . SEQ ID NO: 38 | SEQ ID NO: 13 |
| LD014 | SEQ ID NO: 39 | SEQ ID NO: 40 | SEQ ID NO: 15 |
| LD014_F1 | | | SEQ ID NO: 159 |
| LD014_F2 | | | SEQ ID NO: 160 |
| LD014_C1 | | | SEQ ID NO:161 |
| LD014_C2 | | | SEQ ID NO: 162 |
| LD015 | SEQ ID NO: 41 | SEQ ID NO: 42 | SEQ ID NO:17 |
| LD016 | SEQ ID NO:43 | SEQ lD NO:44 | SEQ ID NO:19 |
| LD018 | SEQ ID NO: 45 | SEQ lD NO:46 | SEQ ID NO:21 G |
| LD027 | SEQ ID NO:47 | SEQ ID NO:48 | SEQ ID NO:23 |

**Table 2-PC**

| **Target ID** | **Primer Forward 5'→3'** | **Primer Reverse 5'→3'** | **cDNA Sequence (sense strand) 5' →3'** |
|---|---|---|---|
| PC001 | SEQ ID NO: 261 CATTTGAAGCG TTTWRMYGCY CC | SEQ ID NO: 262 CTTCGTGCCCT TGCCRATKATR AABACG | SEQ ID NO: 247 CATTTGAAGCGTTTAGCTGCTCCCAAAGCATGGATGTTGGACAAATTGGGGGGTGTCTT CGCCCCTCGTCCATCCACCGGGCCTCACAAGTTGCGCGAATCCCTGCCTTTAGTGATT TTCCTTCGTAACAGGCTGAAGTATGCCCTTACAAACAGTGAAGTCACTAAAATTGTCATG CAAAGGTTGATCAAAGTTGATGGTAAAGTGAGGACTGATTCTAATTACCCTGCTGGTTT CATGGATGTCATTACTATTGAGAAGACTGGTGAATTTTTCCGTCTGATCTATGATGTTAA AGGAAGATTTGCTGTGCACCGTATTACAGCTGAAGAGGCAAAATACAAGTTGTGTAAAG TAAGGAGAGTCCAAACTGGTCCCAAAGGAATCCCATTTTTGGTAACACATGATGGCAGA ACCATTCGTTACCCTGACCCCAACATCAAAGTGAATGACACAATTCAAATGGAAATTGCT ACATCTAAAATTCTTGACTACATCAAATTTGAATCTGGCAACC |
| | | | TCTGCATGATCACGGGGAGG |
| PC003 | SEQ ID NO:263 | SEQ ID NO:264 | SEQ ID NO:249 |
| PC005 | SEQ ID NO:265 | SEQ ID NO:266 | SEQ ID NO: 251 |
| PC010 | SEQ ID NO: 267 | SEQ ID NO:268 | SEQ ID NO:253 |
| PC014 | SEQ lD NO:269 | SEQ ID NO:270 | SEQ lD NO:255 |
| PC016 | SEQ ID NO: 271 | SEQ ID NO:272 | SEQ ID NO: 257 |
| PC027 | SEQ ID NO:273 | SEQ ID NO:274 | SEQ ID NO:259 |

**Table 2-EV**

| **Target ID** | **Primer Forward 5' →3'** | **Primer Reverse 5' →3'** | **cDNA Sequence (sense strand) 5'→3'** |
|---|---|---|---|
| EV005 | SEQ ID NO:523 | (SEQ ID NO: 524 | SEQ ID NO:513 |
| EV009 | SEQ ID NO:525 | SEQ ID NO: 526 | SEQ ID NO:515 |
| EV010 | SEQ ID NO:527 | SEQ ID NO:528 | SEQ ID NO:517 |
| EV015 | SEQ ID NO: 529 | SEQ ID NO: 530 | SEQ ID NO:519 |
| EV016 | SEQ ID NO:531 | SEQ ID NO:532 | SEQ ID NO:521 |

**Table 2-AG**

| **Target ID** | **Primer Forward 5'→3'** | **Primer Reverse 5'→3'** | **cDNA Sequence (sense strand) 5'→3'** |
|---|---|---|---|
| AG001 | SEQ ID NO: 611 CATTTGAAGCG TTTWRMYGCYC C | SEQ ID NO: 612 CGCTTGTCCC GCTCCTCNGC RAT | SEQ ID NO: 601 CATTTGAAGCGTTTTGCTGCCCCCAAAGCATGGATGTTGGACAAATTGGGGGGTGTGTTCGCCC CCAGGCCCTCCACCGGGCCACACAAGCTCAGGGAGTCCCTTCCATTAGTGATTTTCTTGCGTAA CAGGTTGAAGTACGCCCTGACAAACTGTGAGGTGACCAAGATCGTTATGCAGAGACTTATTAAG GTCGACGGCAAAGTCAGGACTGATCCTAACTATCCTGCTGGATTCATGGATGTGATCACCATTGA AAAAACTGGTGAATTCTTCCGTTTGATCTATGATGTTAAGGGAAGATTCACTATTCACAGGATCAC TGCTGAAGAAGCAAAATACAAATTGTGCAAAGTCCGCAAGGTGCAAACCGGACCAAAAGGTATTC CATTCTTGGTCACCCACGATGGTAGGACCATTAGGTACCCTGACCCAATGATCAAGGTAAACGAC ACCATCCAACTGGAAATCGCCACCTCAAAGATCCTGGACTTTATCAAATTCGAATCCGGCAACTT GTGCATGATCACCGGAGGCAGGAATTTGGGTAGAGTGGGAACGGTAGTGAACAGGGAAAGGCA TCCGGGATCATTCGATATTGTCCACATTAGGGACGCTAATGATCACGTGTTCGCCACTAGATTAA ACAACGTATTCGTCATCGGTAAAGGAAGCAAAGCTTTCGTGTCTCTGCCAAGGGGCAAGGGAGT GAAACTGTCCATCGCTG |
| AG005 | SEQ ID NO:613 | SEQ ID NO:614 | SEQ lD NO:603 |
| AG010 | SEQ ID NO:615 | SEQ ID NO: 616 | SEQ ID NO:605 |
| AG014 | SEQ ID NO:617 | SEQ ID NO:618 | SEQ ID NO:607 |
| AG016 | SEQ ID NO:619 | SEQ ID NO:620 | SEQ ID NO:609 |

**Table 2-TC**

| **Target ID** | **Primer Forward 5'→3'** | **Primer Reverse 5'→3'** | **cDNA Sequence (sense strand) 5' →3'** |
|---|---|---|---|
| TC001 | SEQ ID NO: 803 GGCCCCAAGA AGCATTTGAAG CG | SEQ ID NO: 804 CGCTTGTCCC GCTCCTCNGC RAT | SEQ ID NO: 793 GGCCCCAAGAAGCATTTGAAGCGTCTCAATGCGCCCAAAGCATGGATGTTGGATAAACTG GGGGGTGTGTTTGCCCCTCGGCCTTCCACCGGCCCCACAAGCTACGGAGTCGCTACC TTTGGTTATCTTCCTGCGAAACAGGCTGAAGTATGCCTTGACCAACTCAGAAGTGACGAA GATTGTTATGCAAAGATTGATTAAAGTTGACGGAAAAGTTAGGACAGACCCCAACTACCCC GCGGGTTTCATGGATGTTGTGACTATTGAGAAAACTGGGGAATTCTTCCGCTTGATTTATG ATGTTAAGGGAAGGTTCACAATCCATCGCATTACTGGAGAAGAGGCCAAATATAAATTGTG CAAAGTGAAGAAAGTACAGACAGGCCCCAAGGGCATTCCCTTCTTGGTGACCCGCGACG GACGCACTATCAGATACCCAGACCCCATGATCAAAGTGAATGACACCATTCAATTGGAGAT TGCCACTTCGAAAATTCTTGATTTTATCAAATTTGAGTCCGGTAATTTGTGTATGATTACTG GAGGTCGTAACTTGGGGCGTGTCGGTACAGTGGTGAGCCGAGAACGTCACCCAGGTTCC TTCGACATCGTTCATATTAAGGATGCAAATGGGCACACC |
| TC002 | SEQ ID NO:805 | SEQ ID NO: 806 | SEQ ID NO: 795 |
| TC010 | SEQ ID NO:807 | SEQ ID NO: 808 | SEQ ID NO: 797 |
| TC014 | SEQ ID NO:809 | SEQ ID NO:810 | SEQ ID NO:799 |
| TC015 | SEQ ID NO:811 | SEQ ID NO:812 | SEQ ID NO:801 |

**Table 2-MP**

| **Target ID** | **Primer Forward 5' →3'** | **Primer Reverse 5' →3'** | **cDNA Sequence (sense strand) 5' → 3'** |
|---|---|---|---|
| MP001 | SEQ ID NO: 898 GGCCCCAAGAA GCATTTGAAGC G | SEQ ID NO: 899 CGCTTGTCCC GCTCCTCNGC RAT | SEQ ID NO: 888 GGCCCCAAGAAGCATTTGAAGCGTTTAAACGCACCCAAAGCATGGATGTTGGACAAATCGGG GGGTGTCTTCGCTCCACGTCCAAGCACCGGTCCACACAAACTTCGTGAATCACTACCGTTATT GATCTTCTTGCGTAATCGTTTGAAGTATGCACTTACTGGTGCCGAAGTCACCAAGATTGTCAT GCAAAGATTAATCAAGGTTGATGGCAAAGTCCGTACCGACCCTAATTATCCAGCCGGTTTTAT GGATGTTATATCTATCCAAAAGACCAGTGAGCACTTTAGATTGATCTATGATGTGAAAGGTCG TTTCACCATCCACAGAATTACTCCTGAAGAAGCAAAATACAAGTTGTGTAAAGTAAAGAGGGT ACAAACTGGACCCAAAGGTGTGCCATTTTTAACTACTCATGATGGCCGTACTATTCGCTACCC TGACCCTAACATCAAGGTTAATGACACTATTAGATACGATATTGCATCATCTAAAATTTTGGAT CATATCCGTTTTGAAACTGGAAACTTGTGCATGATAACTGGAGGTCGCAATTTAGGGCGTGTT GGTATTGTTACCAACAGGGAAAGACATCCAGGATCTTTTGATATTGTTCACATTAAGGATGCA AATGAACATATTTTTGCTACCCGGATGAACAATGTTTTTATTATTGGAAAAGGTCAAAAGAACT ACATTTCTCTACCAAGGAGTAAGGGAGTTAAATTGACTAT |
| MP002 | SEQ ID NO:900 | SEQ ID NO:901 | SEQ ID NO: 890 |
| MP010 | SEQ ID NO: 902 | SEQ ID NO:903 | SEQ ID NO:892 |
| MP016 | SEQ ID NO:904 | SEQ ID NO:905 | SEQ ID NO: 894 |
| MP027 | SEQ ID NO:906 | SEQ ID NO:907 | SEQ ID NO:896 |

**Table 2-NL**

| **Target ID** | **Primer Forward 5' → 3'** | **Primer Reverse 5' → 3'** | **cDNA Sequence (sense strand) 5' → 3'** |
|---|---|---|---|
| NL001 | SEQ ID NO: 1117 GAAATCATGGAT GTTGGACAAATT GG | SEQ ID NO: 1118 ACTGAGCTTCACAC CCTTGCCC | SEQ ID NO: 1071 GAAATCATGGATGTTGGACAAATTGGGTGGTGTGTATGCACCCCGACCCAGCACAGG TCCACACAAGCTGCGAGAATCTCTCCCACTTGTCATATTTTTGCGTAATCGGCTCAAG TACGCTTTAACTAACTGTGAAGTGAAGAAAATTGTGATGCAGCGTCTCATCAAGGTTG ACGGCAAAGTGAGGACTGACCCCAACTATCCTGCAGGTTTTATGGACGTTGTTCAAAT CGAAAAGACAAACGAGTTCTTCCGTTTGATCTATGATGTTAAGGGACGTTTCACCATC CACAGGATCACAGCTGAAGAAGCTAAGTACAAGCTGTGCAAAGTGAAGAGGGTTCAG ACAGGACCCAAGGGCATTCCATTTTTGACCACTCACGATGGACGCACCATCAGGTAT CCAGACCCCTTGGTAAAAGTCAATGACACCATCCAATTGGACATTGCCACATCCAAAA TCATGGACTTCATCAGATTCGACTCTGGTAACCTGTGTATGATCACTGGAGGTCGTAA CTTGGGTCGTGTGGGCACTGTCGTGAACAGGGAGCGACACCCGGGGTCTTTCGACA TCGTGCACATCAAGGACGTGTTGGGACACACTTTTGCCACTAGGTTGAACAACGTTTT CATCATCGGCAAGGGTAGTAAAGCATACGTGTCTCTGCCCAAGGGCAAGGGTGTGAA GCTCAGT |
| NL002 | SEQ ID NO:1119 | SEQ ID NO: 1120 | SEQ ID NO:1073 |
| NL003 | SEQ ID NO: 1121 | SEQ ID NO: 1122 | SEQ ID NO: 1075 |
| NL004 | SEQ ID NO: 1123 | SEQ ID NO: 1124 | SEQ ID NO: 1077 |
| NL005 | SEQ ID NO: 1125 | SEQ ID NO: 1126 | SEQ ID NO: 1079 |
| NL006 | SEQ ID NO: 127 | SEQ ID NO: 1128 | SEQ ID NO: 1081 |
| NL007 | SEQ ID NO: 1129 | SEQ ID NO: 1130 | SEQ ID NO: 1083 |
| NL008 | SEQ ID NO: 1131 | SEQ ID NO: 1132 | SEQ ID NO: 1085 |
| NL009 | SEQ ID NO: 1133 | SEQ ID NO: 1134 | SEQ ID NO: 1087 |
| NL010 | SEQ ID NO: 1135 | SEQ ID NO: 1136 | SEQ ID NO: 1089 (amino terminus) |
| NL011 | SEQ ID NO: 1137 | SEQ ID NO: 1138 | SEQ ID NO: 1091 |
| NL012 | SEQ ID NO: 1139 | SEQ ID NO: 1140 | SEQ ID NO: 1093 |
| NL013 | SEQ ID NO: 1141 | SEQ ID NO: 1142 | SEQ ID NO: 1095 |
| NL014 | SEQ ID NO: 1143 | SEQ ID NO: 1144 | SEQ ID NO: 1097 |
| NL015 | SEQ ID NO: 1145 | SEQ ID NO: 1146 | SEQ ID NO: 1099 |
| NL016 | SEQ ID NO: 1147 | SEQ ID NO: 1148 | SEQ ID NO: 1101 |
| NL018 | SEQ ID NO: 1149 | SEQ ID NO: 1150 | SEQ ID NO: 1103 |
| NL019 | SEQ ID NO: 1151 | SEQ ID NO: 1152 | SEQ ID NO: 1105 |
| NL021 | SEQ ID NO: 1153 | SEQ ID NO: 1154 | SEQ ID NO: 1107 |
| NL022 | SEQ ID NO: 1155 | SEQ ID NO: 1156 | SEQ ID NO: 109 |
| NL023 | SEQ ID NO: 1157 | SEQ ID NO: 1158 | SEQ ID NO: 1111 |
| NL027 | SEQ ID NO: 1159 | SEQ ID NO: 1160 | SEQ ID NO: 1113 |

**Table 2-CS**

| **Target ID** | **Primer Forward 5' → 3'** | **Primer Reverse 5' → 3'** | **cDNA Sequence (sense strand) 5' → 3'** |
|---|---|---|---|
| CS001 | SEQ ID NO: 1706 CATTTGAAGCGT TTWRMYGCYCC | SEQ ID NO: 1707 CTTCGTGCCCTT GCCRATKATRAA BACG | SEQ ID NO: 1682 TAAAGCATGGATGTTGGACAAACTGGGTGGCGTGTACGCGCCGCGGCCGTCGACCGG CCCCCACAAGTTGCGCGAGTGCCTGCCGCTGGTGATCTTCCTCAGGAACCGGCTCAA GTACGCGCTCACCGGAAATGAAGTGCTTAAGATTGTAAAGCAGCGACTTATCAAAGTTG ACGGCAAAGTCAGGACAGACCCCACATATCCCGCTGGATTTATGGATGTTGTTTCCATT GAAAAGACAAATGAGCTGTTCCGTCTTATATATGATGTCAAAGGCAGATTTACTATTCAC CGTATTACTCCTGAGGAGGCTAAATACAAGCTGTGCAAGGTGCGGCGCGTGGCGACG GGCCCCAAGAACGTGCCTTACCTGGTGACCCACGACGGACGCACCGTGCGATACCCC GACCCACTCATCAAGGTCAACGACTCCATCCAGCTCGACATCGCCACCTCCAAGATCA |
| | | | TGGACTTCATCAAGTTTGAATCTGGTAACCTATGTATGATCACGGAGGCCGTAACTTG GGGCGCGTGGGCACCATCGTGTCCCGCGAGCGACATCCCGGGTCCTTCGACATCGTG CATATACGGGACTCCACCGGACATACCTTCGCTACCAGATTGAACAACGTGTTCATAAT CGGCAAGGGCACGAAG |
| CS002 | SEQ ID NO: 1708 | SEQ ID NO: 1709 | SEQ ID NO: 1684 |
| CS003 | SEQ ID NO: 1710 | SEQ ID NO: 1711 | SEQ ID NO: 1686 |
| CS006 | SEQ ID NO: 1712 | SEQ ID NO: 1713 | SEQ ID NO: 1688 |
| CS007 | SEQ ID NO: 1714 | SEQ ID NO: 1715 | SEQ ID NO: 1690 |
| CS009 | SEQ ID NO: 1716 | SEQ ID NO: 1717 | SEQ ID NO: 1692 |
| CS011 | SEQ ID NO: 1718 | SEQ ID NO: 1719 | SEQ ID NO: 1694 |
| CS013 | SEQ ID NO: 1720 | SEQ ID NO: 1721 | SEQ ID NO: 1696 |
| CS014 | SEQ ID NO:1722 | SEQ ID NO: 1723 | SEQ ID NO: 1698 |
| CS015 | SEQ ID NO: 1724 | SEQ ID NO: 1725 | SEQ ID NO: 1700 |
| CS016 | SEQ ID NO: 1726 | SEQ ID NO: 1727 | SEQ ID NO: 1702 |
| CS018 | SEQ ID NO: 1728 | SEQ ID NO: 1729 | SEQ ID NO: 1704 |

**Table 2-PX**

| **Target ID** | **Primer Forward 5' → 3'** | **Primer Reverse 5' → 3'** | **cDNA Sequence (sense strand) 5' → 3'** |
|---|---|---|---|
| PX001 | SEQ ID NO: 2110 GGCCCCAAGAAG CATTTGAAGCG | SEQ ID NO: 2111 CTTCGTGCCCTTGC CRATKATRAABACG | SEQ ID NO: 2100 GGCCCCAAGAAGCATTTGAAGCGCCTGAACGCCGCGCGCATGGATGCTGGA CAAGCTCGGCGGCGTGTACGCGCCGCGGCCCAGCACGGGCCCGCACAAGCTG |
| | | | CGCGAGTGCCTGCCGCTCGTCATCTTCCTGCAACCGCCTCAAGTACGCGCTCAG CGGCAACGAGGTGCTGAAGATCGTGAAGCAGCGCCTCATCAAGGTGGACGGCA AGGTCCGCACCGACCCCACCTACCCGGCTGGATTCATGGATGTTGTGTCGATTG AAAAGACCAATGAGCTGTTCCGTCTGATCTACGATGTGAAGGGACGCTTCACCAT CCACCGCATCACTCCCGAGGAGGCCAAGTACAAGCTGTGCAAGGTGAAGCGCG TGGCGACGGGCCCCAAGAACGTGCCGTACATCGTGACGCACAACGGCCGCACG CTGCGCTACCCCGACCCGCTCATCAAGGTCAACGACTCCATCCAGCTCGACATC GCCACCTGCAAGATCATGGACATCATCAAGTTCGACTCAGGTAACCTGTGCATGA TCACGGGAGGGCGTAACTTGGGGCGAGTGGGCACCATCGTGTCCCGCGAGAGG CACCCCGGGAGCTTCGACATCGTCCACATCAAGGACACCACCGGACACACCTTC GCCACCAGGTTGAACAACGTGTTCATCATCGGCAAGGGCACGAAG |
| PX009 | SEQ ID NO: 2112 | SEQ ID NO: 2113 | SEQ ID NO: 2102 |
| PX010 | SEQ ID NO: 2114 | SEQ ID NO: 2115 | SEQ ID NO: 2104 |
| PX015 | SEQ ID NO: 2116 | SEQ ID NO: 2117 | SEQ ID NO: 2106 |
| PX016 | SEQ ID NO: 2118 | SEQ ID NO: 2119 | SEQ ID NO: 2108 |

**Table 2-AD**

| **Target ID** | **Primer Forward 5' → 3'** | **Primer Reverse 5' → 3'** | **cDNA Sequence (sense strand) 5' → 3'** |
|---|---|---|---|
| AD001 | SEQ ID NO: 2374 GGCCCCAAGAAGCA TTTGAAGCG | SEQ ID NO: 2375 CGCTTGTCCCG CTCCTCNGCRA T | SEQ ID NO: 2364 GGCCCCAAGAAGCATTTGAAGCGTTTAAATGCTCCTAAAGCATGGATGTTGGACAA ACTCGGAGGAGTATTCGCTCCTCGCCCCAGTACTGGCCCCCACAAATTGCGTGAA TGTTTACCTTTGGTGATTTTTCTTCGCAATCGGCTCAAGTATGCTCTGACGAACTGT GAAGTAACGAAGATTGTTATGCAGCGACTTATCAAAGTTGACGGCAAGGTGCGAAC CGATCCGAATTATCCCGCTGGTTTCATGGATGTTGTCACCATTGAGAAGACTGGAG AGTTCTTCAGGCTGGTGTATGATGTGAAAGGCCGTTTCACAATTCACAGAATTAGT GCAGAAGAAGCCAAGTACAAGCTCTGCAAGGTCAGGAGAGTTCAAACTGGGCCAA AAGGTATTCCATTCTTGGTGACCCATGATGGCCGTACTATCCGTTATCCTGACCCA GTCATTAAAGTTAATGACTCAATCCAATTGGATATTGCCACTTGTAAAATCATGGAC CACATCAGATTTGAATCTGGCAACCTGTGTATGATTACTGGTGGACGTAACTTGGG TCGAGTGGGGACTGTTGTGAGTCGAGAACGTCACCCAGGCTCGTTTGATATTGTT CATATCAAGGATACCCAAGGACATACTTTTGCCACAAGATTGAATAATGTATTCATC ATTGGAAAAGCTACAAAGCCTTACATTTCATTGCCAAAGGGTAAGGGTGTGAAATT GAGTATCGCCGAGGAGCGGGACAAGCG |
| AD002 | SEQ lD NO: 2376 | SEQ ID NO: 2377 | SEQ ID NO: 2366 |
| AD009 | SEQ ID NO: 2378 | SEQ ID NO: 2379 | SEQ ID NO: 2368 |
| AD015 | SEQ lD NO: 2380 | SEQ ID NO: 2381 | SEQ ID NO: 2370 |
| AD016 | SEQ ID NO: 2382 | SEQ ID NO: 2383 | SEQ ID NO: 2372 |

**Table 3-LD**

| **Target ID** | **CDNA SEQ ID NO** | **Corresponding amino acid sequence of cDNA clone** |
|---|---|---|
| LD001 | 1 | SEQ ID NO: 2 (frame +1) GPKKHLKRLNAPKAWMLDKLGGVFAPRPSTGPHKLRESLPLVIFLRNRLKYALTNSEVTKIVMQRLIKVDGKVRTD SNYPAGFMDVITIEKTGEFFRLIYDVKGRFAVHRITAEEAKYKLCKVRRMQTGPKGIPFIVTHDGRTIR |
| LD002 | 3 | SEQ ID NO: 4 (frame -3) |
| LD003 | 5 | SEQ ID NO: 6 (frame -2) |
| LD006 | 7 | SEQ ID NO: 8 (frame +1) |
| LD007 | 9 | SEQ ID NO: 10 (frame +1) |
| LD010 | 11 | SEQ ID NO: 12 (frame +1) |
| LD011 | 13 | SEQ ID NO: 14 (frame -1) |
| LD014 | 15 | SEQ ID NO: 16 (frame +3) |
| LD015 | 17 | SEQ ID NO: 18 (frame -1) |
| LD016 | 19 | SEQ ID NO: 20 (frame -2) |
| LD018 | 21 | SEQ ID NO: 22 (frame +2) |
| LD027 | 23 | SEQ ID NO: 24 (frame +1) |

**Table 3-PC**

| **Target ID** | **CDNA SEQ ID NO** | **Corresponding amino acid sequence of cDNA clone** |
|---|---|---|
| PC001 | 247 | SEQ ID NO: 248 (frame +1) AWMLDKLGGVFAPRPSTGPHKLRESLPLVIFLRNRLKYALTNSEVTKIVMQRLIKVDGKVRTDSNYPAGFMDVITIE KTGEFFRLIYDVKGRFAVHRITAEEAKYKLCKVRRVQTGPKGIPFLVTHDGRTIRYPDPNIKVNDTIQMEIATSKILDY IKFES |
| PC003 | 249 | SEQ ID NO: 250 (frame: +2) |
| PC005 | 251 | SEQ ID NO: 252 (frame +3) |
| PC010 | 253 | SEQ ID NO: 254 (frame +3) |
| PC014 | 255 | SEQ ID NO: 256 (frame +3) |
| PC016 | 257 | SEQ ID NO: 258 (frame +2) |
| PC027 | 259 | SEQ ID NO: 260 (frame +1) |

**Table 3-EV**

| **Target ID** | **cDNA SEQ ID NO** | **Corresponding amino acid sequence of cDNA clone** |
|---|---|---|
| EV005 | 513 | SEQ ID NO: 514 (frame +3) |
| EV009 | 515 | SEQ ID NO: 516 (frame +1) |
| EV010 | 517 | SEQ ID NO: 518 (frame +3) |
| EV015 | 519 | SEQ ID NO: 520 (frame +1) |
| EV016 | 521 | SEQ ID NO: 522 (frame +2) |

**Table 3-AG**

| **Target ID** | **cDNA SEQ ID NO** | **Corresponding amino acid sequence of cDNA clone** |
|---|---|---|
| AG001 | 601 | SEQ ID NO: 602 (frame +1) HLKRFAAPKAWMLDKLGGVFAPRPSTGPHKLRESLPLVIFLRNRLKYALTNCEVTKIVMQRLIKVDGKVRTDPNYPAG FMDVITIEKTGEFFRLIYDVKGRFTIHRITAEEAKYKLCKVRKVQTGPKGIPFLVTHDGRTIRYPDPMIKVNDTIQLEIATS KILDFIKFESGNLCMITGGRNLGRVGTVVNRERHPGSFDIVHIRDANDHVFATRLNNVFVIGKGSKAFVSLPRGKGVK LSIA |
| AG005 | 603 | SEQ ID NO: 604 (frame +2) |
| AG010 | 605 | SEQ ID NO: 606 (frame +3) |
| AG014 | 607 | SEQ ID NO: 608 (frame +3) |
| AG016 | 609 | SEQ ID NO: 610 (frame +1) |

**Table 3-TC**

| **Target ID** | **cDNA SEQ ID NO** | **Corresponding amino acid sequence of cDNA clone** |
|---|---|---|
| TC001 | 793 | SEQ ID NO: 794 (frame +1) GPKKHLKRLNAPKAWMLDKLGGVFAPRPSTGPHKLRESLPLVIFLRNRLKYALTNSEVTKIVMQRLIKVDGKVRTD PNYPAGFMDVVTIEKTGEFFRLIYDVKGRFTIHRITGEEAKYKLCKVKKVQTGPKGIPFLVTRDGRTIRYPDPMIKVN DTIQLEIATSKILDFIKFESGNLCMITGGRNLGRVGTVVSRERHPGSFDIVHIKDANGHTFATRLNNVFIIGKGSKPYV SLPRGKGVKLSI |
| TC002 | 795 | SEQ ID NO: 796 (frame +1) |
| TC010 | 797 | SEQ ID NO: 798 (frame +3) |
| TC014 | 799 | SEQ ID NO: 800 (frame +1) |
| TC015 | 801 | SEQ ID NO: 802 (frame +2) |

**Table 3-MP**

| **Target ID** | **cDNA SEQ ID NO** | **Corresponding amino acid sequence of cDNA clone** |
|---|---|---|
| MP001 | 888 | SEQ ID NO: 889 (frame +1) GPKKHLKRLNAPKAWMLDKSGGVFAPRPSTGPHKLRESLPLLIFLRNRLKYALTGAEVTKIVMQRLIKVDGKVRTDPN YPAGFMDVISIQKTSEHFRLIYDVKGRFTIHRITPEEAKYKLCKVKRVQTGPKGVPFLTTHDGRTIRYPDPNIKVNDTIR YDIASSKILDHIRFETGNLCMITGGRNLGRVGIVTNRERHPGSFDIVHIKDANEHIFATRMNNVFIIGKGQKNYISLPRSK GVKLT |
| MP002 | 890 | SEQ ID NO: 891 (frame +2) |
| MP010 | 892 | SEQ ID NO: 893 (frame +3) |
| MP016 | 894 | SEQ ID NO: 895 (frame +1) |
| MP027 | 896 | SEQ ID NO: 897 (frame +3) |

**Table 3-NL**

| **Target ID** | **cDNA SEQ ID NO** | **Corresponding amino acid sequence of cDNA clone** |
|---|---|---|
| NL001 | 1071 | SEQ ID NO: 1072 (frame +2) KSWMLDKLGGVYAPRPSTGPHKLRESLPLVIFLRNRLKYALTNCEVKKIVMQRLIKVDGKVRTDPNYPAGFMDVVQIEK TNEFFRLIYDVKGRFTIHRITAEEAKYKLCKVKRVQTGPKGIPFLTTHDGRTIRYPDPLVKVNDTIQLDIATSKIMDFIRFDS GNLCMITGGRNLGRVGTVVNRERHPGSFDIVHIKDVLGHTFATRLNNVFIIGKGSKAYVSLPKGKGVKLS |
| NL002 | 1073 | SEQ ID NO: 1074 (frame +1) |
| NL003 | 1075 | SEQ ID NO: 1076 (frame +2) |
| NL004 | 1077 | SEQ ID NO: 1078 (frame +1) |
| NL005 | 1079 | SEQ ID NO: 1080 (frame +1) |
| NL006 | 1081 | SEQ ID NO: 1082 (frame +3) |
| NL007 | 1083 | SEQ ID NO: 1084 (frame +2) |
| NL008 | 1085 | SEQ ID NO: 1086 (frame +1) |
| NL009 | 1087 | SEQ ID NO: 1088 (frame +1) |
| NL010 | 1089 | SEQ ID NO: 1090 (amino terminus end) (frame +2) |
| | 1115 | SEQ ID NO: 1116 (carboxy terminus end) (frame +3) |
| NL011 | 1091 | SEQ ID NO: 1092 (frame +2) |
| NL012 | 1093 | SEQ ID NO: 1094 (frame +2) |
| NL013 | 1095 | SEQ ID NO: 1096 (frame +2) |
| NL014 | 1097 | SEQ ID NO: 1098 (frame +2) |
| NL015 | 1099 | SEQ ID NO: 1100 (frame +1) |
| NL016 | 1101 | SEQ ID NO: 1102 (frame +2) |
| NL018 | 1103 | SEQ ID NO: 1104 (frame +2) |
| NL019 | 1105 | SEQ ID NO: 1106 (frame +2) |
| NL021 | 1107 | SEQ ID NO: 1108 (frame +2) |
| NL022 | 1109 | SEQ ID NO: 1110 (frame +2) |
| NL023 | 1111 | SEQ ID NO: 1112 (frame +2) |
| NL027 | 1113 | SEQ ID NO: 1114 (frame +2) |

**Table 3-CS**

| **Target ID** | **cDNA SEQ ID NO** | **Corresponding amino acid sequence of cDNA clone** |
|---|---|---|
| CS001 | 1682 | SEQ ID NO: 1683 (frame +1) KAWMLDKLGGVYAPRPSTGPHKLRECLPLVIFLRNRLKYALTGNEVLKIVKQRLIKVDGKVRTDPTYPAGFMDVV SIEKTNELFRLIYDVKGRFTIHRITPEEAKYKLCKVRRVATGPKNVPYLVTHDGRTVRYPDPLIKVNDSIQLDIATSK IMDFIKFESGNLCMITGGRNLGRVGTIVSRERHPGSFDIVHIRDSTGHTFATRLNNVFIIGKGTKAYISLPRGKGVR LT |
| CS002 | 1684 | SEQ ID NO: 1685 (frame +1) |
| CS003 | 1686 | SEQ ID NO: 1687 (frame +1) |
| CS006 | 1688 | SEQ ID NO: 1689 (frame +1) |
| CS007 | 1690 | SEQ ID NO: 1691 (frame +3) |
| CS009 | 1692 | SEQ ID NO: 1693 (frame +1) |
| CS011 | 1694 | SEQ ID NO: 1695 (frame +1) |
| CS013 | 1696 | SEQ ID NO: 1697 (frame +2) |
| CS014 | 1698 | SEQ ID NO: 1699 (frame +2) |
| CS015 | 1700 | SEQ ID NO: 1701 (frame +1) |
| CS016 | 1702 | SEQ ID NO: 1703(frame -3) |
| CS018 | 1704 | SEQ ID NO: 1705 (frame +2) |

**Table 3-PX**

| **Target ID** | **cDNA SEQ ID NO** | **Corresponding amino acid sequence of cDNA clone** |
|---|---|---|
| PX001 | 2100 | SEQ ID NO: 2101 (frame +1) GPKKHLKRLNAPRAWMLDKLGGVYAPRPSTGPHKLRECLPLVIFLQPPQVRAQRQRGAEDREAAPHQGGRQGPH RPHLPGWIHGCCVD*KDQ*AVPSDLRCEGTLHHPPHHSRGGQVQAVQGEARGDGPQERAVHRDAQRPHAALPRP AHQGQRLHPARHRHLQDHGHHQVRLR*PVHDHGRA*LGASGHHRVPREAPRELRHRPHQGHHRTHLRHQVEQRV HHRQGHE |
| PX009 | 2102 | SEQ ID NO: 2103 (frame +3) |
| PX010 | 2104 | SEQ ID NO: 2105 (frame +3) |
| PX015 | 2106 | SEQ ID NO: 2107 (frame +3) |
| PX016 | 2108 | SEQ ID NO: 2109 (frame +2) |

**Table 3-AD**

| **Target ID** | **cDNA SEQ ID NO** | **Corresponding amino acid sequence of cDNA clone** |
|---|---|---|
| AD001 | 2364 | SEQ ID NO: 2365 (frame +1) GPKKHLKRLNAPKAWMLDKLGGVFAPRPSTGPHKLRECLPLVIFLRNRLKYALTNCEVTKIVMQRLIKVDGKVRTDPN YPAGFMDVVTIEKTGEFFRLVYDVKGRFTIHRISAEEAKYKLCKVRRVQTGPKGIPFLVTHDGRTIRYPDPVIKVNDSI QLDIATCKIMDHIRFESGNLCMITGGRNLGRVGTVVSRERHPGSFDIVHIKDTQGHTFATRLNNVFIIGKATKPYISLPK GKGVKLSIAEERDK |
| AD002 | 2366 | SEQ ID NO: 2367 (frame +2) |
| AD009 | 2368 | SEQ ID NO: 2369 (frame +3) |
| AD015 | 2370 | SEQ ID NO: 2371 (frame +2) |
| AD016 | 2372 | SEQ ID NO: 2373 (frame +2) |

**Table 4-LD/PC/AG/TC/MP/NUCS/PX/AD**

| **Target ID** | **SEQ ID NO** | **Sequences*** | **Example Gi-number and species** |
|---|---|---|---|
| LD001 | 49 | GGCCCCAAGAAGCATTTGAAGCGTTT | 3101175 (Drosophila melanogaster), 92477283 (Drosophila erecta) |
| LD001 | 50 | AATGCCCCAAAAGCATGGATGTTGGATAAA TTGGGAGGTGT | 70909480 (Carabus granulatus), 77325294 (Chironomus tentans), 900945 (Ctenocephalides felis), 60297219 (Diaprepes abbreviatus), 37951951 (Ips pini), 75735533 (Tribolium castaneum), 22039624 (Ctenocephalides felis) |
| LD001 | 51 | GAAGTTACTAAGATTGTTATGCA | 33368080 (Glossina morsitans) |
| LD001 | 52 | ATTGAAAAAACTGGTGAATTTTTCCG | 60297219 (Diaprepes abbreviatus) |
| LD001 | 53 | ACACACGACGGCCGCACCATCCGCT | 27555937 (Anopheles gambiae), 33355008 (Drosophila yakuba), 22474232 (Helicoverpa armigera), 3738704 (Manduca sexta) |
| LD001 | 54 | ACACACGACGGCCGCACCATCCGCTA | 92477283 (Drosophila erecta) |
| LD001 | 55 | CCCAAGAAGCATTTGAAGCGTTTG | 92954810 (Drosophila ananassae), 92231605 (Drosophila willistoni) |
| PC001 | 275 | AAAATTGTCATGCAAAGGTTGAT | 37952206 (Ips pini) |
| PC001 | 276 | AAAGCATGGATGTTGGACAAA | 98994282 (Antheraea mylitta) 109978109 (Gryllus pennsylvanicus) 55904580 (Locusta migratoria) |
| PC001 | 277 | AAAGCATGGATGTTGGACAAATT | 31366663 (Toxoptera citricida) |
| PC001 | 278 | AAAGCATGGATGTTGGACAAATTGGG | 60311985 (Papilio dardanus) |
| PC001 | 279 | AAAGCATGGATGTTGGACAAATTGGGGGGTGT | 37951951 (Ips pini) |
| PC001 | 280 | AAATACAAGTTGTGTAAAGTAA | 84647793 (Myzus persicae) |
| PC001 | 281 | AAGCATGGATGTTGGACAAATTGGGGGGTGT | 70909486 (Mycetophagus quadripustulatus) |
| PC001 | 282 | ATGGATGTCATTACTATTGAGAA | 25957367 (Carabus granulatus) |
| PC001 | 283 | CATCAAATTTGAATCTGGCAACCT | 37952206 (Ips pini) |
| PC001 | 284 | CATGATGGCAGAACCATTCGTTA | 60303405 (Julodis onopordi) |
| PC001 | 285 | CCAAAGCATGGATGTTGGACAA | 90138164 (Spodoptera frugiperda) |
| PC001 | 286 | CCATTTTTGGTAACACATGATGG | 111011915 (Apis mellifera) |
| PC001 | 287 | CCCAAAGCATGGATGTTGGACAA | 50565112 (Homalodisca coagulata) |
| PC001 | 288 | CCCAAAGCATGGATGTTGGACAAA | 103790417 (Heliconius erato) 101419954 (Plodia interpunctella) |
| PC001 | 289 | CCCAAAGCATGGATGTTGGACAAATT | 73612809 (Aphis gossypii) |
| PC001 | 290 | CCCAAAGCATGGATGTTGGACAAATTGGG | 77329254 (Chironomus tentans) |
| PC001 | 291 | CCCAAAGCATGGATGTTGGACAAATTGGGGGGTGT | 60305420 (Mycetophagus quadripustulatus) |
| PC001 | 292 | CCCAAAGCATGGATGTTGGACAAATTGGGGGGTGTCTTCGC | 84647995 (Myzus persicae) |
| PC001 | 293 | CGTTACCCTGACCCCAACATCAA | 73613065 (Aphis gossypii) |
| PC001 | 294 | GCAAAATACAAGTTGTGTAAAGTAA | 83662334 (Myzus persicae) |
| PC001 | 295 | GCATGGATGTTGGACAAATTGGG | 92969396 (Drosophila grimshawi) |
| PC001 | 296 | GCATGGATGTTGGACAAATTGGGGG | 67885868 (Drosophila pseudoobscura) |
| PC001 | 297 | GCATGGATGTTGGACAAATTGGGGGGTGT | 25956479 (Biphyllus lunatus) |
| PC001 | 298 | GCATGGATGTTGGACAAATTGGGGGGTGTCT | 90814901 (Nasonia vitripennis) |
| PC001 | 299 | GCTCCCAAAGCATGGATGTTGGA | 110260785 (Spodoptera frugiperda) |
| PC001 | 300 | GCTCCCAAAGCATGGATGTTGGACAA | 76551269 (Spodoptera frugiperda) |
| PC001 | 301 | GCTCCCAAAGCATGGATGTTGGACAAA | 56085210 (Bombyx mori) |
| PC001 | 302 | GCTCCCAAAGCATGGATGTTGGACAAATTGGG | 22474232 (Helicoverpa armigera) |
| PC001 | 303 | GGTCCCAAAGGAATCCCATTTTTGGT | 50565112 (Homalodisca coagulata) |
| PC001 | 304 | GGTGTCTTCGCCCCTCGTCCA | 82575022 (Acyrthosiphon pisum) |
| PC001 | 305 | GTGAAGTCACTAAAATTGTCATGCAAAG | 25956820 (Biphyllus lunatus) |
| PC001 | 306 | TCCACCGGGCCTCACAAGTTGCG | 58371410 (Lonomia obliqua) |
| PC001 | 307 | TCCCAAAGCATGGATGTTGGA | 110263957 (Spodoptera frugiperda) |
| PC001 | 308 | TGCTCCCAAAGCATGGATGTTGGACAA | 48927129 (Hydropsyche sp.) |
| PC001 | 309 | TGGATGTTGGACAAATTGGGGGGTGTCT | 90814560 (Nasonia vitripennis) |
| AG001 | 621 | AAAACTGGTGAATTCTTCCGTTTGAT | 37953169 (Ips pini) |
| AG001 | 622 | AAAGCATGGATGTTGGACAAA | 98994282 (Antheraea mylitta) 109978109 (Gryllus pennsylvanicus) 55904580 (Locusta migratoria) |
| AG001 | 623 | AAAGCATGGATGTTGGACAAATT | 31366663 (Toxoptera citricida) |
| AG001 | 624 | AAAGCATGGATGTTGGACAAATTGGG | 60311985 (Papilio dardanus) |
| AG001 | 625 | AAAGCATGGATGTTGGACAAATTGGGGGGTGT | 37951951 (Ips pini) 109195107 (Myzus persicae) |
| AG001 | 626 | AAATACAAATTGTGCAAAGTCCG | 25958703 (Curculio glandium) |
| AG001 | 627 | AACTTGTGCATGATCACCGGAG | 22039624 (Ctenocephalides felis) |
| AG001 | 628 | AAGCATGGATGTTGGACAAATTGGGGG | 112433559 (Myzus persicae) |
| AG001 | 629 | AAGCATGGATGTTGGACAAATTGGGGGGTGTGTT | 70909486 (Mycetophagus quadripustulatus) |
| AG001 | 630 | ACTGGTGAATTCTTCCGTTTGAT | 77327303 (Chironomus tentans) |
| AG001 | 631 | ATTGAAAAAACTGGTGAATTCTTCCGTTTGATCTATGATGTTA A | 22039624 (Ctenocephalides felis) |
| AG001 | 632 | CCAAAGCATGGATGTTGGACAA | 90138164 (Spodoptera frugiperda) |
| AG001 | 633 | CCCAAAGCATGGATGTTGGACAA | 48927129 (Hydropsyche sp.) 76551269 (Spodoptera frugiperda) |
| AG001 | 634 | CCCAAAGCATGGATGTTGGACAAA | 91835558 (Bombyx mori) 103783745 (Heliconius erato) 101419954 (Plodia interpunctella) |
| AG001 | 635 | CCCAAAGCATGGATGTTGGACAAATT | 73619372 (Aphis gossypii) |
| AG001 | 636 | CCCAAAGCATGGATGTTGGACAAATTGGG | 77329254 (Chironomus tentans) 22474232 (Helicoverpa armigera) |
| AG001 | 637 | CCCAAAGCATGGATGTTGGACAAATTGGGGG | 84647382 (Myzus persicae) |
| AG001 | 638 | CCCAAAGCATGGATGTTGGACAAATTGGGGGGTGT | 84647995 (Myzus persicae) |
| AG001 | 639 | CCCAAAGCATGGATGTTGGACAAATTGGGGGGTGTGTT | 60305420 (Mycetophagus quadripustulatus) |
| AG001 | 640 | CTGGATTCATGGATGTGATCA | 27617172 (Anopheles gambiae) |
| AG001 | 641 | GAATTCTTCCGTTTGATCTATGATGT | 50565112 (Homalodisca coagulata) 71049326 (Oncometopia nigricans) |
| AG001 | 642 | GCATGGATGTTGGACAAATTGGG | 92969396 (Drosophila grimshawi) 93001617 (Drosophila mojavensis) 92929731 (Drosophila virilis) |
| AG001 | 643 | GCATGGATGTTGGACAAATTGGGGG | 67885868 (Drosophila pseudoobscura) |
| AG001 | 644 | GCATGGATGTTGGACAAATTGGGGGGTGT | 90814901 (Nasonia vitripennis) |
| AG001 | 645 | GCATGGATGTTGGACAAATTGGGGGGTGTGTTCGCCCC | 25956479 (Biphyllus lunatus) |
| AG001 | 646 | GCCCCCAAAGCATGGATGTTGGACAA | 50565112 (Homalodisca coagulata) |
| AG001 | 647 | GCTGGATTCATGGATGTGATC | 103775903 (Heliconius erato) |
| AG001 | 648 | GGATCATTCGATATTGTCCACAT | 113017118 (Bemisia tabaci) |
| AG001 | 649 | GGCAACTTGTGCATGATCACCGGAGG | 25958703 (Curculio glandium) |
| AG001 | 650 | TACAAATTGTGCAAAGTCCGCAA | 56161193 (Rhynchosciara americana) |
| AG001 | 651 | TATCCTGCTGGATTCATGGATGT | 40934103 (Bombyx mori) |
| AG001 | 652 | TCACCATTGAAAAAACTGGTGAATTCTTC | 62083410 (Lysiphlebus testaceipes) |
| AG001 | 653 | TGCATGATCACCGGAGGCAGGAA | 3478550 (Antheraea yamamai) |
| AG001 | 654 | TGCATGATCACCGGAGGCAGGAATTTGGG | 14627585 (Drosophila melanogaster) 33355008 (Drosophila yakuba) |
| AG001 | 655 | TGGATGTTGGACAAATTGGGGGGTGT | 90814560 (Nasonia vitripennis) |
| AG001 | 656 | TGTGCATGATCACCGGAGGCAG | 92949859 (Drosophila ananassae) 92999306 (Drosophila grimshawi) |
| AG001 | 657 | TGTGCATGATCACCGGAGGCAGGAATTTGGG | 67842487 (Drosophila pseudoobscura) |
| TC001 | 813 | AAAGCATGGATGTTGGATAAA | 70909480 (Carabus granulatus) 16898765 (Ctenocephalides felis) 60298000 (Diaprepes abbreviatus) |
| TC001 | 814 | AATTTGTGTATGATTACTGGAGG | 55904576 (Locusta migratoria) |
| TC001 | 815 | ACTGGAGGTCGTAACTTGGGGCGTGT | 60298000 (Diaprepes abbreviatus) |
| TC001 | 816 | ATGATTACTGGAGGTCGTAACTTGGGGCGTGT | 73619372 (Aphis gossypii) 37804548 (Rhopalosiphum padi) |
| TC001 | 817 | ATGCAAAGATTGATTAAAGTTGACGG | 70909478 (Biphyllus lunatus) |
| TC001 | 818 | ATTAAAGTTGACGGAAAAGTT | 110763874 (Apis mellifera) |
| TC001 | 819 | ATTGAGAAAACTGGGGAATTCTTCCG | 37952206 (Ips pini) |
| TC001 | 820 | ATTGTTATGCAAAGATTGATTAAAGTTGACGGAAAAGT | 70909486 (Mycetophagus quadripustulatus) |
| TC001 | 821 | CCAAGAAGCATTTGAAGCGTCT | 55904580 (Locusta migratoria) |
| TC001 | 822 | CCAAGAAGCATTTGAAGCGTCTC | 83935971 (Lutzomyia longipalpis) |
| TC001 | 823 | GCGCCCAAAGCATGGATGTTGGA | 103790417 (Heliconius erato) 101419954 (Plodia interpunctella) |
| TC001 | 824 | GGCCCCAAGAAGCATTTGAAGCGT | 14700642 (Drosophila melanogaster) |
| TC001 | 825 | TGATTACTGGAGGTCGTAACTTGGGGCGTGT | 73612212 (Aphis gossypii) |
| TC001 | 826 | TGTATGATTACTGGAGGTCGTAACTTGGGGCGTGT | 70909478 (Biphyllus lunatus) |
| TC001 | 827 | TTGATTTATGATGTTAAGGGA | 77325485 (Chironomus tentans) |
| TC001 | 828 | TTGTGTATGATTACTGGAGGTCGTAA | 60305816 (Mycetophagus quadripustulatus) |
| MP001 | 908 | AAAGCATGGATGTTGGACAAA | 98994282 (Antheraea mylitta) 108789768 (Bombyx mori) 109978109 (Gryllus pennsylvanicus) 55904580 (Locusta migratoria) |
| MP001 | 909 | AAAGCATGGATGTTGGACAAAT | 77325485 (Chironomus tentans) 37951951 (Ips pini) 60311985 (Papilio dardanus) 30031258 (Toxoptera citricida) |
| MP001 | 910 | AAGAAGCATTTGAAGCGTTTAAACGCACC | 3658572 (Manduca sexta) |
| MP001 | 911 | AAGCATTTGAAGCGTTTAAACGC | 103790417 (Heliconius erato) 22474232 (Helicoverpa armigera) |
| MP001 | 912 | AAGCATTTGAAGCGTTTAAACGCACC | 25957217 (Carabus granulatus) |
| MP001 | 913 | AAGTCCGTACCGACCCTAATTATCCAGC | 46994131 (Acyrthosiphon pisum) |
| MP001 | 914 | ACGCACCCAAAGCATGGATGTT | 46999037 (Acyrthosiphon pisum) |
| MP001 | 915 | ACTATTAGATACGATATTGCA | 46998791 (Acyrthosiphon pisum) |
| MP001 | 916 | ACTGGACCCAAAGGTGTGCCATTTTTAACTACTCATGATGGC CGTACTAT | 46997137 (Acyrthosiphon pisum) |
| MP001 | 917 | AGAAGCATTTGAAGCGTTTAAA | 27620566 (Anopheles gambiae) |
| MP001 | 918 | AGAAGCATTTGAAGCGTTTAAACGCACC | 98994282 (Antheraea mylitta) |
| MP001 | 919 | AGAAGCATTTGAAGCGTTTAAACGCACCCAAAGCATGGATGT TGGACAAAT | 73619191 (Aphis gossypii) |
| MP001 | 920 | AGTAAGGGAGTTAAATTGACTA | 46998791 (Acyrthosiphon pisum) |
| MP001 | 921 | ATACAAGTTGTGTAAAGTAAAG | 29553519 (Bombyx mori) |
| MP001 | 922 | ATGGATGTTATATCTATCCAAAAGACCAGTGAGCACTTTAGAT TGATCTATGATGTGAAAGGTCGTTTCAC | 46998791 (Acyrthosiphon pisum) |
| MP001 | 923 | ATTGATCTATGATGTGAAAGGTCGTTTCAC | 46999037 (Acyrthosiphon pisum) |
| MP001 | 924 | CAAAAGACCAGTGAGCACTTTAGATTGAT | 30031258 (Toxoptera citricida) |
| MP001 | 925 | CACAGAATTACTCCTGAAGAAGC | 73619191 (Aphis gossypii) |
| MP001 | 926 | CACAGAATTACTCCTGAAGAAGCAAAATACAAG | 46998791 (Acyrthosiphon pisum) 30031258 (Toxoptera citricida) |
| MP001 | 927 | CATCCAGGATCTTTTGATATTGTTCACATTAA | 31364848 (Toxoptera citricida) |
| MP001 | 928 | CATCCAGGATCTTTTGATATTGTTCACATTAAGGATGCAAATG AACATATTTTTGCTAC | 37804548 (Rhopalosiphum padi) |
| MP001 | 929 | CATCTAAAATTTTGGATCATATCCGTTTTGAAACTGGAAACTT GTGCATGAT | 46998791 (Acyrthosiphon pisum) |
| MP001 | 930 | CATTTGAAGCGTTTAAACGCACC | 30031258 (Toxoptera citricida) |
| MP001 | 931 | CATTTGAAGCGTTTAAACGCACCCAAAGCATGGATGTT | 46998791 (Acyrthosiphon pisum) |
| MP001 | 932 | CCAAAGCATGGATGTTGGACAA | 90138164 (Spodoptera frugiperda) |
| MP001 | 933 | CCAAGGAGTAAGGGAGTTAAATTGACTA | 73615238 (Aphis gossypii) 31364848 (Toxoptera citricida) |
| MP001 | 934 | CCCAAAGCATGGATGTTGGAC | 108789768 (Bombyx mori) |
| MP001 | 935 | CCCAAAGCATGGATGTTGGACAA | 50565112 (Homalodisca coagulata) 48927129 (Hydropsyche sp.) 76551269 (Spodoptera frugiperda) |
| MP001 | 936 | CCCAAAGCATGGATGTTGGACAAA | 56085210 (Bombyx mori) 103792451 (Heliconius erato) 101419954 (Plodia interpunctella) |
| MP001 | 937 | CCCAAAGCATGGATGTTGGACAAAT | 22474095 (Helicoverpa armigera) |
| MP001 | 938 | CGTCCAAGCACCGGTCCACACAAACT | 47537863 (Acyrthosiphon pisum) |
| MP001 | 939 | CTGGAAACTTGTGCATGATAACTGGAGG | 78524585 (Glossina morsitans) |
| MP001 | 940 | GAAAGACATCCAGGATCTTTTGATATTGTTCACATTAAGGATG CAAATGAACATATTTTGCTACCCGGATGAACAATGTTTTTAT TATTGGAAAAGGTCAAAAGAACTACATTTCTCTACCAAG | 46997137 (Acyrthosiphon pisum) |
| MP001 | 941 | GATCATATCCGTTTTGAAACTGGAAACTTGTGCATGAT | 73614725 (Aphis gossypii) |
| MP001 | 942 | GATGCAAATGAACATATTTTTGCTAC | 31364848 (Toxoptera citricida) |
| MP001 | 943 | GCACCCAAAGCATGGATGTTGGA | 70909486 (Mycetophagus quadripustulatus) |
| MP001 | 944 | GCACCCAAAGCATGGATGTTGGACAAAT | 77329254 (Chironomus tentans) 60305420 (Mycetophagus quadripustulatus) |
| MP001 | 945 | GGATCTTTTGATATTGTTCACAT | 60303405 (Julodis onopordi) |
| MP001 | 946 | GGATCTTTTGATATTGTTCACATTAAGGATGCAAATGAACATA | 73619191 (Aphis gossypii) |
| | | TTTTTGCTAC | |
| MP001 | 947 | GGCCCCAAGAAGCATTTGAAGCGTTTAA | 14693528 (Drosophila melanogaster) |
| MP001 | 948 | GGGCGTGTTGGTATTGTTACCAACAG | 31365398 (Toxoptera citricida) |
| MP001 | 949 | GGGCGTGTTGGTATTGTTACCAACAGGGAAAG | 73612212 (Aphis gossypii) 37804548 (Rhopalosiphum padi) |
| MP001 | 950 | GGTACAAACTGGACCCAAAGG | 60297572 (Diaprepes abbreviatus) |
| MP001 | 951 | GTTTTTATTATTGGAAAAGGTCAAAAGAACTACATTTCTCT | 73619191 (Aphis gossypii) 31364848 (Toxoptera citricida) |
| MP001 | 952 | TGAAGTATGCACTTACTGGTGC | 73619191 (Aphis gossypii) |
| MP001 | 953 | TGTAAAGTAAAGAGGGTACAAACTGGACCCAAAGGTGT | 73619191 (Aphis gossypii) |
| MP001 | 954 | TGTGTAAAGTAAAGAGGGTACAAACTGGACCCAAAGGTGT | 30031258 (Toxoptera citricida) |
| MP001 | 955 | TTCTTGCGTAATCGTTTGAAGTATGCACTTACTGGTGCCGAA GTCACCAAGATTGTCATGCAAAGATTAATCAAGGTTGATGGC AAAGTCCGTACCGACCCTAATTATCCAGC | 46998791 (Acyrthosiphon pisum) |
| NL001 | 1161 | CTGAAGAAGCTAAGTACAAGCT | 16566724 (Spodoptera frugiperda) |
| NL001 | 1162 | TTCTTCCGTTTGATCTATGATGTTAA | 16900870 (Ctenocephalides felis) |
| NL001 | 1163 | CAGCTGAAGAAGCTAAGTACAA | 16900870 (Ctenocephalides felis), 56199521 (Culicoides sonorensis) |
| NL001 | 1164 | GAGTTCTTCCGTTTGATCTATGATGTTAA | 16900945 (Ctenocephalides felis) |
| NL001 | 1165 | AAGTACAAGCTGTGCAAAGTGAAG | 22474232 (Helicoverpa armigera) |
| NL001 | 1166 | TTCGACATCGTGCACATCAAGGAC | 22474232 (Helicoverpa armigera) |
| NL001 | 1167 | ATCACAGCTGAAGAAGCTAAGTACAAG | 25956820 (Biphyllus lunatus) |
| NL001 | 1168 | TGTGTATGATCACTGGAGGTCGTAA | 25957367 (Carabus granulatus) |
| NL001 | 1169 | AACGTTTTCATCATCGGCAAG | 27613698 (Anopheles gambiae) |
| NL001 | 1170 | CCAAAATCATGGACTTCATCA | 3738704 (Manduca sexta) |
| NL001 | 1171 | TGATCTATGATGTTAAGGGACG | 3738704 (Manduca sexta) |
| NL001 | 1172 | CATGGATGTTGGACAAATTGGG | 37951951 (Ips pini), 56772312 (Drosophila virilis), 60305420 (Mycetophagus quadripustulatus), 67885868 (Drosophila pseudoobscura), 77321575 (Chironomus tentans), 25956479 (Biphyllus lunatus), 22474232 (Helicoverpa armigera); |
| NL001 | 1173 | TTTTGCCACTAGGTTGAACAACGT | 37953169 (Ips pini) |
| NL001 | 1174 | GCAGCGTCTCATCAAGGTTGACGGCAA | 48927129 (Hydropsyche sp.) |
| NL001 | 1175 | AAGGGACGTTTCACCATCCAC | 50818668 (Heliconius melpomene) |
| NL001 | 1176 | AACCTGTGTATGATCACTGGAGG | 60293875 (Homalodisca coagulata) |
| NL001 | 1177 | ACTAACTGTGAAGTGAAGAAAATTGT | 60293875 (Homalodisca coagulata) |
| NL001 | 1178 | TTCTTCCGTTTGATCTATGATGT | 60293875 (Homalodisca coagulata), 71047771 (Oncometopia nigricans) |
| NL001 | 1179 | TGTATGATCACTGGAGGTCGTAACTTGGG | 60297219 (Diaprepes abbreviatus) |
| NL001 | 1180 | CATGGATGTTGGACAAATTGGGTGG | 60311985 (Papilio dardanus) |
| NL001 | 1181 | GCTGAAGAAGCTAAGTACAAG | 68758383 (Acanthoscurria gomesiana) |
| NL001 | 1182 | GGAGGTCGTAACTTGGGTCGTGT | 77327303 (Chironomus tentans) |
| NL001 | 1183 | TATGATGTTAAGGGACGTTTCACCAT | 71327303 (Chironomus tentans) |
| NL001 | 1184 | CATGGATGTTGGACAAATTGGG | 93002561 (Drosophila grimshawi) 93001617 (Drosophila mojavensis) 92939328 (Drosophila virilis) 112433559 (Myzus persicae) 90814922 (Nasonia vitripennis) |
| NL001 | 1185 | CTGAAGAAGCTAAGTACAAGCT | 110264122 (Spodoptera frugiperda) |
| NL001 | 1186 | GAAGAAGCTAAGTACAAGCTGTG | 90820001 (Graphocephala atropunctata) |
| NL001 | 1187 | TTGCACAGCTTGTACTTAGCTTCTTC | 90134075 (Bicyclus anynana) |
| NL001 | 1188 | AAGTACAAGCTGTGCAAAGTGAAG | 112350104 (Helicoverpa armigera) |
| NL001 | 1189 | ATGATCACTGGAGGTCGTAACTTGGGTCG | 113017118 (Bemisia tabaci) |
| NL001 | 1190 | GGTCGTAACTTGGGTCGTGTGGG | 109978109 (Gryllus pennsylvanicus) |
| NL001 | 1191 | TTCGACATCGTGCACATCAAGGAC | 112350104 (Helicoverpa armigera) |
| NL001 | 1192 | ACATCGTGCACATCAAGGACG | 90981811 (Aedes aegypti) |
| CS001 | 1730 | AAAGCATGGATGTTGGACAAA | 73619372 (Aphis gossypii); 77325485 (Chironomus tentans); 22474232 (Helicoverpa armigera); 37951951 (Ips pini); 60305420 (Mycetophagus quadripustulatus); 84647995 (Myzus persicae) |
| CS001 | 1731 | AAAGCATGGATGTTGGACAAACT | 40877657 (Bombyx mori); 103783745 (Heliconius erato); 55904580 (Locusta migratoria); 101413238 (Plodia interpunctella) |
| CS001 | 1732 | AACCGGCTCAAGTACGCGCTCAC | 22474232 (Helicoverpa armigera) |
| CS001 | 1733 | AACCGGCTCAAGTACGCGCTCACCGG | 90134075 (Bicyclus anynana) |
| CS001 | 1734 | AAGATCATGGACTTCATCAAGTT | 90134075 (Bicyclus anynana) |
| CS001 | 1735 | ACCAGATTGAACAACGTGTTCAT | 71536878 (Diaphorina citri) 3658573 (Manduca sexta) |
| CS001 | 1736 | ATCATGGACTTCATCAAGTTTGAATC | 103783745 (Heliconius erato) |
| CS001 | 1737 | CAAGATCATGGACTTCATCAAGTT | 3478550 (Antheraea yamamai) |
| CS001 | 1738 | CCCCACAAGTTGCGCGAGTGC | 63011732 (Bombyx mori) |
| CS001 | 1739 | CCCGCTGGATTTATGGATGTTGT | 101403940 (Plodia interpunctella) |
| CS001 | 1740 | CCTCCAAGATCATGGACTTCATCAAGTT | 22474232 (Helicoverpa armigera) |
| CS001 | 1741 | CCTGCCGCTGGTGATCTTCCT | 27597800 (Anopheles gambiae) |
| CS001 | 1742 | CGACGGGCCCCAAGAACGTGCC | 22474232 (Helicoverpa armigera) |
| CS001 | 1743 | CTCATCAAGGTCAACGACTCC | 103783745 (Heliconius erato) 112350001 (Helicoverpa armigera) 101418268 (Plodia interpunctella) |
| CS001 | 1744 | CTCATCAAGGTCAACGACTCCATCCAGCTCGACAT | 3738704 (Manduca sexta) |
| CS001 | 1745 | CTCATCAAGGTCAACGACTCCATCCAGCTCGACATC GCCACCT | 53884106 (Plutella xylostella) |
| CS001 | 1746 | CTGCCGCTGGTGATCTTCCTC | 27603050 (Anopheles gambiae) |
| CS001 | 1747 | GACCCCACATATCCCGCTGGATT | 103783745 (Heliconius erato) |
| CS001 | 1748 | GCAGCGACTTATCAAAGTTGA | 109978109 (Gryllus pennsylvanicus) |
| CS001 | 1749 | GCATGGATGTTGGACAAACTGGG | 67899746 (Drosophila pseudoobscura) |
| CS001 | 1750 | GCCACCTCCAAGATCATGGACTTCAT | 110259010 (Spodoptera frugiperda) |
| CS001 | 1751 | GCGCGTGGCGACGGGCCCCAAGAACGTGCC | 53884106 (Plutella xylostella) |
| CS001 | 1752 | GCTGGATTTATGGATGTTGTTT | 29553519 (Bombyx mori) |
| CS001 | 1753 | GGCTCAAGTACGCGCTCACCGG | 5498893 (Antheraea yamamai) |
| CS001 | 1754 | GTGGGCACCATCGTGTCCCGCGAG | 3953837 (Bombyx mandarina) 53884106 (Plutelia xylostella) |
| CS001 | 1755 | GTGGGCACCATCGTGTCCCGCGAGCG | 3478550 (Antheraea yamamai) |
| CS001 | 1756 | GTGGGCACCATCGTGTCCCGCGAGCGACATCCCGG | 22474232 (Helicoverpa armigera) |
| CS001 | 1757 | TAAAGCATGGATGTTGGACAA | 58371410 (Lonomia obliqua) |
| CS001 | 1758 | TAAAGCATGGATGTTGGACAAA | 60311985 (Papilio dardanus) 31366663 (Toxoptera citricida) |
| CS001 | 1759 | TAAAGCATGGATGTTGGACAAACT | 109978109 (Gryllus pennsylvanicus) |
| CS001 | 1760 | TAAAGCATGGATGTTGGACAAACTGGG | 98994282 (Antheraea mylitta) |
| CS001 | 1761 | TACAAGCTGTGCAAGGTGCGGCGCGTGGCGACGGG CCC | 98993531 (Antheraea mylitta) |
| CS001 | 1762 | TACAAGCTGTGCAAGGTGCGGCGCGTGGCGAC GGGCCCCAA | 5498893 (Antheraea yamamai) |
| CS001 | 1763 | TACCCCGACCCACTCATCAAGGT | 90134075 (Bicyclus anynana) |
| CS001 | 1764 | TGAACAACGTGTTCATAATCGG | 98993531 (Antheraea mylitta) |
| CS001 | 1765 | TGCGCGAGTGCCTGCCGCTGGT | 22474232 (Helicoverpa armigera) |
| CS001 | 1766 | TGTATGATCACGGGAGGCCGTAACTTGGG | 60311445 (Euclidia glyphica) |
| CS001 | 1767 | TGTATGATCACGGGAGGCCGTAACTTGGGGCG | 3953837 (Bombyx mandarina) |
| CS001 | 1768 | TGTATGATCACGGGAGGCCGTAACTTGGGGCG CGTGGGCACCATCGTGTCCCGCGAG | 91826697 (Bombyx mori) |
| CS001 | 1769 | TGTGCAAGGTGCGGCGCGTGGCGACGGGCCC CAAG | 3478550 (Antheraea yamamai) |
| CS001 | 1770 | TTGAACAACGTGTTCATAATCGGCAAGGGCACG AA | 3953837 (Bombyx mandarina) 40915191 (Bombyx mori) |
| PX001 | 2120 | AACAACGTGTTCATCATCGGCAAGGGCACGAA | 112350001 (Helicoverpa armigera) |
| PX001 | 2121 | AACGTGTTCATCATCGGCAAG | 27562760 (Anopheles gambiae) 58378595 (Anopheles gambiae str. PEST) |
| PX001 | 2122 | AACGTGTTCATCATCGGCAAGG | 42764924 (Armigeres subalbatus) |
| PX001 | 2123 | AACGTGTTCATCATCGGCAAGGG | 71048604 (Oncometopia nigricans) |
| PX001 | 2124 | AACGTGTTCATCATCGGCAAGGGCACGAA | 112783858 (Anopheles funestus) |
| PX001 | 2125 | AACTTGGGGCGAGTGGGCACCATCGTGTC | 90132259 (Bicyclus anynana) |
| PX001 | 2126 | AACTTGGGGCGAGTGGGCACCATCGTGTCCCGCGAG | 112350001 (Helicoverpa armigera) |
| PX001 | 2127 | AAGATCGTGAAGCAGCGCCTCATCAAGGTGGACGGCAAGGT | 112350001 (Helicoverpa armigera) |
| PX001 | 2128 | AAGGTCCGCACCGACCCCACCTA | 14627585 (Drosophila melanogaster) |
| PX001 | 2129 | AAGTACAAGCTGTGCAAGGTG | 5498893 (Antheraea yamamai) 90132259 (Bicyclus anynana) 92969396 (Drosophila grimshawi) 50818668 (Heliconius melpomene) 58371410 (Lonomia obliqua) |
| PX001 | 2130 | ACAACGTGTTCATCATCGGCAAGGGCACGAA | 103783745 (Heliconius erato) |
| PX001 | 2131 | ACGGCAAGGTCCGCACCGACCC | 77890923 (Aedes aegypti) |
| PX001 | 2132 | ACGGCCGCACGCTGCGCTACCCCGACCCGCTCATCAAGGTC AACGACTCC | 101413238 (Plodia interpunctella) |
| PX001 | 2133 | ACGTGTTCATCATCGGCAAGGGCAC | 109509107 (Culex pipiens) |
| PX001 | 2134 | AGGAGGCCAAGTACAAGCTGT | 27566312 (Anopheles gambiae) 67889891 (Drosophila pseudoobscura) |
| PX001 | 2135 | AGGAGGCCAAGTACAAGCTGTGCAAGGT | 92944919 (Drosophila ananassae) 67886177 (Drosophila pseudoobscura) 92045792 (Drosophila willistoni) |
| PX001 | 2136 | AGGAGGCCAAGTACAAGCTGTGCAAGGTG | 92929731 (Drosophila virilis) |
| PX001 | 2137 | CAACGTGTTCATCATCGGCAA | 109509107 (Culex pipiens) |
| PX001 | 2138 | CAACGTGTTCATCATCGGCAAGGGCA | 55816641 (Drosophila yakuba) |
| PX001 | 2139 | CACACCTTCGCCACCAGGTTGAACAACGTGTT | 3986403 (Antheraea yamamai) |
| PX001 | 2140 | CCCCAAGAAGCATTTGAAGCG | 2886669 (Drosophila melanogaster) |
| PX001 | 2141 | CCGAGGAGGCCAAGTACAAGCT | 92944919 (Drosophila ananassae) |
| PX001 | 2142 | CCGAGGAGGCCAAGTACAAGCTGTGCAAGGT | 15480750 (Drosophila melanogaster) |
| PX001 | 2143 | CCGCACAAGCTGCGCGAGTGCCTGCCGCT | 22474232 (Helicoverpa armigera) |
| PX001 | 2144 | CGACGGGCCCCAAGAACGTGCC | 112350001 (Helicoverpa armigera) |
| PX001 | 2145 | CGAGGAGGCCAAGTACAAGCT | 58378595 (Anopheles gambiae str. PEST) |
| PX001 | 2146 | CGAGGAGGCCAAGTACAAGCTG | 18914191 (Anopheles gambiae) |
| PX001 | 2147 | CGAGTGGGCACCATCGTGTCCCGCGAG | 3986403 (Antheraea yamamai) |
| PX001 | 2148 | CGCTACCCCGACCCGCTCATCAAGGTCAACGACTCC | 112350001 (Helicoverpa armigera) |
| PX001 | 2149 | CGCTTCACCATCCACCGCATCAC | 103783745 (Heliconius erato) |
| PX001 | 2150 | CGGCAACGAGGTGCTGAAGATCGT | 90132259 (Bicyclus anynana) |
| PX001 | 2151 | CGTAACTTGGGGCGAGTGGGCAC | 60311985 (Papilio dardanus) |
| PX001 | 2152 | CTACCCGGCTGGATTCATGGATGT | 42764924 (Armigeres subalbatus) |
| PX001 | 2153 | CTCATCAAGGTCAACGACTCC | 103783745 (Heliconius erato) |
| PX001 | 2154 | CTCATCAAGGTCAACGACTCCATCCAGCTCGACAT | 3738704 (Manduca sexta) |
| PX001 | 2155 | GACGGCAAGGTCCGCACCGAC | 109509107 (Culex pipiens) |
| PX001 | 2156 | GACGGCAAGGTCCGCACCGACCC | 77759638 (Aedes aegypti) |
| PX001 | 2157 | GAGGAGGCCAAGTACAAGCTGTGCAAGGT | 67841491 (Drosophila pseudoobscura) |
| PX001 | 2158 | GAGGAGGCCAAGTACAAGCTGTGCAAGGTG | 56772971 (Drosophila virilis) |
| PX001 | 2159 | GAGGCCAAGTACAAGCTGTGCAA | 112350001 (Helicoverpa armigera) |
| PX001 | 2160 | GAGGCCAAGTACAAGCTGTGCAAGGTG | 98993531 (Antheraea mylitta) |
| PX001 | 2161 | GCCAAGTACAAGCTGTGCAAGGT | 67838306 (Drosophila pseudoobscura) 109978109 (Gryllus pennsylvanicus) |
| PX001 | 2162 | GCCCCAAGAAGCATTTGAAGCG | 2151718 (Drosophila melanogaster) |
| PX001 | 2163 | GCGCGTGGCGACGGGCCCCAA | 5498893 (Antheraea yamamai) |
| PX001 | 2164 | GCGCGTGGCGACGGGCCCCAAG | 3986403 (Antheraea yamamai) |
| PX001 | 2165 | GGAGGCCAAGTACAAGCTGTGCAAGGT | 92942537 (Drosophila ananassae) |
| PX001 | 2166 | GGCCCCAAGAAGCATTTGAAGCG | 4459798 (Drosophila melanogaster) |
| PX001 | 2167 | GGCGGCGTGTACGCGCCGCGGCCC | 98994282 (Antheraea mylitta) |
| PX001 | 2168 | GTCCGCACCGACCCCACCTACCC | 92472430 (Drosophila erecta) 55854272 (Drosophila yakuba) |
| PX001 | 2169 | GTGGGCACCATCGTGTCCCGCGAGAG | 3953837 (Bombyx mandarina) |
| | | | 29554802 (Bombyx mori) |
| PX001 | 2170 | TCAAGGTGGACGGCAAGGTCCGCACCGACCC | 92944919 (Drosophila ananassae) |
| PX001 | 2171 | TGATCTACGATGTGAAGGGACG | 83935965 (Lutzomyia longipalpis) |
| PX001 | 2172 | TTCATGGATGTTGTGTCGATTGAAAA | 90132259 (Bicyclus anynana) |
| PX001 | 2173 | GCTGGATTCATGGATGTTGTG | 10707240 (Amblyomma americanum) |
| PX001 | 2174 | AAGCAGCGCCTCATCAAGGTGGACGGCAAGGTCCGCACCGA C | 49545866 (Rhipicephalus appendiculatus) |
| AD001 | 2384 | AAAGCATGGATGTTGGACAAA | 73619372 (Aphis gossypii); 77325485 (Chironomus tentans); 22474232 (Helicoverpa armigera); 37951951 (Ips pini); 60305420 (Mycetophagus quadripustulatus); 84647995 (Myzus persicae) |
| AD001 | 2385 | AAAGCATGGATGTTGGACAAACT | 94432102 (Bombyx mori); 103790417 (Heliconius erato); 55904580 (Locusta migratoria); 101419954 (Plodia interpunctella) |
| AD001 | 2386 | AAAGGTATTCCATTCTTGGTGACCCATGATGGCC GTACTATCCGTTATCCTGACCCAGTCATTAAAGT | 109978109 (Gryllus pennsylvanicus) |
| AD001 | 2387 | AACTGTGAAGTAACGAAGATTGTTATGCAGCGACT TATCAAAGTTGA | 109978109 (Gryllus pennsylvanicus) |
| AD001 | 2388 | AAGAAGCATTTGAAGCGTTTAAA | 3658572 (Manduca sexta) |
| AD001 | 2389 | AAGGGTAAGGGTGTGAAATTGAGTAT | 109978109 (Gryllus pennsylvanicus) |
| AD001 | 2390 | AATGTATTCATCATTGGAAAAGC | 55904577 (Locusta migratoria) |
| AD001 | 2391 | AGAAGCATTTGAAGCGTTTAAA | 98994282 (Antheraea mylitta) 73619372 (Aphis gossypii) |
| AD001 | 2392 | AGAAGCATTTGAAGCGTTTAAATGC | 27620566 (Anopheles gambiae) |
| AD001 | 2393 | AGTACTGGCCCCCACAAATTGCG | 109978109 (Gryllus pennsylvanicus) |
| AD001 | 2394 | AGTGCAGAAGAAGCCAAGTACAAGCT | 109978109 (Gryllus pennsylvanicus) |
| AD001 | 2395 | ATCGCCGAGGAGCGGGACAAGC | 3953837 (Bombyx mandarina) 94432102 (Bombyx mori) |
| AD001 | 2396 | CAAGGACATACTTTTGCCACAAGATTGAATAATGT ATTCATCATTGGAAA | 109978109 (Gryllus pennsylvanicus) |
| AD001 | 2397 | CAGAAGAAGCCAAGTACAAGCT | 42764924 (Armigeres subalbatus) |
| AD001 | 2398 | CATGATGGCCGTACTATCCGTTA | 73613065 (Aphis gossypii) |
| AD001 | 2399 | CATGATGGCCGTACTATCCGTTATCCTGACCC | 31365398 (Toxoptera citricida) |
| AD001 | 2400 | CATTTGAAGCGTTTAAATGCTCC | 27557322 (Anopheles gambiae) |
| AD001 | 2401 | CCTAAAGCATGGATGTTGGAC | 77324536 (Chironomus tentans) |
| AD001 | 2402 | CCTAAAGCATGGATGTTGGACAA | 58371410 (Lonomia obliqua) |
| AD001 | 2403 | CCTAAAGCATGGATGTTGGACAAA | 60311985 (Papilio dardanus) |
| | | | 30031258 (Toxoptera citricida) |
| AD001 | 2404 | CCTAAAGCATGGATGTTGGACAAACT | 98994282 (Antheraea mylitta) |
| AD001 | 2405 | CGTACTATCCGTTATCCTGACCC | 37804548 (Rhopalosiphum padi) |
| AD001 | 2406 | GAATGTTTACCTTTGGTGATTTTTCTTCGCAATCG GCT | 109978109 (Gryllus pennsylvanicus) |
| AD001 | 2407 | GCAGAAGAAGCCAAGTACAAGCT | 37953169 (Ips pini) |
| AD001 | 2408 | GCATGGATGTTGGACAAACTCGG | 83935968 (Lutzomyia longipalpis) |
| AD001 | 2409 | GCTGGTTTCATGGATGTTGTCAC | 109978109 (Gryllus pennsylvanicus) |
| AD001 | 2410 | GGCCCCAAGAAGCATTTGAAGCGTTTAA | 14693528 (Drosophila melanogaster) |
| AD001 | 2411 | GGTTTCATGGATGTTGTCACCAT | 25958683 (Curculio glandium) |
| AD001 | 2412 | TATGATGTGAAAGGCCGTTTCACAATTCACAGAAT | 109978109 (Gryllus pennsylvanicus) |
| AD001 | 2413 | TCATTGCCAAAGGGTAAGGGT | 77324972 (Chironomus tentans) |
| AD001 | 2414 | TGGATATTGCCACTTGTAAAATCATGGACCACATC AGATTTGAATCTGG | 109978109 (Gryllus pennsylvanicus) |
| AD001 | 2415 | TTAAATGCTCCTAAAGCATGGATGTTGGACAAACT | 109978109 (Gryllus pennsylvanicus) |
| AD001 | 2416 | TTTGAATCTGGCAACCTGTGTATGAT | 60311985 (Papilio dardanus) |
| AD001 | 2417 | TTTGATATTGTTCATATCAAGGATAC | 109978109 (Gryllus pennsylvanicus) |

**Table 5-LD/PC/AG/MP/NL/CS/PX**

| **Target ID** | **SEQ ID No** | **Sequences*** | **Example Gi-number and species** |
|---|---|---|---|
| LD001 | 124 | AAGAAGCATTTGAAGCGTTTG | 8005678 (Meloidogyne incognita ), 9829015 (Meloidogyne javanica) |
| AG001 | 739 | GCTGGATTCATGGATGTGATCA | 15666884 (Ancylostoma ceylanicum) |
| AG001 | 740 | ATGGATGTTGGACAAATTGGG | 18081843 (Globodera rostochiensis) |
| AG001 | 741 | TTCATGGATGTGATCACCATTGA | 27002091 (Ascaris suum) |
| MP001 | 1011 | GATCTTTTGATATTGTTCACATTAA | 13099294 (Strongyloides ratti) |
| MP001 | 1012 | ACATCCAGGATCTTTTGATATTGTTCAC | 15275671 (Strongyloides ratti) |
| MP001 | 1013 | TCTTTTGATATTGTTCACATTAA | 32183548 (Meloidogyne chitwoodi) |
| NL001 | 1438 | AGTACAAGCTGTGCAAAGTGAAGA | 18087933 (Globodera rostochiensis), 54547517 (Globodera pallida) |
| NL001 | 1439 | ATGGATGTTGGACAAATTGGGTGG | 7143612 (Globodera rostochiensis) |
| NL001 | 1440 | TGGATGTTGGACAAATTGGGTGG | 7235910 (Meloidogyne incognita) |
| NL001 | 1441 | AGTACAAGCTGTGCAAAGTGAAGA | 111164813 (Globodera rostochiensis) |
| CS001 | 1988 | ATACAAGCTGTGCAAGGTGCG | 10803803 (Trichuris muris) |
| PX001 | 2291 | CTCGACATCGCCACCTGCAAG | 11069004 (Haemonchus contortus); 27770634 (Teladorsagia circumcincta) |
| PX001 | 2292 | GACGGCAAGGTCCGCACCGAC | 32320500 (Heterodera glycines) |
| PX001 | 2293 | CCCGGCTGGATTCATGGATGT | 51334233 (Radopholus similis) |
| PX001 | 2294 | ATCAAGGTGGACGGCAAGGTCCGCAC | 108959807 (Bursaphelenchus xylophilus) |
| PX001 | 2295 | ACAACGTGTTCATCATCGGCAA | 111166840 (Globodera rostochiensis) |

**Table 6-LD/PC/AG/TC/NL/PX/AD**

| | | | |
|---|---|---|---|
| **Target ID** | **SEQ ID No** | **Sequences*** | **Example Gi-number and species** |
| LD001 | 136 | TAGCGGATGGTGCGGCCGTCGTG | 54625255 (Phlebiopsis gigantea) |
| PC001 | 447 | CCCTGCTGGTTTCATGGATGTCAT | 110469463 (Rhizopus oryzae) |
| AG001 | 752 | CGTAACAGGTTGAAGTACGCCCT | 16931515 (Coccidioides posadasii) |
| AG001 | 753 | AAGGTCGACGGCAAAGTCAGGACTGAT | 33515688 (Cryptococcus neoformans var.) |
| AG001 | 754 | CCATTCTTGGTCACCCACGATG | 38132640 (Hypocrea jecorina) |
| AG001 | 755 | ATCAAGGTAAACGACACCATC | 56939474 (Puccinia graminis f. sp.) |
| TC001 | 855 | AACAGGCTGAAGTATGCCTTGACC | 90545567 (Gloeophyllum trabeum) |
| NL001 | 1474 | CCAAGGGCAAGGGTGTGAAGCTCA | 30418788 (Magnaporthe grisea) |
| NL001 | 1475 | TCTCTGCCCAAGGGCAAGGGTGT | 22500578 (Gibberella zeae), 46128672 (Gibberella zeae PH-1), 70662858 (Gibberella moniliformis), 71000466 (Aspergillus fumigatus) |
| NL001 | 1476 | TCTGCCCAAGGGCAAGGGTGT | 14664568 (Fusarium sporotrichioides) |
| NL001 | 1477 | TCTCTGCCCAAGGGCAAGGGT | 50550586 (Yarrowia lipolytica) |
| NL001 | 1478 | TCTCTGCCCAAGGGCAAGGGTGT | 71000466 (Aspergillus fumigatus) 92459259 (Gibberella zeae) |
| PX001 | 2299 | CTCATCAAGGTGGACGGCAAGGT | 85080580 (Neurospora crassa) |
| PX001 | 2300 | TCGGTGCGGACCTTGCCGTCCACCTTGA | 70768092 (Gibberella moniliformis) |
| PX001 | 2301 | GACGGCAAGGTCCGCACCGAC | 109745014 (Allomyces macrogynus); 60673542 (Alternaria brassicicola); 90368699 (Aureobasidium pullulans); 59299145 (Blastocladiella emersonii); 27438899 (Chaetomium globosum); 90623992 (Corynascus heterothallicus); 89975695 (Hypocrea lixii); 99039195 (Leptosphaeria maculans); 39970560 (Magnaporthe grisea); 47731115 (Metarhizium anisopliae); 90036859 (Trichophyton rubrum); 29427127 (Verticillium dahliae) |
| PX001 | 2302 | GACGGCAAGGTCCGCACCGACCC | 70823112 (Aspergillus niger); 90633197 (Thermomyces lanuginosus) |
| PX001 | 2303 | AAGGTCCGCACCGACCCCACCTACCC | 71015993 (Ustilago maydis) |
| PX001 | 2304 | CGCTTCACCATCCACCGCATCAC | 90639458 (Trametes versicolor) |
| PX001 | 2305 | CGAGGAGGCCAAGTACAAGCTG | 78177454 (Chaetomium cupreum); 27438899 (Chaetomium globosum) |
| PX001 | 2306 | GAGGCCAAGTACAAGCTGTGCAAGGT | 109745014 (Allomyces macrogynus) |
| PX001 | 2307 | GCCAAGTACAAGCTGTGCAAG | 45923813 (Coccidioides posadasii) |
| PX001 | 2308 | CCCGACCCGCTCATCAAGGTCAACGAC | 78177454 (Chaetomium cupreum) |
| PX001 | 2309 | CGACATCGTCCACATCAAGGAC | 82603501 (Phanerochaete chrysosporium) |
| PX001 | 2310 | CCGCACAAGCTGCGCGAGTGCCTGCCGCTC | 109745014 (Allomyces macrogynus) |
| AD001 | 2441 | CCCGCTGGTTTCATGGATGTT | 58259586 (Cryptococcus neoformans) |
| AD001 | 2442 | GACAACATCCATGAAACCAGCGGG | 21649877 (Conidiobolus coronatus) |
| AD001 | 2443 | TTCATGGATGTTGTCACCATTG | 90616000 (Ophiostoma piliferum) |
| AD001 | 2444 | GAAGAAGCCAAGTACAAGCTCTG | 110469512 (Rhizopus oryzae) |
| AD001 | 2445 | AAGAAGCCAAGTACAAGCTCTG | 110469518 (Rhizopus oryzae) |
| AD001 | 2446 | GCCAAGTACAAGCTCTGCAAGGT | 98996590 (Spizellomyces punctatus) |
| AD001 | 2447 | GCCAAGTACAAGCTCTGCAAGGTCA | 109743129 (Allomyces macrogynus) |
| AD001 | 2448 | AGTACAAGCTCTGCAAGGTCA | 71000466 (Aspergillus fumigatus); 67537247 (Aspergillus nidulans); 70823112 (Aspergillus niger); 40886470 (Emericella nidulans) |

**Table 8-LD**

| **Target ID** | **Primers Forward 5' → 3'** | **Primers Reverse 5' → 3'** | **dsRNA DNA Sequence (sense strand) 5' → 3'** |
|---|---|---|---|
| LD001 | SEQ ID NO: 164 GCGTAATACGACTC ACTATAGGGGCCCC AAGAAGCATTTGAA GCG | SEQ ID NO: 165 CCTTTGGGGCCAGT TTGCATC | SEQ ID NO: 163 GGCCCCAAGAAGCATTTGAAGCGTTTGAATGCCCCAAAAGCATGGATGTTGG ATAAATTGGGAGGTGTTTTCGCACCTCGCCCATCTACAGGACCTCACAAATTG CGAGAGTCTTTGCCCTTGGTGATCTTCCTACGTAACCGATTGAAGTATGCTTT GACTAACAGCGAAGTTACTAAGATTGTTATGCAAAGGTTAATCAAAGTAGATG GAAAAGTGAGGACCGACTCCAATTACCCTGCTGGGTTTATGGATGTTATTACC |
| | SEQ ID NO: 166 GGCCCCAAGAAGCA TTTGAAGCG | SEQ ID NO: 167 GCGTAATACGACTC ACTATAGGCCTTTG GGGCCAGTTTGCAT C | ATTGAAAAAACTGGTGAATTTTTCCGACTCATCTATGATGTTAAAGGACGATTT GCAGTGCATCGTATTACTGCTGAGGAAGCAAAGTACAAACTATGCAAAGTCAG GAGGATGCAAACTGGCCCCAAAGG |
| LD002 | SEQ ID NO: 169 | SEQ ID NO: 170 | SEQ ID NO: 168 |
| | | SEQ ID NO: 172 | |
| | SEQ ID NO: 171 | | |
| LD003 | SEQ ID NO: 174 | SEQ ID NO: 175 | |
| | | SEQ ID NO: 177 | |
| | SEQ ID NO: 176 | | |
| LD006 | SEQ lD NO: 1 79 | SEQ ID NO: 180 | SEQ ID NO: 178 |
| | | SEQ ID NO: 182 | |
| | SEQ ID NO: 181 | | |
| LD007 | SEQ ID NO: 184 | SEQ ID NO: 185 | SEQ ID NO: 183 |
| | | SEQ ID NO: 187 | |
| | SEQ ID NO: 186 | | |
| LD010 | SEQ ID NO: 189 | SEQ ID NO: 190 | SEQ ID NO: 188 |
| | SEQ ID NO: 191 | SEQ ID NO: 192 | |
| LD011 | SEQ ID NO:194 | SEQ ID NO: 195 | SEQ ID NO: 193 |
| | SEQ ID NO: 196 | SEQ ID NO: 197 | |
| LD014 | SEQ ID NO: 199 | SEQ ID NO: 200 | SEQ ID NO: 198 |
| | SEQ ID NO: 201 | SEQ ID NO: 202 | |
| LD014_F1 | SEQ ID NO: 204 | SEQ lD NO: 205 | SEQ ID NO: 203 |
| | SEQ ID NO: 206 | SEQ ID NO: 207 | |
| LD014_F2 | SEQ lD NO: 209 | SEQ ID NO: 210 | SEQ ID NO: 208 |
| | SEQ ID NO: 21 | SEQ ID NO: 212 | |
| LD014 C1 | | | SEQ ID NO: 21 |
| | | | AGAGGAGGCGCGTAAACGACTTGGTCAGGTCACAAACGC |
| LD014_C2 | | | SEQ ID NO: 214 |
| LD015 | SEQ ID NO: 216 | SEQ ID NO: 217 | SEQ ID NO: 215 |
| | SEQ ID NO: 218 | SEQ ID NO: 219 | |
| LD016 | SEQ ID NO: 221 | SEQ ID NO: 222 | SEQ ID NO: 220 |
| | SEQ ID NO: 223 | SEQ ID NO: 224 | |
| LD018 | SEQ ID NO: 226 | SEQ ID NO: 227 | SEQ ID NO: 225 |
| | SEQ ID NO: 228 | SEQ ID NO: 229 | |
| LD027 | SEQ ID NO: 231 | SEQ ID NO: 232 | SEQ ID NO: 230 |
| | | SEQ ID NO: 234 | |
| | SEQ ID NO: 233 | | |
| gfp | SEQ ID NO: 236 | SEQ ID NO: 237 | SEQ ID NO: 235 |
| | SEQ ID NO: 238 | SEQ ID NO: 239 | |

**Table 8-PC**

| **Target ID** | **Primers Forward 5'→3'** | **Primers Reverse 5'→3'** | **dsRNA DNA Sequence (sense strand) 5'→3'** |
|---|---|---|---|
| PC001 | SEQ ID NO: 474 GCATGGATGTTGGA CAAATTGGG | SEQ ID NO: 475 GCGTAATACGACTC ACTATAGGAGATTCA AATTTGATGTAGTCA AGAATTTTAG | SEQ ID NO: 473 GCATGGATGTTGGACAAATTGGGGGGTGTCTTCGCCCCTCGTCCATCCACCGGG CCTCACAAGTTGCGCGAATCCCTGCCTTTAGTGATTTTCCTTCGTAACAGGCTGAA GTATGCCCTTACAAACAGTGAAGTCACTAAAATTGTCATGCAAAGGTTGATCAAAG TTGATGGTAAAGTGAGGACTGATTCTAATTACCCTGCTGGTTTCATGGATGTCATT ACTATTGAGAAGACTGGTGAATTTTTCCGTCTGATCTATGATGTTAAAGGAAGATT TGCTGTGCACCGTATTACAGCTGAAGAGGCAAAATACAAGTTGTGTAAAGTAAGG AGAGTCCAAACTGGTCCCAAAGGAATCCCATTTTTGGTAACACATGATGGCAGAA CCATTCGTTACCCTGACCCCAACATCAAAGTGAATGACACAATTCAAATGGAAATT |
| | SEQ ID NO: 476 GCGTAATACGACTC ACTATAGGGCATGG ATGTTGGACAAATTG GG | SEQ ID NO: 477 AGATTCAAATTTGAT GTAGTCAAGAATTTT AG | GCTACATCTAAAATTCTTGACTACATCAAATTTGAATCT |
| PC003 | SEQ ID NO: 479 | SEQ ID NO: 480 | SEQ ID NO: 478 |
| | SEQ ID NO: 481 | SEQ ID NO: 482 | |
| PC005 | SEQ ID NO: 484 | SEQ ID NO: 485 | SEQ ID NO: 483 |
| | SEQ ID NO: 486 | SEQ ID NO: 487 | |
| PC010 | SEQ ID NO: 489 | SEQ ID NO: 490 | SEQ ID NO: 488 |
| | SEQ ID NO: 491 | SEQ ID NO: 492 | |
| PC014 | SEQ ID NO: 494 | SEQ ID NO: 495 | SEQ ID NO: 493 |
| | SEQ ID NO: 496 | SEQ ID NO: 497 | |
| PC016 | SEQ ID NO: 499 | SEQ ID NO: 500 | SEQ ID NO: 498 |
| | SEQ ID NO: 501 | SEQ ID NO: 502 | |
| PC027 | SEQ ID NO: 504 | SEQ ID NO: 505 | SEQ ID NO: 503 |
| | SEQ ID NO: 506 | SEQ ID NO: 507 | |

**Table 8-EV**

| **Target ID** | **Primers Forward 5'→3'** | **Primers Reverse 5'→3'** | **dsRNA DNA Sequence (sense strand) 5'→'** |
|---|---|---|---|
| EV005 | SEQ ID NO: 577 | SEQ ID NO: 578 | SEQ ID NO: 576 |
| | SEQ ID NO: 579 | SEQ ID NO: 580 | |
| EV009 | SEQ ID NO: 582 | SEQ ID NO: 583 | SEQ ID NO: 581 |
| | SEQ ID NO: 584 | SEQ ID NO: 585 | |
| EV010 | SEQ ID NO: 587 | SEQ ID NO: 588 | SEQ ID NO: 586 |
| | SEQ ID NO: 589 | SEQ ID NO: 590 | |
| EV015 | SEQ ID NO: 592 | SEQ ID NO: 593 | SEQ ID NO: 591 |
| | SEQ ID NO: 594 | | |
| | | SEQ ID NO: 595 | |
| EV016 | SEQ ID NO: 597 | SEQ ID NO: 598 | SEQ ID NO: 596 |

**Table 8-AG**

| **Target ID** | **Primers Forward 5'→3'3'** | **Primers Reverse 5'→3'** | **dsRNA DNA Sequence (sense strand) 5'→3'** |
|---|---|---|---|
| AG001 | SEQ ID NO: 769 GCGTAATACGACTC ACTATAGGGCATGG ATGTTGGACAAATTG G | SEQ ID NO: 770 GATTTCCAGTTGGAT GGTGTCG | SEQ ID NO: 768 |
| | SEQ ID NO: 771 GCATGGATGTTGGA CAAATTGG | SEQ ID NO: 772 GCGTAATACGACTC ACTATAGGGATTTCC AGTTGGATGGTGTC G | GCATGGATGTTGGACAAATTGGGGGGTGTGTTCGCCCCCAGGCCCTCCACCGGG CCACACAAGCTCAGGGAGTCCCTTCCATTAGTGATTTTCTTGCGTAACAGGTTGAA GTACGCCCTGACAAACTGTGAGGTGACCAAGATCGTTATGCAGAGACTTATTAAG GTCGACGGCAAAGTCAGGACTGATCCTAACTATCCTGCTGGATTCATGGATGTGA TCACCATTGAAAAAACTGGTGAATTCTTCCGTTTGATCTATGATGTTAAGGGAAGA TTCACTATTCACAGGATCACTGCTGAAGAAGCAAAATACAAATTGTGCAAAGTCCG CAAGGTGCAAACCGGACCAAAAGGTATTCCATTCTTGGTCACCCACGATGGTAGG ACCATTAGGTACCCTGACCCAATGATCAAGGTAAACGACACCATCCAACTGGAAA TC |
| AG005 | SEQ ID NO: 774 | SEQ ID NO: 775 | SEQ ID NO: 773 |
| | SEQ ID NO: 776 | SEQ ID NO: 777 | |
| AG010 | SEQ ID NO: 779 | SEQ ID NO: 780 | SEQ ID NO: 778 |
| | SEQ ID NO: 781 | SEQ ID NO: 782 | |
| AG014 | SEQ ID NO: 784 | SEQ ID NO: 785 | SEQ ID NO: 783 |
| | SEQ ID NO: 786 | SEQ ID NO: 787 | |
| AG016 | SEQ ID NO: 789 | SEQ ID NO: 790 | SEQ ID NO: 788 |

**Table 8-TC**

| **Target ID** | **Primers Forward 5'→3'** | **Primers Reverse 5'→3'** | **dsRNA DNA Sequence (sense strand) 5'→3'** |
|---|---|---|---|
| TC001 | SEQ ID NO: 864 GCGTAATACGACTC ACTATAGGCTGCGA AACAGGCTGAAGTA TGC | SEQ ID NO: 865 GGTGTGCCCATTTG CATCCT | SEQ ID NO: 863 |
| | SEQ ID NO: 866 CTGCGAAACAGGCT GAAGTATGC | SEQ ID NO: 867 GCGTAATACGACTC ACTATAGGGGTGTG CCCATTTGCATCCT | CTGCGAAACAGGCTGAAGTATGCCTTGACCAACTCAGAAGTGACGAAGATTGTTA TGCAAAGATTGATTAAAGTTGACGGAAAAGTTAGGACAGACCCCAACTACCCCGC GGGTTTCATGGATGTTGTGACTATTGAGAAAACTGGGGAATTCTTCCGCTTGATTT ATGATGTTAAGGGAAGGTTCACAATCCATCGCATTACTGGAGAAGAGGCCAAATA TAAATTGTGCAAAGTGAAGAAAGTACAGACAGGCCCCAAGGGCATTCCCTTCTTG GTGACCCGCGACGGACGCACTATCAGATACCCAGACCCCATGATCAAAGTGAAT GACACCATTCAATTGGAGATTGCCACTTCGAAAATTCTTGATTTTATCAAATTTGAG TCCGGTAATTTGTGTATGATTACTGGAGGTCGTAACTTGGGGCGTGTCGGTACAG TGGTGAGCCGAGAACGTCACCCAGGTTCCTTCGACATCGTTCATATTAAGGATGC AAATGGGCACACC |
| TC002 | SEQ ID NO: 869 | SEQ ID NO: 870 | SEQ ID NO: 868 |
| | | SEQ ID NO: 872 | |
| | SEQ ID NO: 871 | | |
| TC010 | SEQ ID NO: 874 | SEQ ID NO: 875 | SEQ ID NO: 873 |
| | SEQ ID NO: 876 | SEQ ID NO: 877 | |
| TC014 | SEQ ID NO: 879 | SEQ ID NO: 880 | SEQ ID NO: 878 |
| | SEQ ID NO: 881 | SEQ ID NO: 882 | |
| TC015 | SEQ ID NO: 884 | SEQ ID NO: 885 | SEQ ID NO: 883 |
| | SEQ ID NO: 886 | SEQ ID NO: 887 | |

**Table 8-MP**

| **Target ID** | **Primers Forward 5'→3'** | **Primers Reverse 5'→3'** | **dsRNA DNA Sequence (sense strand) 5'→3'** |
|---|---|---|---|
| MP001 | SEQ ID NO: 1042 GCGTAATACGACTC ACTATAGGGTTTAAA CGCACCCAAAGCAT GG | SEQ ID NO: 1043 CAATACCAACACGC CCTAAATTGC | SEQ ID NO: 1041 |
| | SEQ ID NO: 1044 GTTTAAACGCACCC AAAGCATGG | SEQ ID NO: 1045 GCGTAATACGACTC ACTATAGGCAATAC CAACACGCCCTAAA TTGC | GTTTAAACGCACCCAAAGCATGGATGTTGGACAAATCGGGGGGTGTCTTCGCTCC ACGTCCAAGCACCGGTCCACACAAACTTCGTGAATCACTACCGTTATTGATCTTCT TGCGTAATCGTTTGAAGTATGCACTTACTGGTGCCGAAGTCACCAAGATTGTCATG CAAAGATTAATCAAGGTTGATGGCAAAGTCCGTACCGACCCTAATTATCCAGCCG GTTTTATGGATGTTATATCTATCCAAAAGACCAGTGAGCACTTTAGATTGATCTATG ATGTGAAAGGTCGTTTCACCATCCACAGAATTACTCCTGAAGAAGCAAAATACAAG TTGTGTAAAGTAAAGAGGGTACAAACTGGACCCAAAGGTGTGCCATTTTTAACTAC TCATGATGGCCGTACTATTCGCTACCCTGACCCTAACATCAAGGTTAATGACACTA TTAGATACGATATTGCATCATCTAAAATTTTGGATCATATCCGTTTTGAAACTGGAA ACTTGTGCATGATAACTGGAGGTCGCAATTTAGGGCGTGTTGGTATTG |
| MP002 | SEQ ID NO: 1047 | SEQ ID NO: 1048 | SEQ ID NO: 1046 |
| | SEQ ID NO: 1049 | SEQ ID NO: 1050 | |
| MP010 | SEQ ID NO: 1052 | SEQ ID NO: 1053 | SEQ ID NO: 1051 |
| | SEQ ID NO: 1054 | SEQ ID NO: 1055 | |
| MP016 | SEQ ID NO: 1057 | SEQ ID NO: 1058 | SEQ ID NO: 1056 |
| | SEQ ID NO: 1059 | SEQ ID NO: 1060 | |
| MP027 | SEQ ID NO: 1062 | SEQ ID NO: 1063 | SEQ ID NO: 1061 |
| | SEQ ID NO: 1064 | SEQ ID NO: 1065 | |

**Table 8-NL**

| **Target ID** | **Primers Forward 5'→3'** | **Primers Reverse 5'→3'** | **dsRNA DNA Sequence 5'→3'** |
|---|---|---|---|
| NL001 | SEQ ID NO: 1573 GCGTAATACGACTCA CTATAGGGAAATCAT GGATGTTGGACAAAT TGG | SEQ ID NO: 1574 ACTGAGCTTCACA CCCTTGCCC | SEQ ID NO: 1572 |
| | SEQ ID NO: 1575 GAAATCATGGATGTT GGACAAATTGG | SEQ ID NO: 1576 GCGTAATACGACT CACTATAGGACTG AGCTTCACACCCT TGCCC | GAAATCATGGATGTTGGACAAATTGGGTGGTGTGTATGCACCCCGACCCAGCACA GGTCCACACAAGCTGCGAGAATCTCTCCCACTTGTCATATTTTTGCGTAATCGGCT CAAGTACGCTTTAACTAACTGTGAAGTGAAGAAAATTGTGATGCAGCGTCTCATCA AGGTTGACGGCAAAGTGAGGACTGACCCCAACTATCCTGCAGGTTTTATGGACGT TGTTCAAATCGAAAAGACAAACGAGTTCTTCCGTTTGATCTATGATGTTAAGGGAC GTTTCACCATCCACAGGATCACAGCTGAAGAAGCTAAGTACAAGCTGTGCAAAGT GAAGAGGGTTCAGACAGGACCCAAGGGCATTCCATTTTTGACCACTCACGATGGA CGCACCATCAGGTATCCAGACCCCTTAGTAAAAGTCAATGACACCATCCAATTGG ACATTGCCACATCCAAAATCATGGACTTCATCAGATTCGACTCTGGTAACCTGTGT ATGATCACTGGAGGTCGTAACTTGGGTCGTGTGGGCACTGTCGTGAACAGGGAG CGACACCCGGGGTCTTTCGACATCGTGCACATCAAGGACGTGTTGGGACACACTT TTGCCACTAGGTTGAACAACGTTTTCATCATCGGCAAGGGTAGTAAAGCATACGT GTCTCTGCCCAAGGGCAAGGGTGTGAAGCTCAGT |
| NL002 | SEQ ID NO: 1578 | SEQ ID NO: 1579 | SEQ ID NO: 1577 |
| | SEQ ID NO: 1580 | SEQ ID NO: 1581 | |
| NL003 | SEQ ID NO: 1583 | SEQ ID NO: 1584 | SEQ ID NO: 1582 |
| | SEQ ID NO: 1585 | SEQ ID NO: 1586 | |
| NL004 | SEQ ID NO: 1588 | SEQ ID NO: 1589 | SEQ ID NO: 1587 |
| | SEQ ID NO: 1590 | SEQ ID NO: 1591 | |
| NL005 | SEQ ID NO: 1593 | SEQ ID NO: 1594 | SEQ ID NO: 1592 |
| | SEQ ID NO: 1595 | SEQ ID NO: 1596 | |
| NL006 | SEQ ID NO: 1598 | SEQ ID NO: 1599 | SEQ ID NO: 1597 |
| | SEQ ID NO: 1600 | SEQ ID NO: 1601 | |
| NL007 | SEQ ID NO: 1603 | SEQ ID NO: 1604 | SEQ ID NO: 1602 |
| | SEQ ID NO: 1605 | SEQ ID NO: 1606 | |
| NL008 | SEQ ID NO: 1608 | SEQ ID NO: 1609 | SEQ ID NO: 1607 |
| | SEQ ID NO: 1610 | SEQ ID NO: 1611 | |
| NL009 | SEQ ID NO: 1613 | SEQ ID NO: 1614 | SEQ ID NO: 1612 |
| | SEQ ID NO: 1615 | SEQ ID NO: 1616 | GCGACTATGATCGACCGCCGGGACGCGGTCAGGTGTGCGACGTCGACGTCAAG AACTGGTTTCCCTGCACCTCTGAGAACAATTTCAACTACCATCAATCGAGCCCTTG TGTTTTTCTCAAACTGAACAAGATAATTGGTTGGCAACCGGAGTACTACAATGAGA CTGAAGGCTTTCCAGATAATATGCCAGGTGACCTCAAGCGACACATTGCCCAACA GAAGAGTATCAACAAGCTGTTTATGCAAACAATCTGGATAACTTGCGAAGGAGAG GGTCCTCTAGACAAGGAGAATGCAGGGGAGATCCAGTACATCCCTAGACAGGGA TTTCCGGGCTACTTCTACCCTTACAC |
| NL010 | SEQ ID NO: 1618 | SEQ ID NO: 1619 | SEQ ID NO: 1617 |
| | SEQ ID NO: 1620 | SEQ ID NO: 1621 | |
| NL011 | SEQ ID NO: 1623 | SEQ ID NO: 1624 | SEQ ID NO: 1622 |
| | SEQ ID NO: 1625 | SEQ ID NO: 1626 | |
| NL012 | SEQ ID NO: 1628 | SEQ ID NO: 1629 | SEQ ID NO: 1627 |
| | SEQ ID NO: 1630 | SEQ ID NO: 1631 | |
| NL013 | SEQ ID NO: 1633 | SEQ ID NO: 1634 | SEQ ID NO: 1632 |
| | SEQ ID NO: 1635 | SEQ ID NO: 1636 | |
| NL014 | SEQ ID NO: 1638 | SEQ ID NO: 1639 | SEQ ID NO: 1637 |
| | SEQ ID NO: 1640 | SEQ ID NO: 1641 | |
| NL015 | SEQ ID NO: 1643 | SEQ ID NO: 1644 | SEQ ID NO: 1642 |
| | SEQ ID NO: 1645 | SEQ ID NO: 1646 | |
| NL016 | SEQ ID NO: 1648 | SEQ ID NO: 1649 | SEQ ID NO: 1647 |
| | SEQ ID NO: 1650 | SEQ ID NO: 1651 | |
| NL018 | SEQ ID NO: 1653 | SEQ ID NO: 1654 | SEQ ID NO: 1652 |
| | SEQ ID NO: 1655 | SEQ ID NO: 1656 | |
| NL019 | SEQ ID NO: 1658 | SEQ ID NO: 1659 | SEQ ID NO: 1657 |
| | SEQ ID NO: 1660 | SEQ ID NO: 1661 | |
| NL021 | SEQ ID NO: 1663 | SEQ ID NO: 1664 | SEQ ID NO: 1662 |
| | SEQ ID NO: 1665 | SEQ ID NO: 1666 | |
| NL022 | SEQ ID NO: 1668 | SEQ ID NO: 1669 | SEQ ID NO: 1667 |
| | SEQ ID NO: 1670 | SEQ ID NO: 1671 | |
| NL023 | SEQ ID NO: 1673 | SEQ ID NO: 1674 | SEQ ID NO: 1672 |
| | SEQ ID NO: 1675 | SEQ ID NO: 1676 | |
| NL027 | SEQ ID NO: 1678 | SEQ ID NO: 1679 | SEQ ID NO: 1677 |
| | SEQ ID NO: 1680 | SEQ ID NO: 1681 | |

**Table 8-CS**

| **Target ID** | **Primers Forward 5' → 3'** | **Primers Reverse 5' → 3'** | **dsRNA DNA Sequence (sense strand) 5'→ 3'** |
|---|---|---|---|
| CS001 | SEQ ID NO: 2041 TAAAGCATGGATGTT GGACAAACTGGG | SEQ ID NO: 2042 GCGTAATACGACTC ACTATAGGGTGAG TCGCACGCCCTTGC C | SEQ ID NO: 2040 |
| | SEQ ID NO: 2043 GCGTAATACGACTC ACTATAGGTAAAGC ATGGATGTTGGACA AACTGGG | SEQ ID NO: 2044 GGTGAGTCGCACGC CCTTGCC | TAAAGCATGGATGTTGGACAAACTGGGTGGCGTGTACGCGCCGCGGCCGTCGAC CGGCCCCCACAAGTTGCGCGAGTGCCTGCCGCTGGTGATCTTCCTCAGGAACCG GCTCAAGTACGCGCTCACCGGAAATGAAGTGCTTAAGATTGTAAAGCAGCGACTT ATCAAAGTTGACGGCAAAGTCAGGACAGACCCCACATATCCCGCTGGATTTATGG ATGTTGTTTCCATTGAAAAGACAAATGAGCTGTTCCGTCTTATATATGATGTCAAAG GCAGATTTACTATTCACCGTATTACTCCTGAGGAGGCTAAATACAAGCTGTGCAAG GTGCGGCGCGTGGCGACGGGCCCCAAGAACGTGCCTTACCTGGTGACCCACGA CGGACGCACCGTGCGATACCCCGACCCACTCATCAAGGTCAACGACTCCATCCA GCTCGACATCGCCACCTCCAAGATCATGGACTTCATCAAGTTTGAATCTGGTAAC CTATGTATGATCACGGGAGGCCGTAACTTGGGGCGCGTGGGCACCATCGTGTCC CGCGAGCGACATCCCGGGTCCTTCGACATCGTGCATATACGGACTCCACCGGA CATACCTTCGCTACCAGATTGAACAACGTGTTCATAATCGGCAAGGGCACGAAGG CGTACATCTCGCTGCCGCGCGGCAAGGGCGTGCGACTCACC |
| CS002 | SEQ ID NO: 2046 | SEQ ID NO: 2047 | SEQ ID NO: 2045 |
| | SEQ ID NO: 2048 | SEQ ID NO: 2049 | |
| CS003 | SEQ ID NO: 2051 | SEQ ID NO: 2052 | SEQ ID NO: 2050 |
| | SEQ ID NO: 2053 | SEQ ID NO: 2054 | |
| CS006 | SEQ ID NO: 2056 | SEQ ID NO: 2057 | SEQ ID NO: 2055 |
| | SEQ ID NO: 2058 | SEQ ID NO: 2059 | |
| CS007 | SEQ 10 NO: 2061 | SEQ ID NO: 2062 | SEQ ID NO: 2060 |
| | SEQ ID NO: 2063 | SEQ ID NO: 2064 | GACATGCCG |
| CS009 | SEQ ID NO: 2066 | SEQ ID NO: 2067 | SEQ ID NO: 2065 |
| | SEQ ID NO: 2068 | SEQ ID NO: 2069 | |
| CS011 | SEQ ID NO 2071 | SEQ ID NO: 2072 | SEQ ID NO: 2070 |
| | SEQ ID NO: 2073 | SEQ ID NO: 2074 | |
| CS013 | SEQ ID NO: 2076 | SEQ ID NO: 2077 | SEQ ID NO: 2075 |
| | SEQ ID NO: 2078 | SEQ ID NO: 2079 | |
| CS014 | SEQ ID NO: 2081 | SEQ ID NO: 2082 | SEQ ID NO: 2080 |
| | SEQ ID NO: 2083 | SEQ ID NO: 2084 | |
| CS015 | SEQ ID NO: 2086 | SEQ ID NO: 2087 | SEQ ID NO: 2085 |
| | SEQ ID NO: 2088 | SEQ ID NO: 2089 | |
| CS016 | SEQ ID NO: 2091 | SEQ ID NO: 2092 | SEQ ID NO: 2090 |
| | SEQ ID NO: 2093 | SEQ ID NO: 2094 | |
| CS018 | SEQ ID NO: 2096 | SEQ ID NO: 2097 | SEQ ID NO: 2095 |

**Table 8-PX**

| **Target ID** | **Primers Forward 5' → 3'** | **Primers Reverse 5' → 3'** | **dsRNA DNA Sequence (sense strand) 5' → 3'** |
|---|---|---|---|
| PX001 | SEQ ID NO: 2340 GCGTAATACGACTC ACTATAGGCGAGGT GCTGAAGATCGTGA AG | SEQ ID NO: 2341 CTTGCCGATGATGA ACACGTTG | SEQ ID NO: 2339 CGAGGTGCTGAAGATCGTGAAGCAGCGCCTCATCAAGGTGGACGGCAAGGTCCG CACCGACCCCACCTACCCGGCTGGATTCATGGATGTTGTGTCGATTGAAAAGACC AATGAGCTGTTCCGTCTGATCTACGATGTGAAGGGACGCTTCACCATCCACCGCA TCACTCCCGAGGAGGCCAAGTACAAGCTGTGCAAGGTGAAGCGCGTGGCGACG GGCCCCAAGAACGTGCCGTACATCGTGACGCACAACGGCCGCACGCTGCGCTAC CCCGACCCGCTCATCAAGGTCAACGACTCCATCCAGCTCGACATCGCCACCTGC AAGATCATGGACATCATCAAGTTCGACTCAGGTAACCTGTGCATGATCACGGGAG GGCGTAACTTGGGGCGAGTGGGCACCATCGTGTCCCGCGAGAGGCACCCCGGG AGCTTCGACATCGTCCACATCAAGGACACCACCGGACACACCTTCGCCACCAGGT TGAACAACGTGTTCATCATCGGCAAG |
| | SEQ ID NO: 2342 | SEQ ID NO: 2343 | |
| | TCGTGAAG | ACTATAGGCTTGCC GATGATGAACACGT TG | |
| PX009 | SEQ ID NO: 2345 | SEQ ID NO: 2346 | SEQ ID NO: 2344 |
| | SEQ ID NO: 2347 | SEQ ID NO: 2348 | |
| PX010 | SEQ ID NO: 2350 | SEQ ID NO: 2351 | SEQ ID NO: 2349 |
| | SEQ ID NO: 2352 | SEQ ID NO: 2353 | |
| PX015 | SEQ ID NO: 2355 | SEQ ID NO: 2356 | SEQ ID NO: 2354 |
| | SEQ ID NO: 2357 | SEQ ID NO: 2358 | |
| PX016 | SEQ ID NO: 2360 | SEQ ID NO: 2361 | SEQ ID NO: 2359 |
| | SEQ ID NO: 2362 | SEQ ID NO: 2363 | |

**Table 8-AD**

| **Target ID** | **Primers Forward 5' → 3'** | **Primers Reverse 5' → 3'** | **dsRNA DNA Sequence (sense strand) 5' → 3'** |
|---|---|---|---|
| AD001 | SEQ ID NO: 2462 | SEQ ID NO: 2463 | SEQ ID NO: 2461 |
| | GCGTAATACGACTC ACTATAGGGCTCCT AAAGCATGGATGTT GG SEQ ID NO: 2464 GCTCCTAAAGCATG GATGTTGG | CAATATCAAACGAG CCTGGGTG SEQ ID NO: 2465 GCGTAATACGACTC ACTATAGGCAATATC AAACGAGCCTGGGT G | GCTCCTAAAGCATGGATGTTGGACAAACTCGGAGGAGTATTCGCTCCTCGCCCCAG TACTGGCCCCCACAAATTGCGTGAATGTTTACCTTTGGTGATTTTTCTTCGCAATCG GCTCAAGTATGCTCTGACGAACTGTGAAGTAACGAAGATTGTTATGCAGCGACTTAT CAAAGTTGACGGCAAGGTGCGAACCGATCCGAATTATCCCGCTGGTTTCATGGATG TTGTCACCATTGAGAAGACTGGAGAGTTCTTCAGGCTGGTGTATGATGTGAAAGGC CGTTTCACAATTCACAGAATTAGTGCAGAAGAAGCCAAGTACAAGCTCTGCAAGGTC AGGAGAGTTCAAACTGGGCCAAAAGGTATTCCATTCTTGGTGACCCATGATGGCCG TACTATCCGTTATCCTGACCCAGTCATTAAAGTTAATGACTCAATCCAATTGGATATT GCCACTTGTAAAATCATGGACCACATCAGATTTGAATCTGGCAACCTGTGTATGATT ACTGGTGGACGTAACTTGGGTCGAGTGGGGACTGTTGTGAGTCGAGAACGTCACC CAGGCTCGTTTGATATTG |
| AD002 | SEQ ID NO: 2467 | SEQ ID NO: 2468 | SEQ ID NO: 2466 |
| | SEQ ID NO: 2469 | SEQ ID NO: 2470 | |
| AD009 | SEQ ID NO: 2472 | SEQ ID NO: 2473 | SEQ ID NO: 2471 |
| | SEQ ID NO: 2474 | SEQ ID NO: 2475 | |
| AD015 | SEQ ID NO: 2477 | SEQ ID NO: 2478 | SEQ ID NO: 2476 |
| | SEQ ID NO: 2479 | SEQ ID NO: 2480 | |
| AD016 | SEQ ID NO: 2482 | SEQ ID NO: 2483 | SEQ ID NO: 2481 |
| | SEQ ID NO: 2484 | SEQ ID NO: 2485 | |

**Table 9-PC/MP**

| | |
|---|---|
| PC001 | SEQ ID NO: 508 AGATTCAAATTTGATGTAGTCAAGAATTTTAGATGTAGCAATTTCCATTTGAATTGTGTCATTCACTTTGATGTTGGGGTCAG GGTAACGAATGGTTCTGCCATCATGTGTTACCAAAAATGGGATTCCTTTGGGACCAGTTTGGACTCTCCTTACTTTACACAA CTTGTATTTTGCCTCTTCAGCTGTAATACGGTGCACAGCAAATCTTCCTTTAACATCATAGATCAGACGGAAAAATTCACCA GTCTTCTCAATAGTAATGACATCCATGAAACCAGCAGGGTAATTAGAATCAGTCCTCACTTTACCATCAACTTTGATCAACC TTTGCATGACAATTTTAGTGACTTCACTGTTTGTAAGGGCATACTTCAGCCTGTTACGAAGGAAAATCACTAAAGGCAGGGA TTCGCGCAACTTGTGAGGCCCGGTGGATGGACGAGGGGCGAAGACACCCCCCAATTTGTCCAACATCCATGCAAGGGCG AATTCGACCCAGCTTTCTTGTACAAAGTGGTGATATCACTAGTGCGGCCGCCTGCAGGTCGACCATATGGTCGACCTGCA GGCGGCCGCACTAGTGATGCTGTTATGTTCAGTGTCAAGCTGACCTGCAAACACGTTAAATGCTAAGAAGTTAGAATATAT GAGACACGTTAACTGGTATATGAATAAGCTGTAAATAACCGAGTATAAACTCATTAACTAATATCACCTCTAGAGTATAATAT AATCAAATTCGACAATTTGACTTTCAAGAGTAGGCTAATGTAAAATCTTTATATATTTCTACAATGTTCAAAGAAACAGTTGC ATCTAAACCCCTATGGCCATCAAATTCAATGAACGCTAAGCTGATCCGGCGAGATTTTCAGGAGCTAAGGAAGCTAAAATG GAGAAAAAAATCACTGGATATACCACCGTTGATATATCCCAATGGCATCGTAAAGAACATTTTGAGGCATTTCAGTCAGTTG CTCAATGTACCTATAACCAGACCGTTCAGCTGGATATTACGGCCTTTTTAAAGACCGTAAAGAAAAATAAGCACAAGTTTTA TCCGGCCTTTATTCACATTCTTGCCCGCCTGATGAATGCTCATCCGGAATTCCGTATGGCAATGAAAGACGGTGAGCTGGT GATATGGGATAGTGTTCACCCTTGTTACACCGTTTTCCATGAGCAAACTGAAACGTTTTCATCGCTCTGGAGTGAATACCAC GACGATTTCCGGCAGTTTCTACACATATATTCGCAAGATGTGGCGTGTTACGGTGAAAACCTGGCCTATTTCCCTAAAGGG TTTATTGAGAATATGTTTTTCGTCTCAGCCAATCCCTGGGTGAGTTTCACCAGTTTTGATTTAAACGTGGCCAATATGGACA ACTTCTTCGCCCCCGTTTTCACCATGGGCAAATATTATACGCAAGGCGACAAGGTGCTGATGCCGCTGGCGATTCAGGTT CATCATGCCGTCTGTGATGGCTTCCATGTCGGCAGAATGCTTAATGAATTACAACAGTACTGCGATGAGTGGCAGGGCGG GGCGTAAACGCGTGGATCAGCTTAATATGACTCTCAATAAAGTCTCATACCAACAAGTGCCACCTTATTCAACCATCAAGAA AAAAGCCAAAATTTATGCTACTCTAAGGAAAACTTCACTAAAGAAGACGATTTAGAGTGTTTTACCAAGAATTTCTGTCATCT TACTAAACAACTAAAGATCGGTGTGATACAAAACCTAATCTCATTAAAGTTTATGCTAAAATAAGCATAATTTTACCCACTAA GCGTGACCAGATAAACATAACTCAGCACACCAGAGCATATATATTGGTGGCTCAAATCATAGAAACTTACAGTGAAGACAC AGAAAGCCGTAAGAAGAGGCAAGAGTATGAAACCTTACCTCATCATTTCCATGAGGTTGCTTCTGATCCCGCGGGATATCA CCACTTTGTACAAGAAAGCTGGGTCGAATTCGCCCTTGCATGGATGTTGGACAAATTGGGGGGTGTCTTCGCCCCTCGTC CATCCACCGGGCCTCACAAGTTGCGCGAATCCCTGCCTTTAGTGATTTTCCTTCGTAACAGGCTGAAGTATGCCCTTACAA ACAGTGAAGTCACTAAAATTGTCATGCAAAGGTTGATCAAAGTTGATGGTAAAGTGAGGACTGATTCTAATTACCCTGCTGG TTTCATGGATGTCATTACTATTGAGAAGACTGGTGAATTTTTCCGTCTGATCTATGATGTTAAAGGAAGATTTGCTGTGCAC CGTATTACAGCTGAAGAGGCAAAATACAAGTTGTGTAAAGTAAGGAGAGTCCAAACTGGTCCCAAAGGAATCCCATTTTTG |
| | GTAACACATGATGGCAGAACCATTCGTTACCCTGACCCCAACATCAAAGTGAATGACACAATTCAAATGGAAATTGCTACAT CTAAAATTCTTGACTACATCAAATTTGAATCT |
| MP001 | SEQ ID NO: 1066 GTTTAAACGCACCCAAAGCATGGATGTTGGACAAATCGGGGGGTGTCTTCGCTCCACGTCCAAGCACCGGTCCACACAAACTTCGTG AATCACTACCGTTATTGATCTTCTTGCGTAATCGTTTGAAGTATGCACTTACTGGTGCCGAAGTCACCAAGATTGTCATGCAAAGATTA ATCAAGGTTGATGGCAAAGTCCGTACCGACCCTAATTATCCAGCCGGTTTTATGGATGTTATATCTATCCAAAAGACCAGTGAGCACT TTAGATTGATCTATGATGTGAAAGGTCGTTTCACCATCCACAGAATTACTCCTGAAGAAGCAAAATACAAGTTGTGTAAAGTAAAGAGG GTACAAACTGGACCCAAAGGTGTGCCATTTTTAACTACTCATGATGGCCGTACTATTCGCTACCCTGACCCTAACATCAAGGTTAATG ACACTATTAGATACGATATTGCATCATCTAAAATTTTGGATCATATCCGTTTTGAAACTGGAAACTTGTGCATGATAACTGGAGGTCGC AATTTAGGGCGTGTTGGTATTGAAGGGCGAATTCGACCCAGCTTTCTTGTACAAAGTGGTGATATCACTAGTGCGGCCGCCTGCAGG TCGACCATATGGTCGACCTGCAGGCGGCCGCACTAGTGATGCTGTTATGTTCAGTGTCAAGCTGACCTGCAAACACGTTAAATGCTA AGAAGTTAGAATATATGAGACACGTTAACTGGTATATGAATAAGCTGTAAATAACCGAGTATAAACTCATTAACTAATATCACCTCTAGA GTATAATATAATCAAATTCGACAATTTGACTTTCAAGAGTAGGCTAATGTAAAATCTTTATATATTTCTACAATGTTCAAAGAAACAGTTG CATCTAAACCCCTATGGCCATCAAATTCAATGAACGCTAAGCTGATCCGGCGAGATTTTCAGGAGCTAAGGAAGCTAAAATGGAGAAA AAAATCACTGGATATACCACCGTTGATATATCCCAATGGCATCGTAAAGAACATTTTGAGGCATTTCAGTCAGTTGCTCAATGTACCTA TAACCAGACCGTTCAGCTGGATATTACGGCCTTTTTAAAGACCGTAAAGAAAAATAAGCACAAGTTTTATCCGGCCTTTATTCACATTC TTGCCCGCCTGATGAATGCTCATCCGGAATTCCGTATGGCAATGAAAGACGGTGAGCTGGTGATATGGGATAGTGTTCACCCTTGTT ACACCGTTTTCCATGAGCAAACTGAAACGTTTTCATCGCTCTGGAGTGAATACCACGACGATTTCCGGCAGTTTCTACACATATATTCG CAAGATGTGGCGTGTTACGGTGAAAACCTGGCCTATTTCCCTAAAGGGTTTATTGAGAATATGTTTTTCGTCTCAGCCAATCCCTGGG TGAGTTTCACCAGTTTTGATTTAAACGTGGCCAATATGGACAACTTCTTCGCCCCCGTTTTCACCATGGGCAAATATTATACGCAAGGC GACAAGGTGCTGATGCCGCTGGCGATTCAGGTTCATCATGCCGTCTGTGATGGCTTCCATGTCGGCAGAATGCTTAATGAATTACAA CAGTACTGCGATGAGTGGCAGGGCGGGGCGTAAACGCGTGGATCAGCTTAATATGACTCTCAATAAAGTCTCATACCAACAAGTGCC ACCTTATTCAACCATCAAGAAAAAAGCCAAAATTTATGCTACTCTAAGGAAAACTTCACTAAAGAAGACGATTTAGAGTGTTTTACCAA GAATTTCTGTCATCTTACTAAACAACTAAAGATCGGTGTGATACAAAACCTAATCTCATTAAAGTTTATGCTAAAATAAGCATAATTTTAC CCACTAAGCGTGACCAGATAAACATAACTCAGCACACCAGAGCATATATATTGGTGGCTCAAATCATAGAAACTTACAGTGAAGACAC AGAAAGCCGTAAGAAGAGGCAAGAGTATGAAACCTTACCTCATCATTTCCATGAGGTTGCTTCTGATCCCGCGGGATATCACCACTTT GTACAAGAAAGCTGGGTCGAATTCGCCCTTCAATACCAACACGCCCTAAATTGCGACCTCCAGTTATCATGCACAAGTTTCCAGTTTC AAAACGGATATGATCCAAAATTTTAGATGATGCAATATCGTATCTAATAGTGTCATTAACCTTGATGTTAGGGTCAGGGTAGCGAATAG |
| | TACGGCCATCATGAGTAGTTAAAAATGGCACACCTTTGGGTCCAGTTTGTACCCTCTTTACTTTACACAACTTGTATTTTGCTTCTTCA GGAGTAATTCTGTGGATGGTGAAACGACCTTTCACATCATAGATCAATCTAAAGTGCTCACTGGTCTTTTGGATAGATATAACATCCAT AAAACCGGCTGGATAATTAGGGTCGGTACGGACTTTGCCATCAACCTTGATTAATCTTTGCATGACAATCTTGGTGACTTCGGCACCA GTAAGTGCATACTTCAAACGATTACGCAAGAAGATCAATAACGGTAGTGATTCACGAAGTTTGTGTGGACCGGTGCTTGGACGTGGA GCGAAGACACCCCCCGATTTGTCCAACATCCATGCTTTGGGTGCGTTTAAAC |

**Table10-LD**

| **bioassay** | **bacterial host strain** | **treatment** | **no. of survivors** | **total weight** | **average weight / larvae** |
|---|---|---|---|---|---|
| 1 | | diet only | 8* | 1.0245 | 0.1281 |
| | AB309-105 | pGN29 | 8* | 1.0124 | 0.1266 |
| | | pGBNJ003 clone 1 | 4 | 0.0273 | 0.0068 |
| | | pGBNJ003 clone 2 | 1 | 0.0091 | 0.0091 |
| | | pGBNJ003 clone 3 | 25 | 0.7113 | 0.0285 |
| | | pGBNJ003 clone 4 | 12 | 0.1379 | 0.0115 |
| | | pGBNJ003 clone 5 | 12 | 0.1808 | 0.0151 |
| II | | diet only | 8* | 1.0435 | 0.1304 |
| | BL21(DE3) | pGN29 | 8* | 1.1258 | 0.1407 |
| | | pGBNJ003 clone 1 | 33 | 0.5879 | 0.0178 |
| | | pGBNJ003 clone 2 | 42 | 0.8034 | 0.0191 |
| | | pGBNJ003 clone 3 | 33 | 0.3441 | 0.0104 |
| | | pGBNJ003 clone 4 | 21 | 0.1738 | 0.0083 |
| | | pGBNJ003 clone 5 | 33 | 0.3628 | 0.0120 |

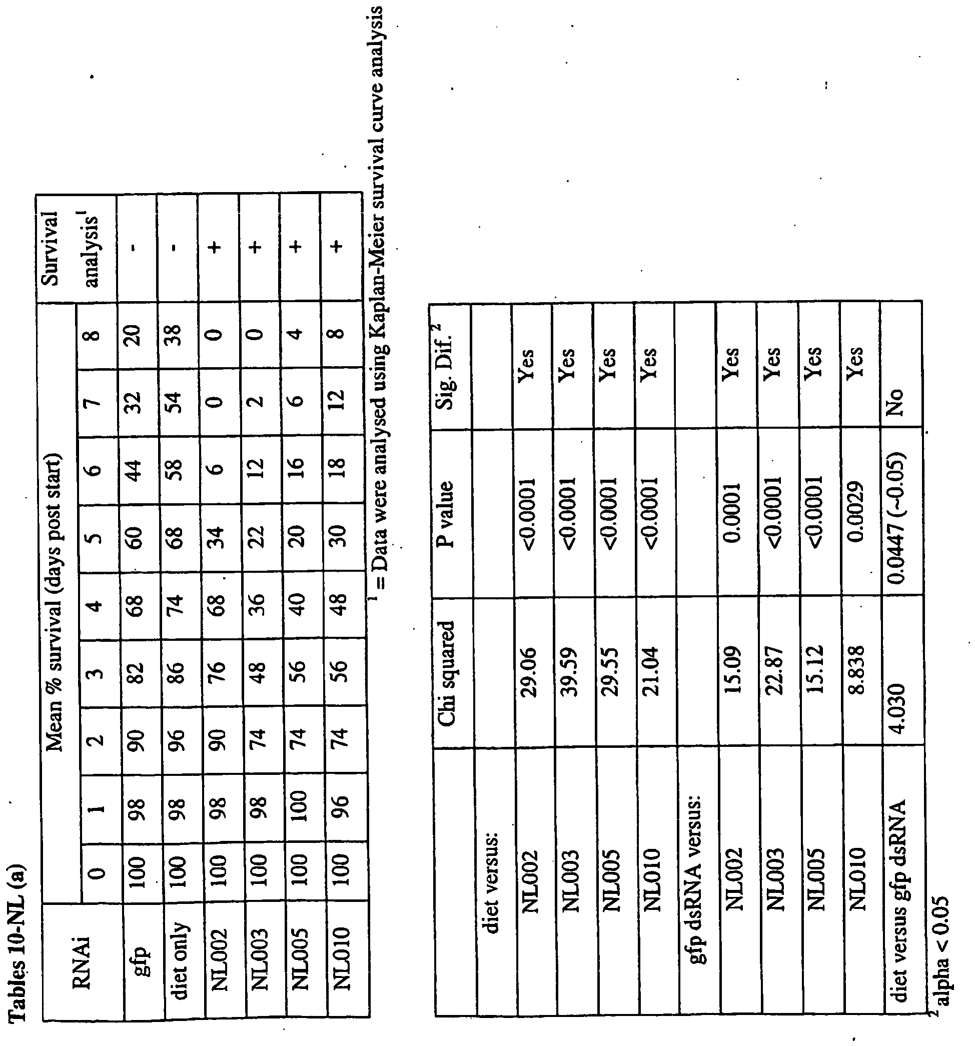

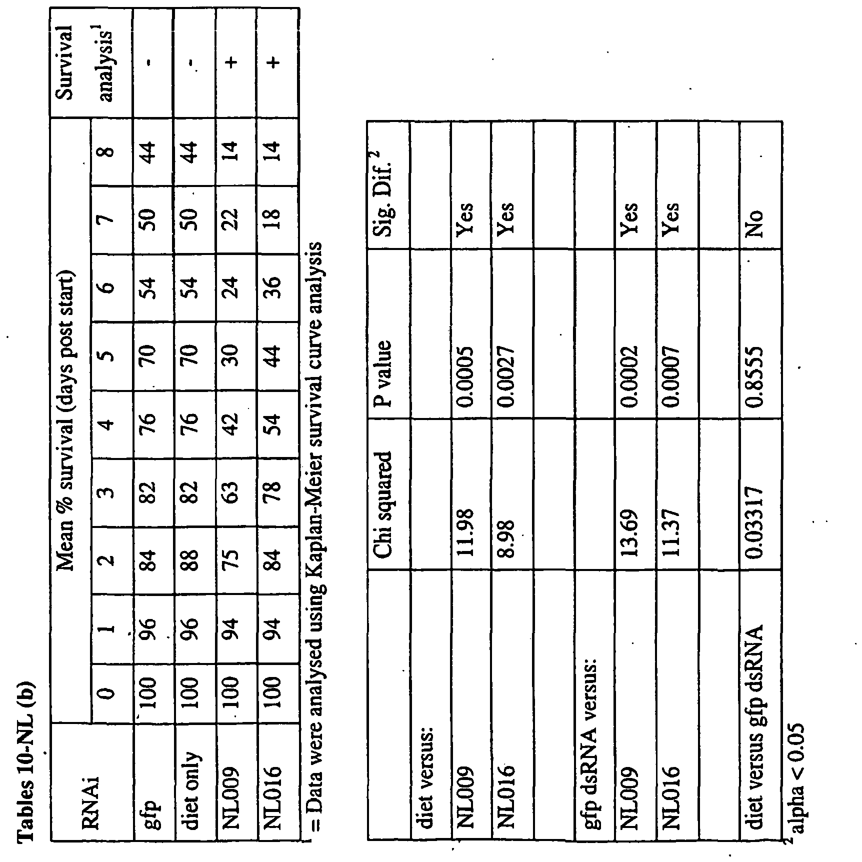

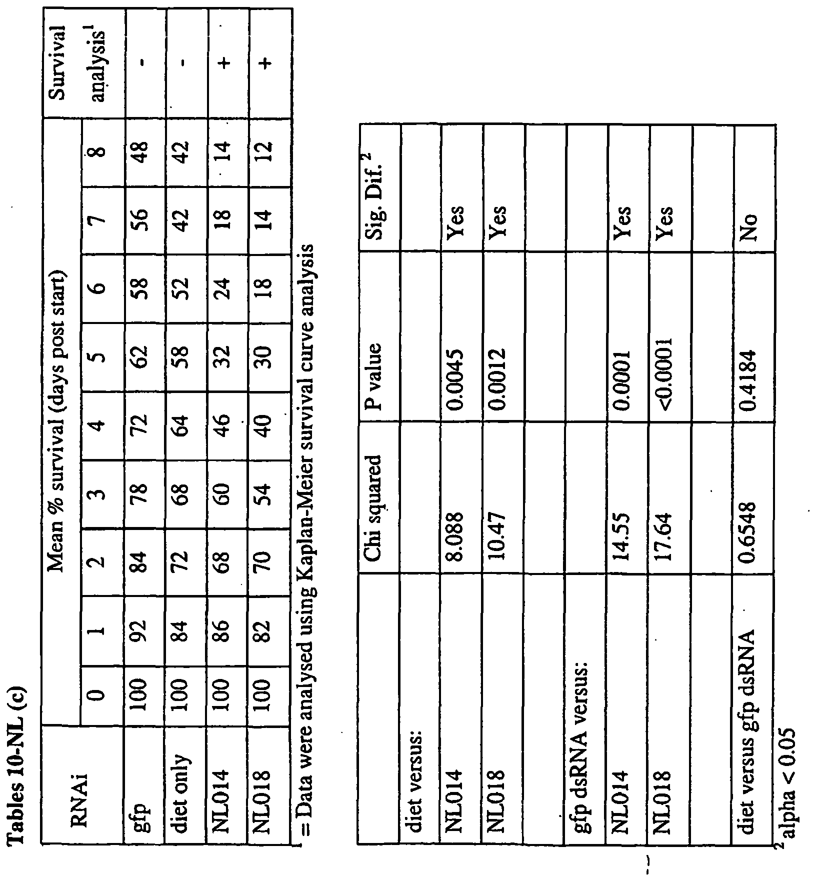

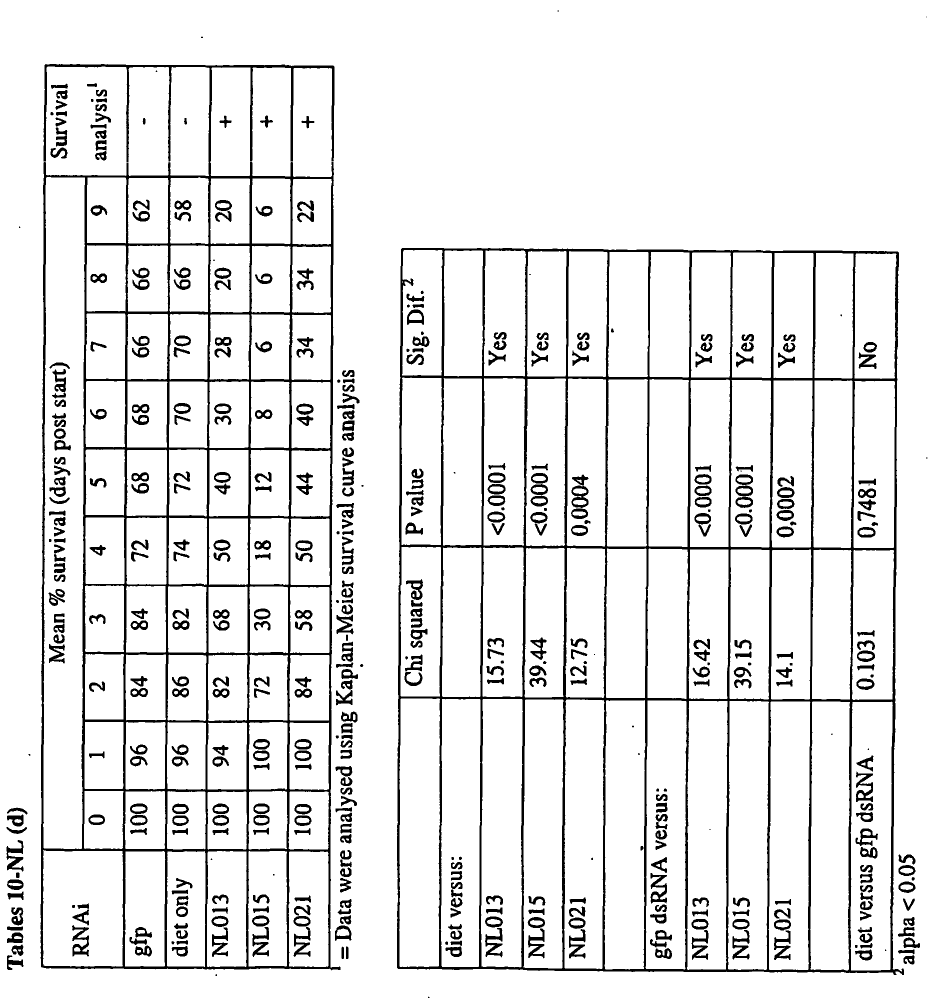

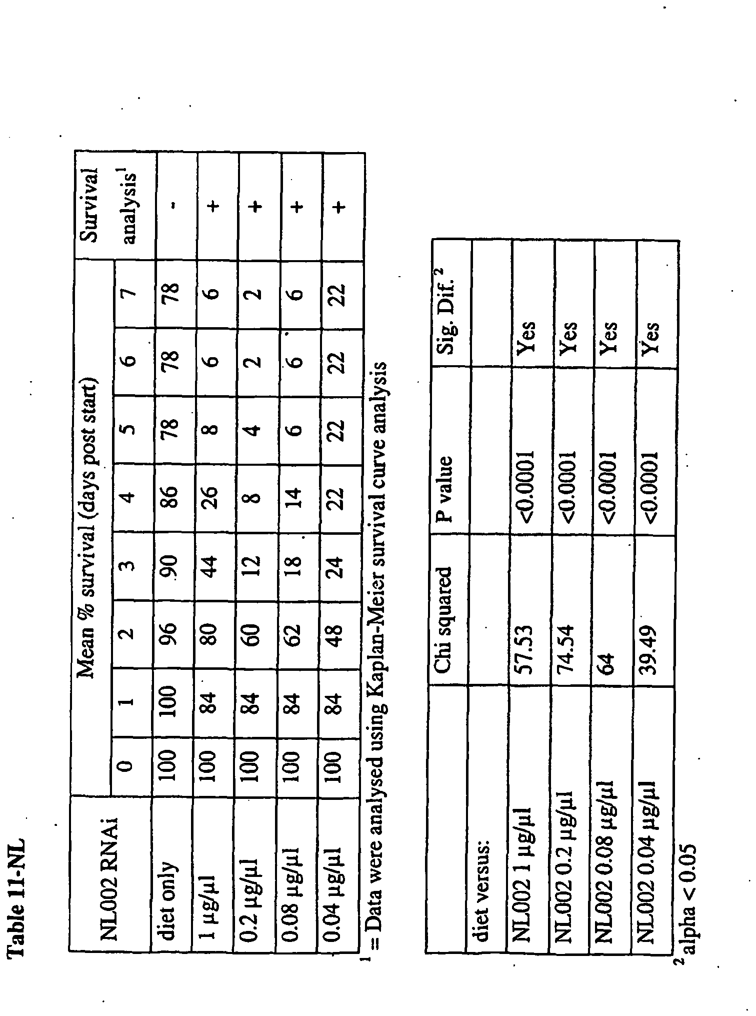

## Claims

1. A composition comprising a cell comprising a double stranded ribonucleotide sequence produced from the expression of a polynucleotide sequence selected from the group consisting of:
(i) a polynucleotide sequence comprising the nucleic add sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth In SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide sequence comprises annealed complementary strands, one of which has a nucleotide sequence which is complementary to any one of the sequences selected from the group consisting of:
(i) a polynucleotide sequence consisting of the nucleic add sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence consisting of a fragment of at least 21 contiguous nucleotides of any of SEQ ID NOs 1, 25, 49 to 55, 163 or 166, or the complement thereof.

2. The composition of claim 1, wherein said cell Is a bacterial, yeast, or algal cell.

3. The composition of claim 2, wherein the bacterial cell is killed by heat treatment.

4. The composition of any one of claims 1 to 3, wherein said composition is selected from the group consisting of a spray, powder, pellet, gel, capsule, food product, garment bag and book.

5. A method for controlling insect pest infestation, comprising exposing a pest to a double stranded ribonucleotide sequence produced from the expression of a polynudeotide sequence selected from the group consisting of:
(i) a polynudeotlde sequence comprising the nucleic add sequence set forth in any of SEQ ID NOs: 1, 25, 49 to 55, 163 or 166, or the complement thereof,
(ii) a polynudeotide sequence having at least 85% sequence identity as determined using the BLASTN alignment tool to the nucleic add sequence set forth in SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide sequence comprises annealed complementary strands, one of which has a nucleotide sequence which is complementary to any one of the sequences selected from the group consisting of:
(i) a polynucleotide sequence consisting of the nucleic add sequence set forth In any of SEQ ID NOs: 1, 25, 49 to 55, 163 or 166, or the complement thereof,
(ii) a polynucleotide sequence having at least 85% sequence identity as determined using the BLASTN alignment tool to the nucleic add sequence set forth in SEQ ID NO 1, or the complement thereof,
(iii) a polynucleotide sequence consisting of a fragment of at least 21 contiguous nucleotides of any of SEQ ID NOs 1, 25, 49 to 55, 163 or 166, or the complement thereof.

6. A method for protecting an object from insect pest infestation, comprising treating said object with a composition comprising a double stranded ribonucleotide sequence produced from the expression of a polynucleotide sequence selected from the group consisting of:
(i) a polynucleotide sequence comprising the nucleic add sequence set forth in any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence comprising a fragment of at least 21 contiguous nucleotides of any of SEQ ID NOs 1, 25, 49 to 55, 163 or 166, or the complement thereof, wherein said double stranded ribonucleotide sequence comprises annealed complementary strands, one of which has a nucleotide sequence which is complementary to any one of the sequences selected from the group consisting of:
(i) a polynucleotide sequence consisting of the nucleic add sequence set forth In any of SEQ ID NOs: 1, 25, 49 to 55, 163 or 166, or the complement thereof,
(ii) a polynucleotide sequence having at least 85% sequence identity as determined using the BLASTN alignment tool to the nucleic acid sequence set forth in SEQ ID NO 1, or the complement thereof,
(iii) a polynucleotide sequence consisting of a fragment of at least 21 contiguous nucleotides of any of SEQ ID NOs 1, 25, 49 to 55, 163 or 166, or the complement thereof.

7. The method of claim 6, wherein said object is selected from the group consisting of wood, tree, book binding, doth and a food storage container.

8. Use of the composition according to any one of claims 1 to 4, for preventing or treating an insect infestation.

## Patentansprüche

1. 2usammensetzung, welche eine Zelle umfasst, die eine doppelsträngige Ribonukleotidsequenz umfasst, welche durch die Expression einer Polynukleotidsequenz hergestellt ist, welche aus der Gruppe ausgewählt ist, die besteht aus:
(i) einer Polynukleotidsequenz, welche die in einer von SEQ ID Nr. 1, 25, 49 bis 55, 163 oder 166 ausgeführte Nukleinsäuresequenz umfasst, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 umfasst, oder das Komplement davon, wobei die doppelsträngige Ribonukleotidsequenz angeheftete komplementäre Stränge umfasst, von denen einer eine Nukleotidsequenz umfasst, der zu einer der Sequenzen komplementär ist, die aus der Gruppe ausgewählt sind, die besteht aus:
(i) einer Polynukleotidsequenz, welche die in einer von SEQ ID Nr. 1, 25, 49 bis 55, 163 oder 166 ausgeführte Nukleinsäuresequenz aufweist, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 aufweist, oder das Komplement davon.

2. Zusammensetzung nach Anspruch 1, wobei es sich bei der Zelle um eine Bakterien-, Hefe- oder Algenzelle handelt.

3. Zusammensetzung nach Anspruch 2, wobei die Bakterienzelle durch Hitzebehandlung getötet ist.

4. Zusammensetzung nach einem der Ansprüche 1 bis 3, wobei die Zusammensetzung aus der Gruppe ausgewählt ist, welche aus einem Spray, einem Pulver, einem Pellet, einem Gel, einer Kapsel, einem Nahrungsmittelprodukt, einem Kleidungsbeutel und einem Buch besteht.

5. Verfahren zum Kontrollieren eines Insektenschädlingsbefalls, umfassend das Aussetzen eines Schädlings gegenüber einer doppelsträngigen Ribonukleotidsequenz, welche durch die Expression einer Polynukleotidsequenz hergestellt ist, welche aus der Gruppe ausgewählt ist, die besteht aus:
(i) einer Polynukleotidsequenz, welche die in einer von SEQ ID Nr. 1, 25, 49 bis 55, 163 oder 166 ausgeführte Nukleinsäuresequenz umfasst, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 umfasst, oder das Komplement davon, wobei die doppelsträngige Ribonukleotidsequenz angeheftete komplementäre Stränge umfasst, von denen einer eine Nukleotidsequenz umfasst, der zu einer der Sequenzen komplementär ist, die aus der Gruppe ausgewählt sind, die besteht aus:
(i) einer Polynukleotidsequenz, welche die in einer von SEQ ID Nr. 1, 25, 49 bis 55, 163 oder 166 ausgeführte Nukleinsäuresequenz aufweist, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 aufweist, oder das Komplement davon.

6. Verfahren zum Schützen eines Gegenstandes vor einem Insektenschädlingsbefall, welches das Behandeln des Gegenstandes mit einer 2zusammensetzung umfasst, welche eine doppelsträngige Ribonukleotidsequenz umfasst, welche durch die Expression einer Polynukleotidsequenz hergestellt ist, welche aus der Gruppe ausgewählt ist, die besteht aus:
(i) einer Polynukleotidsequenz, welche die in einer von SEQ ID Nr. 1, 25, 49 bis 55, 163 oder 166 ausgeführte Nukleinsäuresequenz umfasst, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 umfasst, oder das Komplement davon, wobei die doppelsträngige Ribonukleotidsequenz angeheftete komplementäre Stränge umfasst, von denen einer eine Nukleotidsequenz umfasst, der zu einer der Sequenzen komplementär ist, die aus der Gruppe ausgewählt sind, die besteht aus:
(i) einer Polynukleotidsequenz, welche die in einer von SEQ ID Nr. 1, 25, 49 bis 55, 163 oder 166 ausgeführte Nukleinsäuresequenz aufweist, oder das Komplement davon,
(ii) einer Polynukleotidsequenz, die eine Sequenzidentität von mindestens 85 % mit der in SEQ ID Nr. 1 ausgeführten Nukleinsäuresequenz aufweist, wie mithilfe des Ausrichtungswerkzeugs BLASTN bestimmt wurde, oder das Komplement davon,
(iii) einer Polynukleotidsequenz, welche ein Fragment von mindestens 21 kontigen Nukleotiden einer der SEQ ID Nr. 1, 25, 49 bis 55, 163 oder 166 aufweist, oder das Komplement davon.

7. Verfahren nach Anspruch 6, wobei der Gegenstand aus der Gruppe ausgewählt ist, die aus Holz, Baum, Bucheinband, Tuch und einem Nahrungsmittelaufbewahrungsbehälter besteht.

8. Verwendung der Zusammensetzung nach einem der Ansprüche 1 bis 4, zum Verhindern oder Behandeln eines Insektenbefalls.

## Revendications

1. Composition comprenant une cellule comprenant une séquence ribonucléotidique double brin produite par l'expression d'une séquence polynucléotidique choisie dans le groupe constitué par :
(i) une séquence polynucléotidique comprenant la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique comprenant un fragment d'au moins 21 nucléotides contigus selon l'une quelconque de SEQ ID NO : 1, 25, 49 à 55, 163 ou 166, ou leur complément, ladite séquence ribonucléotidique double brin comprenant des brins complémentaires annelés, l'un d'entre eux ayant une séquence nucléotidique qui est complémentaire de l'une quelconque des séquences choisies dans le groupe constitué par :
(i) une séquence polynucléotidique constituée par la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique constituée par un fragment d'au moins 21 nucléotides contigus selon l'une quelconque de SEQ ID NO : 1, 25, 49 à 55, 163 ou 166, ou leur complément.

2. Composition selon la revendication 1, ladite cellule étant une cellule bactérienne, de levure, ou d'algue.

3. Composition selon la revendication 2, ladite cellule bactérienne étant détruite par traitement thermique.

4. Composition selon l'une quelconque des revendications 1 à 3, ladite composition étant choisie dans le groupe constitué par une pulvérisation, une poudre, une pastille, un gel, une gélule, un produit alimentaire, une housse à vêtement et un livre.

5. Procédé de lutte contre une infestation par les insectes nuisibles, qui comprend l'exposition d'un insecte nuisible à une séquence ribonucléotidique double brin produite par l'expression d'une séquence polynucléotidique choisie dans le groupe constitué par :
(i) une séquence polynucléotidique comprenant la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique comprenant un fragment d'au moins 21 nucléotides contigus selon l'une quelconque de SEQ ID NO : 1, 25, 49 à 55, 163 ou 166, ou leur complément, ladite séquence ribonucléotidique double brin comprenant des brins complémentaires annelés, l'un d'entre eux ayant une séquence nucléotidique qui est complémentaire de l'une quelconque des séquences choisies dans le groupe constitué par :
(i) une séquence polynucléotidique constituée par la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique constituée par un fragment d'au moins 21 nucléotides contigus selon l'une quelconque de SEQ ID NO : 1, 25, 49 à 55, 163 ou 166, ou leur complément.

6. Procédé de protection d'un objet contre une infestation par les insectes nuisibles, qui comprend le traitement dudit objet avec une composition comprenant une séquence ribonucléotidique double brin produite par l'expression d'une séquence polynucléotidique choisie dans le groupe constitué par :
(i) une séquence polynucléotidique comprenant la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique comprenant un fragment d'au moins 21 nucléotides contigus selon l'une quelconque de SEQ ID NO : 1, 25, 49 à 55, 163 ou 166, ou leur complément, ladite séquence ribonucléotidique double brin comprenant des brins complémentaires annelés, l'un d'entre eux ayant une séquence nucléotidique qui est complémentaire de l'une quelconque des séquences choisies dans le groupe constitué par .
(i) une séquence polynucléotidique constituée par la séquence d'acide nucléique exposée dans l'une quelconque des SEQ ID NO : 1, 25, 49 à 55, 163 ou 166, ou leur complément,
(ii) une séquence polynucléotidique ayant une identité de séquence d'au moins 85 % telle que déterminée en utilisant l'outil d'alignement BLASTN avec la séquence d'acide nucléique exposée dans SEQ ID NO : 1, ou son complément,
(iii) une séquence polynucléotidique constituée par un fragment d'au moins 21 nucléotides contigus selon l'une quelconque de SEQ ID NO : 1, 25, 49 à 55, 163 ou 166, ou leur complément.

7. Procédé selon la revendication 6, ledit objet étant choisi dans le groupe constitué par le bois, un arbre, une reliure, un vêtement et un récipient destiné au stockage de nourriture.

8. Utilisation de la composition selon l'une quelconque des revendications 1 à 4, pour la prévention ou le traitement d'une infestation par des insectes.
